# EUROPEAN PATENT APPLICATION

(11) **EP 4 516 320 A1**
(43) Date of publication of application: **05.03.2025**
(21) Application number: 23795600.8
(22) Date of filing: 28.04.2023
(51) Int. Cl.: A61K 47/54, A61K 47/61, A61K 47/68, A61P 35/00

(54) **LIGAND-DRUG CONJUGATE CONTAINING HYDROPHILIC SUGAR STRUCTURE**

(30) Priority: 29.04.2022 CN 202210467568
(71) Applicant: SystImmune, Inc., Redmond WA 98052 (US)
(72) Inventor: ZHU, Yi, Redmond, WA 98052 (US); WAN, Weili, Chengdu, Sichuan 611130 (CN); ZHUO, Shi, Chengdu, Sichuan 611130 (CN); ZHANG, Yiying, Chengdu, Sichuan 611130 (CN); YU, Tianzi, Chengdu, Sichuan 611130 (CN); YANG, Xiujuan, Chengdu, Sichuan 611130 (CN)
(74) Representative: Tirotta, Ilaria
(86) International application number: PCT/CN2023/091469
(87) International publication number: WO 2023/208168

(57) **Abstract**

A ligand-drug conjugate containing a hydrophilic sugar structure (SU), a pharmaceutically acceptable salt or solvate thereof, a preparation method for the ligand-drug conjugate, and use thereof in the preparation of a drug for treating disease. The ligand-drug conjugate has improved hydrophilicity.

## Description

### TECHNICAL FIELD

The present invention discloses a ligand-drug conjugate with improved hydrophilicity, and a preparation method therefor and use thereof. Specifically, the present invention discloses a ligand-drug conjugate containing a hydrophilic sugar structure (SU), a preparation method therefor and use thereof in the preparation of a drug for treating a disease.

### BACKGROUND

Ligand-drug conjugates, especially antibody-drug conjugates, are formed by conjugating a targeted ligand to a small molecule drug (cytotoxin). They not only have the characteristics of strong lethality of cytotoxic drugs, but also combine the high targeting, stability and favorable pharmacokinetic characteristics of the ligand, which are mainly manifested as: high therapeutic efficacy; high specificity; weak immunogenicity and not easy to produce drug resistance; long circulation time in serum; weak toxicity to non-target sites, etc.

At present, there are more than 100 antibody-drug conjugates undergoing clinical trials, most of which have progressed from Phase I to Phase II. Phase III trials of some antibody-drug conjugates show positive outcomes. As of November 2021, there are 14 ADCs (antibody-drug conjugates) on the market worldwide, and their treatment areas are mainly in hematological tumors and solid tumors, and they are mainly used for the second-line treatment of patients, including patients with late-stage, relapsed/refractory and metastatic tumor indications.

A common challenge in the design of ligand-drug conjugates is the hydrophobicity of the effective load, which may cause problems in water solubility, aggregation, and rapid clearance of the conjugate. Studies have shown that the amplification of the MDR1 gene in cancer cells and the overexpression of its product (an energy-dependent transporter P-protein) can pump anticancer drugs in tumor cells out of the cells, reduce the drug concentration in the cells, and weaken or lose the cytotoxic effect of hydrophobic anticancer drugs, which is the main reason for tumor cells to develop multidrug resistance. At present, a main method to overcome this problem is to use polyethylene glycol (PEG) linkers to improve the solubility of ligand-drug conjugates in water (see patent application WO2015057699A2). Sacituzumab-govitecan (IMMU-132), which has been launched on the market, solves the hydrophobicity problem by conjugating a cleavable maleimide linker with a short PEGylation unit to SN-38.

### SUMMARY OF THE INVENTION

In order to solve the hydrophobicity problem of the effective load in a ligand-drug conjugate, the present invention discloses a ligand-drug conjugate containing a hydrophilic sugar structure. The present invention further discloses a linker-drug conjugate containing a hydrophilic sugar structure, and the use of the ligand-drug conjugate or linker-drug conjugate in the preparation of a drug for treating or preventing a disease (such as a tumor or an autoimmune disease).

Specifically, by connecting a sugar structure (such as a monosaccharide, a disaccharide or a polysaccharide) to an effective load, the hydrophilicity of the effective load is reduced by utilizing the hydrophilicity of the sugar, thereby increasing the hydrophilicity of the ligand-drug conjugate. Compared with the prior art, the hydrophilic structure involved in the present invention is a more readily available sugar; it is more easily achievable to connect naturally occurring reaction sites in the sugar with the effective load. In addition, tumor cells have a much greater ability to take up sugars than normal cells, further avoiding the non-targeted side effects of the ligand-drug conjugate.

In one aspect, the present invention provides a ligand-drug conjugate of formula I, formula II or formula III, or a pharmaceutically acceptable salt or solvate thereof: 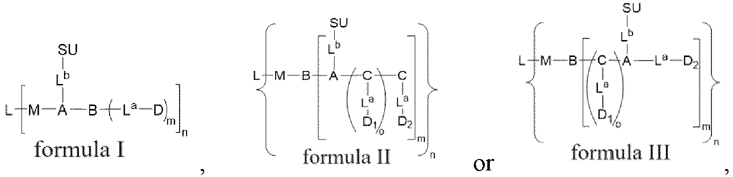 wherein
L is selected from ligands;
M is selected from any linker units or bonds;
A is selected from any linking scaffolds;
B is present or absent, and when present, is selected from any linking structures or bonds;
C is present or absent, and when present, is selected from any branching units or bonds;
D, D₁, D₂ are the same or different, and are each independently selected from drugs;
SU is selected from sugars or derivatives thereof;
L^{a} and L^{b} are the same or different, and are each independently selected from any linking units or bonds;
m is selected from integers of 1 to 5;
n is selected from integers of 1 to 10;
o is selected from integers of 1 to 10.

In some embodiments, the ligand L is selected from an antibody, a functional antibody fragment and a protein with a targeting effect.

In some embodiments, the ligand L is selected from antibodies, including but not limited to chimeric antibodies, humanized antibodies, fully human antibodies or mouse antibodies.

In some embodiments, the linker unit M is non-restrictively selected from structures or bonds represented by the following formulas; when the structure contains a cycloalkyl or a heterocyclyl, the linker unit M may also be selected from a derived structure thereof in which the cycloalkyl or heterocyclyl is in open form, 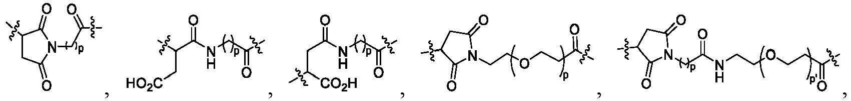 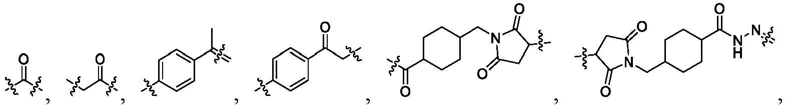 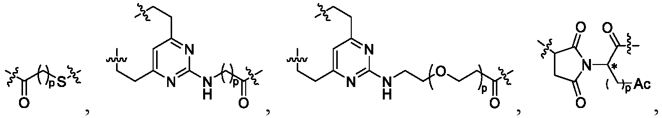 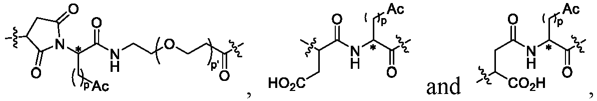 wherein
the position indicated by a wavy line is non-restrictively connected to the ligand, the linking scaffold A, the linking structure B or the branching unit C;
carbon at the position indicated by * is a chiral carbon and has an R or S configuration;
p and p' are each independently selected from integers of 1 to 10;

Ac is non-restrictively selected from a residue of a natural or non-natural amino acid, a polyethylene glycol segment with 1-20 repeating units, a phosphate group, a carboxylic acid group, a sulfonic acid group, a sulfinic acid group or the following structure, 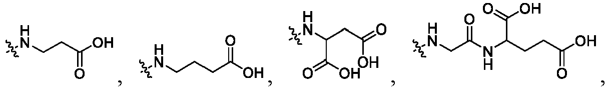 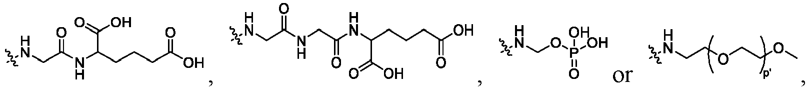 wherein the position indicated by a wavy line is connected to the carbon atom at the position indicated by *.

In some embodiments, the linker unit M is non-restrictively selected from structures represented by the following formulas, or stereoisomers thereof or open ring forms thereof, derived structures thereof in which rings in succinimide groups are in open form, 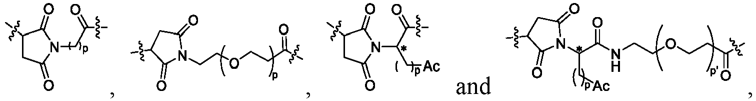 wherein
the position indicated by a wavy line is non-restrictively connected to the ligand, the linking scaffold A, the linking structure B or the branching unit C;
carbon at the position indicated by * is a chiral carbon and has an R or S configuration; p and p' are each independently selected from integers of 1 to 10;

Ac is non-restrictively selected from a residue of a natural or non-natural amino acid, a polyethylene glycol segment with 1-20 repeating units, a phosphate group, a carboxylic acid group, a sulfonic acid group, a sulfinic acid group or the following structure, 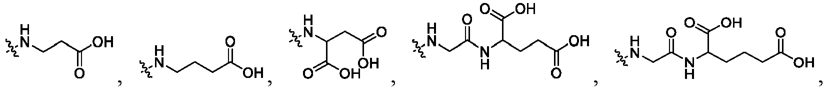 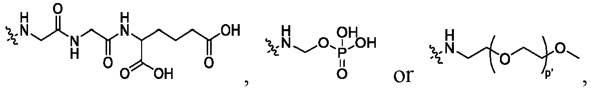 wherein the position indicated by a wavy line is connected to the carbon atom at the position indicated by *.

In some embodiments, the linker unit M is a structure represented by the following formula, or a stereoisomer thereof or a derived structure thereof in which ring in succinimide group is in open form,

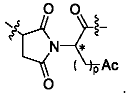

In some embodiments, p is 1. In some embodiments, Ac is a glycine residue.

In some embodiments, the linker unit M is non-restrictively selected from structures represented by the following formulas, or derived structures thereof in which rings in succinimide groups are in open form,

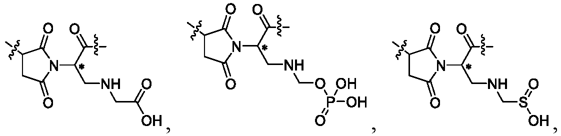

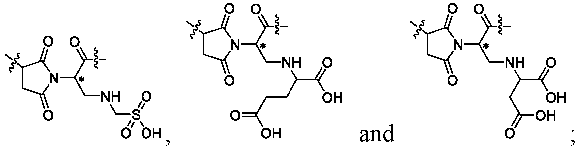

In some embodiments, the linker unit M is 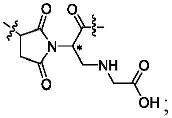 wherein
carbon at the position indicated by * is a chiral carbon and has an R or S configuration;
the position indicated by a wavy line is non-restrictively connected to the ligand, the linking scaffold A, the linking structure B or the branching unit C.

In some embodiments, the linking scaffold A is selected from one or more of natural or non-natural amino acids or from the following structures, 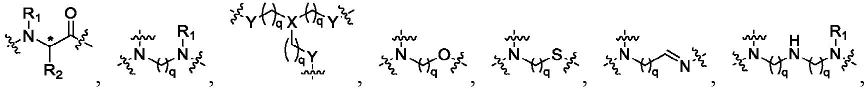 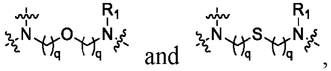 wherein
X is selected from N, CH, C3-C8 cycloalkyl, 3- to 8-membered heterocyclyl, aryl, substituted aryl, and heteroaryl;
Y is selected from -NH-, -O-, -S-, -CO-, -CO₂-, -CONH-, -NHCO-, -SO-, - SO₂-, -OSO₂-, and -OP=O(OH)O-;
q is selected from integers of 1 to 10;
the position indicated by a wavy line is non-restrictively connected to the linker unit M, the linking unit L^{a} or L^{b}, the linking structure B or the branching unit C;
R₁ is selected from hydrogen atom, deuterium atom, halogen, C1-C6 alkyl, C1-C6 substituted alkyl, C3-C8 cycloalkyl C1-C6 alkyl, C1-C6 alkoxy C1-C6 alkyl, carboxyl, 3- to 8-membered heterocyclyl, aryl, substituted aryl and heteroaryl;
R₂ is non-restrictively selected from: 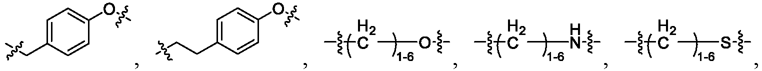 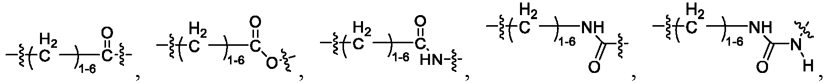 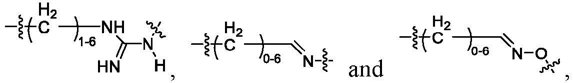
wherein a wavy line on the left indicates the position where R₂ is connected to the chiral carbon indicated by *, and a wavy line on the right indicates one of any three connection sites of the linking scaffold A.

In some embodiments, the linking scaffold A is 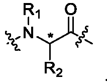 In some embodiments, R₂ is

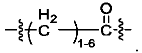

In some embodiments, the linking scaffold A is non-restrictively selected from the following structures or stereoisomers thereof, 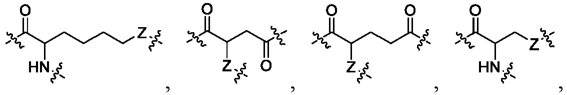 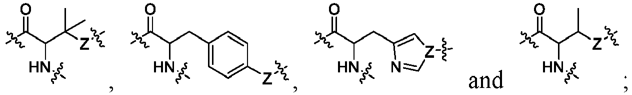 wherein
Z is selected from -NH-, -O- and -S-;
the position indicated by a wavy line is non-restrictively connected to the linker unit M, the linking unit L^{a} or L^{b}, the linking structure B or the branching unit C;

In some embodiments, the linking scaffold A is 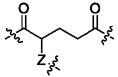 . In some embodiments, Z is -NH-.

In some embodiments, the aryl is a phenyl. In some embodiments, the substituted aryl is a phenyl substituted with one or more substituents selected from deuterium atom, halogen, C1-C6 alkyl, C1-C6 alkoxy, and C1-C6 haloalkyl. In some embodiments, the heteroaryl is a 6-membered monoheteroaryl containing one or more heteroatoms selected from nitrogen, oxygen, and sulfur.

In some embodiments, the B is non-restrictively selected from any linking structures or bonds.

In some embodiments, the C is present or absent, and when present, is non-restrictively selected from one or more of natural or non-natural amino acids.

In some embodiments, the drugs D, D₁, and D₂ are the same or different, and are each independently and non-restrictively selected from an anti-tumor drug, an autoimmune disease drug, an anti-infective disease drug (such as an antiviral drug), a radioactive isotope, a chromogenic molecule, or a pharmaceutically acceptable salt or solvate thereof.

In some embodiments, the drugs D, D₁, and D₂ are the same or different, and are independently and non-restrictively selected from anti-tumor drugs, including but not limited to DNA damaging agents, RNA damaging agents, enzyme inhibitors, or microtubule inhibitors.

In some embodiments, the SU is non-restrictively selected from a natural or non-natural monosaccharide, disaccharide, polysaccharide and derivatives thereof.

In some embodiments, the natural or non-natural monosaccharide, disaccharide, polysaccharide or derivatives thereof are non-restrictively selected from the following structures,

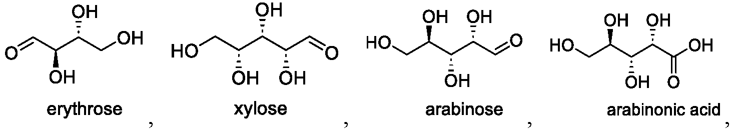

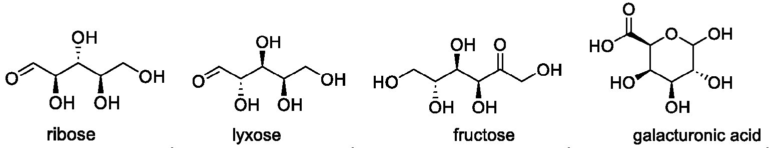

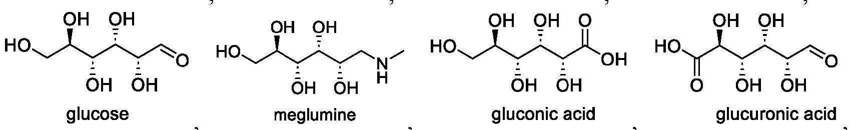

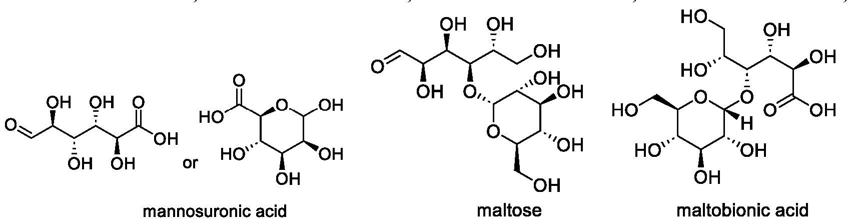

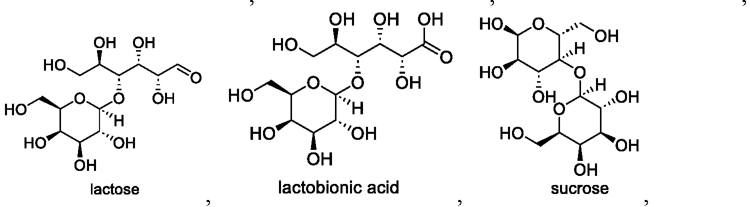

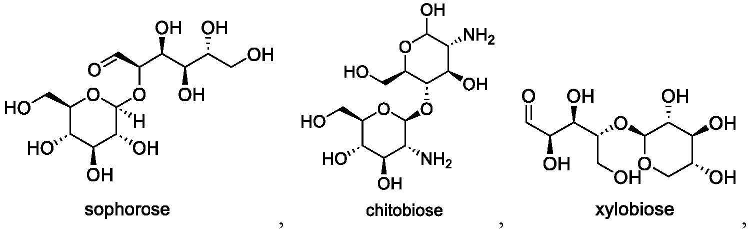

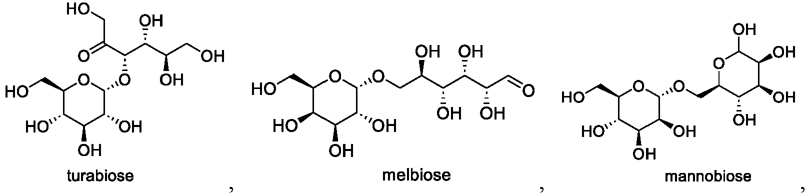

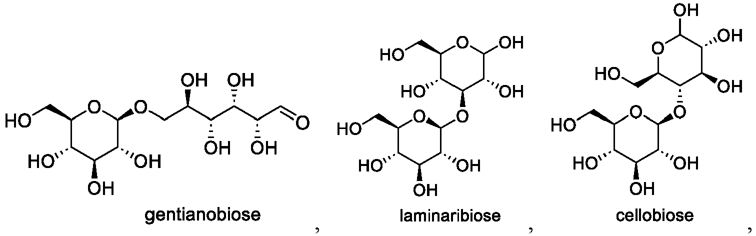

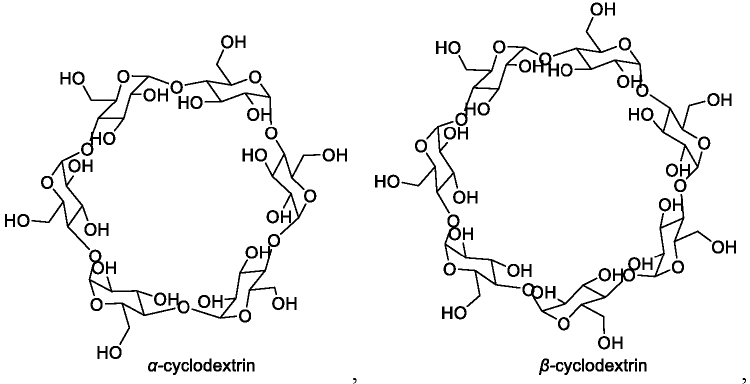

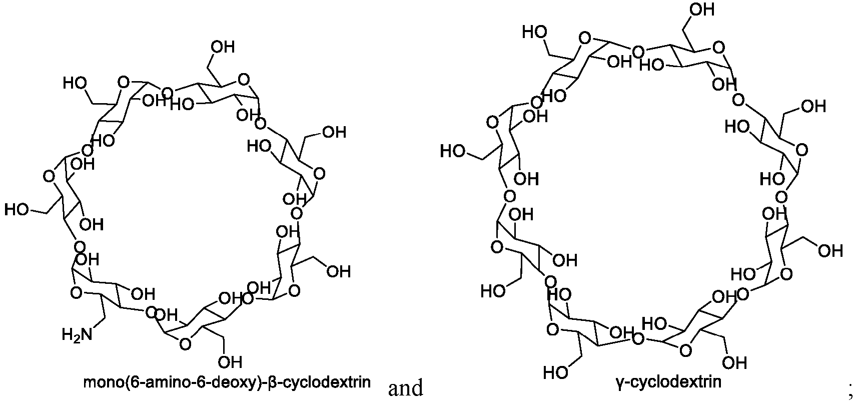

In some embodiments, the SU is selected from methylglucamine, maltose, maltobionic acid, mannuronic acid, β-cyclodextrin and mono(6-amino-6-deoxy)-β-cyclodextrin.

In some embodiments, the SU is non-restrictively linked to L^{b} in a covalent manner.

In some embodiments, the linking units L^{a} and L^{b} are the same or different, and are each independently and non-restrictively selected from one or more of a chemically unstable linking unit, an enzyme-catalyzed cleavage linking unit and a non-cleavable linking unit.

In some embodiments, L^{a} is selected from a chemically unstable linking unit and an enzyme-catalyzed cleavage linking unit.

In some embodiments, the linking unit L^{a} is non-restrictively selected from the following structures or stereoisomers thereof: 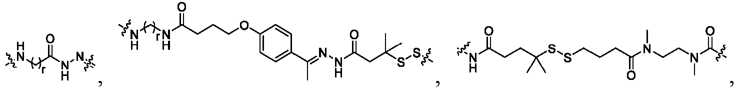 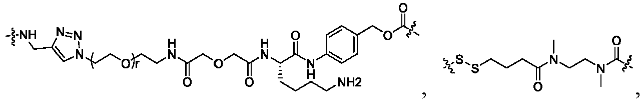 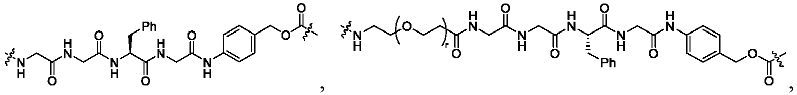 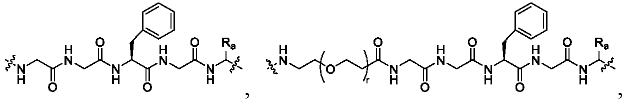 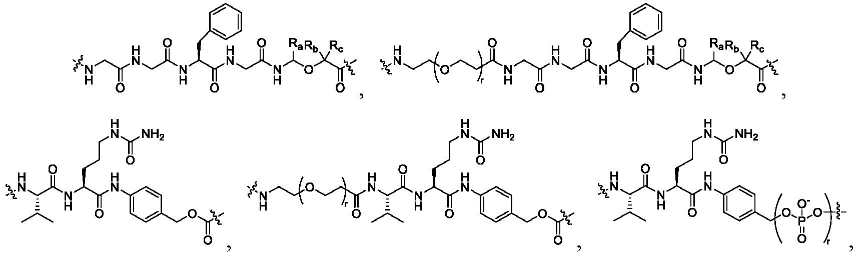 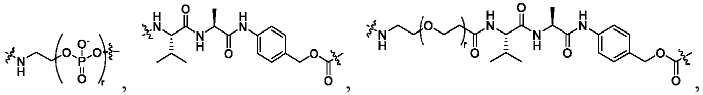 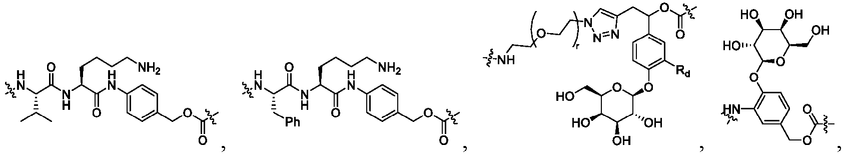 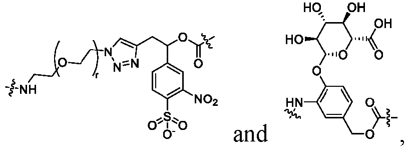 wherein
Rₐ, R_{b}, and R_{c} are the same or different, and are each independently selected from hydrogen atom, deuterium atom, halogen, alkyl, substituted alkyl, deuterated alkyl, cycloalkyl, cycloalkylalkyl, alkoxyalkyl, heterocyclyl, aryl, substituted aryl or heteroaryl;
or, R_{b}, R_{c} and carbon atoms connected to R_{b} and R_{c} constitute a C3-C8 cycloalkyl or a 3- to 8-membered heterocyclyl;
Rₐ is selected from H, NO₂, HO-SO₃- and -OSO₃;
r is selected from integers of 1 to 10;
the position indicated by a wavy line on the left is connected to the linking scaffold A, the linking structure B or the branching unit C;
the position indicated by a wavy line on the right is connected to the drug D, D₁ or D₂.

In some embodiments, the linking units L^{a}-D, L^{a}-D₁ and L^{a}-D₂ are the same or different and are non-restrictively selected from the following structures or stereoisomers thereof:
(1) Auristatins 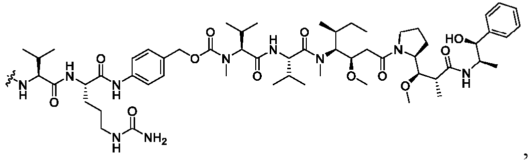 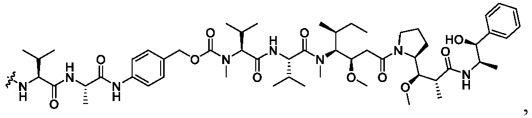 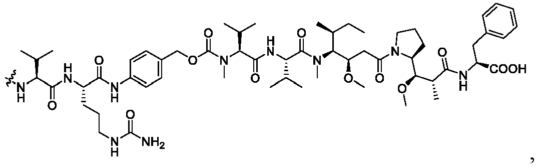 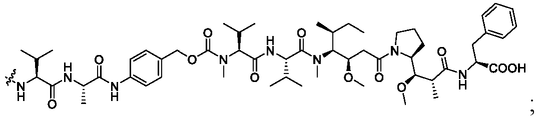
(2) Maytansinoids 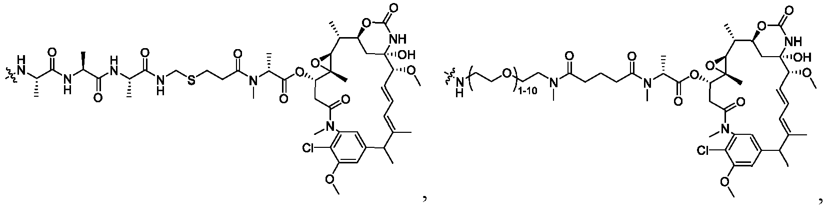 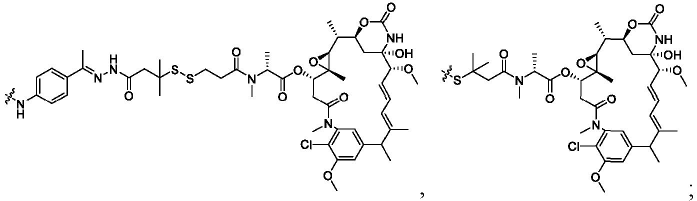
(3) Benzodiazepines 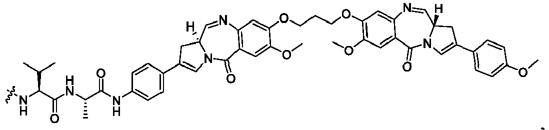 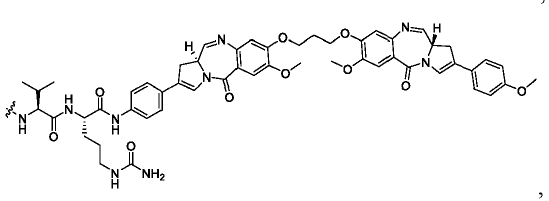 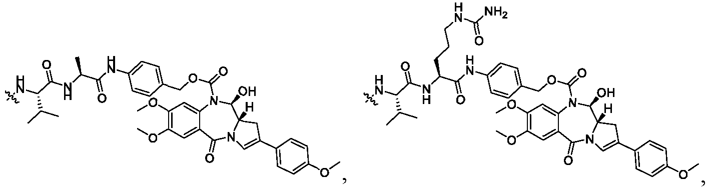 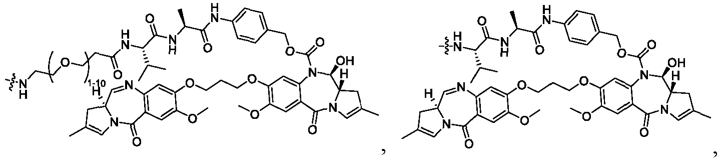 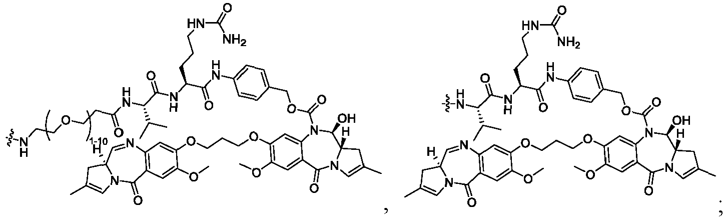
(4) Camptothecin analogues 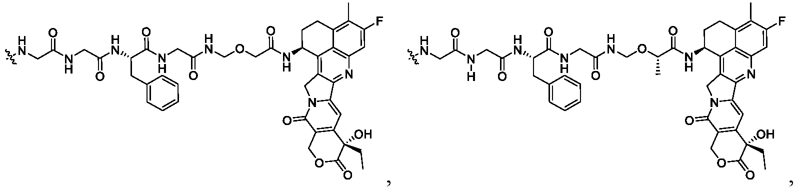 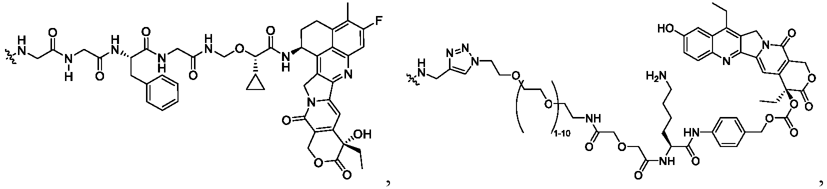 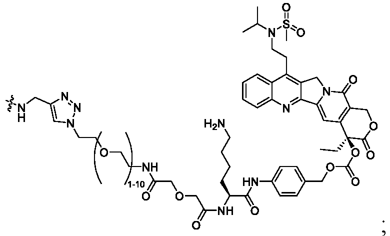
(5) Tubulysins 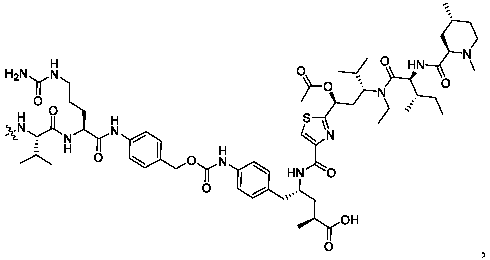 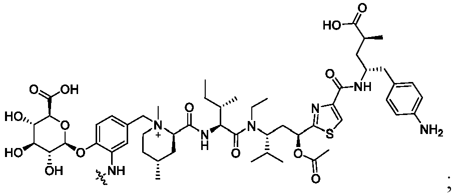
(6) Adriamycins 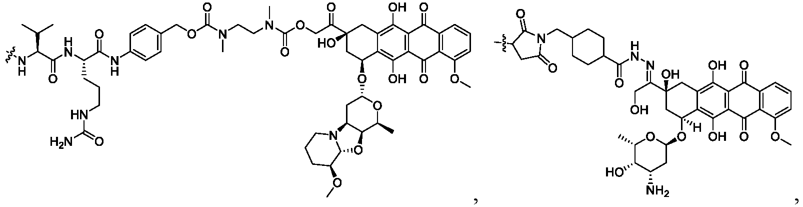 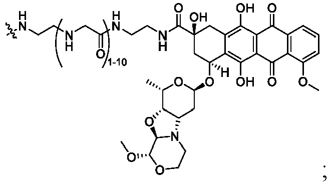
(7) Calicheamicins 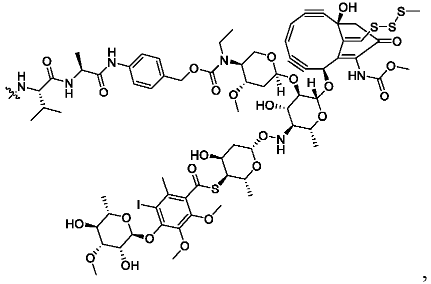 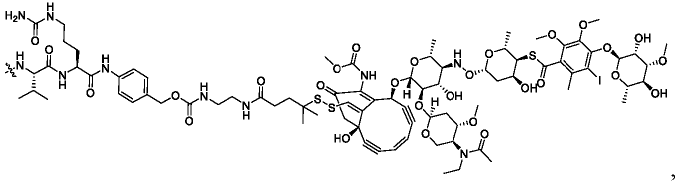 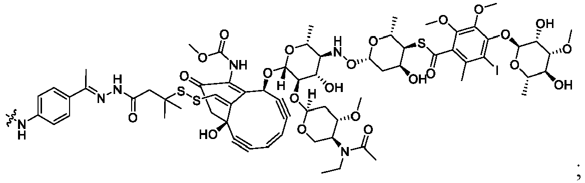
(8) Duocarmycins 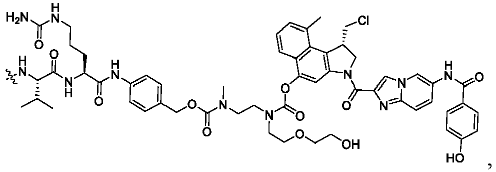 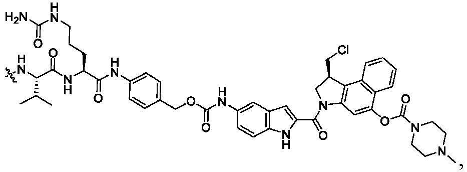 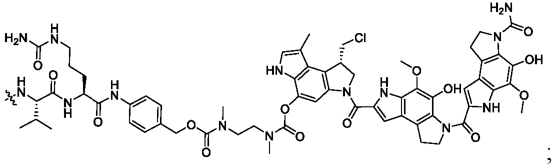
(9) Other antitumor drugs 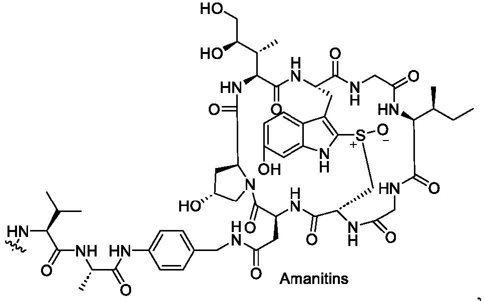 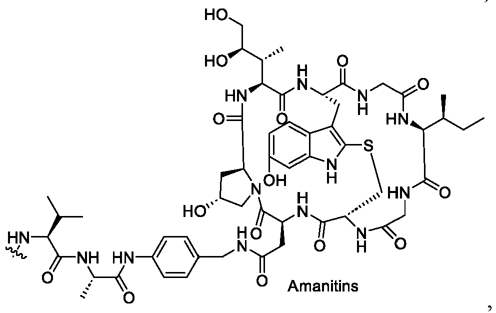 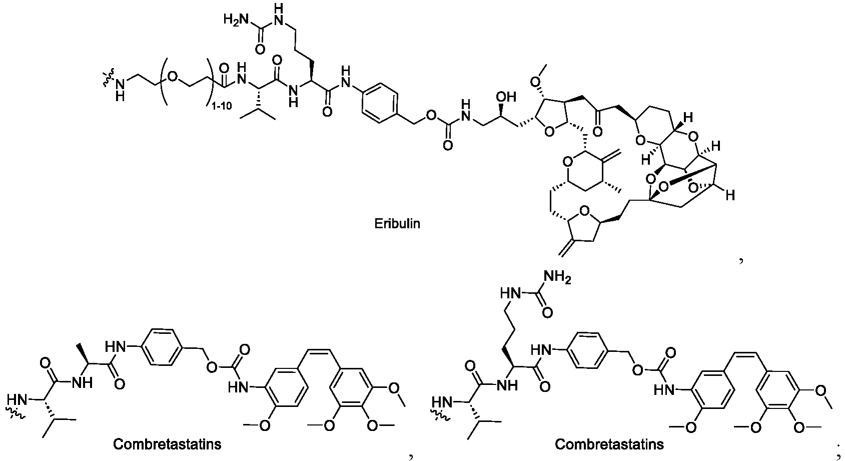
wherein
the position indicated by a wavy line is connected to the linking scaffold A, the linking structure B or the branching unit C;

In some embodiments, the L^{a}-D, L^{a}-D₁ or L^{a}-D₂ is selected from:

Auristatins, for example,

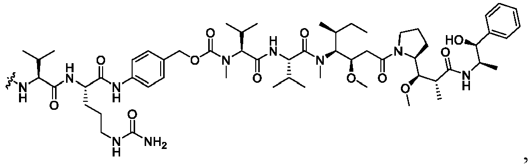

Benzodiazepines, for example,

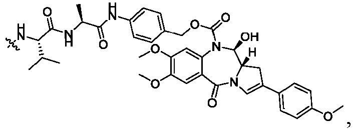

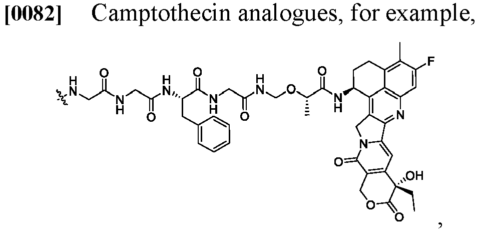

Combretastatins, for example,

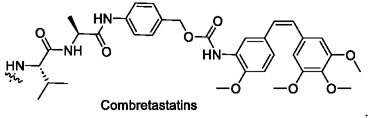

In some embodiments, the linking unit L^{b} is non-restrictively selected from the following structures or stereoisomers thereof: 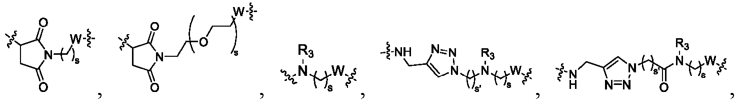 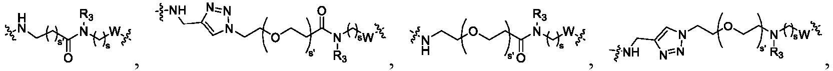 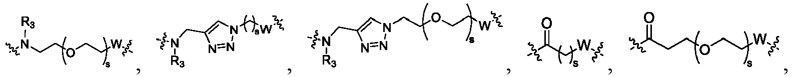 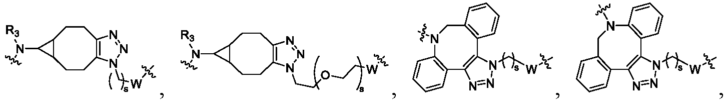 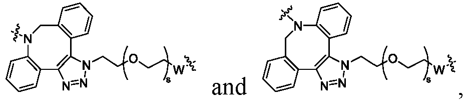 wherein
R₃ is selected from hydrogen atom, deuterium atom, halogen, C1-C6 alkyl, deuterated C1-C6 alkyl, halogenated C1-C6 alkyl, C3-C8 cycloalkyl C1-C6 alkyl,C1-C6 alkoxy C1-C6 alkyl, heterocyclyl, aryl, substituted aryl, and heteroaryl;
W is selected from -NR₄-, -O-, -S-, -CO- and -CONR₅-;
R₄ or R₅ is independently selected from hydrogen atom, deuterium atom, halogen,C1-C6 alkyl, deuterated C1-C6 alkyl, halogenated C1-C6 alkyl, C3-C8 cycloalkyl C1-C6 alkyl,C1-C6 alkoxy C1-C6 alkyl, heterocyclyl, aryl, substituted aryl, and heteroaryl;
s is selected from integers of 1 to 10;
s' is selected from integers of 1 to 10;
the positions indicated by a wavy line on the left and a wavy line on the right are non-restrictively connected to the SU or to the linking scaffold A.

The present application also provides a linker-drug compound of formula IV, V or VI or a pharmaceutically acceptable salt or solvate thereof: 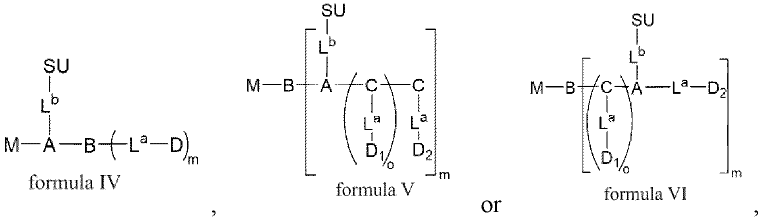 wherein
M is selected from any linker units;
A is selected from any linking scaffolds;
B is present or absent, and when present, is selected from any linking structures or bonds;
C is present or absent, and when present, is selected from any branching units or bonds;
D, D₁, D₂ are the same or different, and are each independently selected from drugs;
SU is selected from sugars or derivatives thereof;
L^{a} and L^{b} are the same or different, and are each independently selected from any linking units or bonds;
m is selected from integers of 1 to 5;
o is selected from integers of 1 to 10.

In some embodiments, the linker unit M is non-restrictively selected from structures represented by the following formulas; when the structure contains a cycloalkyl or a heterocyclyl, the linker unit M may also be selected from a derived structure thereof in which the cycloalkyl or a heterocyclyl is in open form, 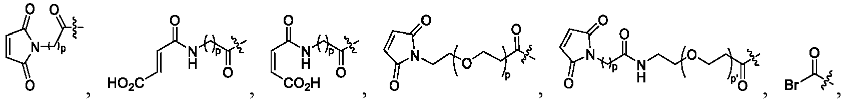 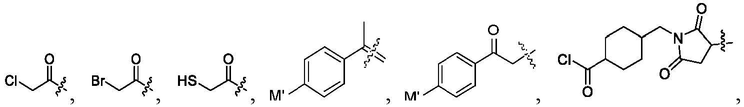 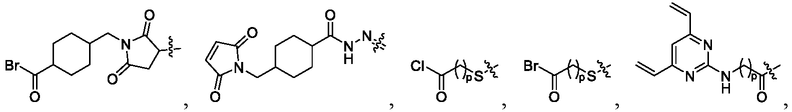 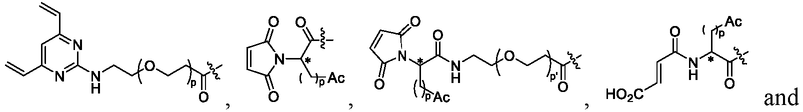 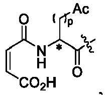 wherein
the position indicated by a wavy line is non-restrictively connected to the linking scaffold A, the linking structure B or the branching unit C;
M' is non-restrictively selected from halogen, OTf and OTs;
carbon at the position indicated by * is a chiral carbon and has an R or S configuration;
p and p' are each independently selected from integers of 1 to 10;
Ac is non-restrictively selected from a residue of a natural or non-natural amino acid, a polyethylene glycol segment with 1-20 repeating units, a phosphate group, a carboxylic acid group, a sulfonic acid group, a sulfinic acid group or the following structure, 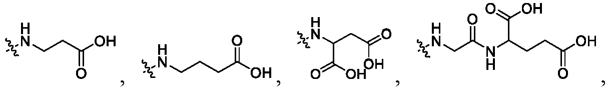 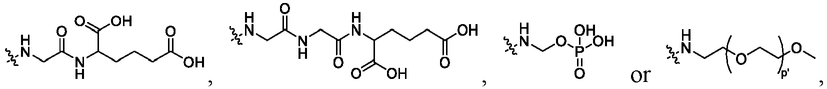
wherein the position indicated by a wavy line is connected to the carbon atom at the position indicated by *.

In some embodiments, the linker unit M is non-restrictively selected from structures represented by the following formulas, or stereoisomers thereof or derived structures thereof in which rings in succinimide groups are in open form, 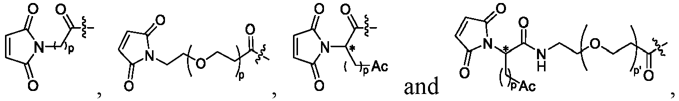 wherein
the position indicated by a wavy line is non-restrictively connected to the linking scaffold A, the linking structure B or the branching unit C;
carbon at the position indicated by * is a chiral carbon and has an R or S configuration; p and p' are each independently selected from integers of 1 to 10;
Ac is non-restrictively selected from a residue of a natural or non-natural amino acid, a polyethylene glycol segment with 1-20 repeating units, a phosphate group, a carboxylic acid group, a sulfonic acid group, a sulfinic acid group or the following structure, 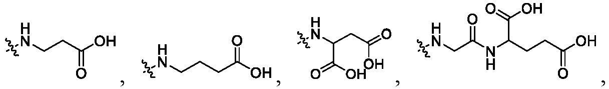 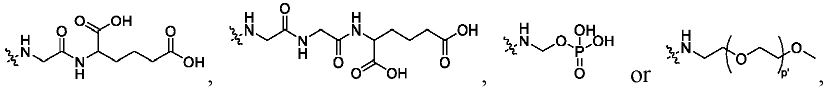
wherein the position indicated by a wavy line is connected to the carbon atom at the position indicated by *.

In some embodiments, the linker unit M is non-restrictively selected from structures represented by the following formulas, or derived structures thereof in which rings in succinimide groups are in open form,

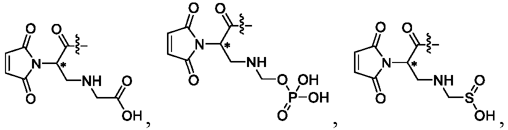

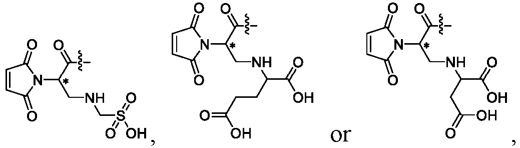

In some embodiments, the linker unit M is 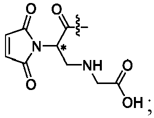 wherein
carbon at the position indicated by * is a chiral carbon and has an R or S configuration;
the position indicated by a wavy line is non-restrictively connected to the linking scaffold A, the linking structure B or the branching unit C.

In some embodiments, the linking scaffold A is selected from one or more of natural or non-natural amino acids or from the following structures: 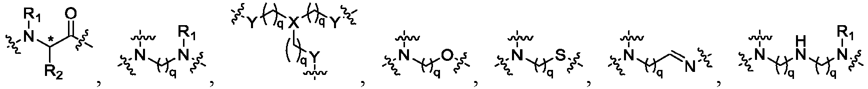 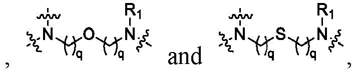 wherein
X is selected from N, CH, C3-C8 cycloalkyl, 3- to 8-membered heterocyclyl, aryl, substituted aryl, and heteroaryl;
Y is selected from -NH-, -O-, -S-, -CO-, -CO₂-, -CONH-, -NHCO-, -SO-, - SO₂-, -OSO₂-, and -OP=O(OH)O-;
q is selected from integers of 1 to 10;
the position indicated by a wavy line is non-restrictively connected to the linker unit M, the linking unit L^{a} or L^{b}, the linking structure B or the branching unit C;
R₁ is selected from hydrogen atom, deuterium atom, halogen, C1-C6 alkyl, C1-C6 substituted alkyl, C3-C8 cycloalkyl C1-C6 alkyl, C1-C6 alkoxy C1-C6 alkyl, carboxyl, 3- to 8-membered heterocyclyl, aryl, substituted aryl and heteroaryl;
R₂ is non-restrictively selected from: 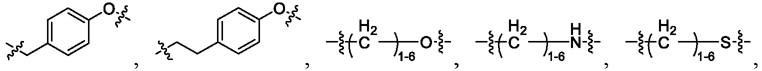 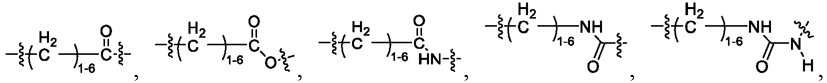 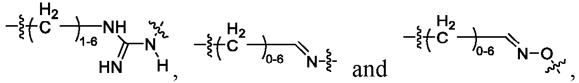
wherein a wavy line on the left indicates the position where R₂ is connected to the chiral carbon indicated by *, and a wavy line on the right indicates one of any three connection sites of the linking scaffold A.

In some embodiments, the linking scaffold A is non-restrictively selected from the following structures or stereoisomers thereof, 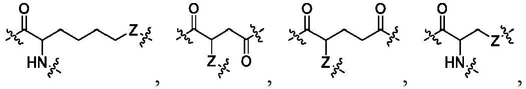 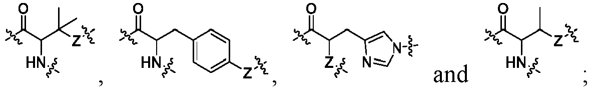 wherein
Z is selected from -NH-, -O- and -S-;
the position indicated by a wavy line is non-restrictively connected to the linker unit M, the linking unit L^{a} or L^{b}, the linking structure B or the branching unit C;

In some embodiments, the linking scaffold A is 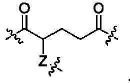 . In some embodiments, Z is -NH-.

In some embodiments, the B is non-restrictively selected from any linking structures or bonds.

In some embodiments, the C is present or absent, and when present, is non-restrictively selected from one or more of natural or non-natural amino acids.

In some embodiments, the drugs D, D₁, and D₂ are the same or different, and are each independently and non-restrictively selected from an anti-tumor drug, an autoimmune disease drug, an anti-infective disease drug (such as an antiviral drug), a radioactive isotope, a chromogenic molecule, or a pharmaceutically acceptable salt or solvate thereof.

In some embodiments, the drugs D, D₁, and D₂ are the same or different, and are independently and non-restrictively selected from anti-tumor drugs, including but not limited to DNA damaging agents, RNA damaging agents, enzyme inhibitors, or microtubule inhibitors.

In some embodiments, the SU is non-restrictively selected from a natural or non-natural monosaccharide, disaccharide, polysaccharide and derivatives thereof.

In some embodiments, the natural or non-natural monosaccharide, disaccharide, polysaccharide or derivatives thereof are non-restrictively selected from the following structures,

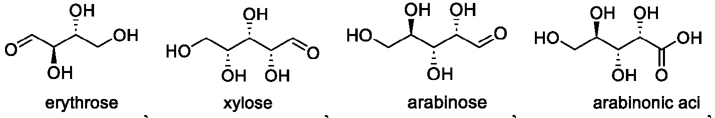

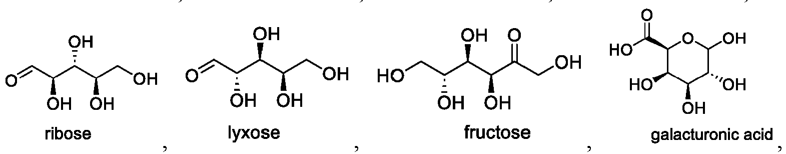

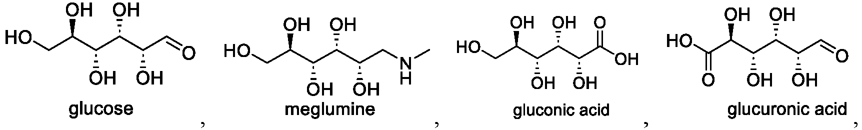

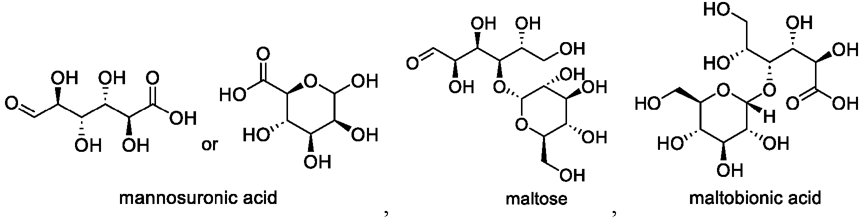

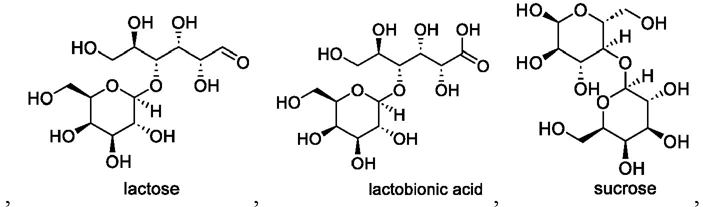

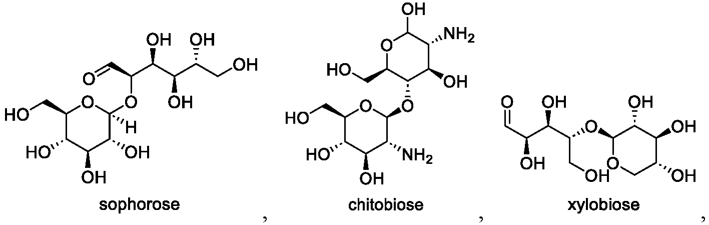

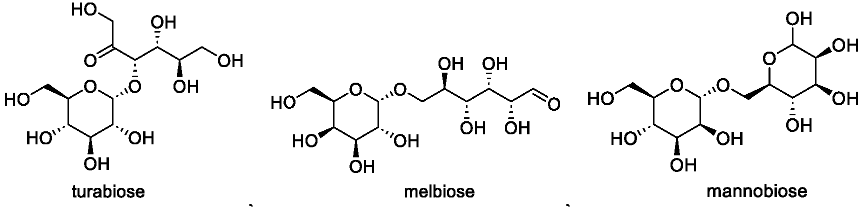

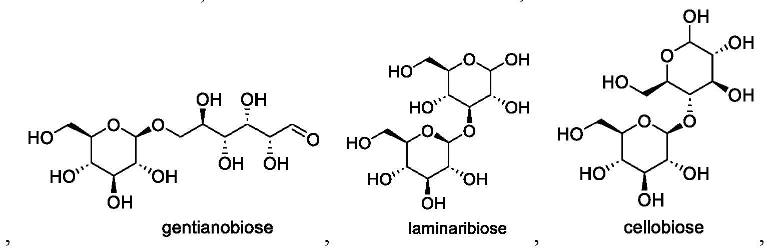

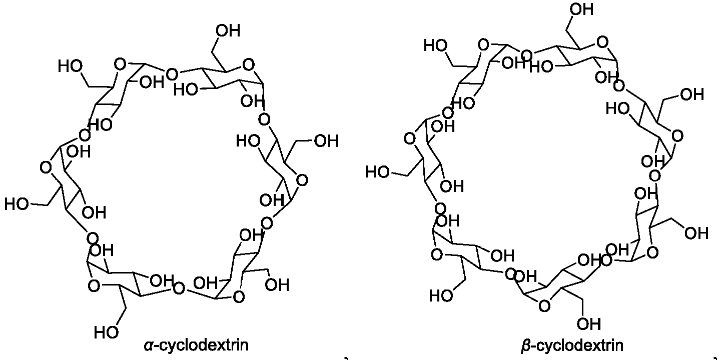

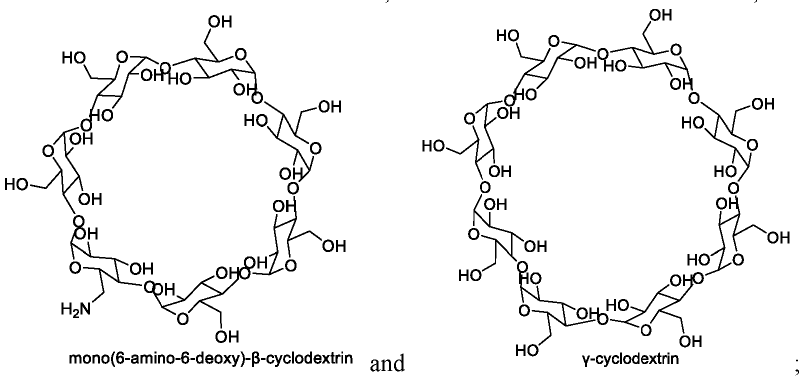

In some embodiments, the SU is selected from methylglucamine, maltose, maltobionic acid, mannuronic acid, β-cyclodextrin and mono(6-amino-6-deoxy)-β-cyclodextrin.

In some embodiments, the SU is non-restrictively linked to L^{b} in a covalent manner.

In some embodiments, the linking units L^{a} and L^{b} are the same or different, and are each independently and non-restrictively selected from one or more of a chemically unstable linking unit, an enzyme-catalyzed cleavage linking unit and a non-cleavable linking unit.

In some embodiments, L^{a} is selected from a chemically unstable linking unit and an enzyme-catalyzed cleavage linking unit.

In some embodiments, the linking unit L^{a} is non-restrictively selected from the following structures or stereoisomers thereof, 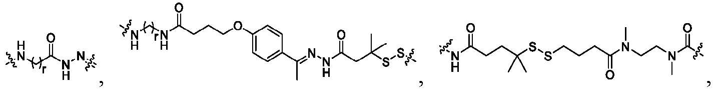 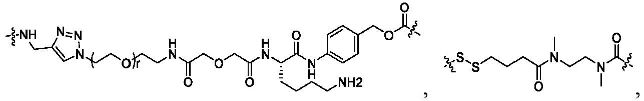 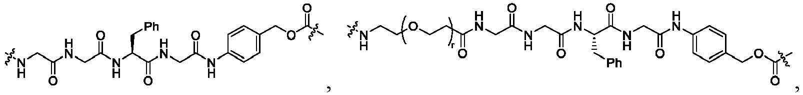 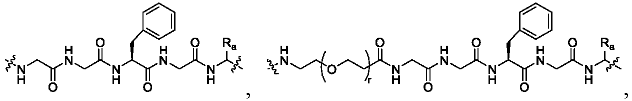 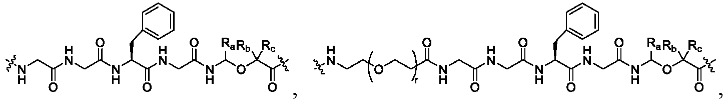 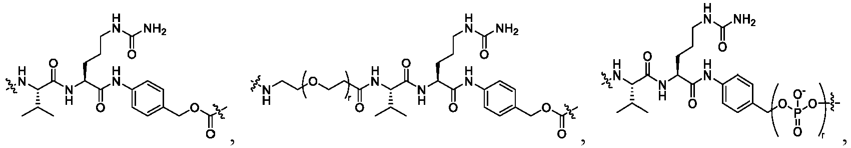 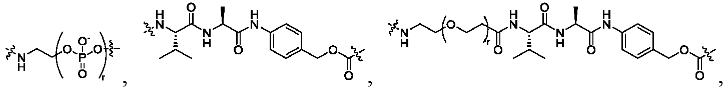 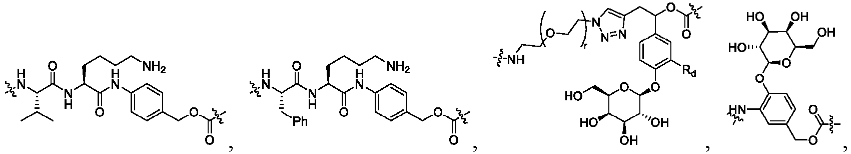 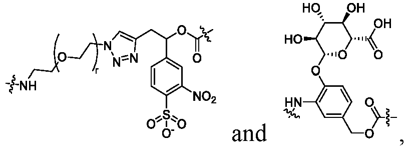 wherein
Rₐ, R_{b}, and R_{c} are the same or different, and are each independently selected from hydrogen atom, deuterium atom, halogen, alkyl, substituted alkyl, deuterated alkyl, cycloalkyl, cycloalkylalkyl, alkoxyalkyl, heterocyclyl, aryl, substituted aryl or heteroaryl;
or, R_{b}, R_{c} and carbon atoms connected to R_{b} and R_{c} constitute a C3-C8 cycloalkyl or a 3- to 8-membered heterocyclyl;
Rₐ is selected from H, NO₂, HO-SO₃ and -OSO₃;
r is selected from integers of 1 to 10;
the position indicated by a wavy line on the left is connected to the linking scaffold A, the linking structure B or the branching unit C;
the position indicated by a wavy line on the right is connected to the drug D, D₁ or D₂.

In some embodiments, the L^{a}-D, L^{a}-D₁ and L^{a}-D₂ are the same or different and are non-restrictively selected from the following structures or stereoisomers thereof:
(1) Auristatins 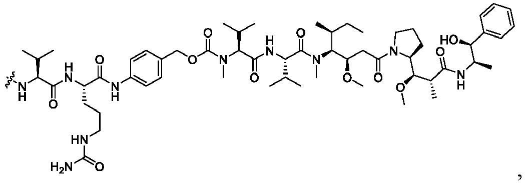 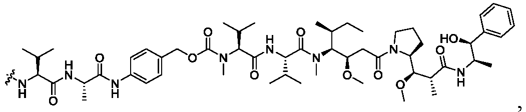 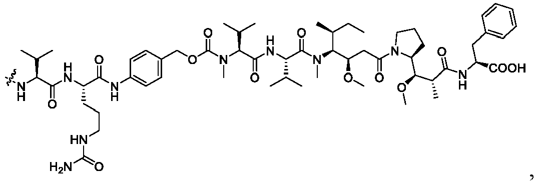 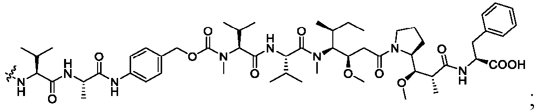
(2) Maytansinoids 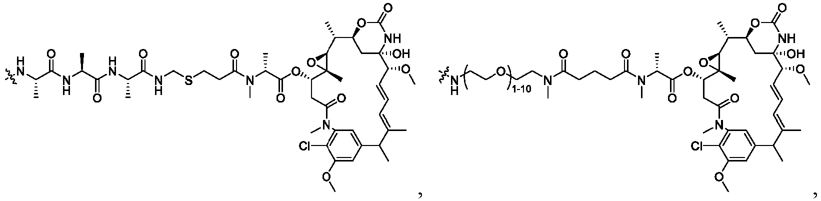 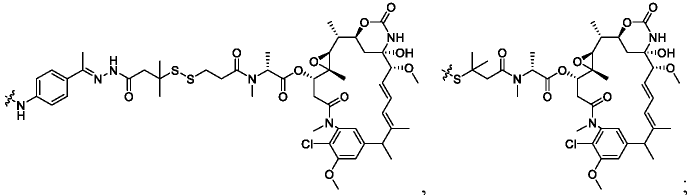
(3) Benzodiazepines 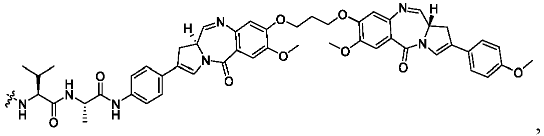 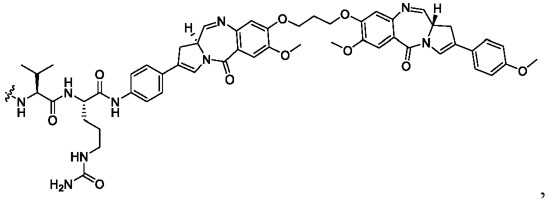 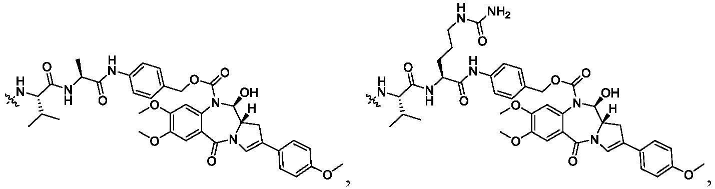 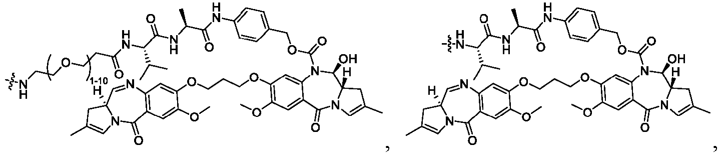 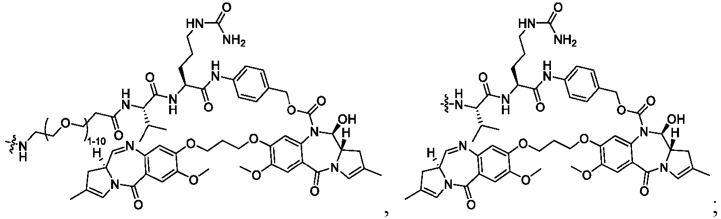
(4) Camptothecin analogues 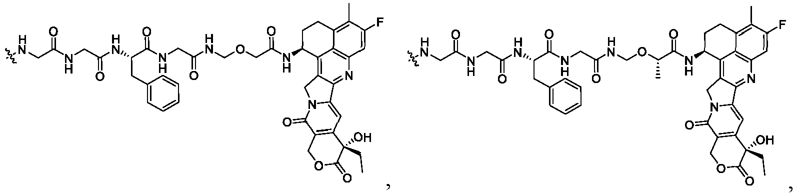 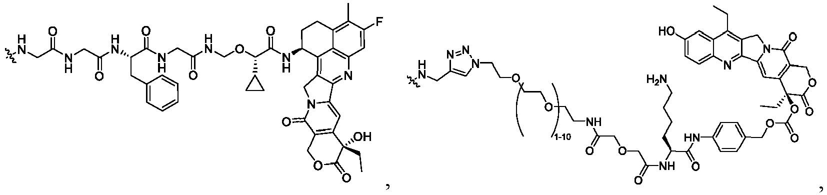 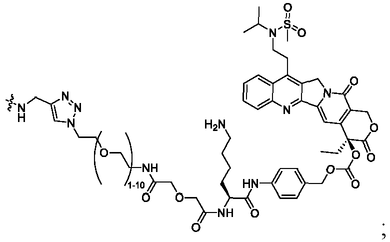
(5) Tubulysins 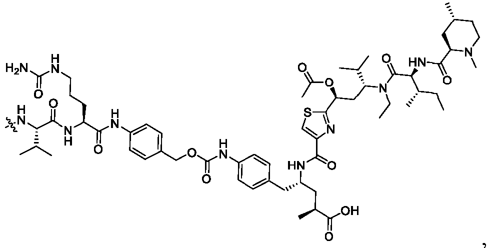 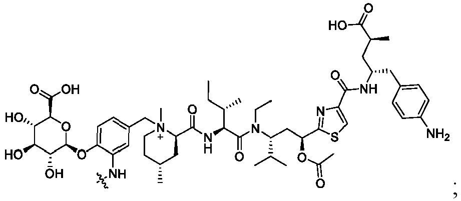
(6) Adriamycins 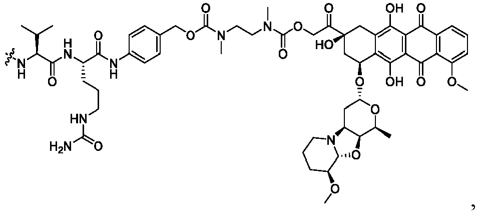 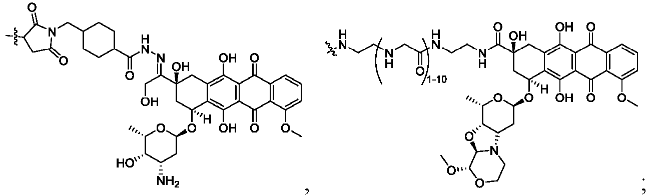
(7) Calicheamicins 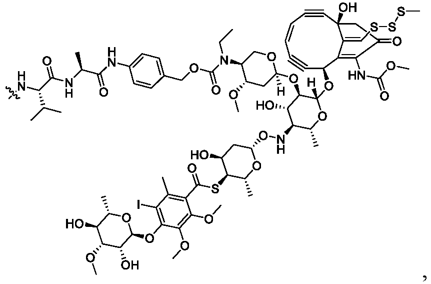 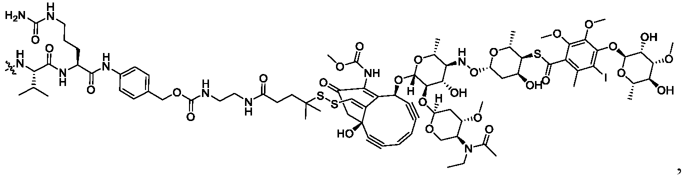 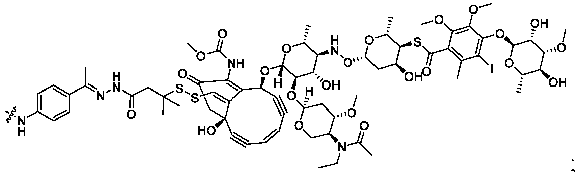
(8) Duocarmycins 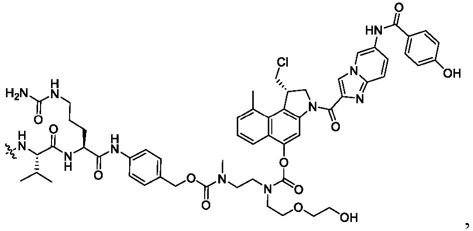 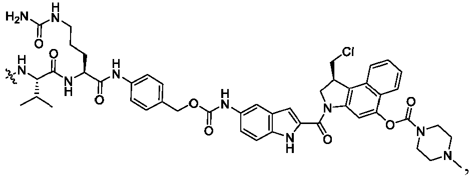 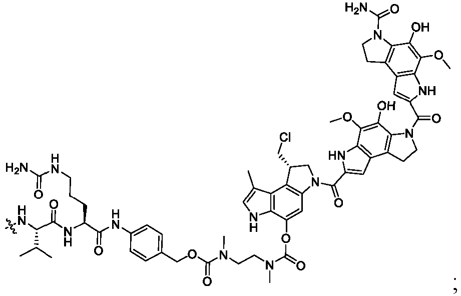
(9) Other antitumor drugs 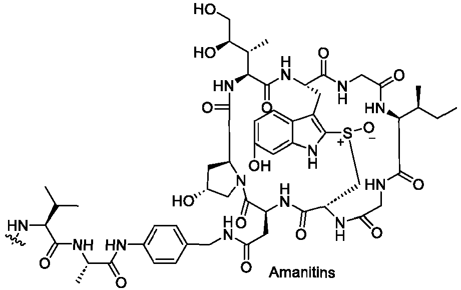 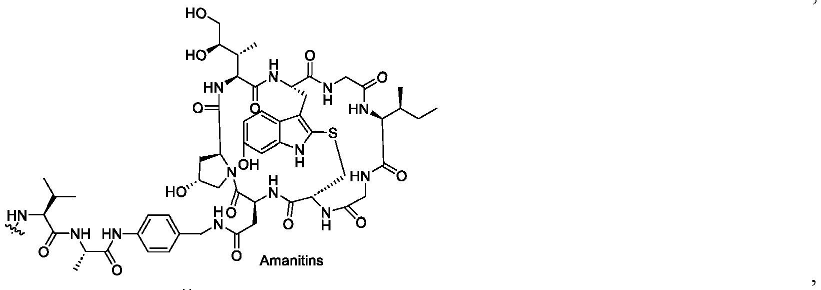 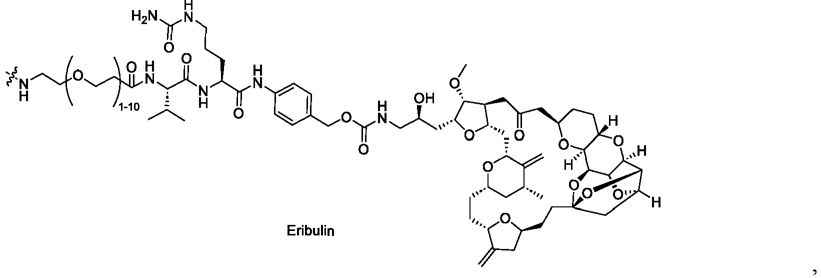 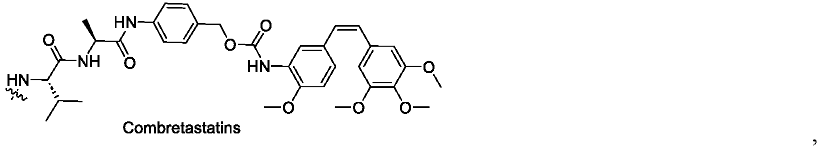 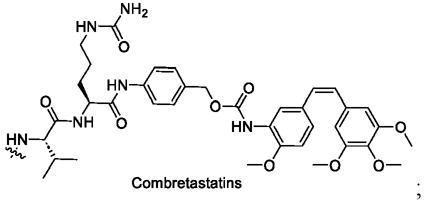
wherein
the position indicated by a wavy line is connected to the linking scaffold A, the linking structure B or the branching unit C.

In some embodiments, the L^{a}-D, L^{a}-D₁ or L^{a}-D₂ is selected from:

Auristatins, for example,

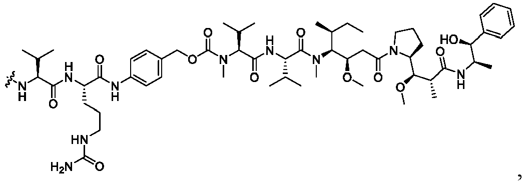

Benzodiazepines, for example,

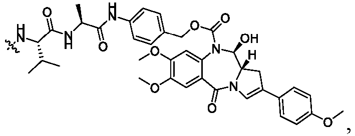

Camptothecin analogues, for example,

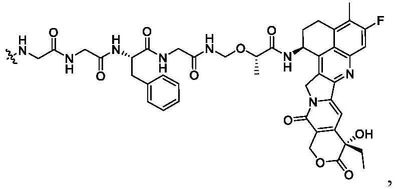

Combretastatins, for example,

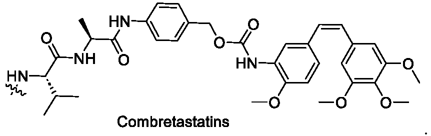

In some embodiments, the linking unit L^{b} is non-restrictively selected from the following structures or stereoisomers thereof: 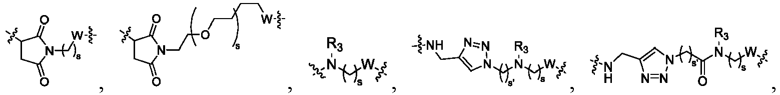 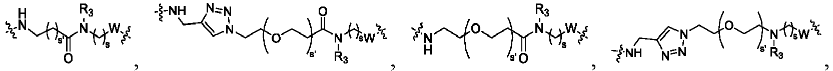 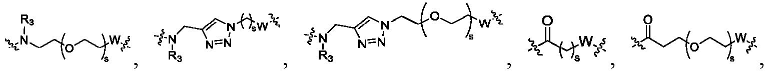 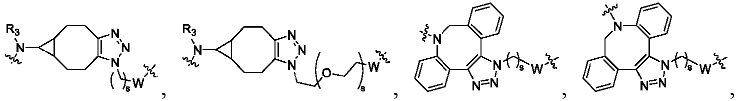 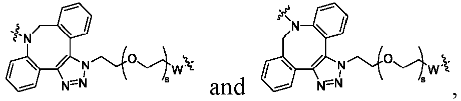 wherein
R₃ is selected from hydrogen atom, deuterium atom, halogen, C1-C6 alkyl, deuterated C1-C6 alkyl, halogenated C1-C6 alkyl, C3-C8 cycloalkyl C1-C6 alkyl,C1-C6 alkoxy C1-C6 alkyl, heterocyclyl, aryl, substituted aryl, and heteroaryl;
W is selected from -NR₄-, -O-, -S-, -CO- and -CONR₅-;
R₄ or R₅ is independently selected from hydrogen atom, deuterium atom, halogen,C1-C6 alkyl, deuterated C1-C6 alkyl, halogenated C1-C6 alkyl, C3-C8 cycloalkyl C1-C6 alkyl,C1-C6 alkoxy C1-C6 alkyl, heterocyclyl, aryl, substituted aryl, and heteroaryl;
s is selected from integers of 1 to 10;
s' is selected from integers of 1 to 10;
the positions indicated by a wavy line on the left and a wavy line on the right are non-restrictively connected to the SU or to the linking scaffold A.

In some embodiments, the L^{b}-SU is non-restrictively selected from the following structures:

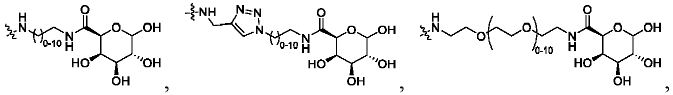

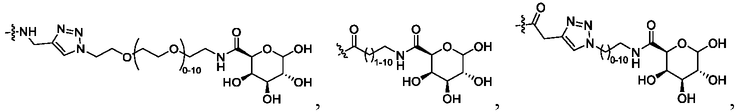

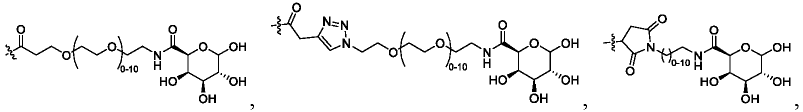

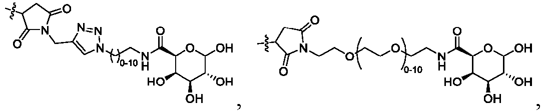

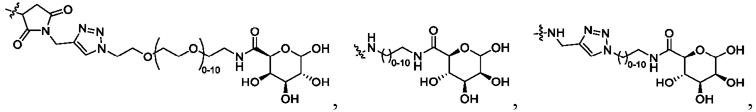

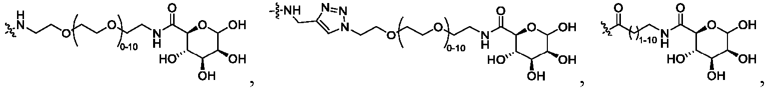

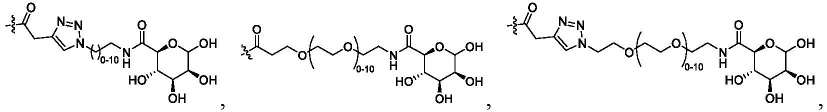

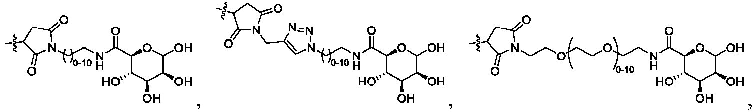

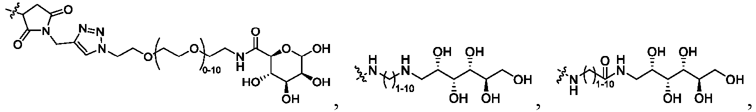

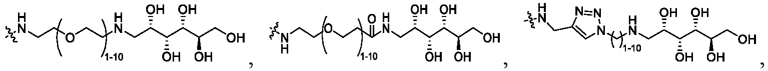

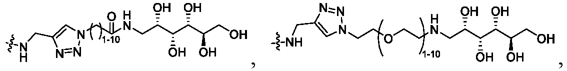

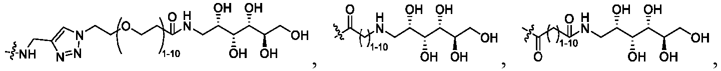

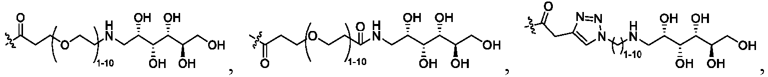

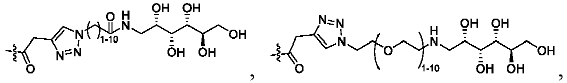

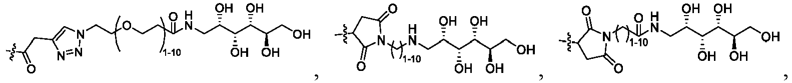

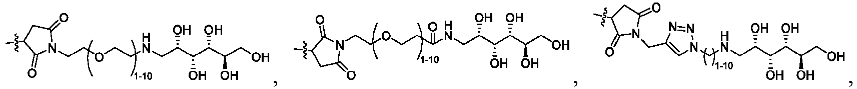

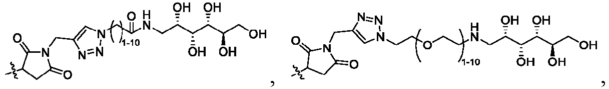

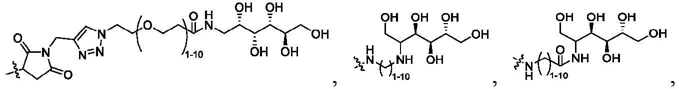

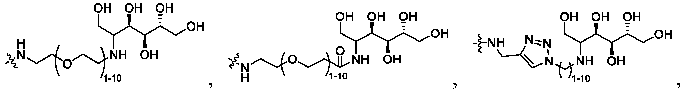

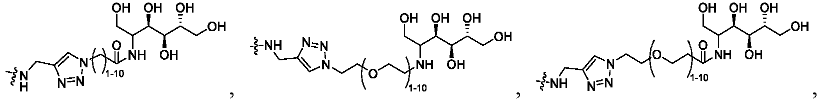

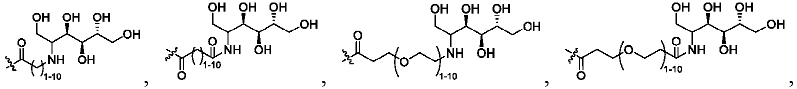

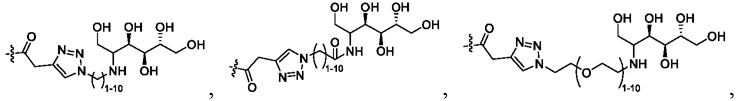

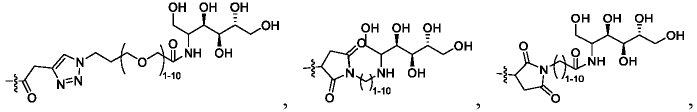

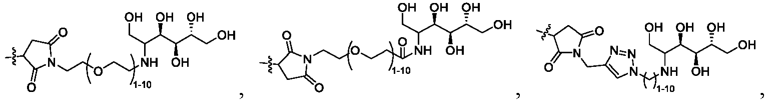

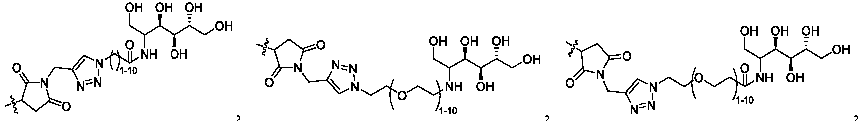

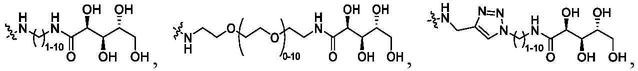

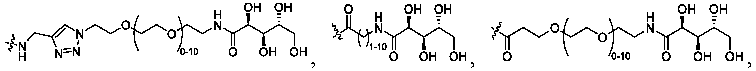

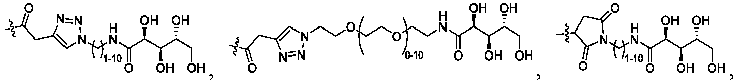

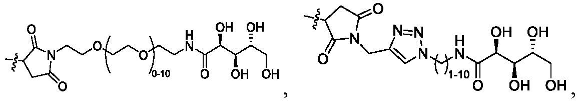

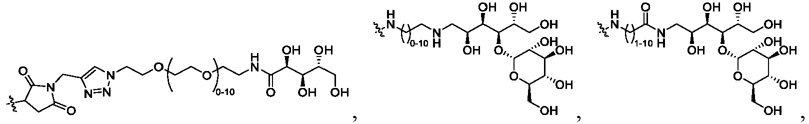

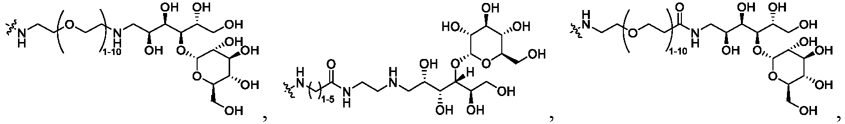

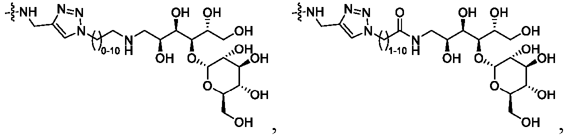

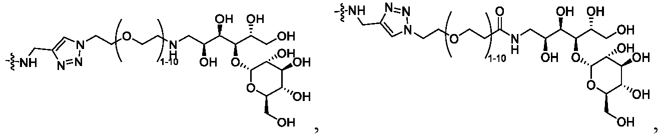

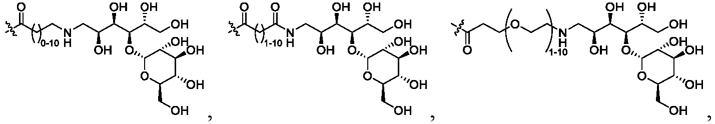

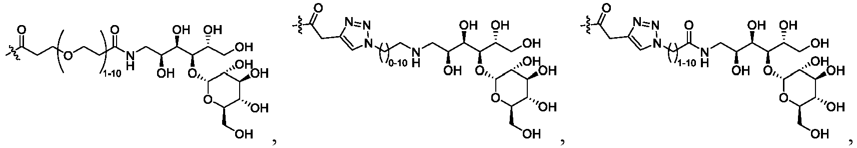

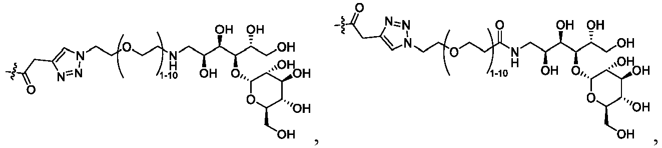

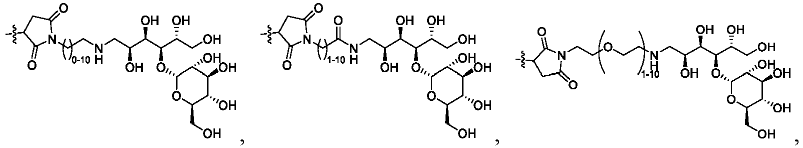

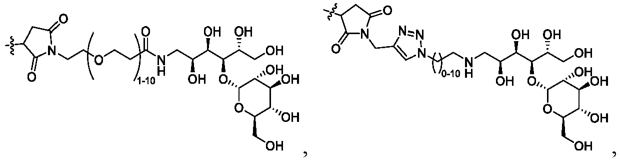

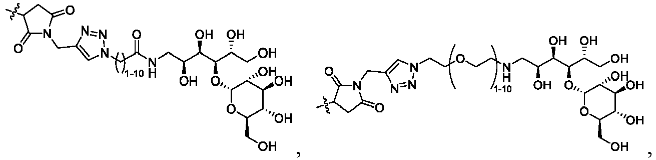

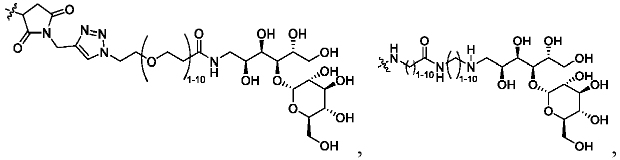

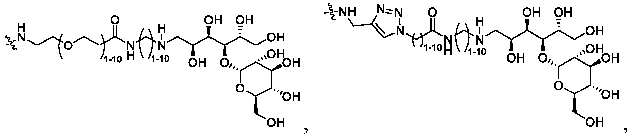

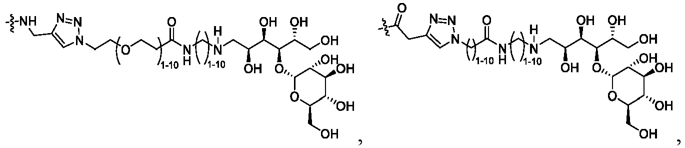

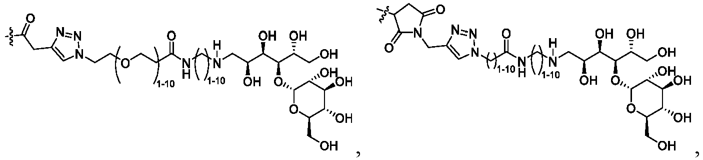

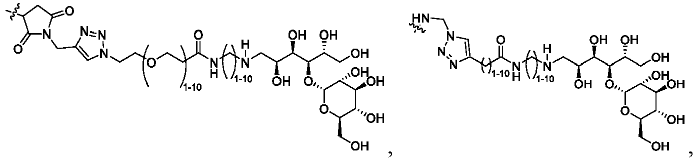

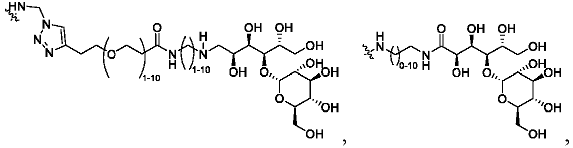

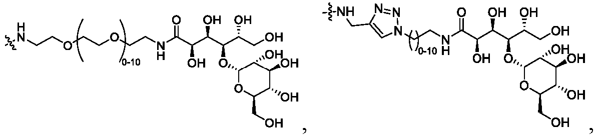

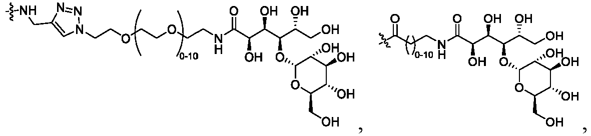

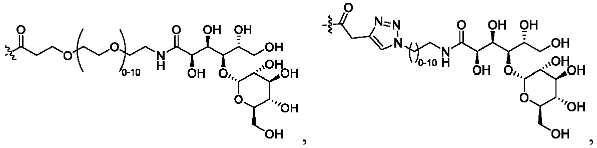

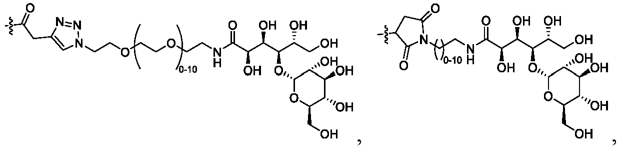

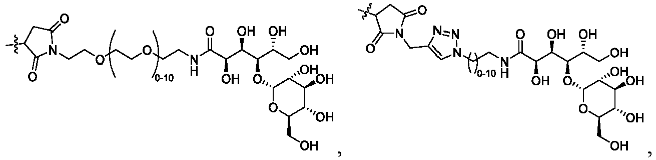

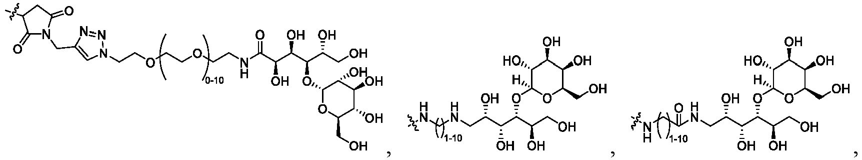

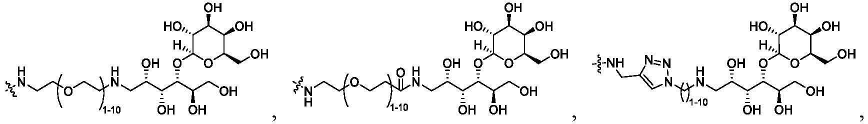

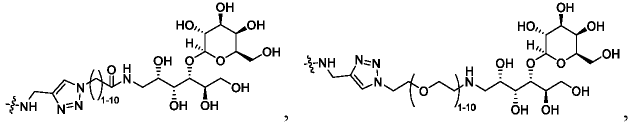

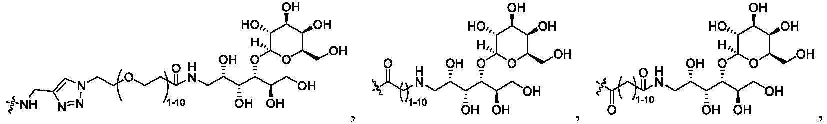

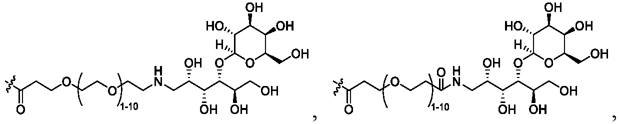

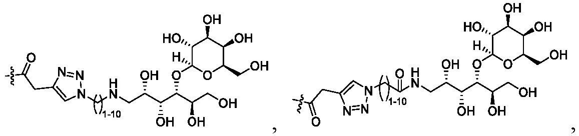

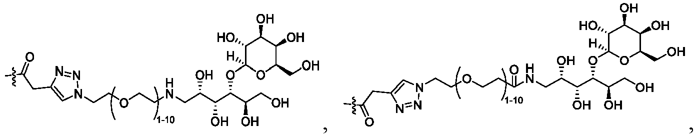

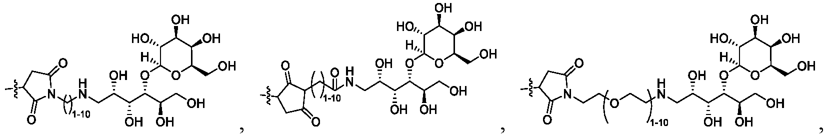

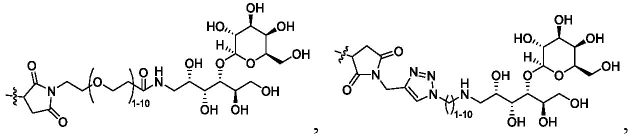

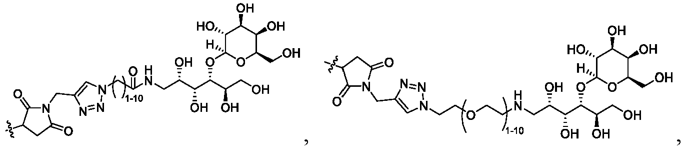

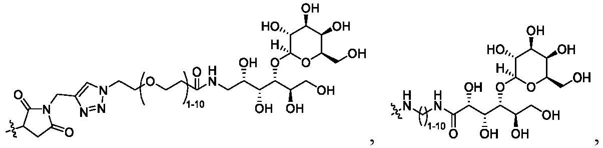

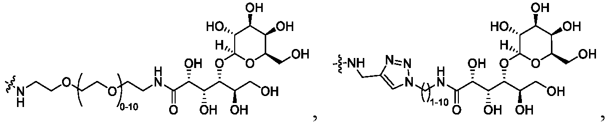

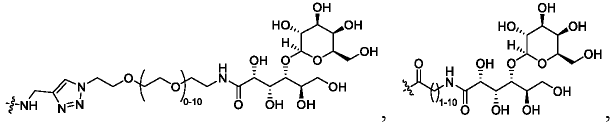

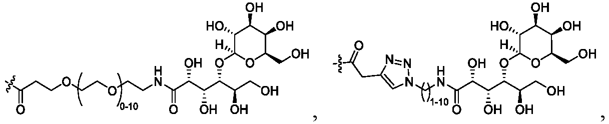

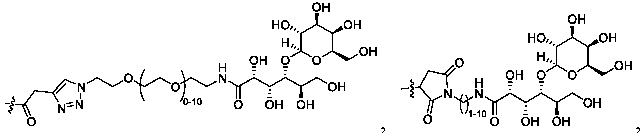

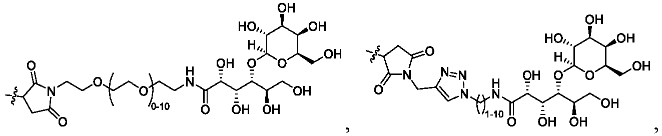

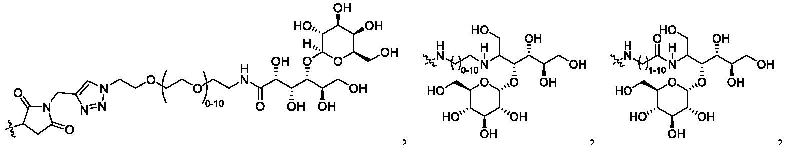

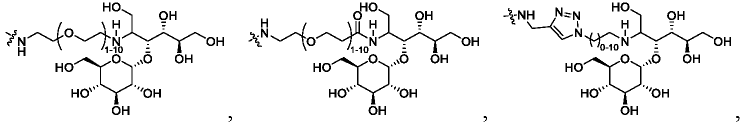

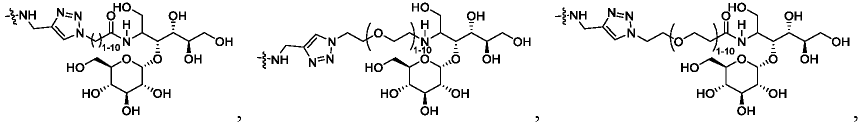

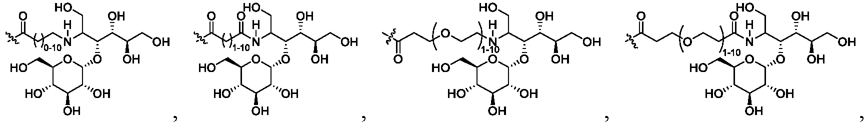

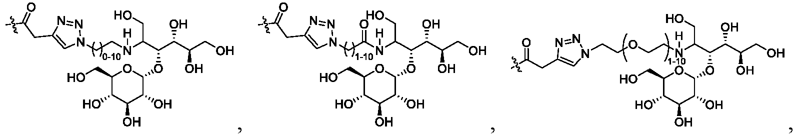

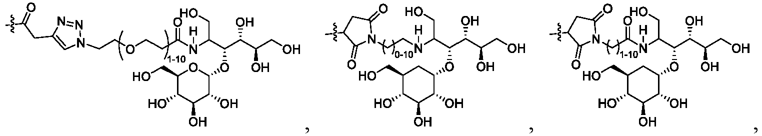

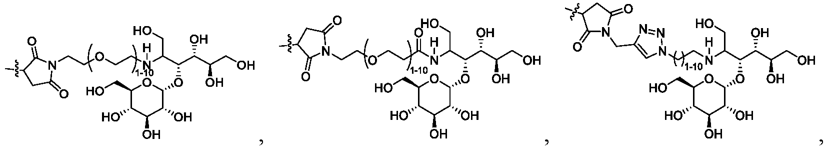

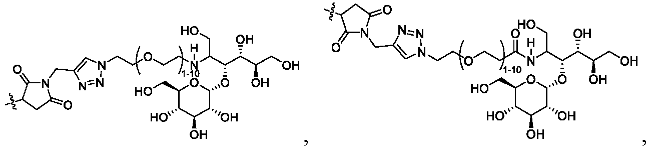

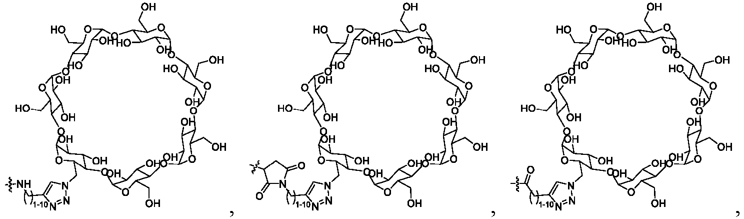

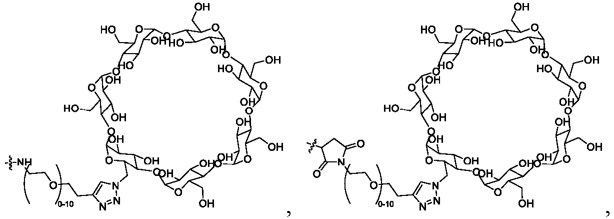

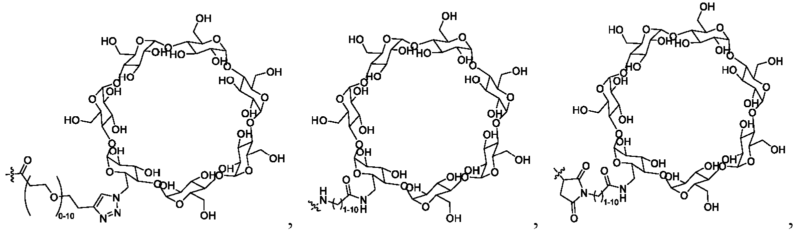

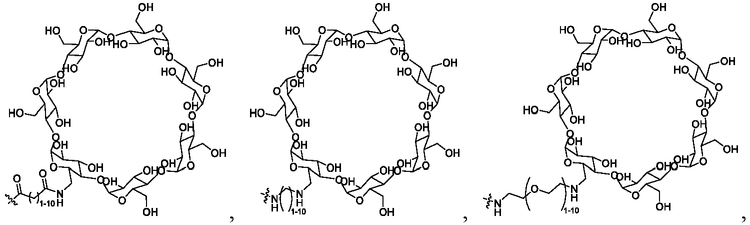

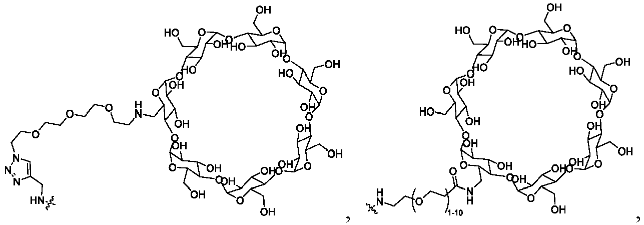

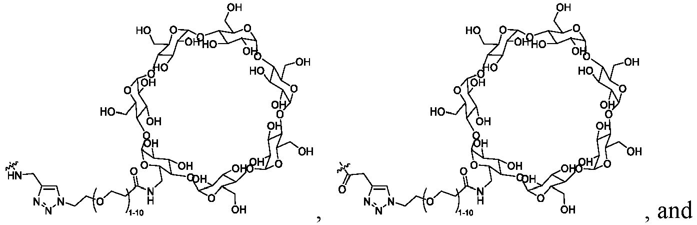

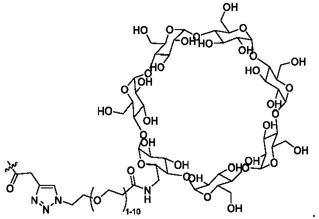

In some embodiments, the L^{b}-SU is non-restrictively selected from the following structures:

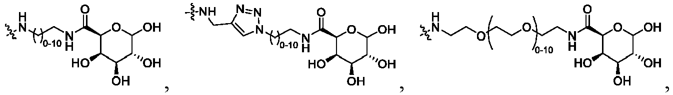

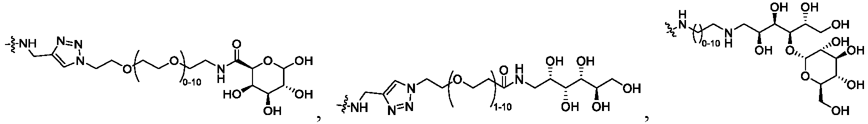

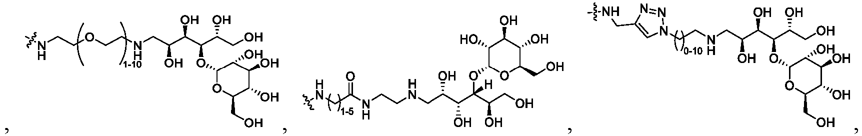

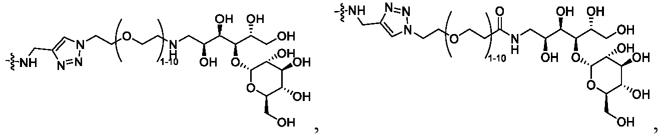

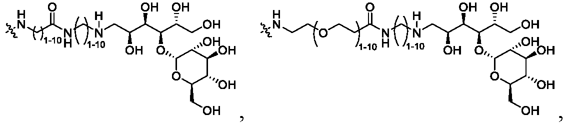

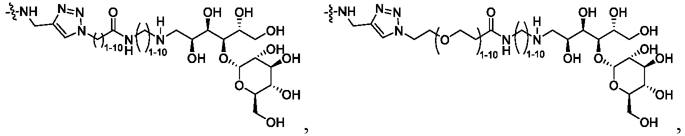

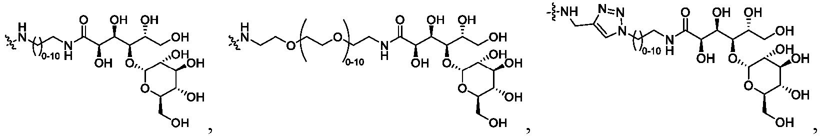

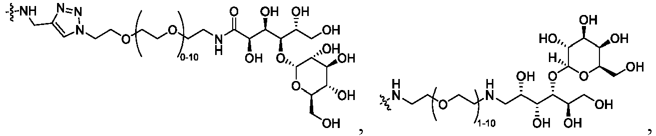

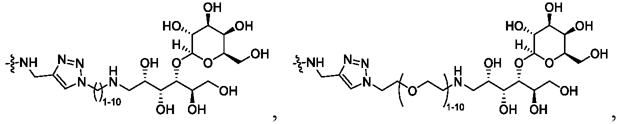

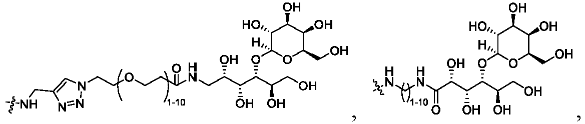

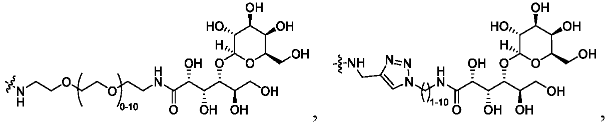

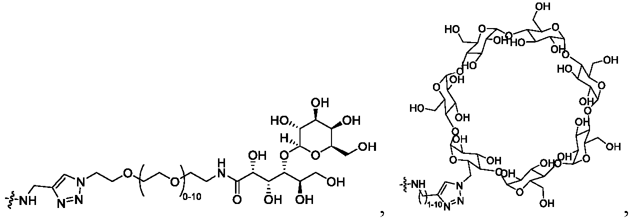

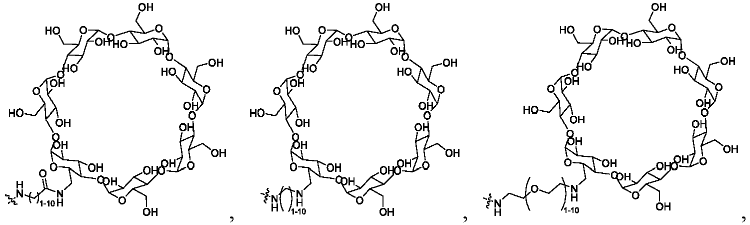

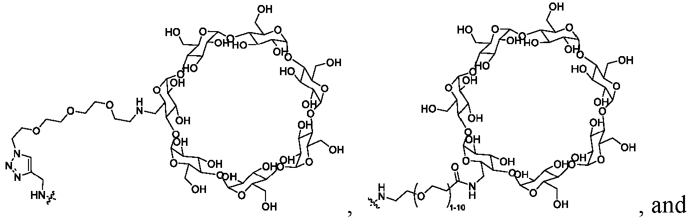

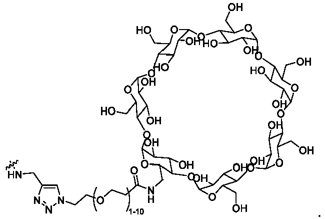

In some embodiments, the linker-drug compound is non-restrictively selected from the following structures: 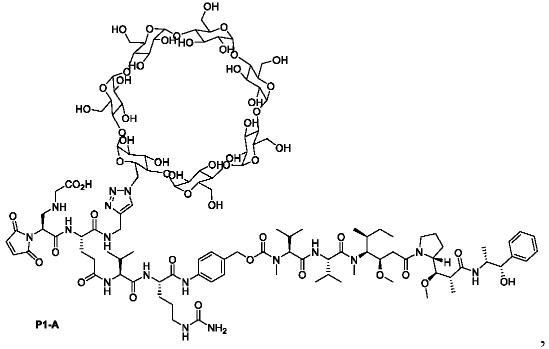 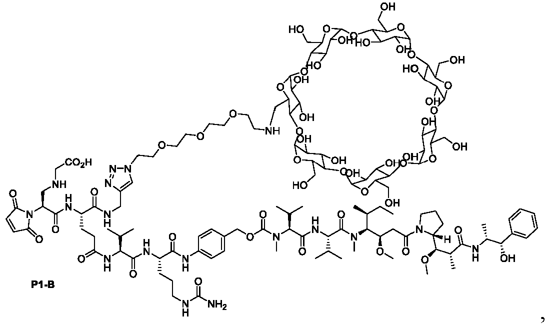 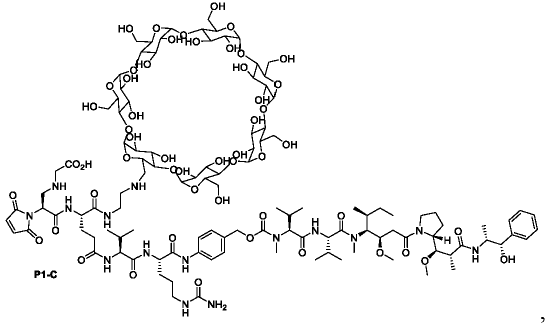 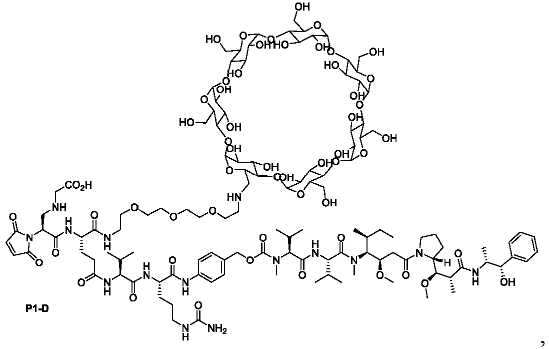 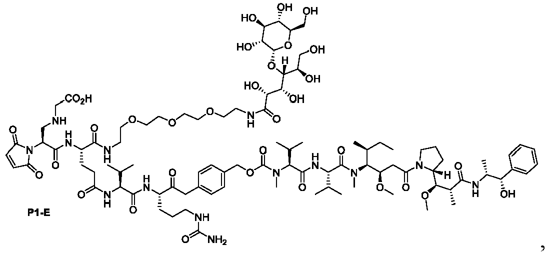 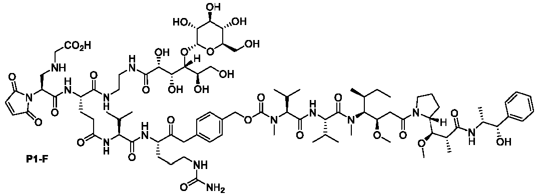 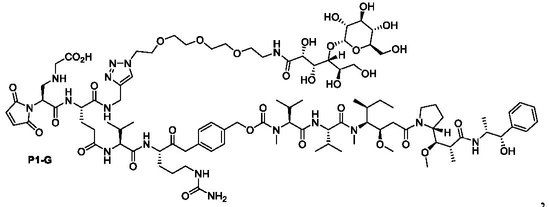 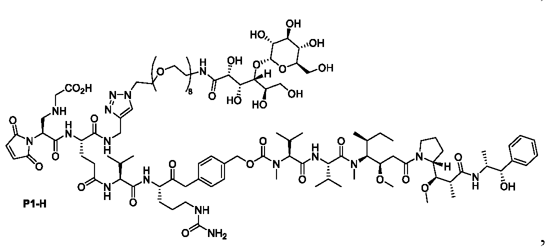 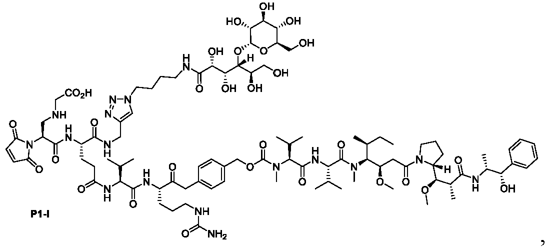 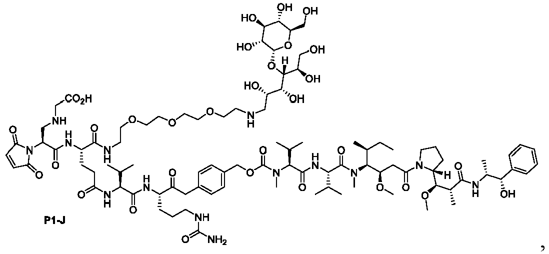 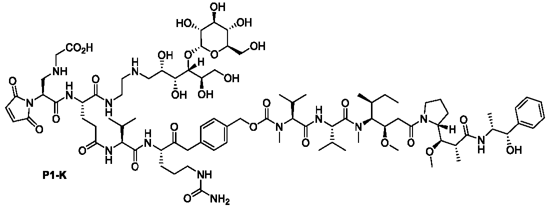 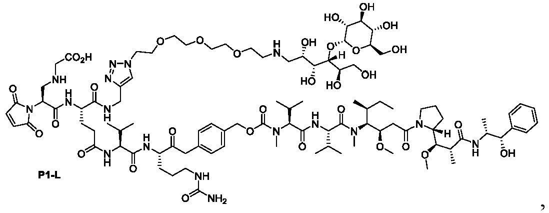 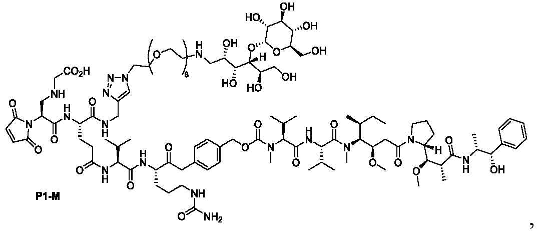 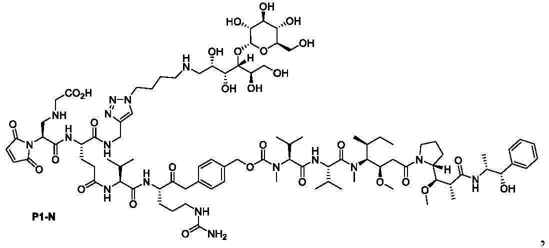 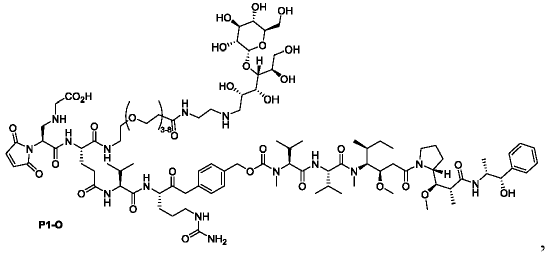 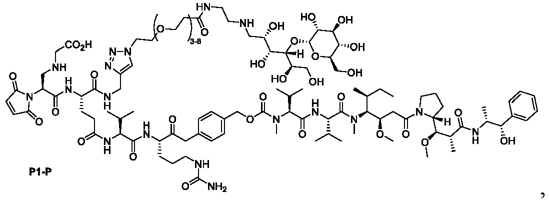 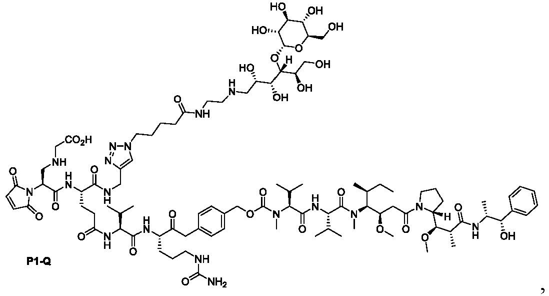 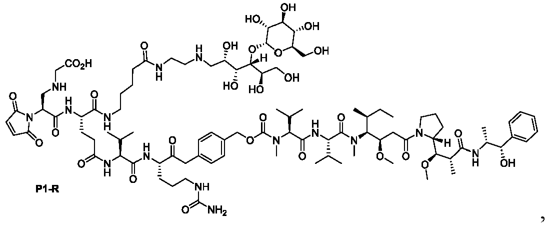 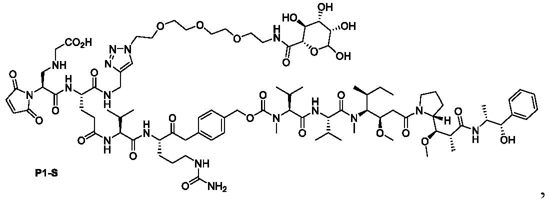 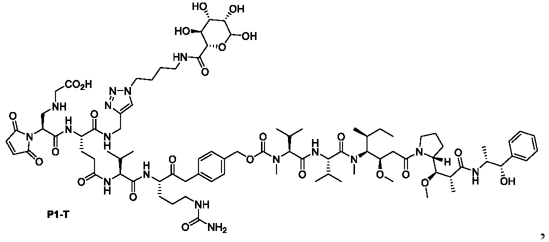 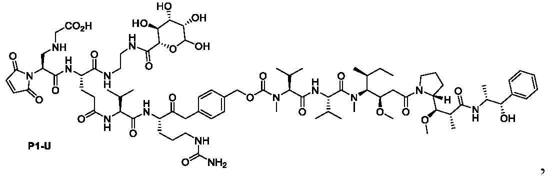 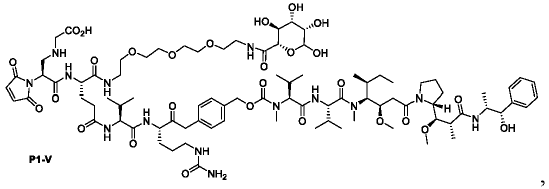 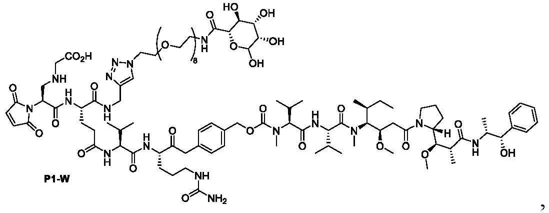 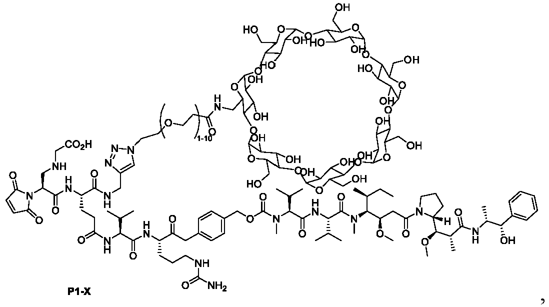 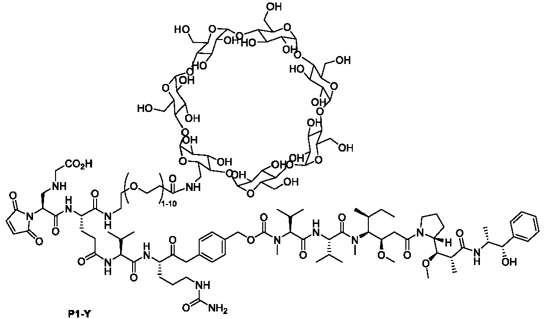 and

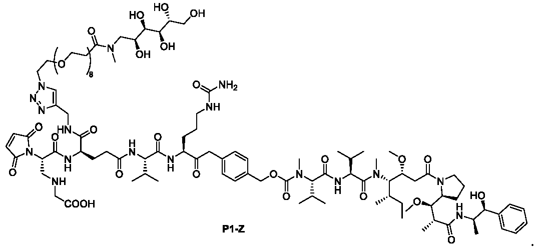

In some embodiments, the linker-drug compound is non-restrictively selected from the following structures:

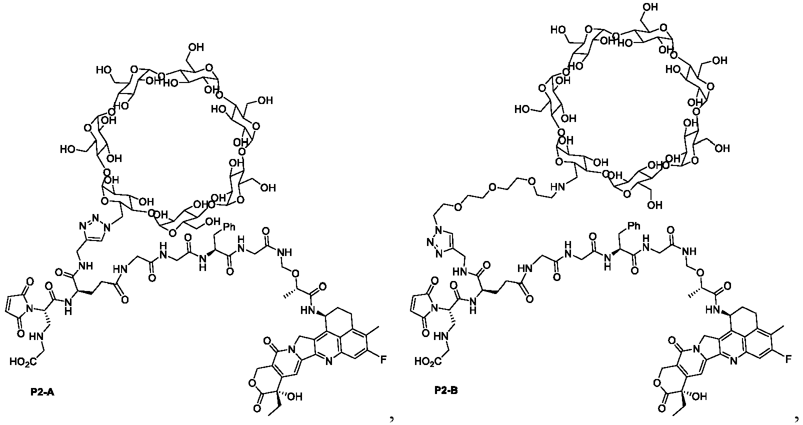

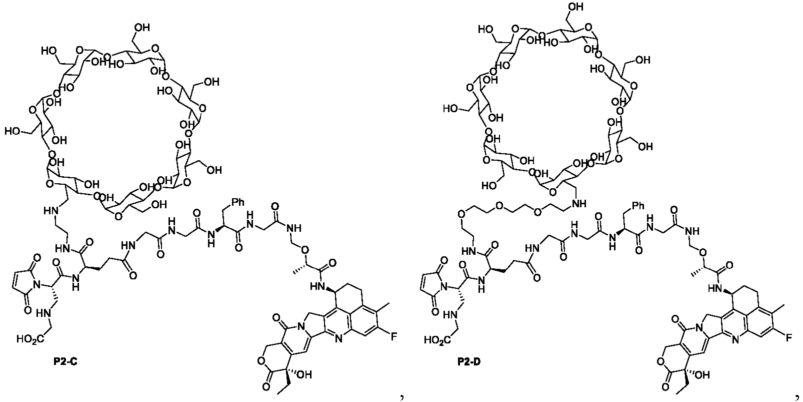

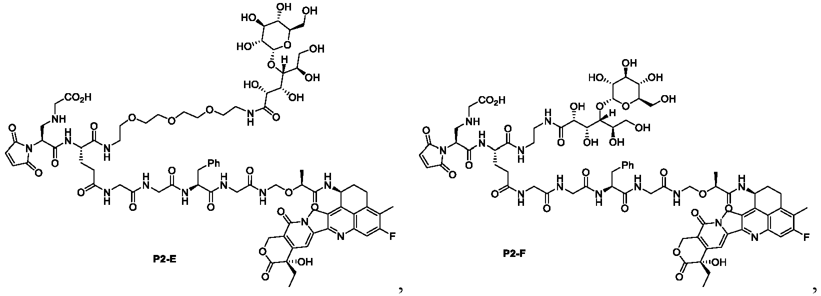

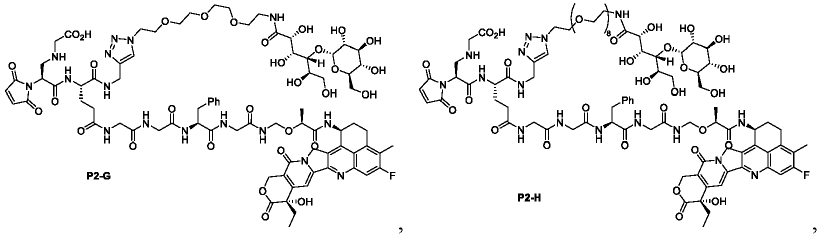

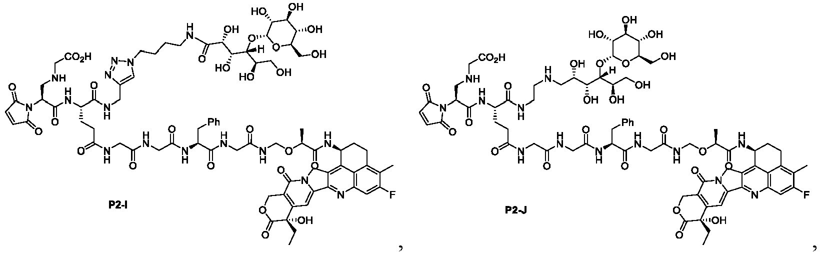

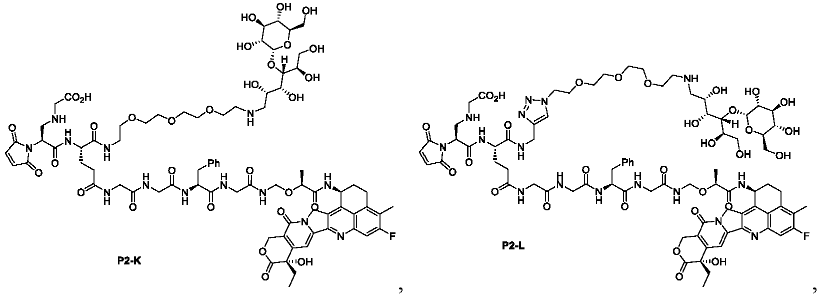

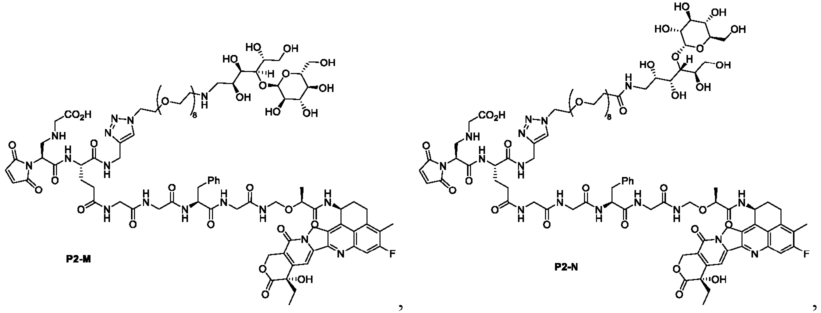

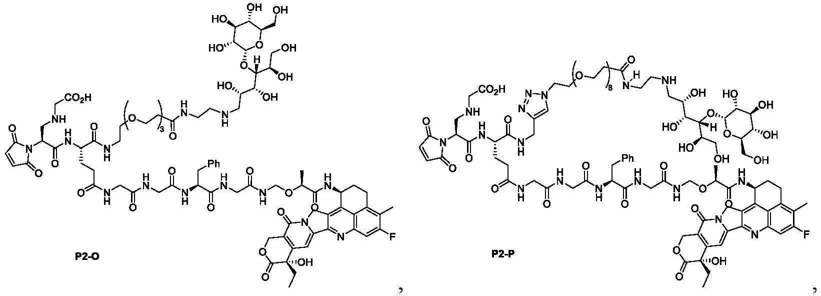

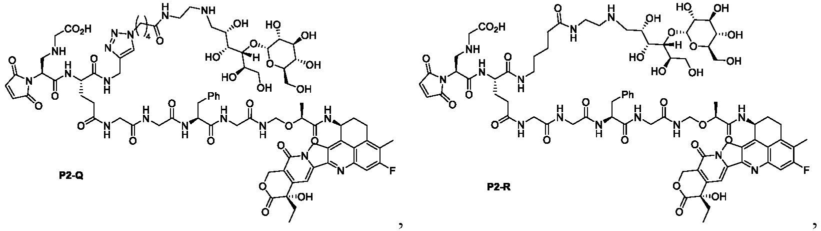

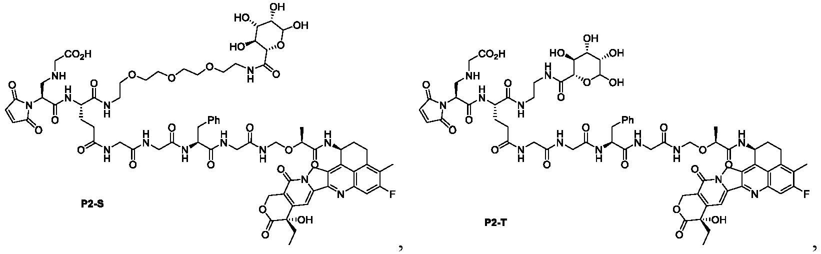

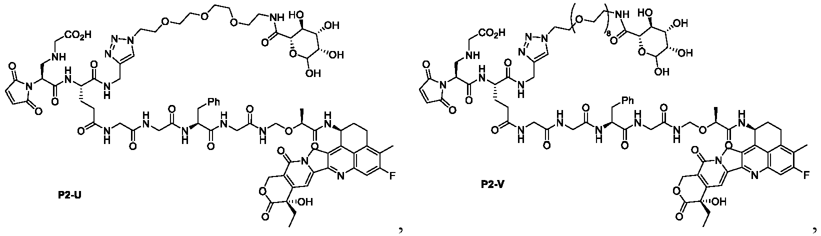

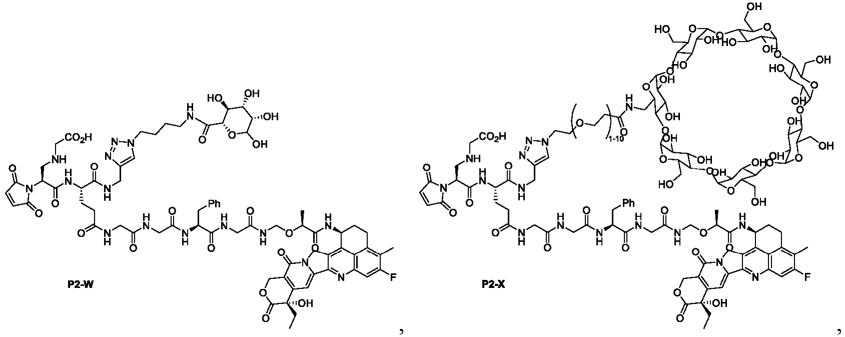

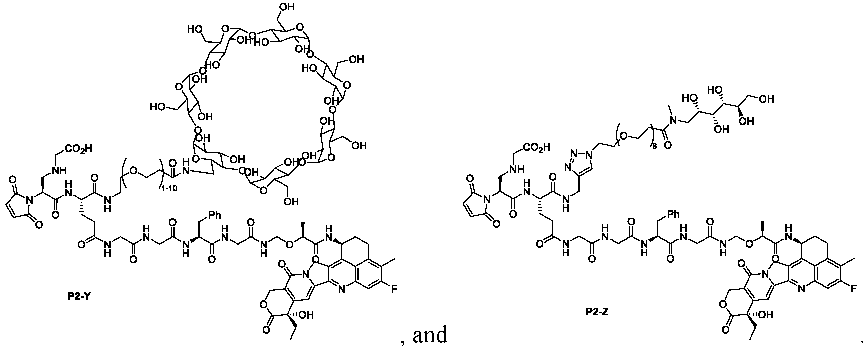

In some embodiments, the linker-drug compound is non-restrictively selected from the following structures:

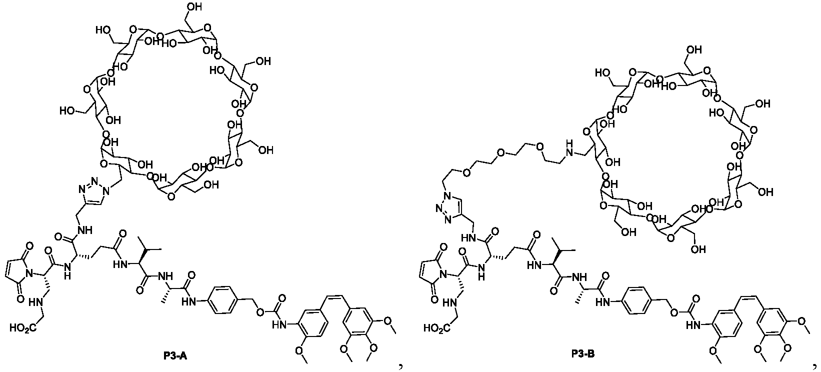

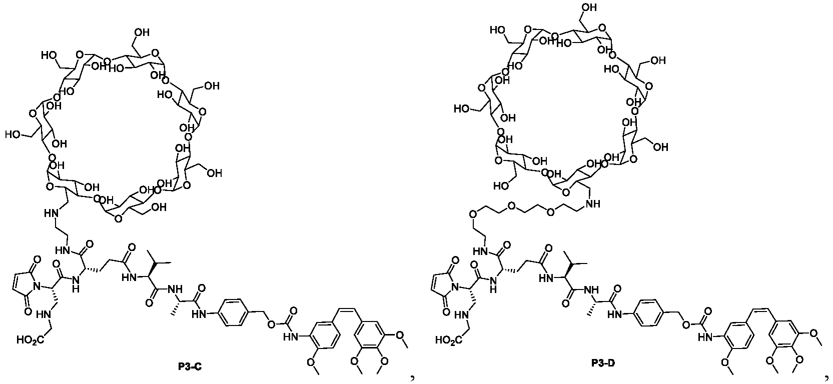

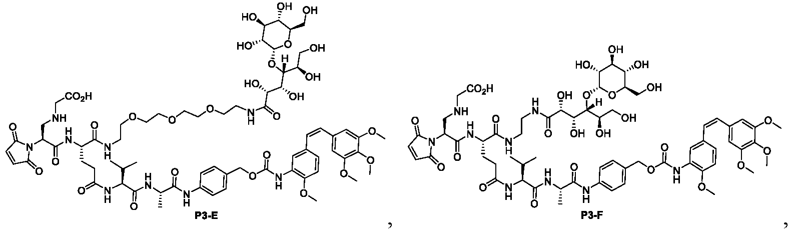

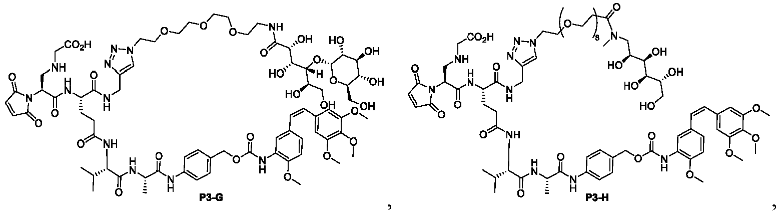

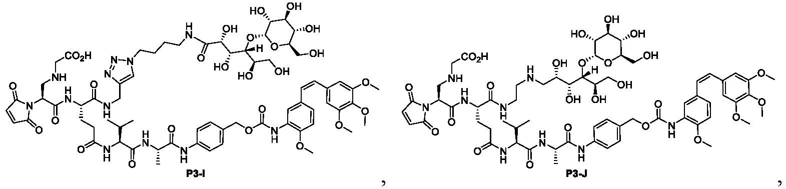

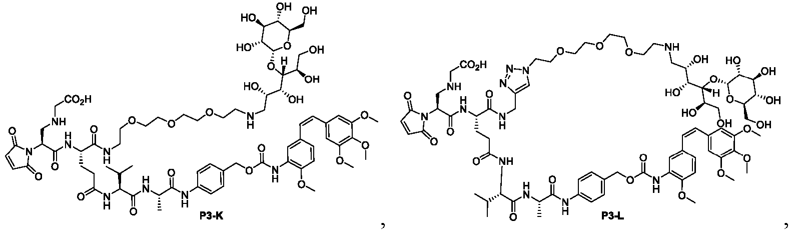

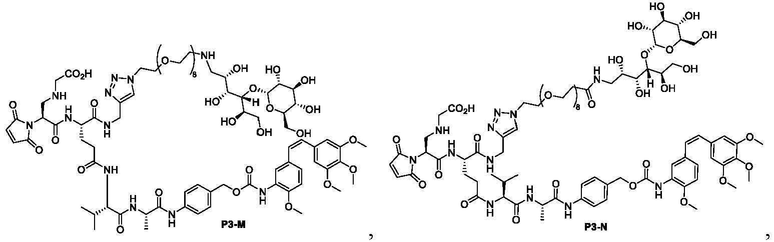

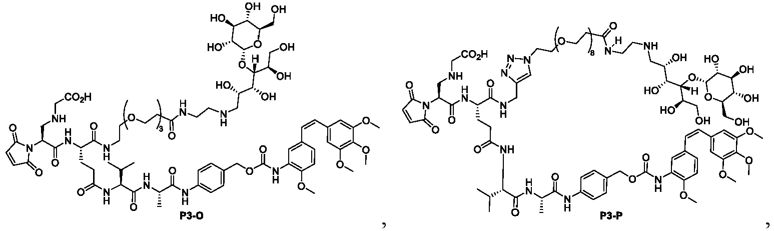

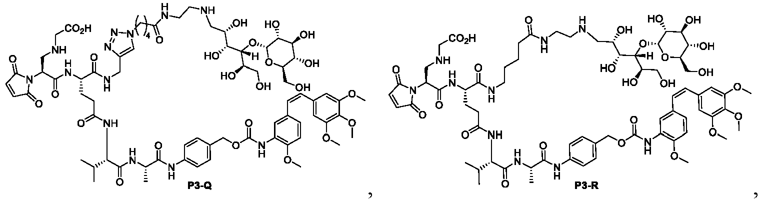

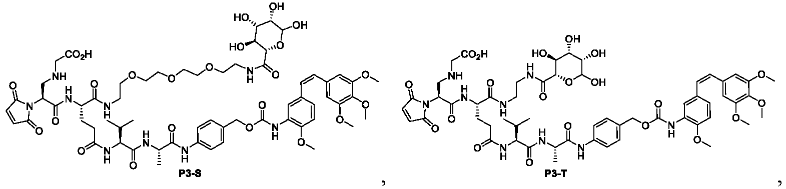

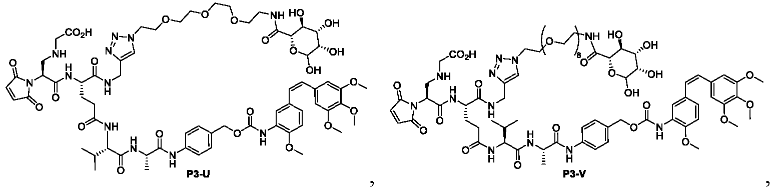

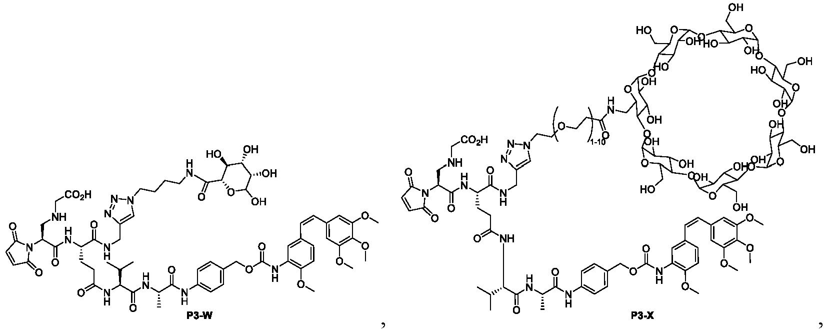

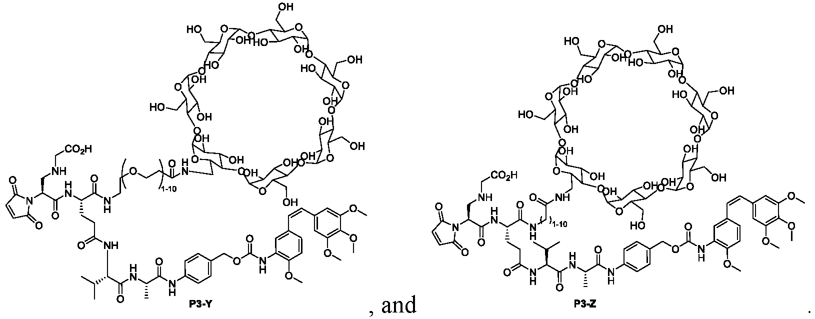

In some embodiments, the linker-drug compound is non-restrictively selected from the following structures:

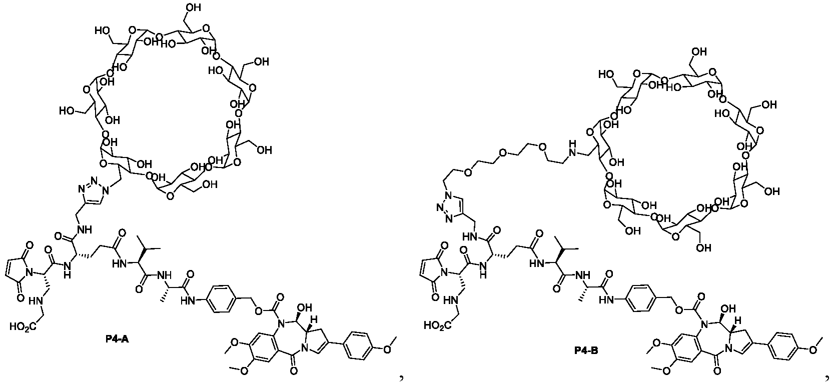

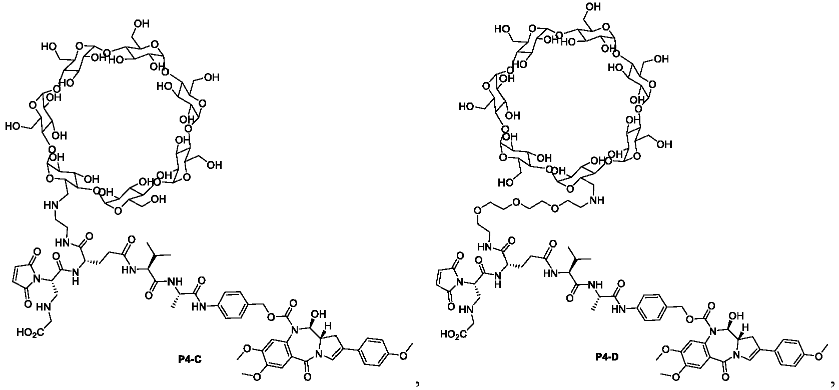

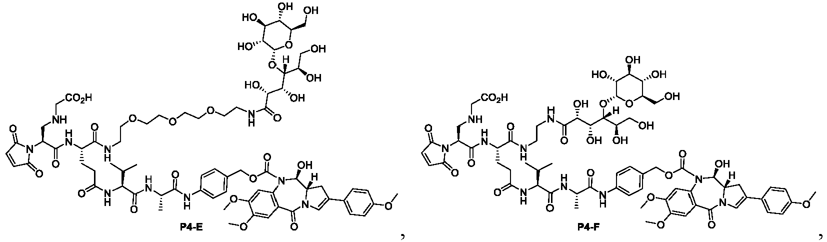

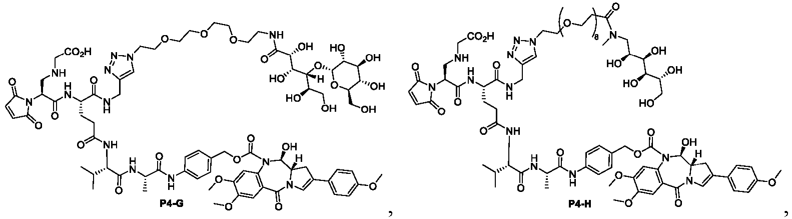

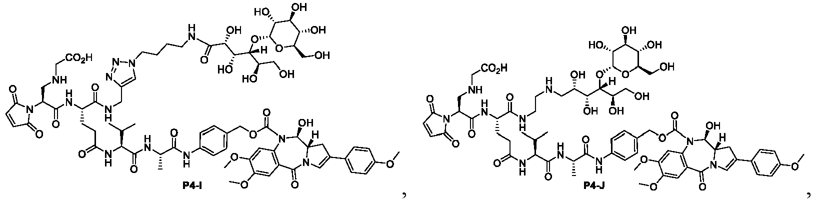

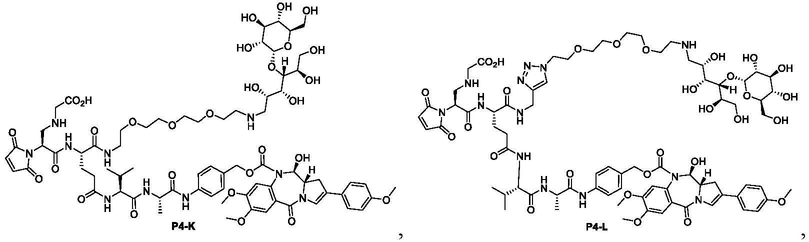

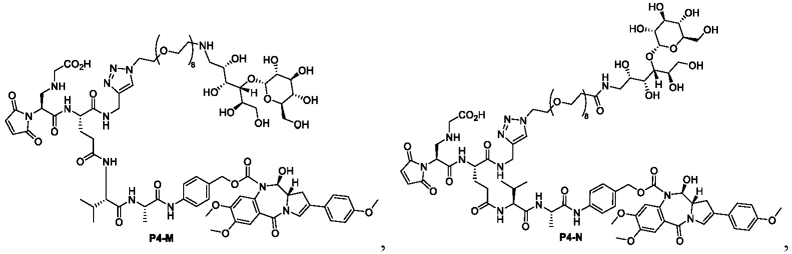

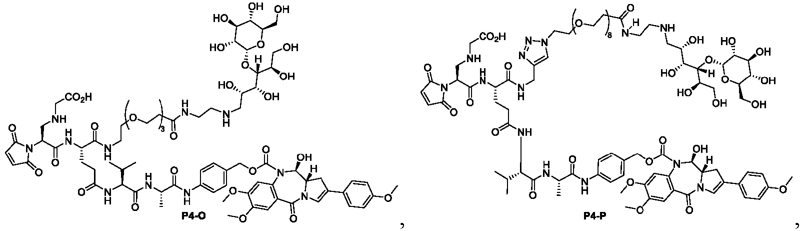

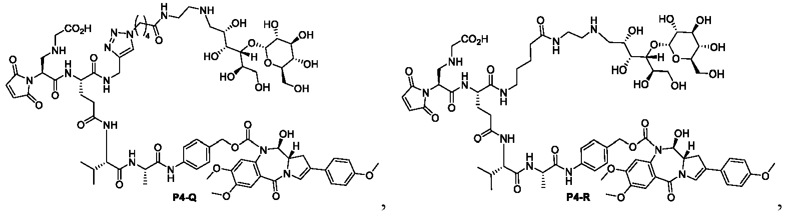

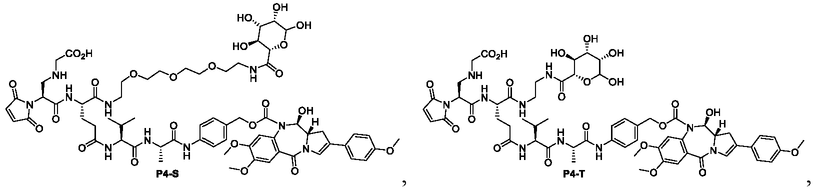

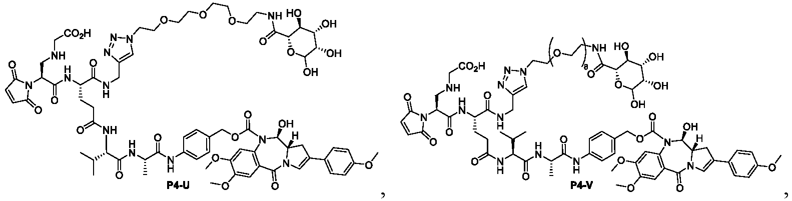

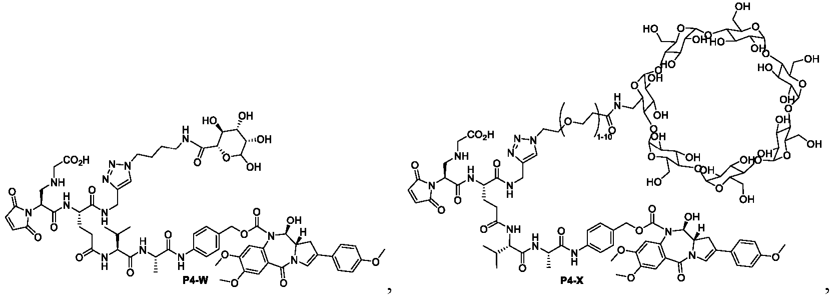

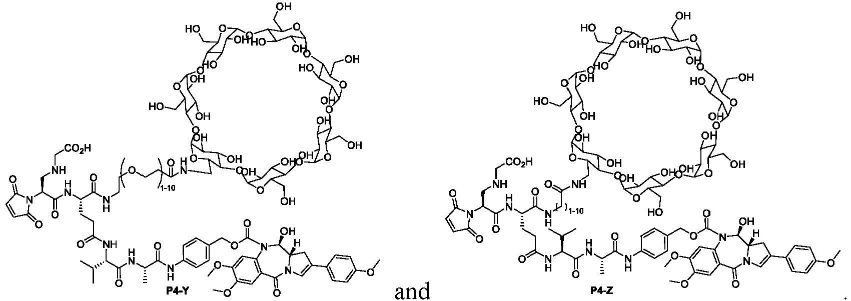

The present application further provides a preparation method for a ligand-drug conjugate of formula I, formula II or formula III, or a pharmaceutically acceptable salt or solvate thereof, comprising the following steps: 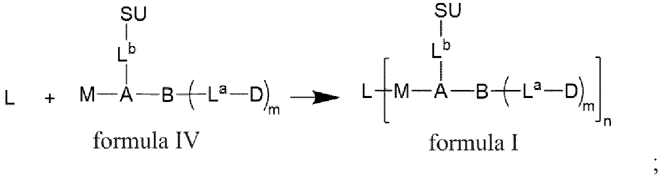 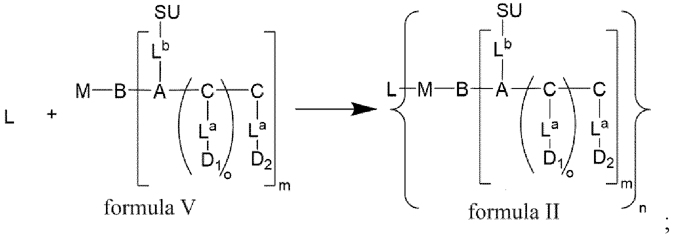 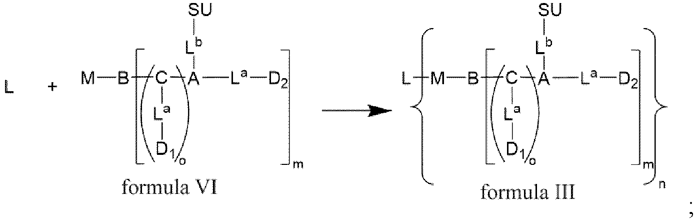
allowing a modified ligand to react with a compound of formula IV, V or VI to obtain a ligand-drug conjugate of formula I, II or III, or a pharmaceutically acceptable salt or solvate thereof,
wherein
L is selected from ligands;
M is selected from any linker units;
A is selected from any linking scaffolds;
B is present or absent, and when present, is selected from any linking structures or bonds;
C is present or absent, and when present, is selected from any branching units or bonds;
D, D₁, D₂ are the same or different, and are each independently selected from drugs;
SU is selected from sugars or derivatives thereof;
L^{a} and L^{b} are the same or different, and are each independently selected from any linking units or bonds;
m is selected from integers of 1 to 5;
n is selected from integers of 1 to 10;
o is selected from integers of 1 to 10.
For the ligand-drug conjugate of the present invention or a pharmaceutically acceptable salt or solvate thereof, or a ligand-drug conjugate containing the linker-drug compound of the invention or a pharmaceutically acceptable salt or solvate of the ligand-drug conjugate, in some embodiments, the ligand-drug conjugate or the pharmaceutically acceptable salt or solvate thereof is non-restrictively selected from the following structures or stereoisomers thereof: 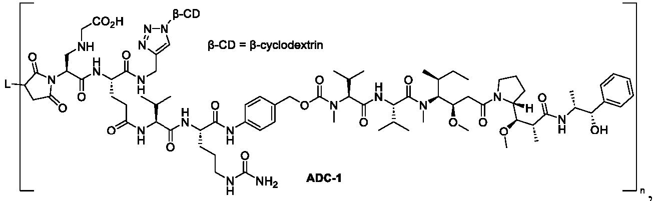 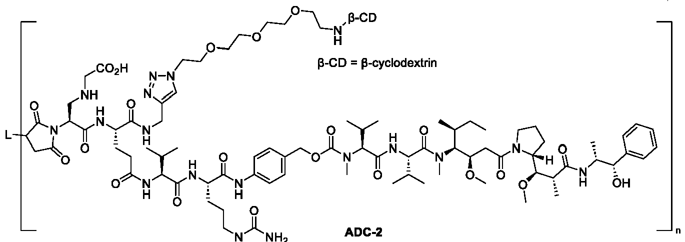 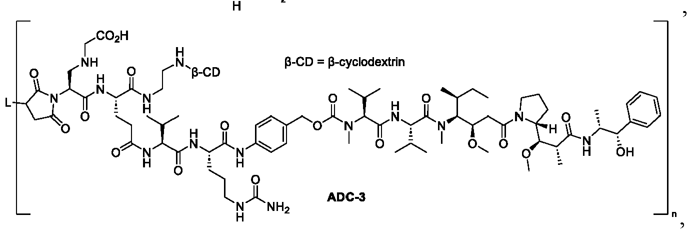 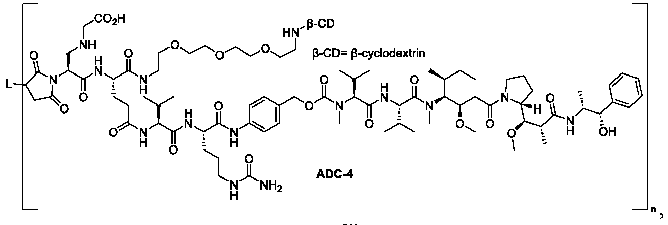 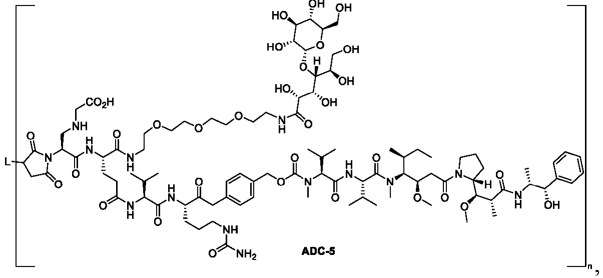 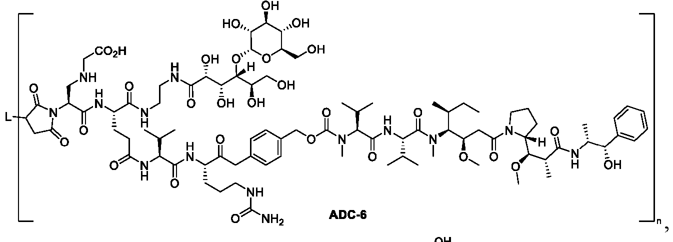 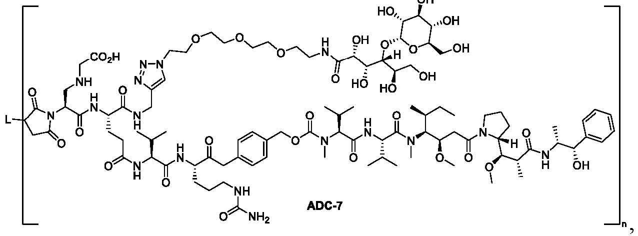 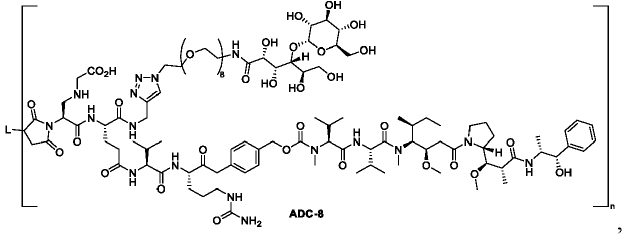 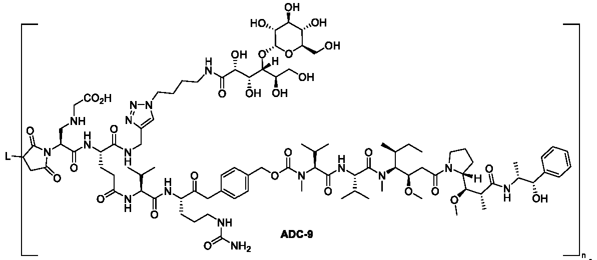 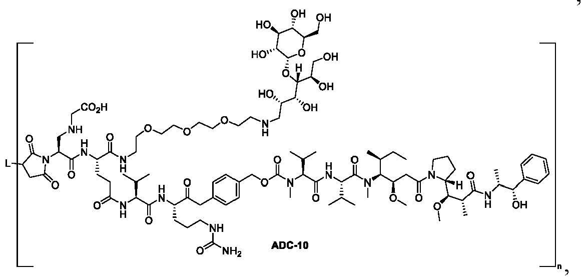 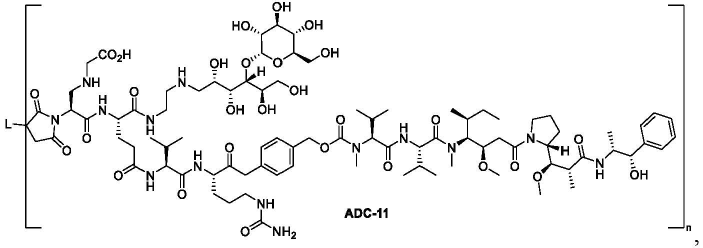 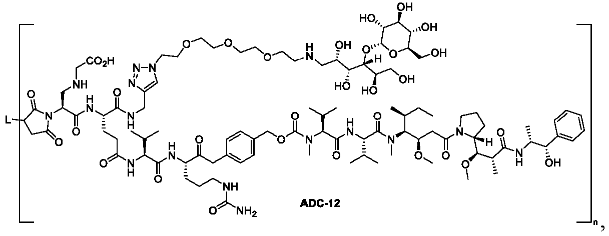 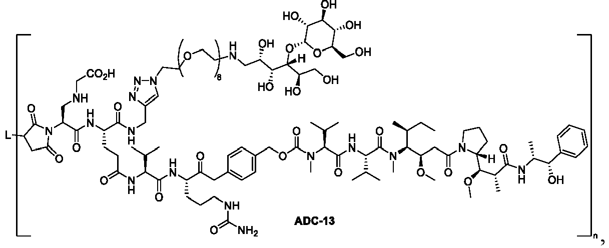 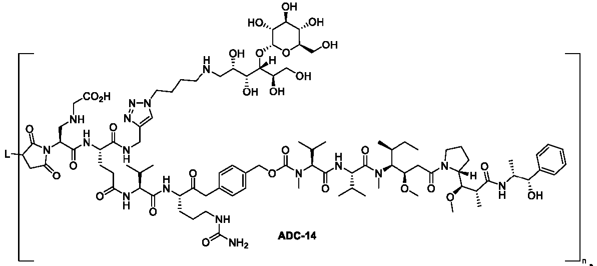 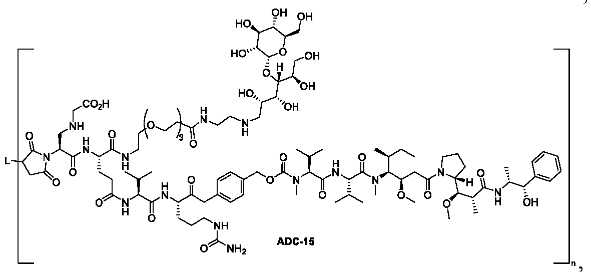 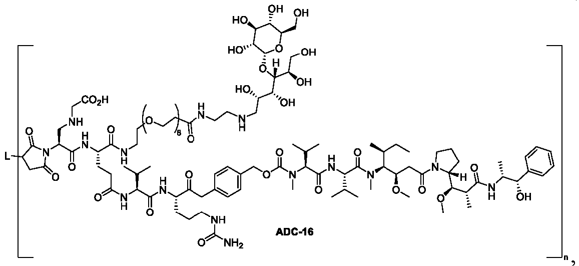 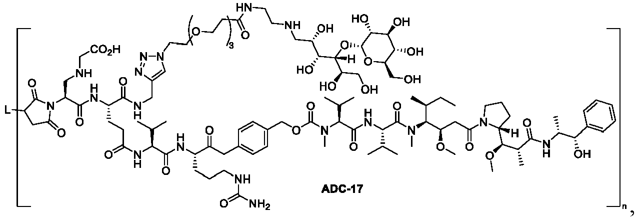 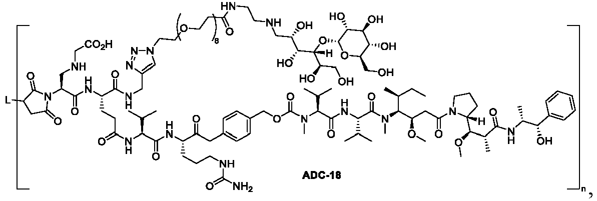 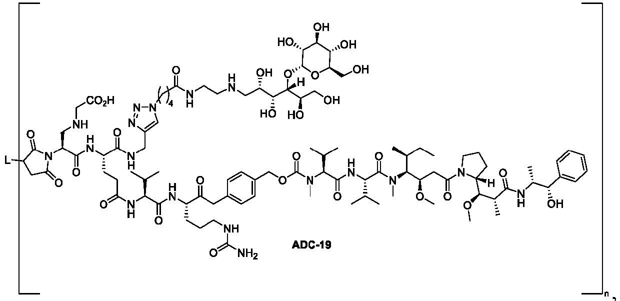 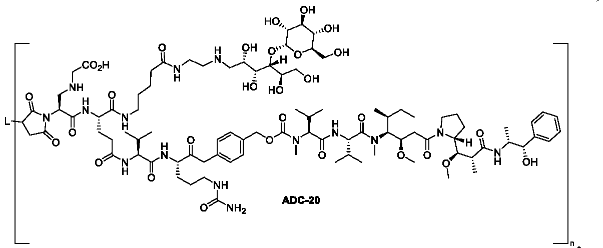 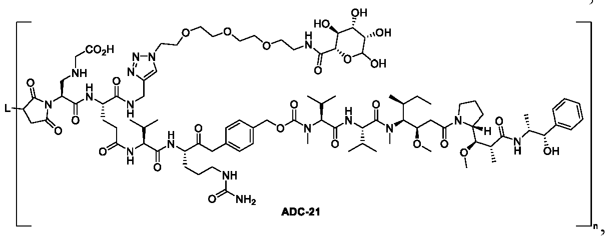 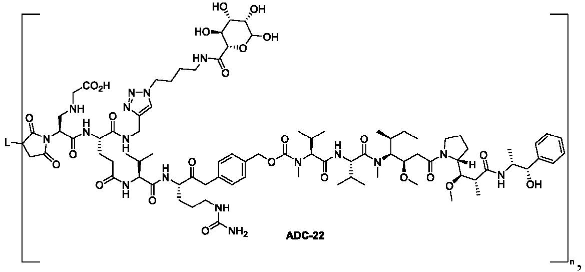 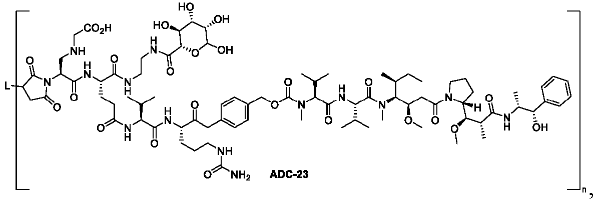 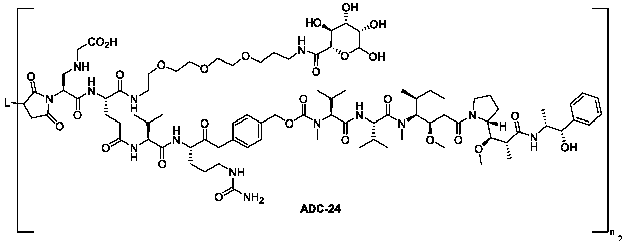 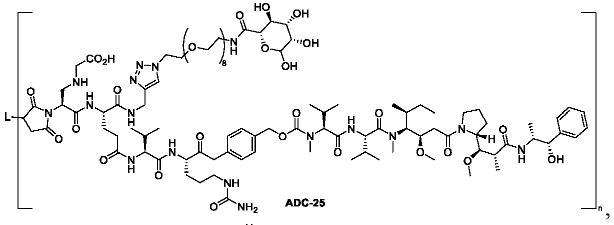 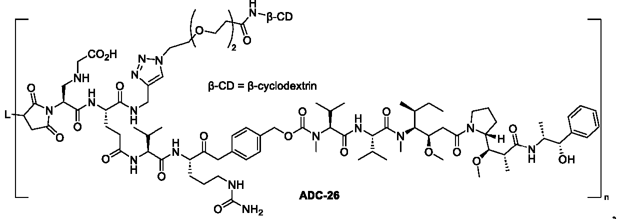 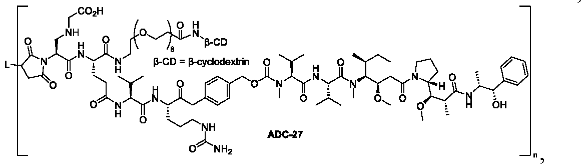 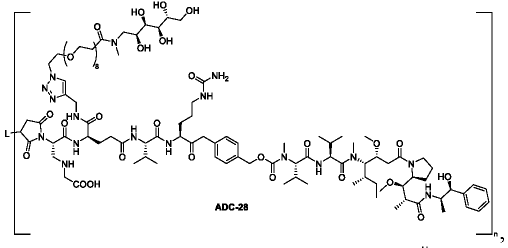 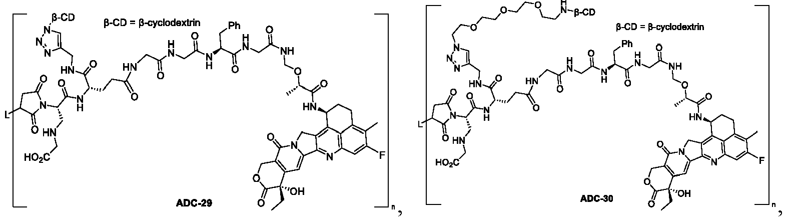 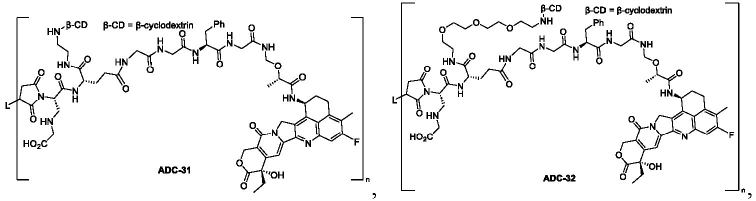 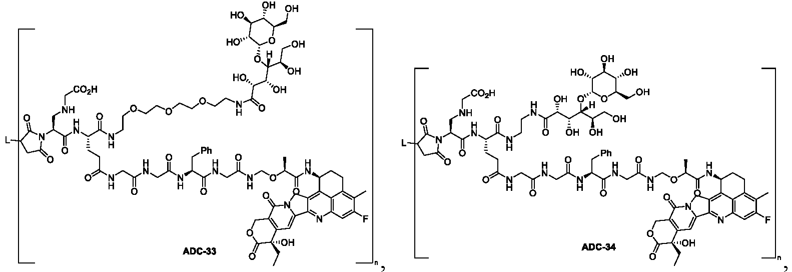 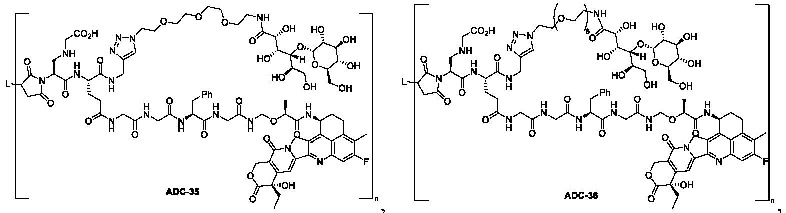 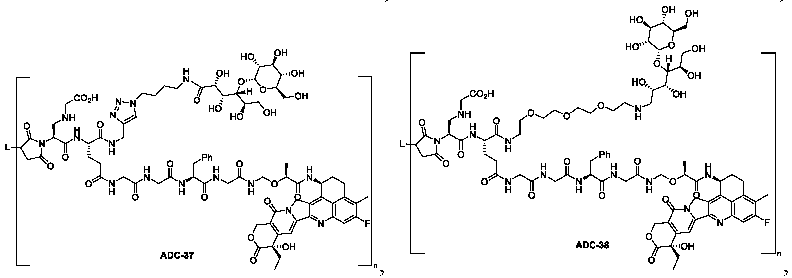 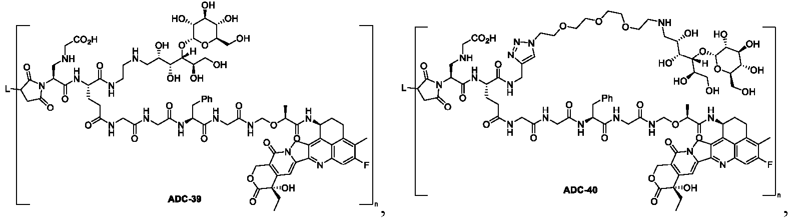 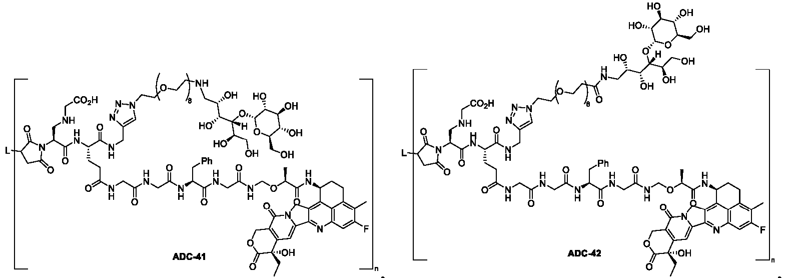 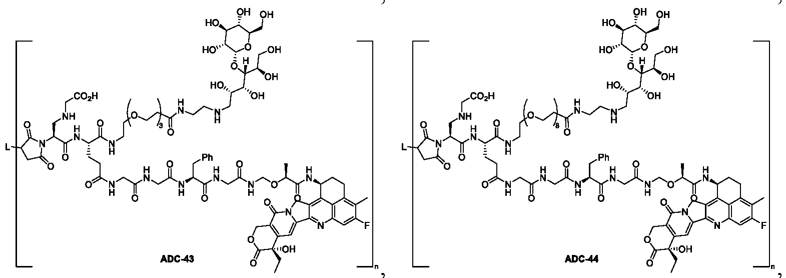 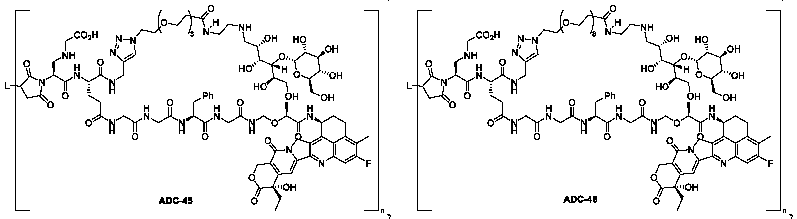 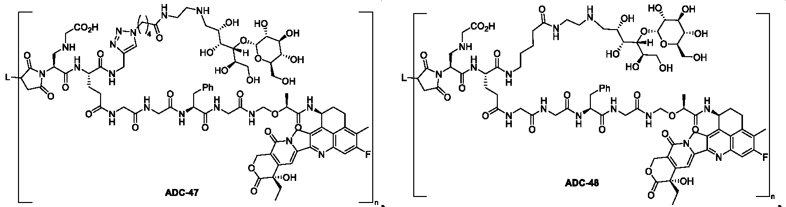 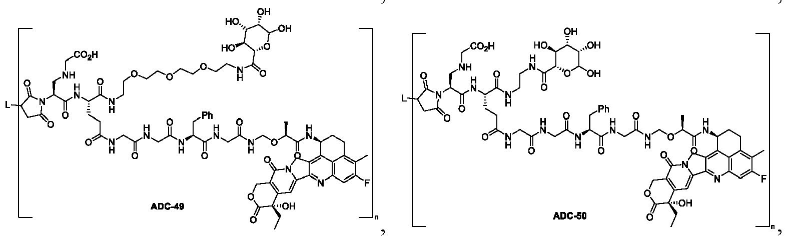 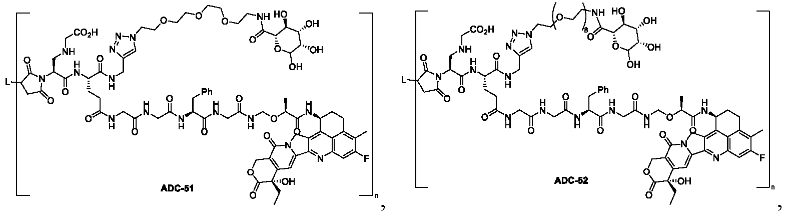 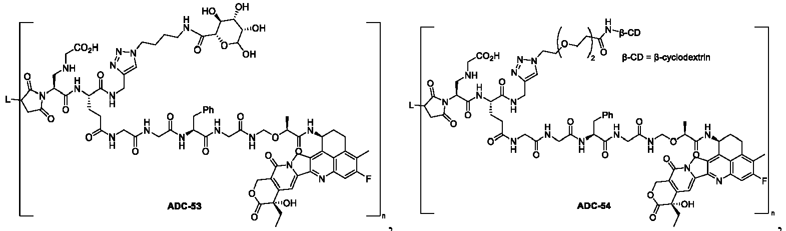 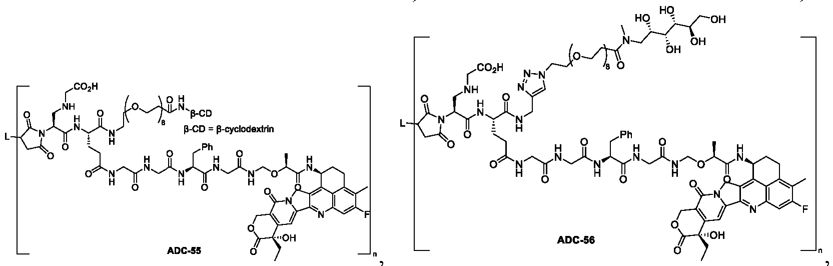 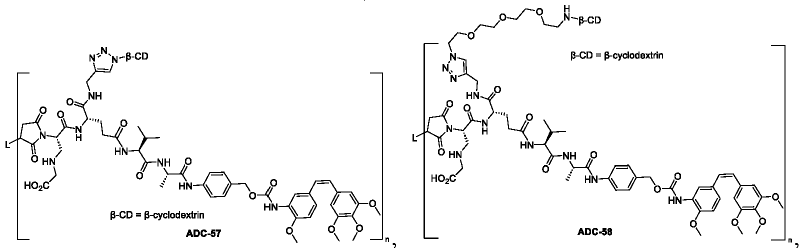 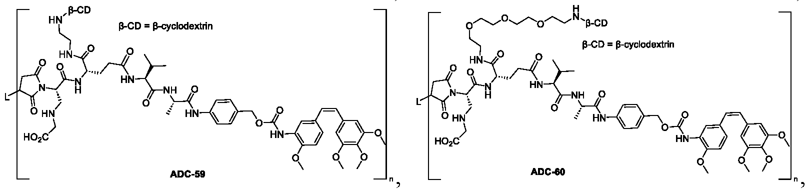 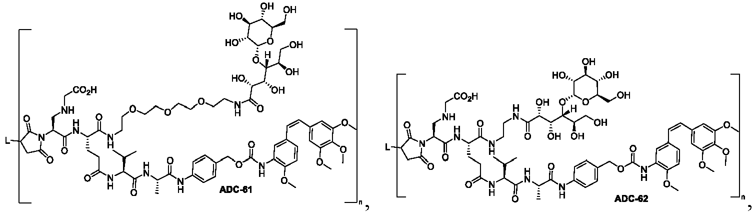 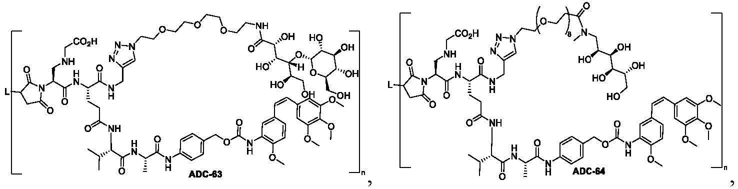 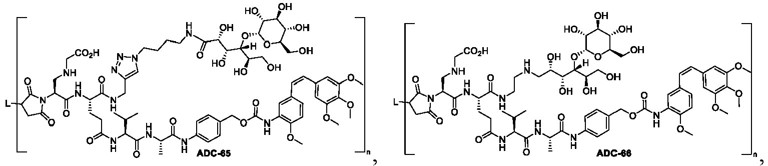 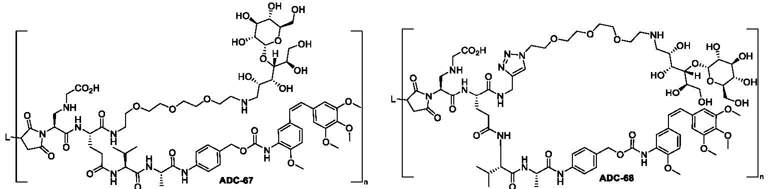 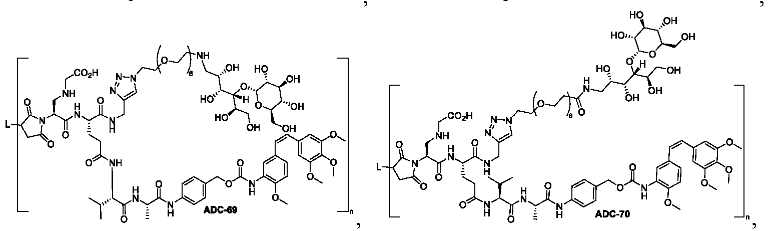 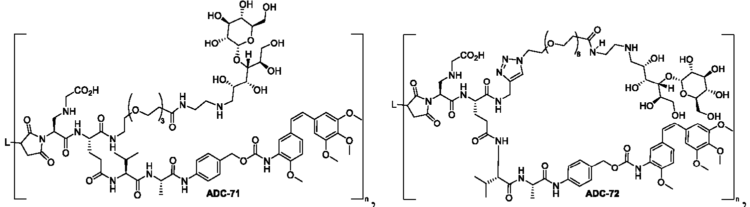 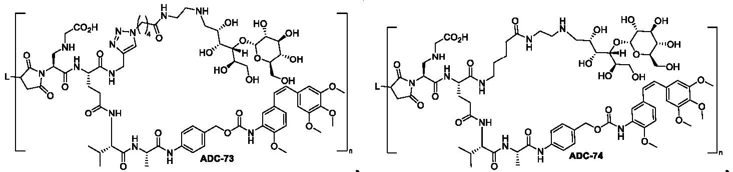 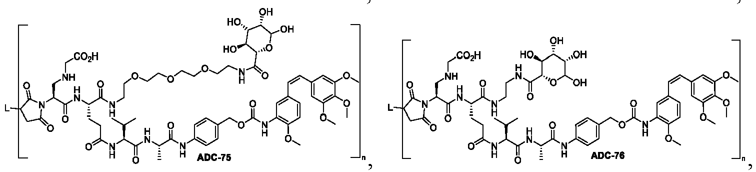 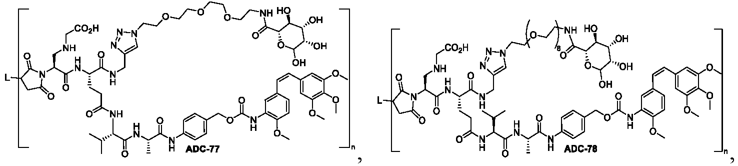 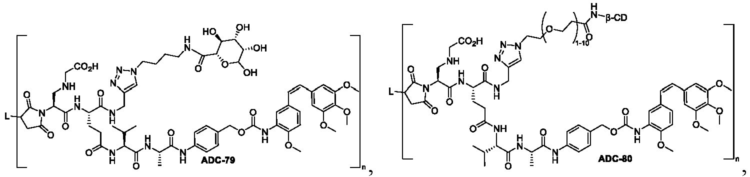 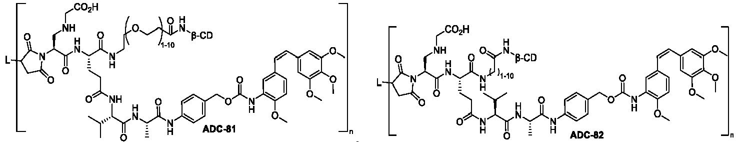 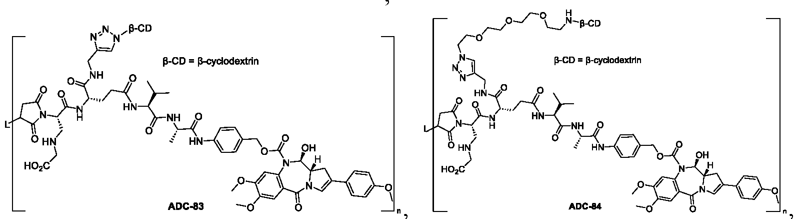 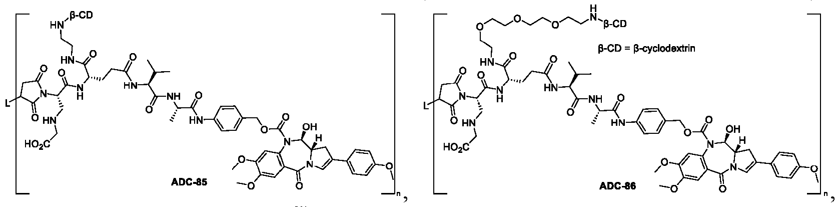 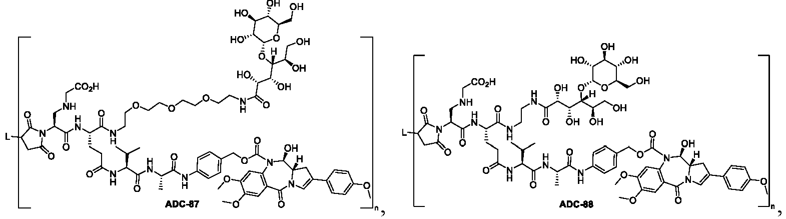 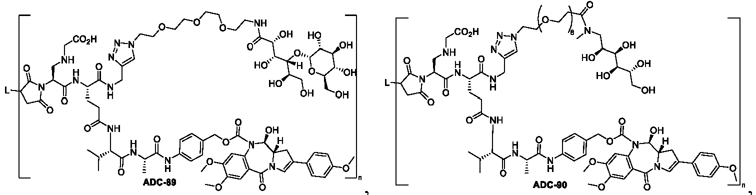 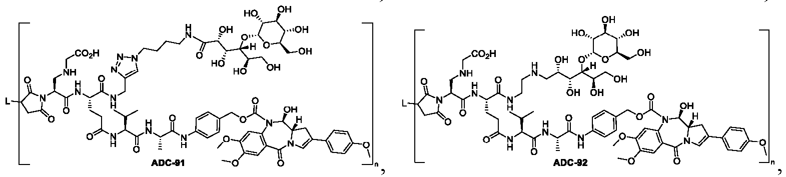 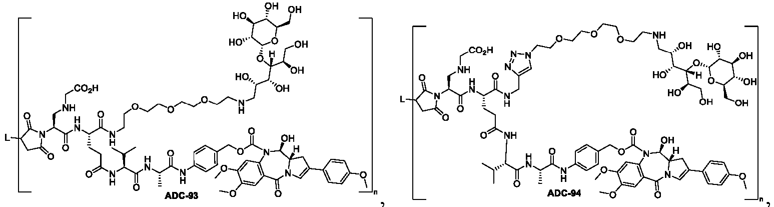 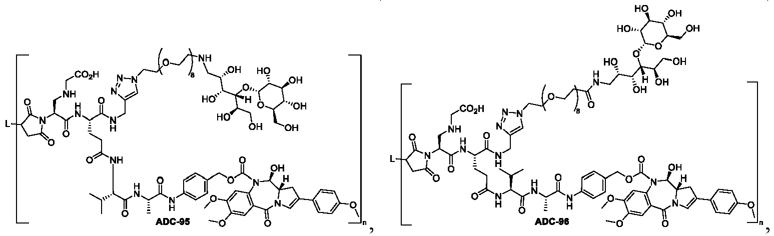 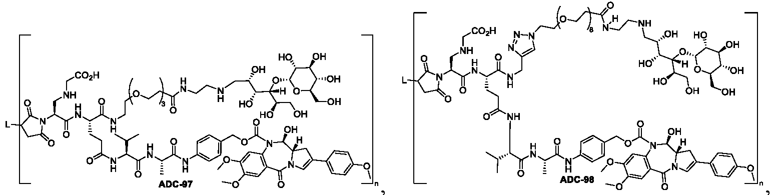 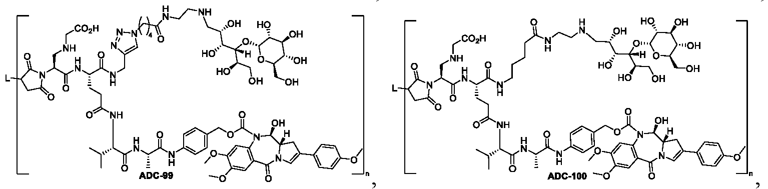 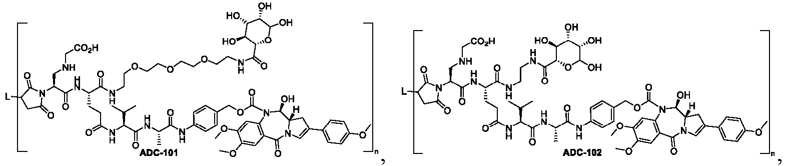 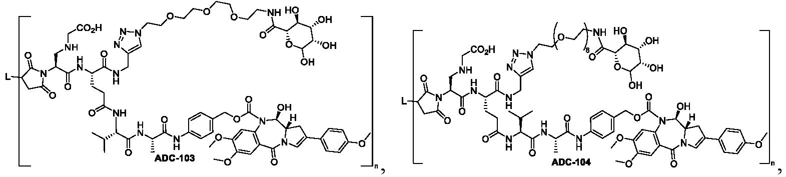 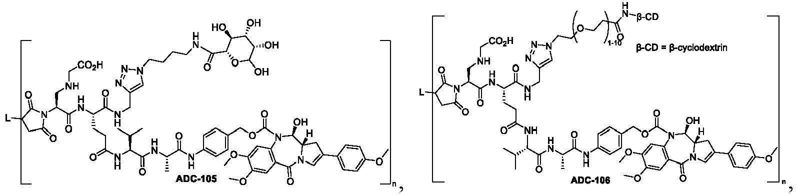 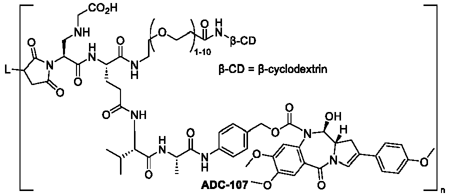 and 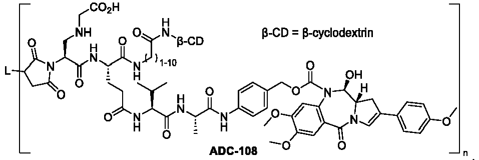 wherein
L is selected from ligands, preferably antibodies;
n is selected from integers of 1 to 10, preferably 2 to 8.
In some embodiments, the pharmaceutically acceptable salt is selected from:
a sodium salt, potassium salt, calcium salt or magnesium salt formed by an acidic functional group in the structural formula and an alkali; and
an acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methanesulfonate, ethanesulfonate, benzenesulfonate or p-toluenesulfonate formed by a basic functional group in the structure and an acid.

In some embodiments, the ligand is selected from monoclonal antibodies and non-restrictively from: an anti-EGFRvIII antibody, an anti-DLL-3 antibody, an anti-PSMA antibody, an anti-CD70 antibody, an anti-MUC16 antibody, an anti-ENPP3 antibody, an anti-TDGF1 antibody, an anti-ETBR antibody, an anti-MSLN antibody, an anti-TIM-1 antibody, an anti-LRRC15 antibody, an anti-LIV-1 antibody, an anti-CanAg/AFP antibody, an anti-cladin18.2 antibody, an anti-Mesothelin antibody, an anti-HER2 (ErbB2) antibody, an anti-EGFR antibody, an anti-C-Met antibody, an anti-SLITRK6 antibody, an anti-KIT/CD117 antibody, an anti-STEAP1 antibody, an anti-SLAMF7/CS1 antibody, an anti-NaPi2B/SLC34A2 antibody, an anti-GPNMB antibody, anti-HER3 (ErbB3) antibody, anti-MUC1/CD227 antibody, an anti-AXL antibody, an anti-CD166 antibody, an anti-B7-H3 (CD276) antibody, an anti-PTK7/CCK4 antibody, an anti-PRLR antibody, an anti-EFNA4 antibody, an anti-5T4 antibody, an anti-NOTCH3 antibody, an anti-Nectin 4 antibody, an anti-Trop-2 antibody, an anti-CD142 antibody, an anti-CA6 antibody, an anti-GPR20 antibody, an anti-CD174 antibody, an anti-CD71 antibody, an anti-EphA2 antibody, an anti-LYPD3 antibody, an anti-FGFR2 antibody, an anti-FGFR3 antibody, an anti-FRα antibody, an anti-CEACAMs antibody an anti-GCC antibody, an anti-Integrin Av antibody, an anti-CAIX antibody, an anti-P-cadherin antibody, an anti-GD3 antibody, an anti-Cadherin 6 antibody, an anti-LAMP1 antibody, an anti-FLT3 antibody, an anti-BCMA antibody, an anti-CD79b antibody, an anti-CD19 antibody, an anti-CD33 antibody, an anti-CD56 antibody Body, an anti-CD74 antibody, an anti-CD22 antibody, an anti-CD30 antibody, an anti-CD37 antibody, an anti-CD138 antibody, an anti-CD352 antibody, an anti-CD25 antibody, and an anti-CD123 antibody;

In some embodiments, the ligand is an anti-Trop-2 antibody. For instance, the light chain and heavy chain sequences of the anti-Trop-2 antibody are shown in SEQ ID NO: 1 and SEQ ID NO: 2, respectively.

In some embodiments, the ligand is an anti-CD33 antibody. For instance, the light chain and heavy chain sequences of the anti-CD33 antibody are shown in SEQ ID NO: 3 and SEQ ID NO: 4, respectively.

The present application further provides a pharmaceutical composition, containing a therapeutically effective amount of the ligand-drug conjugate of the present invention or the pharmaceutically acceptable salt of the ligand-drug conjugate or the linker-drug compound of the present invention or the pharmaceutically acceptable salt or solvate of the linker-drug compound, and a pharmaceutically acceptable carrier, diluent or excipient.

The present application further provides the use of the ligand-drug conjugate of the present invention or the pharmaceutically acceptable salt of the ligand-drug conjugate or the linker-drug compound of the present invention or the pharmaceutically acceptable salt or solvate of the linker-drug compound in the preparation of a drug for treating or preventing a tumor or an autoimmune disease.

In some embodiments, the tumor is selected from solid tumors or non-solid tumors, such as breast cancer, ovarian cancer, cervical cancer, uterine cancer, prostate cancer, kidney cancer, urethral cancer, bladder cancer, liver cancer, stomach cancer, endometrial cancer, salivary gland cancer, esophageal cancer, lung cancer, colon cancer, rectal cancer, colorectal cancer, bone cancer, skin cancer (such as epidermal cell carcinoma of the skin (also known as squamous cell carcinoma of the skin)), thyroid cancer, pancreatic cancer, melanoma, glioma, neuroblastoma, glioblastoma multiforme, sarcoma, lymphoma and leukemia (e.g., acute leukemia and chronic leukemia, such as acute lymphoblastic leukemia (ALL), acute myeloid leukemia (AML), chronic myeloid leukemia (CML), chronic lymphocytic leukemia (CLL), hairy cell leukemia, and prolymphocytic leukemia).

### Definitions

Unless otherwise defined, all technical and scientific terms used herein are consistent with the common understanding of those of ordinary skill in the art to which the invention belongs. Although any methods and materials similar or equivalent to those described herein may be used to practice or test the invention, the invention describes preferred methods and materials. In describing and claiming the invention, the following terms are used in accordance with the definitions below.

When a trade name is used herein, the applicants intend to include formulations of a product of the trade name, generic drugs and active drug portions of the trade name.

Unless otherwise stated, the terms used in the specification and claims have the following meanings.

The term "ligand" is a targeting agent that specifically binds to a target moiety. The ligand is capable of specifically binding to a cellular component or to other target molecules of interest. The targeting moiety or target is usually on the cell surface. In some aspects, the ligand functions to deliver the drug unit to a specific target cell population with which the ligand unit interacts. The ligands include, but are not limited to, proteins, polypeptides and peptides, as well as non-proteins such as sugars. Suitable ligand units include, for example, antibodies, such as full-length (intact) antibodies and antigen-binding fragments thereof. In embodiments where the ligand unit is a non-antibody targeting agent, it may be a peptide or polypeptide, or a non-protein molecule. Examples of such targeting agents include interferons, lymphokines, hormones, growth factors and colony-stimulating factors, vitamins, nutrient transport molecules, or any other cell-binding molecules or substances. In some embodiments, the linker is covalently attached to the sulfur atom of the ligand. In some aspects, the sulfur atom is that of a cysteine residue that forms an interchain disulfide bond of the antibody. In another aspect, the sulfur atom is a sulfur atom of a cysteine residue that has been introduced into the ligand unit, and the sulfur atom forms an interchain disulfide bond of the antibody. In another aspect, the sulfur atom is a sulfur atom of a cysteine residue introduced into the ligand unit by, for example, site-directed mutagenesis or chemical reaction.

The term "drug" refers to a cytotoxic drug, i.e., a molecule that has a strong ability to destroy the normal growth of tumor or cancer cells. In principle, cytotoxic drugs can kill tumor cells at sufficiently high concentrations, but due to the lack of specificity, while killing tumor or cancer cells, they can also cause apoptosis of normal cells, thus easily leading to serious side effects.

The term "ligand-drug conjugate" refers to a molecule formed by connecting a ligand to a drug by a stable linking unit. In the present invention, the "ligand-drug conjugate" is preferably an antibody-drug conjugate (ADC), which refers to connecting a monoclonal antibody or a functional antibody fragment or a targeted protein to a cytotoxic drug by a stable linking unit.

The term "antibody" or "functional antibody fragment" includes, within its scope, any part of the antibody structure. This unit can bind, reactively associate, or complex a receptor, antigen, or other receptor unit present in the target cell population. The antibody may be any protein or proteinaceous molecule that binds, complexes, or reacts with a portion of the cell population to be treated or biomodified.

The antibodies of the present invention include but are not limited to mouse antibodies, chimeric antibodies, humanized antibodies and fully human antibodies, preferably humanized antibodies and fully human antibodies.

The term "linker unit" refers to a chemical structure fragment or bond that is connected to a ligand at one end and directly connected to a drug at the other end or connected to other linkers and then connected to a drug. In the general formulas I to VI of the present application, the linker unit is represented by M.

The term "linking scaffold" refers to a chemical structure fragment or bond with a branched structure that can be connected to a linker unit, a ligand, a drug or a hydrophilic unit, etc., and the linking scaffold is represented by A. The connection manner in the present invention is preferably covalent connection.

The term "linking unit" refers to a chemical structure fragment or bond that can connect different components in an antibody-drug conjugate or a linker-drug compound such as a branching unit, a linking structure, a drug or a hydrophilic unit. According to the mechanism of drug release in cells, the linking units can be divided into two categories: non-cleavable linkers and cleavable linkers, and the linking units are represented by L, L^{a}, and L^{b}.

For ligand-drug conjugates containing non-cleavable linkers, the drug release mechanism is as follows: after the conjugate binds to the antigen and is endocytosed by cells, the antibody is enzymatically hydrolyzed in lysosomes, releasing active molecules composed of small molecule drugs, linkers, and antibody amino acid residues. The resulting change in the structure of the drug molecule does not weaken its cytotoxicity, but because the active molecule is charged (amino acid residue), it cannot penetrate into neighboring cells. Therefore, such active drugs cannot kill adjacent tumor cells (bystander effect) that do not express the target antigen (antigen-negative cells) (Ducry et al., 2010, Bioconjugate Chem. 21: 5-13).

Cleavable linkers, as the name implies, can be cleaved within the target cell and release the active drug (the small molecule drug itself). The cleavable linkers can be divided into two main categories: Chemically labile linkers and enzyme labile linkers.

The chemically labile linkers can be selectively cleaved due to differences in the properties of plasma and cytoplasm. Such properties include pH and glutathione concentration.

pH-sensitive linkers are often called acid-cleavable linkers. Such linkers are relatively stable in the neutral environment of blood (pH 7.3-7.5), but will be hydrolyzed in the weakly acidic endosomes (pH 5.0-6.5) and lysosomes (pH 4.5-5.0). Most of the first generation of antibody-drug conjugates used this type of linker, such as hydrazones, carbonates, acetals, and ketals. Due to the limited plasma stability of acid-cleaved linkers, antibody-drug conjugates based on this type of linker usually have a short half-life (2-3 days). This short half-life limits the application of pH-sensitive linkers in new generation antibody-drug conjugates to a certain extent.

Glutathione-sensitive linkers are also called disulfide linkers. Drug release is caused by the difference between high intracellular glutathione concentration (millimolar range) and relatively low glutathione concentration in the blood (micromolar range). This is especially true for tumor cells, where low oxygen content leads to increased activity of reductase enzymes and thus higher glutathione concentration. Disulfide bonds are thermodynamically stable and therefore have good stability in plasma.

Enzyme-labile linkers, such as peptide linkers, enable better control of drug release. Peptide linkers can be effectively cleaved by lysosomal proteases, such as cathepsin B or plasmin (increased levels of such enzymes in some tumor tissues). Such as peptide linkers are believed to be very stable in the plasma circulation because proteases are usually inactive due to inappropriate extracellular pH and serum protease inhibitors. Enzyme-labile linkers are widely used as cleavable linkers for antibody-drug conjugates due to their high plasma stability and good intracellular cleavage selectivity and effectiveness. Typical enzyme-labile linkers include Val-Cit (VC), Phe-Lys and the like.

A suicide linker may be embedded between the cleavable linker and the active drug, or the suicide linker itself is part of the cleavable linker. The mechanism of action of the suicide linker is as follows: When the cleavable linker is cleaved under appropriate conditions, the suicide linker can spontaneously rearrange its structure and release the active drug connected to it. Common suicide linkers include p-aminobenzyl alcohol (PAB) and *β*-glucuronide.

The term "branching unit" or "linking structure" refers to a chemical structure fragment with a branched structure or bond that connects different components in the ligand-drug conjugate. The connection manner in the present invention is preferably covalent connection.

The term "sugar" is a polyhydroxy aldehyde or ketone compound and can be converted into one of the polyhydroxy aldehyde and ketone compounds after hydrolysis. The sugar can be divided into monosaccharides, disaccharides and polysaccharides.

The three-letter and single-letter codes for amino acids used in the present invention are as described in J. boil. Chem. 1968, 243, 3558.

The term "natural amino acid" refers to amino acids that can be synthesized by organisms. Natural amino acids are generally L-type, but there are a few exceptions, such as glycine, including natural and biosynthesized glycine.

The term "unnatural amino acid" refers to amino acids that can only be synthesized artificially.

The term "alkyl" refers to a saturated aliphatic hydrocarbon group, which is a linear or branched chain group containing 1 to 20 carbon atoms, preferably an alkyl containing 1 to 12 carbon atoms, more preferably an alkyl containing 1 to 10 carbon atoms, most preferably an alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl,n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 (e.g. 1 to 4) carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "substituted alkyl" means that the hydrogen in an alkyl is replaced by a substituent. Unless otherwise specified in the text, the substituent of the alkyl may be one or more groups selected from the group consisting of: -halogen, -OR', -NR'R", - SR', -SiR'R"R"', -OC(O)R', -C(O)R', -CO₂R', -CONR'R", -OC(O)NR'R", -NR"C(O)R', -NR'-C(O)NR' 'R‴, -NR"C(O)₂R', -NH-C(NH₂)=NH, -NR'C(NH₂)=NH, -NH-C(NH₂)=NR', -S(O)R', -S(O)₂R', -S(O)₂NR'R", -NR'S(O)₂R", -CN and -NO₂, the number of substituents is 1 to (2m'+1), where m' is the total number of carbon atoms in the group, such as 1, 2, 3, 4, 5 or 6. R', R" and R‴ respectively refer to hydrogen, C₁₋₈ alkyl, aryl, aryl substituted by 1-3 halogens, C₁₋₈ alkyl substituted by 1-3 halogens, C₁₋₈ alkoxy or C₁₋₈ thioalkoxy, or unsubstituted aryl-C₁₋₄ alkyl. When R' and R" are attached to the same nitrogen atom, they can form a 3-, 4-, 5-, 6- or 7-membered ring together with the nitrogen atom. For example, -NR'R" includes 1-pyrrolidinyl and 4-morpholinyl.

The term "cycloalkyl" refers to a saturated or partially unsaturated monocyclic or polycyclic ring-shaped hydrocarbon group, and the ring of the cycloalkyl contains 3 to 20 carbon atoms, preferably 3 to 12 carbon atoms, more preferably 3 to 10 carbon atoms, and most preferably 3 to 8 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, and cyclooctyl. Non-limiting examples of polycyclic cycloalkyl include spiro, fused and bridged cycloalkyl groups.

The term "cycloalkylalkyl" means that an alkyl is substituted by one or more cycloalkyl groups, preferably by one cycloalkyl group, wherein the alkyl is as defined above and the cycloalkyl is as defined above. The ring of the cycloalkyl preferably contains 3 to 12 carbon atoms, more preferably 3 to 10 carbon atoms, and most preferably 3 to 8 carbon atoms. The alkyl is preferably a lower alkyl having 1 to 6 carbon atoms, more preferably 1 to 4 carbon atoms.

The term "alkoxy" refers to an -O-(alkyl) and an -O-(cycloalkyl) group, wherein the alkyl and cycloalkyl are as defined above. Non-limiting examples of alkoxy include: methoxy, ethoxy, propoxy, butoxy, cyclopropyloxy, cyclobutyloxy, cyclopentyloxy, and cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heterocyclyl" refers to a saturated or partially unsaturated monocyclic or polycyclic ring-shaped hydrocarbon group containing 3 to 20 ring atoms, of which one or more (such as 1, 2, 3 or 4) ring atoms are heteroatoms selected from nitrogen, oxygen or S(O)ₘ (wherein m is 0, 1 or 2), and the remaining ring atoms are carbon. Preferably, the heterocyclyl contains 3 to 12 ring atoms, of which 1 to 4 are heteroatoms. More preferably, the heterocyclyl contains 3 to 10 ring atoms. Non-limiting examples of monocyclic heterocyclyl include pyrrolidinyl, piperidinyl, piperazinyl, morpholinyl, thiomorpholinyl, and homopiperazinyl. Non-limiting examples of polycyclic heterocyclyl include spiro, fused and bridged heterocyclyls.

The term "aryl" refers to a 6- to 14-membered all-carbon monocyclic or fused polycyclic (i.e., rings that shares adjacent pairs of carbon atoms) group having a conjugated *π* electron system, preferably 6- to 10-membered group, such as phenyl. The aryl may be substituted or unsubstituted. When the aryl is substituted, the substituent is preferably one or more of the following groups, selected from, but not limited to, alkyl, alkenyl, alkynyl, alkoxy, alkylthio, alkylamino, halogen, deuterium atom, mercapto, hydroxyl, nitro, cyano, cycloalkyl, heterocycloalkyl, aryl, heteroaryl, cycloalkyloxy, heterocycloalkyloxy, cycloalkylthio, and heterocycloalkylthio.

The term "heteroaryl" includes 5- to 8-membered monocyclic heteroaryls and 8- to 12-membered fused heteroaryls.

The term "5- to 8-membered monocyclic heteroaryl" refers to an aromatic monocyclic ring group containing 5 to 8 ring atoms (at least one of which is a heteroatom, such as a nitrogen atom, an oxygen atom or a sulfur atom). Optionally, the ring atoms (such as carbon atoms, nitrogen atoms or sulfur atoms) in the ring structure may be oxo-substituted. "5- to 8-membered monocyclic heteroaryl" includes, such as, "5- to 7-membered monocyclic heteroaryl", "5- to 6-membered monocyclic heteroaryl", "5- to 6-membered monocyclic nitrogen-containing heteroaryl", and "6-membered monocyclic nitrogen-containing heteroaryl", wherein the heteroatom in the "nitrogen-containing heteroaryl" contains at least one nitrogen atom, for example, only 1 or 2 nitrogen atoms, or, contains one nitrogen atom and other 1 or 2 heteroatoms (for example, oxygen atoms and/or sulfur atoms), or, contains 2 nitrogen atoms and other 1 or 2 heteroatoms (for example, oxygen atoms and/or sulfur atoms). Specific examples of "5- to 8-membered monocyclic heteroaryl" include but are not limited to furyl, thienyl, pyrrolyl, thiazolyl, isothiazolyl, thiadiazolyl, oxazolyl, isoxazolyl, oxadiazolyl, imidazolyl, pyrazolyl, 1,2,3-triazole base,1,2,4-triazolyl,1,2,3-oxadiazolyl,1,2,4-oxadiazolyl,1,2,5-oxadiazolyl,1,3,4 -oxadiazolyl, pyridyl, 2-pyridonyl, 4-pyridonyl, pyrimidinyl, pyridazinyl, pyrazinyl, 1,2,3-triazinyl, 1,3,5-triazine base, 1,2,4,5-tetrazinyl, azepantrienyl, 1,3-diazacycloheptatrienyl, azepantetraenyl, etc.

The term "8- to12-membered fused heteroaryl" refers to an unsaturated aromatic ring structure containing 8 to 12 ring atoms (at least one of which is a heteroatom, such as a nitrogen atom, an oxygen atom or a sulfur atom), which is formed by two or more ring structures sharing two adjacent atoms. Optionally, the ring atoms (such as carbon atoms, nitrogen atoms or sulfur atoms) in the ring structure may be oxo-substituted. "8- to 12-membered fused heteroaryl" includes "8- to 10-membered fused heteroaryl" and "8- to 9-membered fused heteroaryl"; specific examples include but are not limited to: pyrrolopyrrole, pyrrolofuran, pyrazopyrrole, pyrazothiophene, furothiophene, pyrazoxazole, benzofuryl, benzisofuryl, benzothienyl, indolyl, isoindolyl, benzoxazolyl, benzimidazolyl, indazolyl, benzotriazolyl, quinolinyl, 2-quinolinonyl, 4-quinolinonyl, 1-isoquinolinonyl, Isoquinolinyl, acridinyl, phenanthridinyl, benzopyridazinyl, phthalazinyl, quinazolinyl, quinoxalinyl, purinyl, and naphthyridinyl.

The term "haloalkyl" refers to an alkyl substituted by one or more halogens, where alkyl is as defined above.

The term "deuterated alkyl" refers to an alkyl substituted by one or more deuterium atoms, where alkyl is as defined above.

The term "hydroxy" refers to an -OH group.

The term "halogen" refers to fluorine, chlorine, bromine or iodine.

The term "amino" refers to a -NH₂ group.

The term "nitro" refers to a -NO₂ group.

The term "derivative" refers to a substance that has a chemical structure similar to that of a compound but also contains at least one chemical group that is not present in the compound and/or lacks at least one chemical group that is present in the compound. The compound to which the derivative is compared is called a "parent" compound. Typically, a "derivative" may be produced from a parent compound in one or more chemical reaction steps.

The term "pharmaceutically acceptable salt" refers to a pharmaceutically acceptable organic or inorganic salt of a compound (such as, a drug, a linker-drug compound, or a ligand-drug conjugate). The compound or conjugate may contain at least one amino or carboxyl group and can therefore form addition salts with the corresponding acid or alkali. Exemplary salts include, but are not limited to sulfate, trifluoroacetate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, salicylate, formate, benzoate, glutamate, methanesulfonate, ethanesulfonate, benzenesulfonate, p-toluenesulfonate, potassium salt, and sodium salt.

The term "solvate" refers to a compound formed by a linker-drug compound or ligand-drug conjugate of the present invention and one or more solvent molecules, where the solvent molecules include but are not limited to water, ethanol, acetonitrile, isopropanol, DMSO, and ethyl acetate.

The term "pharmaceutical composition" refers to a mixture containing one or more compounds of the present invention or physiologically/pharmaceutically acceptable salts or prodrugs thereof and other chemical components, as well as other components such as physiologically/pharmaceutically acceptable carriers and/or excipients. The purpose of the pharmaceutical composition is to promote the administration of drugs to organisms, facilitate the absorption of active ingredients and thus exert biological activity.

The term "carrier" refers to a system that can change the way drugs enter the human body and their distribution in the body, control the release rate of drugs and deliver drugs to the target. Drug carrier release and targeting systems can reduce drug degradation and loss, reduce side effects and improve bioavailability.

The term "excipient" refers to an additive or excipient other than the main drug in a pharmaceutical preparation. Such as adhesives, fillers, disintegrants, lubricants in tablets; matrix backup in semi-solid preparations ointments and creams; preservatives, antioxidants, flavoring agents, fragrances, cosolvents, emulsifiers, permeability enhancers, osmotic pressure regulators, colorants, etc. in liquid preparations can all be called excipients.

The term "diluent" or "filler" is mainly used to increase the weight and/or volume of the preparation. The addition of diluent not only ensures a certain volume, but also reduces the dosage deviation of the main ingredients and improves the compression molding of the drug.

In one embodiment of the present invention, the linker-drug compound is connected to the ligand via a thiol group generated by an opened interchain disulfide bond or a cysteine thiol group introduced by genetic engineering. Generally, the number of drug molecules that can be connected to the ligand in the conjugating reaction will be less than or equal to the theoretical maximum value.

The drug loading of the ligand-drug conjugate may be controlled by the following non-limiting methods, including:
1) controlling a molar ratio of a linking agent to the ligand,
2) controlling reaction time and temperature, and
3) selecting different reaction reagents.

The present invention is further described below in conjunction with the accompanying drawings and specific embodiments. It should be understood that these embodiments are only used to illustrate the invention and are not used to limit the scope of the invention. The experimental methods in the following embodiments that do not specify specific conditions are usually carried out under conventional conditions or under conditions recommended by the manufacturers. Unless otherwise specified, all percentages, proportions, ratios, or parts are calculated by weight.

### BRIEF DESCRIPTION OF THE DRAWINGS

FIG. 1A is a SEC-HPLC spectrum of ADC-29, showing the aggregation of ADC-29.
FIG. 1B is a SEC-HPLC spectrum of ADC-56, showing the aggregation of ADC-56.
FIG. 1C is a SEC-HPLC spectrum of ADC-111, showing the aggregation of ADC-111.
FIG. 2A is an RP-HPLC spectrum of ADC-29, showing the drug to antibody ratio (DAR) of ADC-29.
FIG. 2B is an RP-HPLC spectrum of ADC-56, showing the DAR of ADC-56.
FIG. 2C is an RP-HPLC spectrum of ADC-111, showing the DAR of ADC-111.
FIG. 3A shows the in vitro efficacy of ADC-1, ADC-28, ADC-29, ADC-42, ADC-56, ADC-109, and ADC-111 in A431 cell line.
FIG. 3B shows the in vitro efficacy of ADC-1, ADC-28, ADC-29, ADC-42, ADC-56, ADC-109, and ADC-111 in HL-60 cell line.
FIG. 3C shows the in vitro efficacy of ADC-1, ADC-28, ADC-29, ADC-42, ADC-56, ADC-109, and ADC-111 in TF-1 cell line.
FIG. 3D shows the in vitro efficacy of ADC-1, ADC-28, ADC-29, ADC-42, ADC-56, ADC-109, and ADC-111 in HEL92.1.7 cell line.
FIG. 3E shows the in vitro efficacy of ADC-1, ADC-28, ADC-29, ADC-42, ADC-56, ADC-109, and ADC-111 in MV4-11 cell line.
FIG. 3F shows the in vitro efficacy of ADC-1, ADC-28, ADC-29, ADC-42, ADC-56, ADC-109, and ADC-111 in MOLM-13 cell line.

### DETAILED DESCRIPTION OF EMBODIMENTS

### Example 1: Synthesis of compound 6

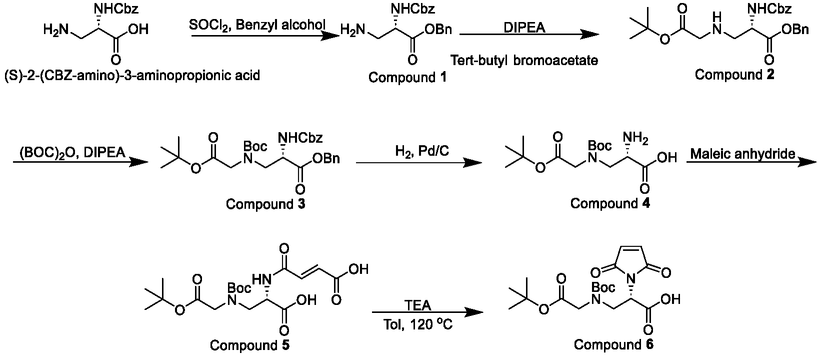

400 mL of benzyl alcohol was added into a 1 L single-neck flask, then SOCl₂ (13.38 mL, 184.8 mmol) was slowly added dropwise under the cooling of a water bath, and the reaction was allowed at the water bath temperature for 1 h. Compound (S)-2-(CBZ-amino)-3-aminopropionic acid (40 g, 168 mmol) was added in three batches, and the resulting reaction solution reacted overnight at room temperature. After the reaction was completed, reduced-pressure evaporation was performed using an oil pump to remove the unreacted benzyl alcohol, and the resulting brown residue was purified by silica gel column chromatography (DCM: MeOH =150:1) to obtain 44 g of compound 1. LC-MS m/z(M+H)⁺:329.1.

Compound 1 (40 g, 121.6 mmol) was dissolved in 600 mL of acetonitrile, followed by addition of DIPEA (18.88 mL, 146.32 mmol) under the cooling of an ice water bath. Tert-butyl bromoacetate (23.9 g, 120 mmol) was added dropwise in the condition of the low temperature, and the resulting reaction solution was stirred for 30 min, then transferred to room temperature for a reaction. After the reaction was found to be completed from the monitoring of TLC (DCM: MeOH=10:1), the reaction solution was concentrated under pressure in a water bath, and the resulting residue was purified by silica gel column chromatography (dichloromethane: methanol=100:1) to obtain 45.5 g of compound 2. LC-MS m/z(M+H)⁺:443.3.

In a 500 mL single-neck reaction flask, compound 2 (45 g, 25.2 mmol), 100 mL of 1,4-dioxane and 100 mL of water were mixed, followed by addition of DIPEA (25.2 m:L, 152.5 mmol). Boc anhydride (110.6 g, 508.7 mmol) was added dropwise at room temperature to obtain a yellow reaction solution, and the reaction was allowed at room temperature for about 3-4 h. After the reaction was found to be completed from the monitoring of TLC (DCM: MeOH=30: 1), the reaction solution was concentrated under reduced pressure in a water bath to remove dioxane, extracted with DCM (100 mL^{x}3). The obtained organic phase was dried with anhydrous sodium sulfate, filtered, and concentrated under reduced pressure to obtain a yellow oily crude product. The crude product was purified by silica gel column chromatography (DCM: MeOH=300: 1) to obtain 50.3 g of compound 3. LC-MS m/z(M+H)⁺:543.2.

50 g of compound 3 was dissolved in 250 mL of methanol, followed by addition of 10 g 5% Pd/C, the atmosphere in the system was replaced with hydrogen 2-3 times, and the reaction was allowed at room temperature. After the reaction was found to be completed from the monitoring of TLC (DCM: MeOH=5: 1), the reaction solution was filtered with two pieces of filter paper. The filter cake was rinsed three times with methanol, and the obtained filtrate was concentrated at 40 °C under reduced pressure to obtain compound 4, which was directly put into the next reaction without purification.
The crude compound 4 was dissolved in 250 mL of glacial acetic acid, maleic anhydride (18.2 g, 184.4 mmol) was added, and the reaction solution reacted with stirring at room temperature. After the reaction was found to be completed from the monitoring of TLC (DCM: MeOH=3:1), the reaction solution was directly concentrated under reduced pressure, and the residue was purified by preparative-grade high-performance liquid chromatography;
Column: YMC-C18, 100mm*450mm, 10µm, testing with acetonitrile/water (0.2% TFA), at a rate of 200 mL/min, λ=215 nm;
Solvent A: 0.2% TFA in water;
Solvent B: acetonitrile;
Gradient: 0-10 min 90% A, 10-25 min, 90% A-45% A, 25-55 min, 45% A-40% A;

The fraction with retention time of 43 min was collected and lyophilized to obtain 41 g of compound 5 as a white solid. LC-MS m/z (M+H)⁺:417.9.

In a 1 L single-neck flask, compound 5 (40 g, 96 mmol, 1.0 eq), triethylamine (26.7 mL, 2.0 eq) and toluene (400 mL) were mixed and then heated to 120 °C to reflux for 2 h. After the reaction was found to be completely from the monitoring of the TLC, the reaction system was cooled to 50 °C and rotary evaporation under reduced pressure was performed to remove the solvent. The reaction product was then dissolved in ethyl acetate (150 mL) and water (40 mL), and 1 M of HCl was then added to adjust the pH to 2-3 with stirring in an ice bath. The resulting solution was allowed for liquid separation; the aqueous layer was extracted once more with ethyl acetate; the organic layers were combined, and dried with anhydrous Na₂SO₄. After filtration, the filtrate was concentrated to obtain a pale yellow oily rude product. The rude product was purified by column chromatography (DCM: MeOH=40:1) to obtain 26.6 g of compound 6; LC-MS m/z (M+H)⁺:399.3.

### Example 2: Synthesis of compound 9

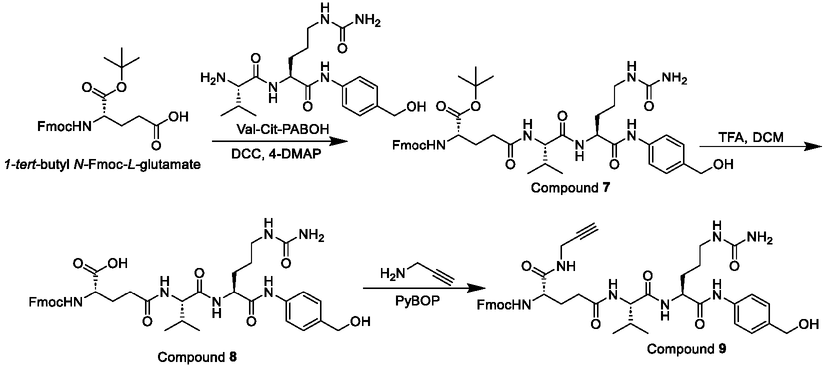

In a 1 L single-neck flask, 1-tert-butyl *N*-Fmoc-*L*-glutamate (21 g, 48.9 mmol), DCC (20.6 g, 97.8 mmol), Val-Cit-PABOH (18.5 g, 48.9 mmol) and dichloromethane (400 mL) were added, and the resulting solution was then cooled to 0 °C in condition of an ice water bath; 4-DMAP (610 mg, 4.89 mmol) was weighed and added into the reaction flask at one time, and the resulting solution was slowly warmed to room temperature to react for 3 h. After the reaction was found to be completed from the monitoring of TLC, the insoluble solid impurities was filtered out, the filter cake was rinsed three times with dichloromethane, rotary evaporation under reduced pressure was performed to remove the solvent, and the residual was concentrated to obtain a pale yellow oily rude product. The rude product was purified by column chromatography (DCM: MeOH=100:1-50:1) to obtain 33.2 g of compound 7; LC-MS m/z (M+H)⁺:787.5.

The above compound 7 was dissolved in 300 mL of dichloromethane, the reaction solution was then cooled to 0 °C in condition of an ice water bath, TFA (150 mL) was slowly added dropwise, and the resulting solution was slowly brought naturally to room temperature to react for 5 h. After the reaction was found to be completed from the monitoring of TLC (DCM: MeOH=10:1), the reaction solution was concentrated under reduced pressure at 45 °C to obtain a yellow oily rude of compound 8, which was directly put into the next reaction without purification.

In a 1 L single-neck flask, the above crude compound 8 (48.9 mmol based on the starting material charge), PyBOP (38.56 g, 73.4 mmol), propargylamine (3.3 g, 58.7 mmol) and dichloromethane (400 mL) were mixed and then cooled to 0 °C in condition of an ice water bath; DIPEA (2.6 mL, 14.7 mmol) was added dropwise into the reaction flask, and the reaction was allowed at room temperature overnight. After the reaction was found to be completed from the monitoring of TLC (20: 1), the reaction solution was diluted with saturated aqueous NaCl solution (100 mL), extracted twice with dichloromethane (100 mL); the organics were combined, dried with anhydrous Na₂SO₄, filtered with suction, and rotary evaporation under reduced pressure was performed to remove the solvent. The residual was concentrated to obtain a pale yellow oily rude product, and the rude product was purified by column chromatography (DCM: MeOH=50:1) to obtain 25.4 g of compound 9; LC-MS m/z (M+H)⁺:768.4.

### Example 3: Synthesis of compound 10

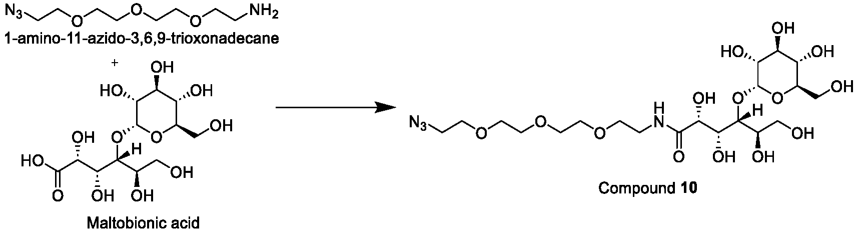

At room temperature, maltobionic acid (7.0 g, 19.5 mmol) was dissolved in 70 mL of anhydrous methanol, and the atmosphere in the system was replaced with N₂ three times. 4Å molecular sieve was added, and the reaction solution was heated to reflux overnight, then cooled to room temperature. 1-amino-11-azido-3,6,9-trioxaundecane was added and stirred at room temperature for 24 h. The reaction solution was directly concentrated under reduced-pressure, the obtained crude product was dissolved in 5 mL of methanol, followed by addition of 30 mL of chloroform and 100 mL of tetrahydrofuran in sequence. The resulting solution was stirred and stayed still to obtain a white solid, filtered, and the filter cake was rinsed twice with tetrahydrofuran. The obtained white solid was dissolved in 30 mL of water and lyophilized to obtain 5.6 g of white solid compound 10; LC-MS m/z (M+H)⁺:559.3.

### Example 4: Synthesis of compound 12

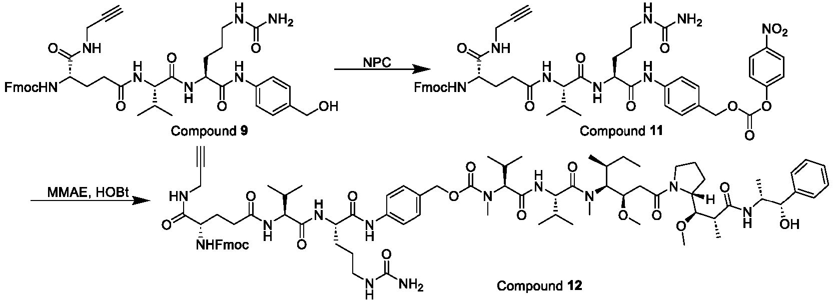

In a 250 mL single-neck flask, compound 9 (5.0 g, 6.5 mmol) and dichloromethane (100 mL) were added at room temperature, followed by addition of NPC (10.1 g, 32.6 mmol) and DIPEA (5.7 mL, 32.6 mmol) in sequence, and the reaction was allowed at room temperature overnight. After the reaction was found to be completed from the monitoring of TLC, the reaction solution was diluted with saturated aqueous NaCl solution (50 mL), and extracted twice with dichloromethane (100 mL). The organics were combined, dried with anhydrous Na₂SO₄ and filtered with suction. Rotary evaporation under reduced pressure was performed to remove the solvent, and the residual was concentrated to obtain a pale yellow oily rude product. The rude product was purified by column chromatography (DCM: MeOH=100:1-50:1) to obtain 5.3 g of compound 11; LC-MS m/z (M+H)⁺:933.5.

In a 50 mL single-neck flask, compound 11 (1.0 g, 1.1 mmol) and DMF (15 mL) were added at room temperature, followed by addition of MMAE (723 mg, 1 mmol), HOBt (304 mg, 2.2 mmol) and DIPEA (390 µL, 2.2 mmol) in sequence, and the reaction was allowed at room temperature overnight. After the reaction was completed, the reaction solution was directly concentrated under reduced pressure by an oil pump. The residue was purified by preparative-grade high-performance liquid chromatography to obtain 1.09 g of compound 12 as a white solid; TOF-MS m/z (M+Na)⁺:1534.0.

### Example 5: Synthesis of compound 15

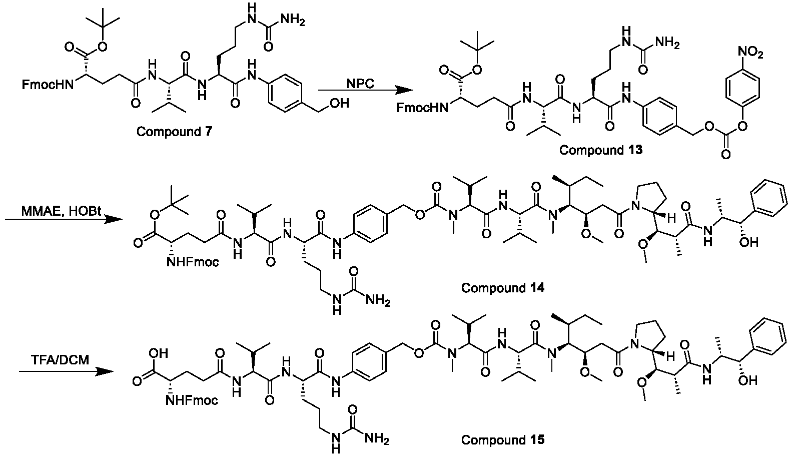

In a 250 mL single-neck flask, compound 7 (10.0 g, 12.7 mmol) and dichloromethane (130 mL) were added at room temperature, followed by addition of NPC (19.7 g, 63.5 mmol) and DIPEA (11.1 mL, 63.5 mmol) in sequence to have a reaction overnight at room temperature. After the reaction was found to be completed from the monitoring of TLC, the reaction solution was diluted with saturated aqueous NaCl solution (100 mL), and extracted three times with dichloromethane (100 mL). The organic phases were combined, dried with anhydrous Na₂SO₄, filtered with suction, and rotary evaporation under reduced pressure was performed to remove the solvent. The residual was concentrated to obtain a pale yellow oily rude product. The rude product was purified by column chromatography (DCM: MeOH=100:1-50:1) to obtain 10.5 g of compound 11; LC-MS m/z (M+H)⁺:952.3.

In a 50 mL single-neck flask, compound 13 (1.0 g, 1.05 mmol) and DMF (15 mL) were added at room temperature, then MMAE (759 mg, 1.05 mmol), HOBt (319 mg, 2.3 mmol) and DIPEA (408 µL, 2.3 mmol) were added in sequence, and the reaction was allowed at room temperature overnight. After the reaction was completed, the reaction solution was directly concentrated under reduced pressure by an oil pump, the residue was purified by preparative-grade high-performance liquid chromatography to obtain 1.22 g of compound 14 as a white solid; TOF-MS m/z (M+H)⁺:1530.9.

In a 25 mL single-neck flask, compound 14 (1 g, 0.653 mmol) and dichloromethane (5 mL) were added at room temperature, then followed by addition of trifluoroacetic acid (2.5 mL), and the reaction was allowed at room temperature for 6 h. After the reaction was completed, the reaction solution was concentrated under reduced pressure in a water bath, the residue was purified by preparative-grade high-performance liquid chromatography to obtain 0.76 g of compound 15 as a white solid; TOF-MS m/z (M+Na)⁺:1496.8.

### Example 6: Synthesis of compound P1-A

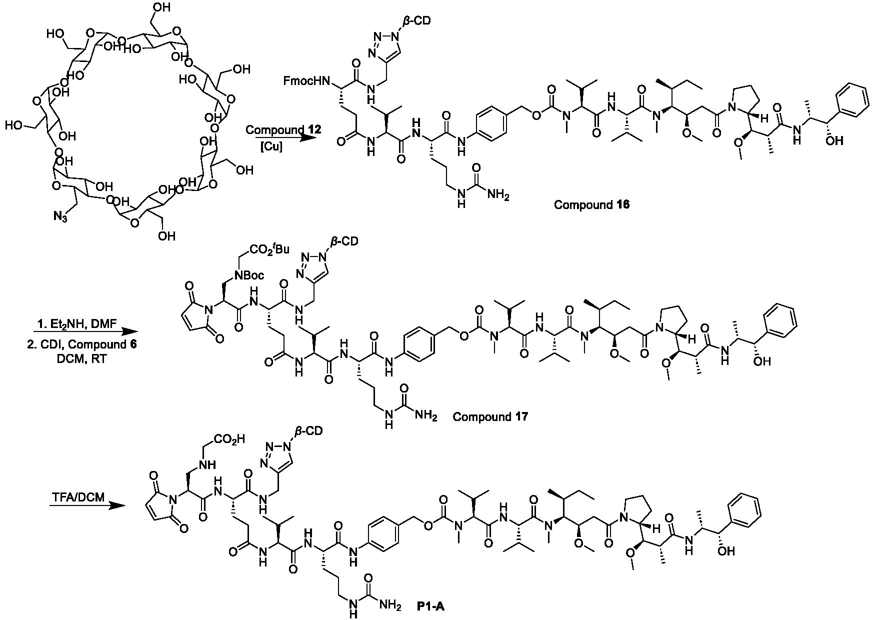

In a 25 mL single-neck flask, compound 12 (30 mg, 0.02 mmol) and tetrahydrofuran (5 mL) were added at room temperature, azide-*β*-cyclodextrin (24.2 mg, 0.02 mmol), cuprous iodide (2.0 mg, 0.1 mmol) and TEA (4 µL, 0.029 mmol) was added in sequence, and the reaction system was heated to 50 °C to react for 10 h. After the reaction was completed, the reaction solution was diluted with 10 mL of ethyl acetate, the organic phase was washed twice with 10 mL of saturated aqueous solution of ammonium chloride, the water phase was extracted three times with 10 mL of ethyl acetate, and the organic phases were combined, dried with anhydrous Na₂SO₄, and filtered. The filtrate was concentrated under reduced pressure, and the residue was purified by preparative-grade high-performance liquid chromatography to obtain 32 mg of compound 16 as a white solid; TOF-MS m/z (M+H)⁺:2671.4.

In a 5 mL single-neck flask, compound 16 (30 mg, 11.2 umol) and DMF (1 mL) were added at room temperature, followed by addition of diethylamine (12 µL, 0.12 mmol), and the reaction was allowed at room temperature for 12 h. DMF solvent was removed under reduced pressure by an oil pump, and the resulting crude yellow oil was directly put into the reaction without further purification.

In the above 5 mL single-neck flask, dichloromethane (1 mL) was added at room temperature, followed by addition of compound 6 (45 mg, 112 umol) and carbonyldiimidazole (CDI) (19.9 mg, 0.12 mmol) in sequence, and the reaction was allowed at room temperature for 10 h. The yellow reaction solution obtained was directly purified by preparative-grade high-performance liquid chromatography to obtain 28 mg of compound 17 as a slightly yellow solid; TOF-MS m/z (M+H)⁺:2829.3.

In a 5 mL single-neck flask, compound 17 (25 mg, 8.84 umol) and dichloromethane (4 mL) were added at room temperature, followed by addition of trifluoroacetic acid (2 mL), and the reaction was allowed at room temperature for 2 h. After the reaction was completed, the reaction solution was directly concentrated under reduced pressure by a water pump. The residue was purified by preparative-grade high-performance liquid chromatography to obtain 13 mg of compound P1-A as a white solid; TOF-MS m/z (M+Na)⁺:2695.3.

### Example 7: Synthesis of compound P1-B

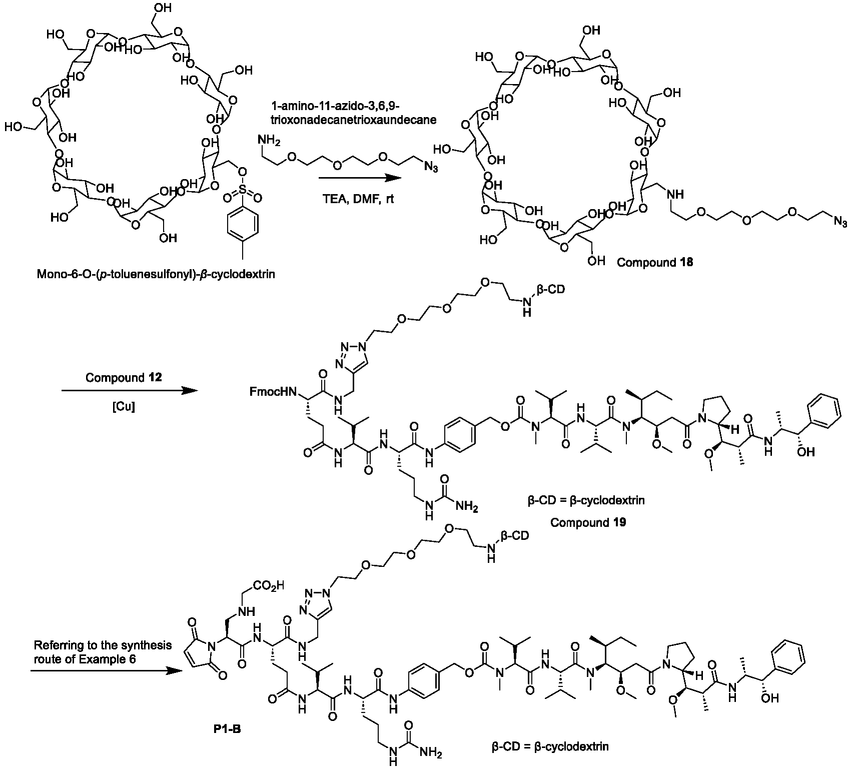

Compound mono-6-O-(*p*-toluenesulfonyl)-*β*-cyclodextrin (45 mg, 0.035 mmol), 1-amino-11-azido-3,6,9-trioxaundecane (9.0 mg, 0.04 mmol) and 5 mL of DMF were added into a 25 mL single-neck reaction flask, followed by addition of DIPEA (13 µL, 0.07 mmol), and the resulting yellow solution reacted at room temperature for about 3-4 h. The reaction solution was purified by preparative-grade high-performance liquid chromatography to obtain 42.3 mg of compound 18 as a white solid. LC-MS m/z(M+H)⁺:1335.6.

The subsequent reaction was implemented according to the synthesis route of Example 6 to prepare 9 mg of compound P1-B; TOF-MS m/z (M+Na)⁺:2870.5.

### Example 8: Synthesis of compound P1-C

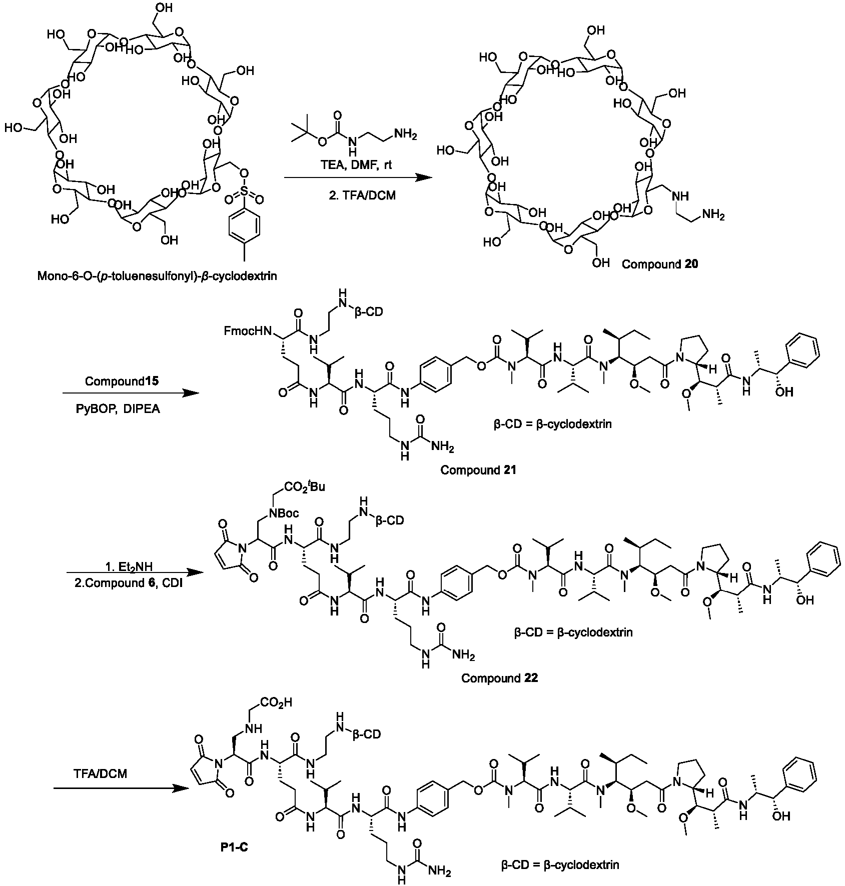

Compound mono-6-O-(*p*-toluenesulfonyl)-*β*-cyclodextrin (90 mg, 0.07 mmol), N-tert-butyloxycarbonyl-1,2-ethylenediamine (13.2 mg, 0.08 mmol) and 5 mL of DMF were added into a 25 mL single-neck reaction flask, followed by addition of DIPEA (26 µL, 0.14 mmol), and the resulting yellow solution reacted at room temperature for about 4 h. The reaction solution was purified by preparative-grade high-performance liquid chromatography to obtain 77 mg of compound 20 as a white solid; LC-MS m/z(M+H)⁺:1177.5.

In a 5 mL single-neck flask, compound 20 (70 mg, 0.06 mmol) and dichloromethane (4 mL) were added at room temperature, followed by addition of trifluoroacetic acid (2 mL), and the reaction was allowed at room temperature for 4 h. After the reaction was completed, rotatory evaporation under reduced-pressure was performed to remove trifluoroacetic acid and dichloromethane, and the residual was directly put into the next reaction.

In a 25 mL single-neck flask, the above crude compound (0.06 mmol based on the starting material charge), PyBOP (63.7 mg, 0.12 mmol), compound 15 (88 mg, 0.06 mmol) and DMF (5 mL) were added, and the resulting solution was then cooled to 0 °C in condition of an ice water bath. DIPEA (22 µL, 0.12 mmol) was added dropwise into a reaction flask, and the resulting reaction solution was slowly brought naturally to room temperature to have a reaction overnight. The yellow reaction solution obtained was directly purified by preparative-grade high-performance liquid chromatography to obtain 55 mg of compound 21; TOF-MS m/z (M+Na)⁺:2655.2.

In a 5 mL single-neck flask, compound 21 (50 mg, 19 umol) and DMF (1 mL) were added at room temperature, followed by addition of diethylamine (35 µL, 0.19 mmol), and reaction was allowed at room temperature overnight. DMF solvent was removed under reduced pressure by an oil pump, and the resulting crude yellow oil was directly put into the reaction without further purification.

In the above 5 mL single-neck flask, dichloromethane (1 mL) was added at room temperature, followed by addition of compound 6 (77 mg, 190 umol) and carbonyldiimidazole (CDI) (32 mg, 0.19 mmol) in sequence, and the reaction was allowed at room temperature for 10 h. The yellow reaction solution obtained was directly purified by preparative-grade high-performance liquid chromatography to obtain 46 mg of compound 22 as a slightly yellow solid; TOF-MS m/z (M+H)⁺:2813.5.

In a 5 mL single-neck flask, compound 22 (45 mg) and dichloromethane (4 mL) were added at room temperature, followed by addition of trifluoroacetic acid (2 mL), and the reaction was allowed at room temperature for 2 h. After the reaction was completed, the reaction solution was directly concentrated under reduced pressure in a water bath. The residue was purified by preparative-grade high-performance liquid chromatography to obtain 20 mg of compound P1-C as a white solid; TOF-MS m/z (M+Na)⁺:2657.2.

### Example 9: Synthesis of compound P1-D

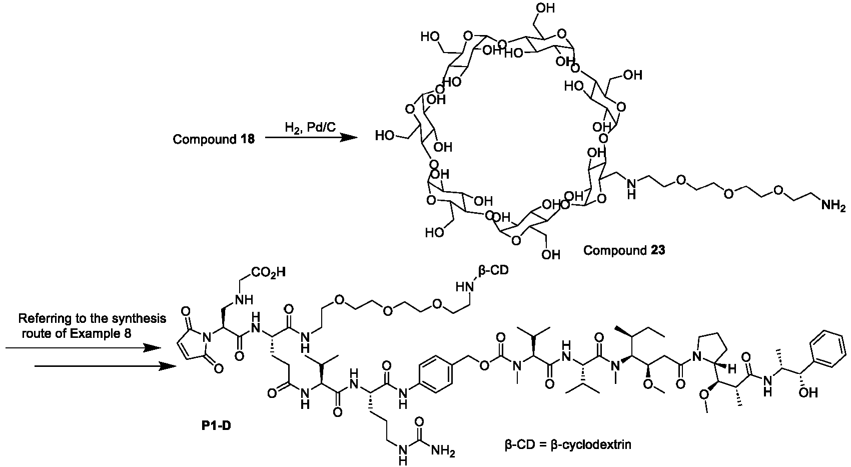

In a 25 mL single-neck flask, compound 18 (40 mg) and DMF (5 mL) were added at room temperature, followed by addition of Pd/C (40 mg), and the atmosphere in the system was replaced with hydrogen three times. The reaction was allowed at room temperature for 1 h. The reaction solution was filtered to remove the catalyst. DMF solvent was removed under reduced pressure by an oil pump, and the resulting crude yellow oil 23 was directly put into the reaction without further purification.

9.9 mg of the slightly yellow solid compound P1-D was prepared according to the synthesis route of Example 8; TOF-MS m/z (M+Na)⁺:2789.4.

### Example 10: Synthesis of compound P1-E

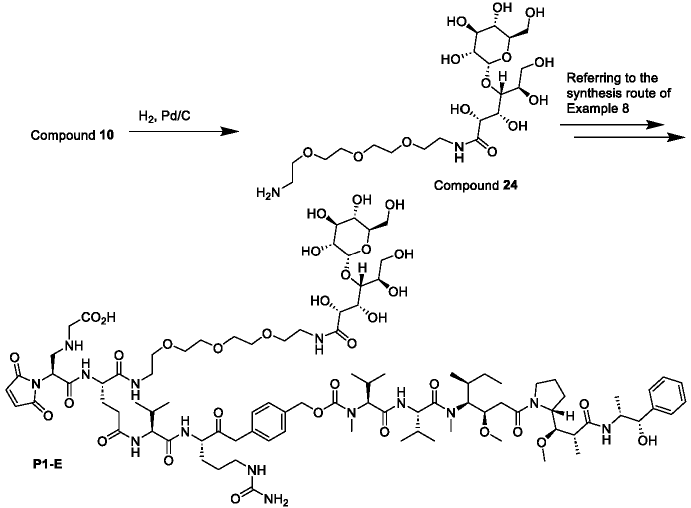

In a 25 mL single-neck flask, compound 10 (100 mg) and DMF (10 mL) were added at room temperature, followed by addition of Pd/C (100 mg), and the atmosphere in the system was replaced with hydrogen three times. The reaction was allowed at room temperature for 3 h. The reaction solution was filtered to remove the catalyst. DMF solvent was removed under reduced pressure by an oil pump. The resulting crude yellow oil 24 was directly put into the reaction without further purification.

23 mg of the slightly yellow solid compound P1-E was prepared according to the synthesis route of Example 8; TOF-MS m/z (M+Na)⁺:2012.5.

### Example 11: Synthesis of compound P1-F

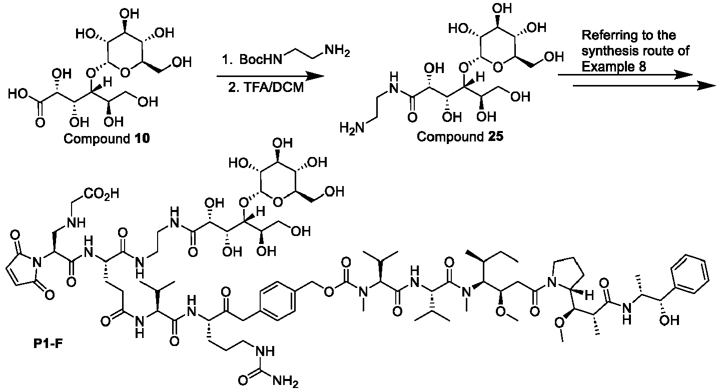

Compound maltobionic acid (200 mg, 0.56 mmol), N-tert-butyloxycarbonyl-1,2-ethylenediamine (196 mg, 1.2 mmol) and 10 mL of DMF were added into a 25 mL single-neck reaction flask, followed by addition of DIPEA (0.22 mL, 1.2 mmol), and the resulting yellow solution reacted at room temperature for about 4 h. The reaction solution was purified by preparative-grade high-performance liquid chromatography to obtain a white solid compound. In a 25 mL single-neck flask, the above compound and dichloromethane (6 mL) were added at room temperature, followed by addition of trifluoroacetic acid (3 mL), and the reaction was allowed at room temperature for 2 h. After the reaction was completed, rotatory evaporation under reduced-pressure was performed to remove trifluoroacetic acid and dichloromethane, which was directly put into the next reaction; LC-MS m/z(M+H)⁺:401.1.

23 mg of the slightly yellow solid compound P1-F was prepared according to the synthesis route of Example 8; TOF-MS m/z (M+H)⁺:1858.1.

### Example 12: Synthesis of compound P1-G

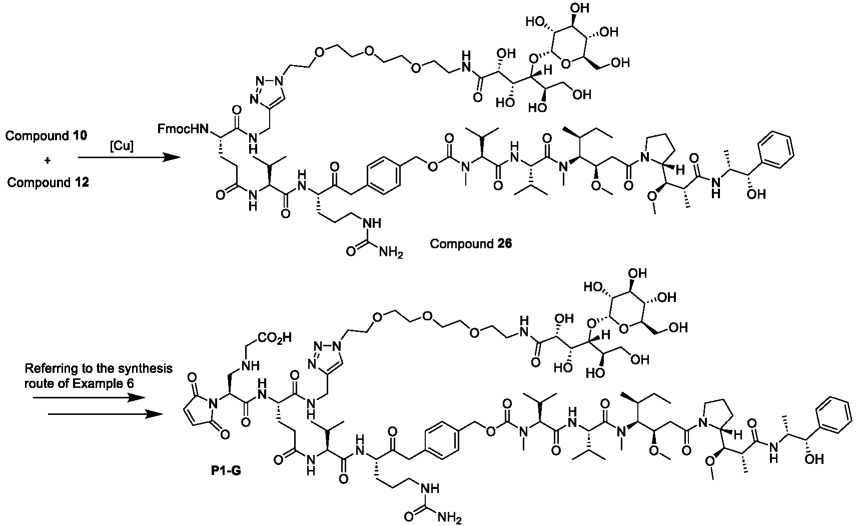

15 mg of the white solid compound P1-G was obtained by compound 10 and compound 12 referring to the synthesis route of Example 6; TOF-MS m/z (M+H)⁺:2071.4.

### Example 13: Synthesis of compound P1-H

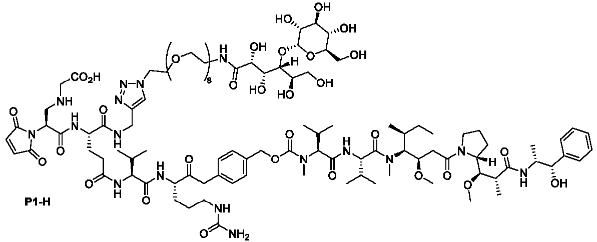

12 mg of the white solid compound P1-H was prepared according to the synthesis route of Example 6; TOF-MS m/z (M+H)⁺:2291.6.

### Example 14: Synthesis of compound P1-I

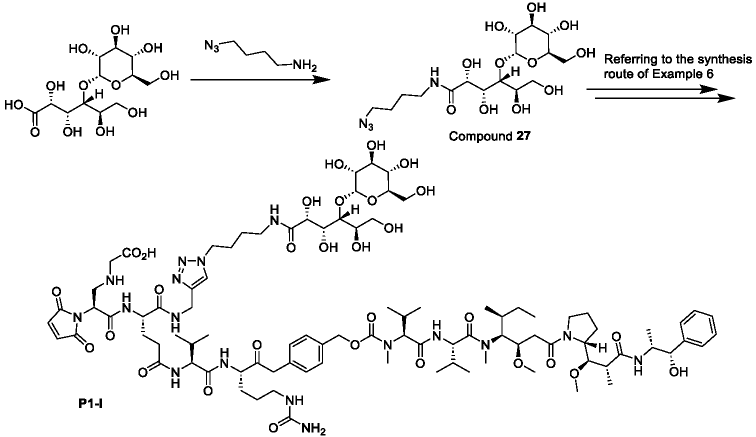

Compound 27 was prepared according to the synthesis route of Example 3; LC-MS m/z (M+H)⁺:455.4.

11 mg of the white solid compound P1-I was prepared according to the synthesis route of Example 6; TOF-MS m/z (M+H)⁺:1989.2.

### Example 15: Synthesis of compound P1-J

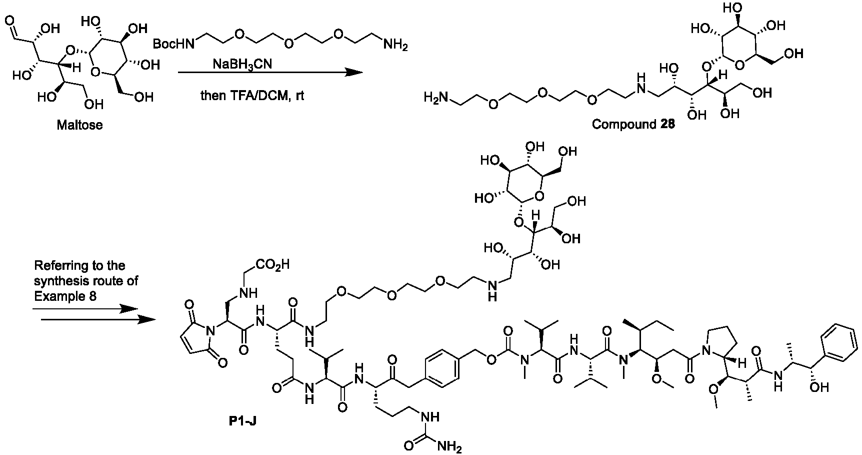

Maltose (500 mg, 1.46 mmol), 1,1-dimethylethyl 13-amino-5,8,11-trioxa-2-azatridecanoate (436 mg, 1.46 mmol) and 15 mL of anhydrous ethanol were added into a 25 mL single-neck reaction flask, and the reaction was allowed at room temperature for about 4 h. The reaction flask was cooled with an ice-water bath, and sodium cyanoborohydride (186 mg, 2.92 mmol) was added twice to react at a constant temperature for 1 h. The reaction solution was purified by preparative-grade high-performance liquid chromatography, then lyophilization was performed to obtain a white solid compound. In a 25 mL single-neck flask, the above compound and dichloromethane (10 mL) were added at room temperature, followed by addition of trifluoroacetic acid (5 mL), and the reaction was allowed at room temperature for 3 h. After the reaction was completed, rotatory evaporation under reduced-pressure was performed to remove trifluoroacetic acid and dichloromethane, and the residual was directly put into the next reaction; LC-MS m/z(M+H)⁺:519.3.

8 mg of the white solid compound P1-J was prepared according to the synthesis route of Example 8; TOF-MS m/z (M+Na)⁺:1998.2.

### Example 16: Synthesis of compound P1-K

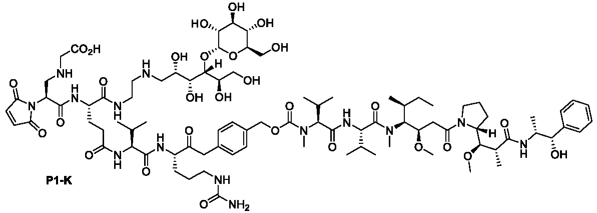

21 mg of the white solid compound P1-K was prepared according to the synthesis route of Example 8 and Example 14; TOF-MS m/z (M+Na)⁺:1866.0.

### Example 17: Synthesis of compound P1-L

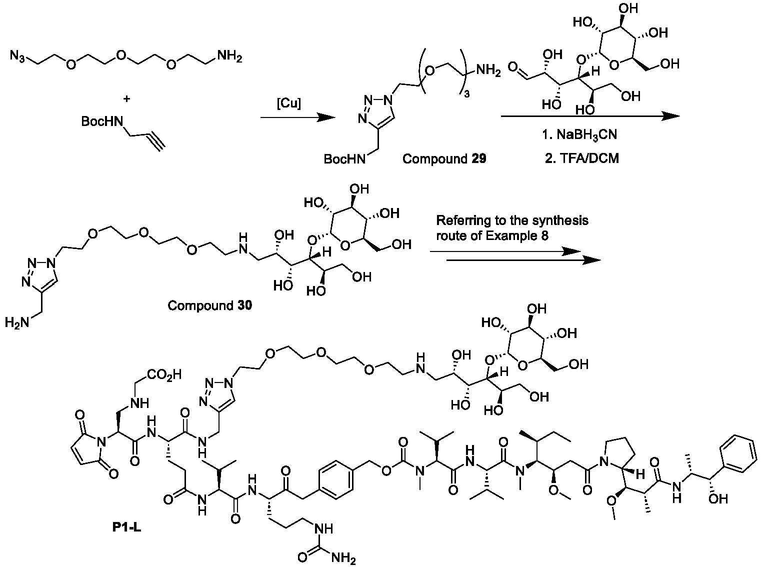

In a 25 mL single-neck flask, 1-amino-11-azido-3,6,9-trioxaundecane (60 mg, 0.27 mmol) and tetrahydrofuran (5 mL) were added at room temperature, followed by addition of N-Boc-aminopropyne (44 mg, 0.27 mmol), cuprous iodide (2.0 mg, 0.1 mmol) and TEA (40 µL, 0. 29 mmol) in sequence, and the mixed solution was heated to 50 °C to react for 10 h, then filtered, and the residue was purified by preparative-grade high-performance liquid chromatography to obtain 82 mg of compound 29 as a white solid; LC-MS m/z (M+H)⁺:374.3.

Compound 30 was prepared according to the synthesis route of Example 14; LC-MS m/z (M+H)⁺:600.5.

17 mg of the white solid compound P1-L was prepared according to the synthesis route of Example 8; TOF-MS m/z (M+Na)⁺:2079.1.

### Example 18: Synthesis of compound P1-M

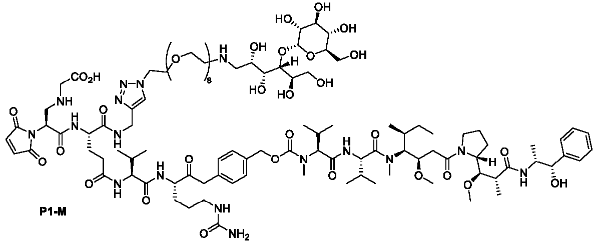

21 mg of the white solid compound P1-M was prepared according to the synthesis route of Example 16; TOF-MS m/z (M+Na)⁺:2299.1.

### Example 19: Synthesis of compound P1-N

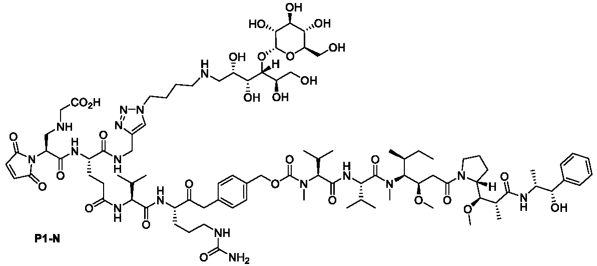

13 mg of the solid compound P1-N was prepared according to the synthesis route of Example 16; TOF-MS m/z (M+H)⁺:1953.2.

### Example 20: Synthesis of compound P1-O8

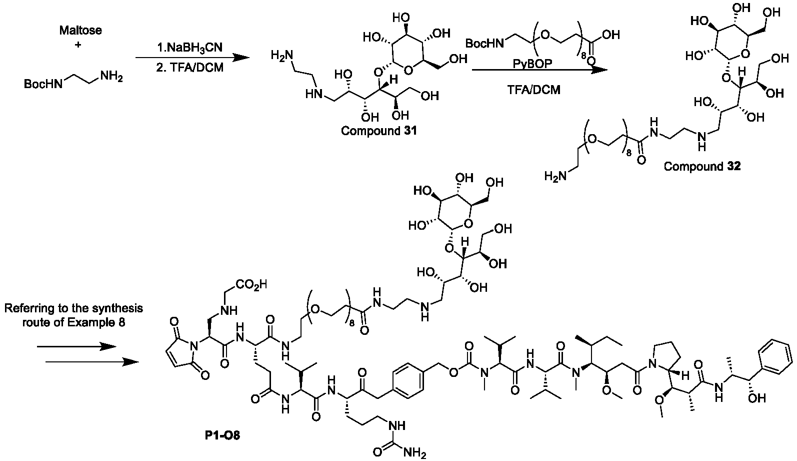

Maltose (50 mg, 0.146 mmol), N-Boc-ethylenediamine (24 mg, 0.146 mmol) and 5 mL of anhydrous ethanol were added into a 25 mL single-neck reaction flask, and the reaction was allowed at room temperature for about 4 h. The reaction flask was cooled with an ice-water bath, and sodium cyanoborohydride (18.6 mg, 0.292 mmol) was added twice to react at room temperature for 1 h. The reaction solution was purified by preparative-grade high-performance liquid chromatography, then lyophilization was performed to obtain a solid compound. In a 25 mL single-neck flask, the above compound and dichloromethane (4 mL) were added at room temperature, followed by addition of trifluoroacetic acid (2 mL), and the reaction was allowed at room temperature for 3 h. After the reaction was completed, rotatory evaporation under reduced-pressure was performed to remove trifluoroacetic acid and dichloromethane to obtain a crude compound 31, which was directly put into the next reaction without purification; LC-MS m/z(M+H)⁺:387.2.

In a 25 mL single-neck flask, the above crude compound (0.146 mmol based on the starting material charge), N-*tert*-butyloxycarbonyl-heptaethylene glycol-carboxylic acid (81 mg, 0.146 mmol), PyBOP (116 mg, 0.219 mmol) and DMF (5 mL) were added, and the resulting solution was then cooled to 0 °C in condition of an ice water bath. DIPEA (40 µL, 0.219 mmol) was added dropwise into a reaction flask, and the resulting solution was slowly brought naturally to room temperature to have a reaction overnight. The resulting yellow reaction solution was directly concentrated under reduced pressure by an oil pump to obtain a yellow oil. In the above 25 mL single-neck flask with the oil, dichloromethane (4 mL) was added at room temperature, followed by addition of trifluoroacetic acid (2 mL), and the reaction was allowed at room temperature for 2 h. After the reaction was completed, rotatory evaporation under reduced-pressure was performed to remove trifluoroacetic acid and dichloromethane to obtain a crude product. The crude product was purified by preparative-grade high-performance liquid chromatography column and lyophilized to obtain 66 mg of compound 32 as a white solid; LC-MS m/z (M+H)⁺:810.9.

15 mg of the solid compound P1-O8 was prepared according to the synthesis route of Example 8; TOF-MS m/z (M+Na)⁺:2289.2.

### Example 21: Synthesis of compound P1-P8

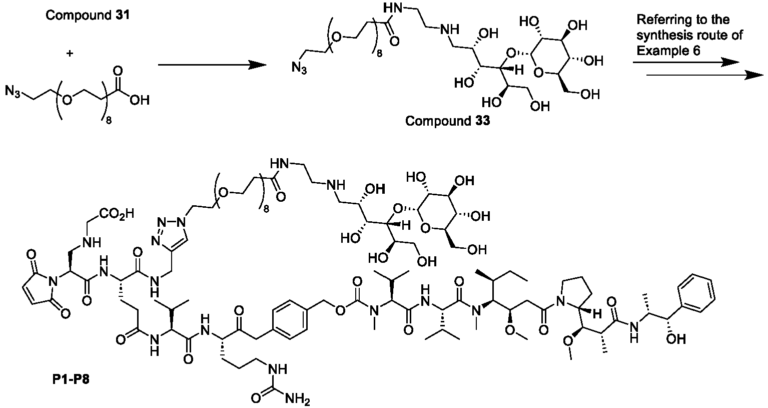

Compound 33 was prepared according to the synthesis route of Example 19; LC-MS m/z (M+H)⁺:836.9.

23 mg of the solid compound P1-P8 was prepared according to the synthesis route of Example 6; TOF-MS m/z (M+Na)⁺:2289.2.

### Example 22: Synthesis of compound P1-Q

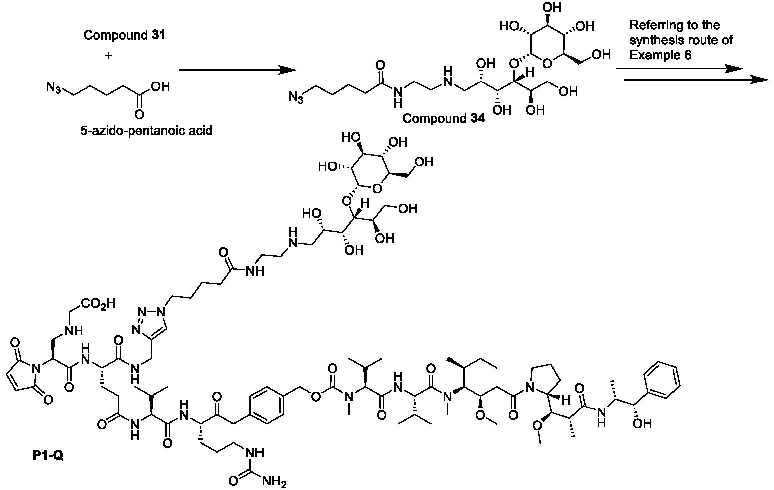

Compound 34 was prepared according to the synthesis route of Example 19; LC-MS m/z (M+H)⁺:512.5

9 mg of the solid compound P1-Q was prepared according to the synthesis route of Example 6; TOF-MS m/z (M+H)⁺:2024.1.

### Example 23: Synthesis of compound P1-R

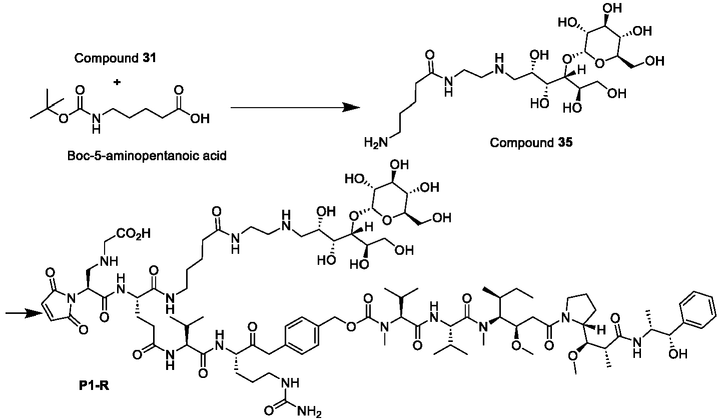

Compound 35 was prepared according to the synthesis route of Example 19; LC-MS m/z (M+H)⁺:486.5

14 mg of the solid compound P1-R was prepared according to the synthesis route of Example 8; TOF-MS m/z (M+H)⁺:1943.2.

### Example 24: Synthesis of compound P1-S

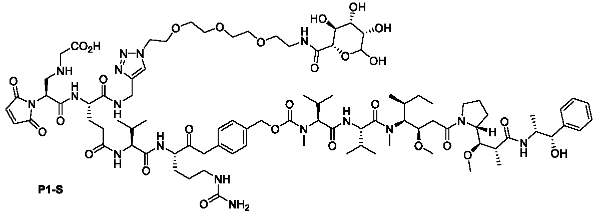

24 mg of the solid compound P1-S was prepared according to the synthesis route of Example 6; TOF-MS m/z (M+Na)⁺:1929.1.

### Example 25: Synthesis of compound P1-T

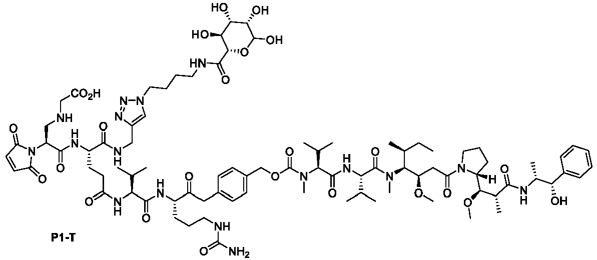

16 mg of the solid compound P1-T was prepared according to the synthesis route of Example 6; TOF-MS m/z (M+H)⁺:1803.5.

### Example 26: Synthesis of compound P1-U

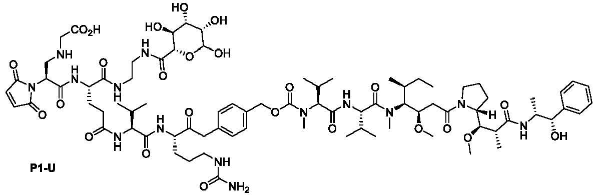

12 mg of the solid compound P1-U was prepared according to the synthesis route of Example 8; TOF-MS m/z (M+H)⁺:1693.9.

### Example 27: Synthesis of compound P1-V

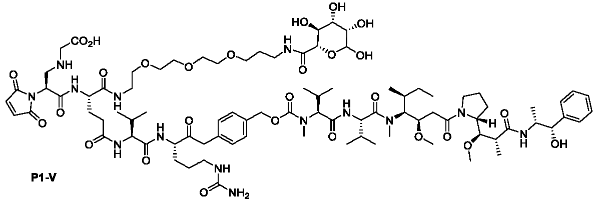

19 mg of the solid compound P1-V was prepared according to the synthesis route of Example 8; TOF-MS m/z (M+H)⁺:1840.1.

### Example 28: Synthesis of compound P1-W

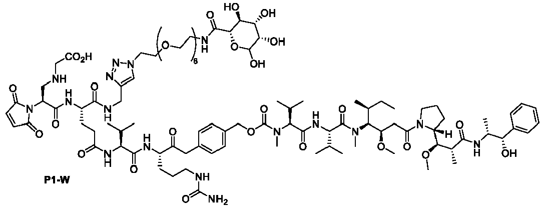

32 mg of the solid compound P1-W was prepared according to the synthesis route of Example 6; TOF-MS m/z (M+H)⁺:2127.1291.

### Example 29: Synthesis of compound P1-X2

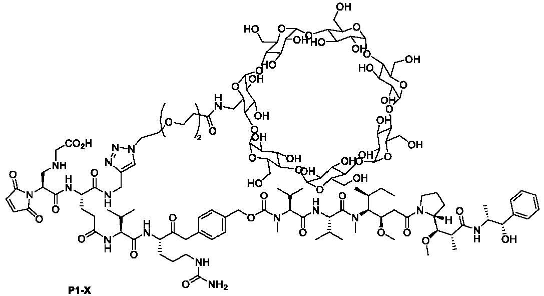

27 mg of the solid compound P1-W was prepared according to the synthesis route of Example 6; TOF-MS m/z (M+Na)⁺:2853.2939.

### Example 30: Synthesis of compound P1-Y8

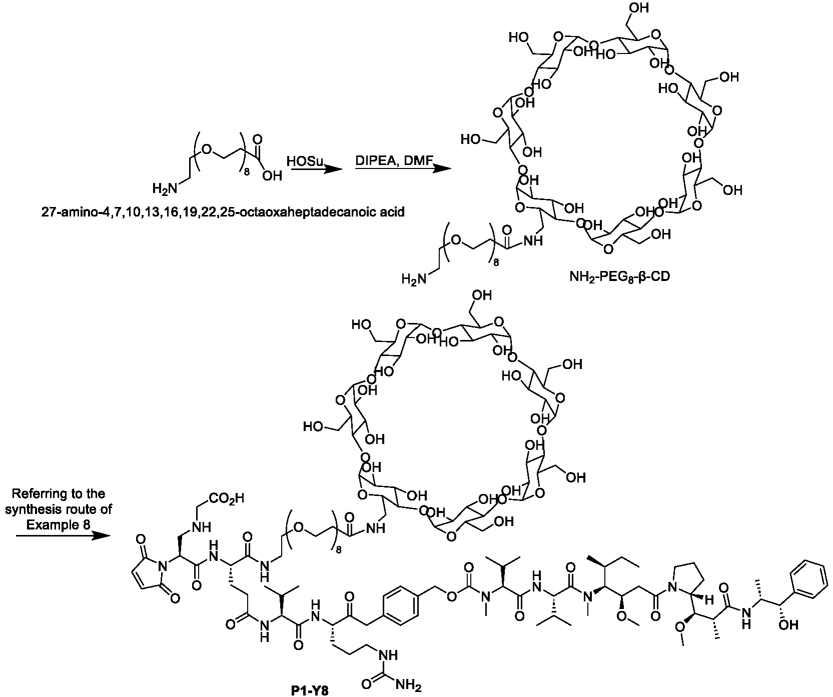

### Step 1: Synthesis of compound NH₂-PEG₈-β-CD

In a 25 mL single-neck flask, compound 27-amino-4,7,10,13,16,19,22,25-octaoxaheptadecanoic acid (100 mg, 0.22 mmol) and DMF (5 mL) were added at room temperature, followed by addition of HOSu (28 mg, 0.23 mmol) and DCC (48 mg, 0.23 mmol), and the reaction was allowed at room temperature for 3 h. DMF solvent was removed under reduced pressure by an oil pump. The resulting crude yellow oil was dissolved in 10 mL of DCM, filtered to remove insoluble solids; the filter cake was washed three times with DCM; and the organics were combined and spin-dried with water pump in 45 °C water bath, and the residual was directly put into the next reaction.

In a 25 mL single-neck flask, the above crude compound (0.22 mmol) and DMF (5 mL) were added at room temperature, followed by addition of mono(6-amino-6-deoxy)-β-cyclodextrin (275 mg, 0.23 mmol) and DIPEA (74 µL, 0.44 mmol), and the reaction was allowed at room temperature for 2 h. After the reaction was found to be completed from the monitoring of HPLC, the reaction solution was purified by preparative-grade high-performance liquid chromatography to obtain a purified solution. The purified solution was lyophilized to obtain compound NH₂-PEG₈-β-CD(145 mg); LC-MS m/z (M+H)⁺: 1157.7.

### Step 2: Synthesis of compound P1-Y8

Taking of compound 15 and NH₂-PEG₈-β-CD as starting raw materials, 13 mg of solid compound P1-Y8 was prepared according to the synthesis route of Example 8; TOF-MS m/z (M+H)⁺:3014.4188.

### Example 31: Synthesis of compound P1-Z

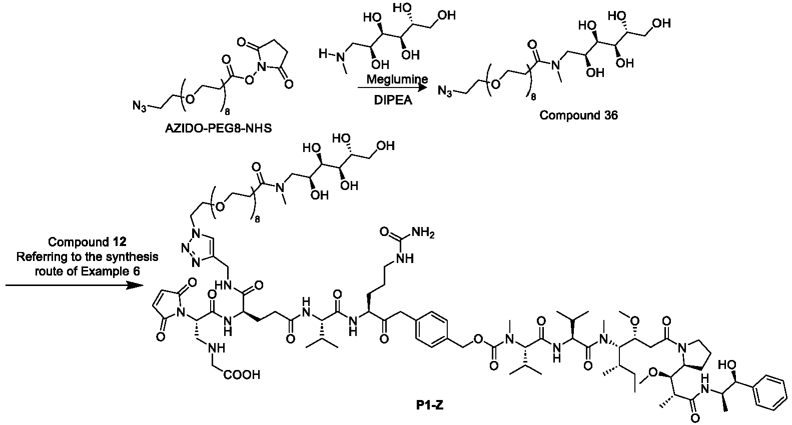

### Step 1: Synthesis of compound 36

In a 25 mL single-neck flask, AZIDO-PEG8-NHS (100 mg, 0.17 mmol) and DMF (5 mL) were added at room temperature, followed by addition of meglumine (34 mg, 0.17 mmol) and DIPEA (66 µL, 0.4 mmol), and the reaction was allowed at room temperature for 2 h. After the reaction was found to be completed from the monitoring of HPLC, the reaction solution was purified by preparative-grade high-performance liquid chromatography to obtain a purified solution. The purified solution was lyophilized to obtain compound 36 (120 mg); LC-MS m/z (M+H)⁺:645.7.

### Step 2: Synthesis of compound P1-Z

Taking of compound 12 and compound 36 as starting raw materials, 18 mg of solid compound P1-Z was prepared according to the synthesis route of example 6; TOF-MS m/z (M+H)⁺:2157.1796.

### Example 32: Preparation of compound 37

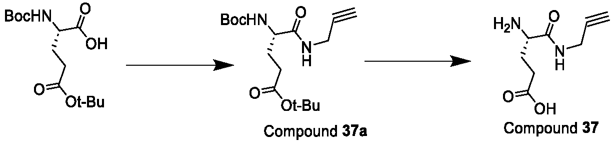

In a 1 L single-neck flask, 5-tert-butyl Boc-L-glutamate (50 g, 164.8 mmol, 1.0 eq) was completely dissolved in 250 mL of THF, and propargylamine (9.97 g, 181.3 mmol, 1.1 eq), HOBt (22.25 g, 164.8 mmol, 1.0 eq), DIEA (54.3 mL, 329.6 mmol, 2.0 eq) and EDCI (37.7 g, 197.7 mmol, 1.2 eq) were added in sequence in condition of an ice water bath. The reaction was allowed at room temperature to react for 2 h. After the reaction was found to be completed from the monitoring of TLC, the reaction solution was poured into 600 mL of ice water and extracted three times with ethyl acetate (200 mL×3); the organics were combined, washed twice with saturated salt solution (300 mL×2), dried with anhydrous Na₂SO₄, filtered, and concentrated to obtain a crude product. The crude product was refined by petroleum ether/ethyl acetate (2/1) to obtain 35.5 g of compound 37a; LC-MS m/z (M+H)⁺: 341.2.

In a 1 L single-neck flask, compound 37a (20 g) was completely dissolved in 150 mL of dichloromethane, followed by addition of 150 mL of trifluoroacetic acid, and the reaction was allowed at room temperature for 1 h. After the reaction was found to be completed from the monitoring of TLC, the reaction solution was concentrated at 45 °C by a water pump to obtain an oily crude product. The crude product was added to methyl *tert*-ether for crystallization overnight. The reaction solution was filtered, and the filter cake was dried at 45 °C for 4 h by blowing air to obtain 7.68 g of the product; LC-MS m/z (M+H)⁺:184.2.

### Example 33: Preparation of compound 39

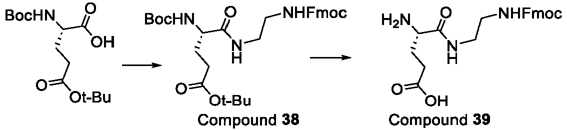

In a 1 L single-neck flask, 5-*tert*-butyl Boc-*L*-glutamate (50 g, 164.8 mmol, 1.0 eq) was completely dissolved in 300 mL of THF. FmocNH-(CH₂)₂-NH₂ (51.1 g, 181.3 mmol, 1.1 eq), HOBt (22.25 g, 164.8 mmol, 1.0 eq), DIEA (54.3 mL, 329.6 mmol, 2.0 eq) and EDCI (37.7 g, 197.7 mmol, 1.2 eq) were added in sequence in condition of an ice water bath, and the reaction was allowed at room temperature to react for 2 h. After the reaction was found to be completed from the monitoring of TLC, the reaction solution was poured into 600 mL of ice water, extracted three times with ethyl acetate (300 mL×3); the organics were combined, washed twice with Saturated salt solution (300 mL×2), dried with anhydrous Na₂SO₄, filtered, and concentrated to obtain a crude product. The crude product was refined by petroleum ether/ethyl acetate (2/1) to obtain 57.5 g of pure product; LC-MS m/z (M+H)⁺:567.2.

In a 1 L single-neck flask, compound 38 (20 g) was completely dissolved in 150 mL of dichloromethane, followed by addition of 150 mL of trifluoroacetic acid, and the reaction was allowed at room temperature for 1 h. After the reaction was found to be completed from the monitoring of TLC, the reaction solution was concentrated at 45 °C to obtain a crude oil. The crude product was added to methyl *tert*-ether for crystallization overnight and filtered. The filter cake was dried at 45 °C for 4 h by blowing air, and weighed to obtain 11.7 g of the product; LC-MS m/z (M+H)⁺:412.2.

### Example 34: Preparation of compound 40

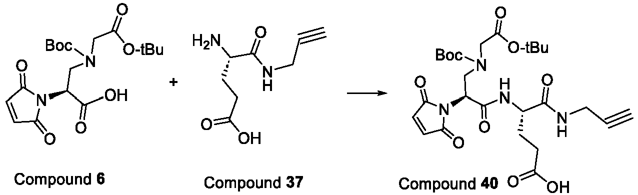

In a 50 mL single-neck flask, compound 37 (5.0 g, 27.1 mmol) and compound 6 (10.8 g, 27.1 mmol) were mixed and completely dissolved in 20 mL of DMF, and EEDQ (13.4 g, 54.2 mmol) was then added in condition of an ice water bath. After the addition was completed, the reaction was allowed at room temperature for 2 h. After the reaction was found to be completed from the monitoring of HPLC, the reaction solution was purified by preparative high-performance liquid phase chromatography and lyophilized to obtain 7.84 g of the product; LC-MS m/z (M+H)⁺:465.2.

### Example 35: Preparation of compound 44

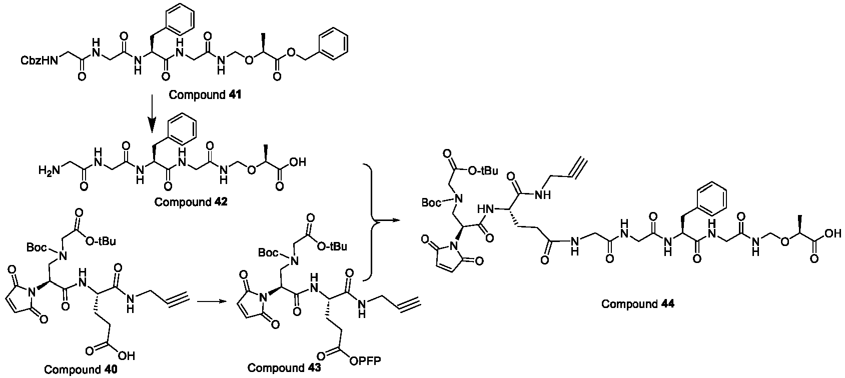

In a 50 mL single-neck flask, compound 41 (1.0 g, 1.51 mmol, referring to patent application WO2020063676A1) was completely dissolved in 15 mL of DMF, and 5% Pd/C (1.0 g) was added to have a hydrogenation reaction at room temperature for 2 h. After the reaction was found to be completed from the monitoring of HPLC, filtering was performed to obtain the filtrate, denoted filtrate 1;

In a 25 mL single-neck flask, compound 40 (0.85 g, 1.51 mmol) was completely dissolved in 15 mL of DMF, then DCC (1.55 g, 7.56 mmol) and pentafluorophenol (0.31 g, 1.66 mmol) were added in condition of an ice water bath, and the reaction was allowed at room temperature for 2 h. After the reaction was found to be completed from the monitoring of TLC, filtering was performed to obtain the filtrate, denoted filtrate 2;

DIEA (0.37 mL, 2.26 mmol) was added into filtrated 1 in condition of an ice water bath, followed by addition of filtrated 2, and the reaction was allowed at room temperature for 1 h. After the reaction was found to be completed from the monitoring of HPLC, the reaction solution was purified by preparative high-performance liquid phase chromatography to obtain a preparation solution. The preparation solution was lyophilized to obtain 502 mg of compound 44 as a white solid; LC-MS m/z (M+H)⁺:985.2.

### Example 36: Preparation of compound 45

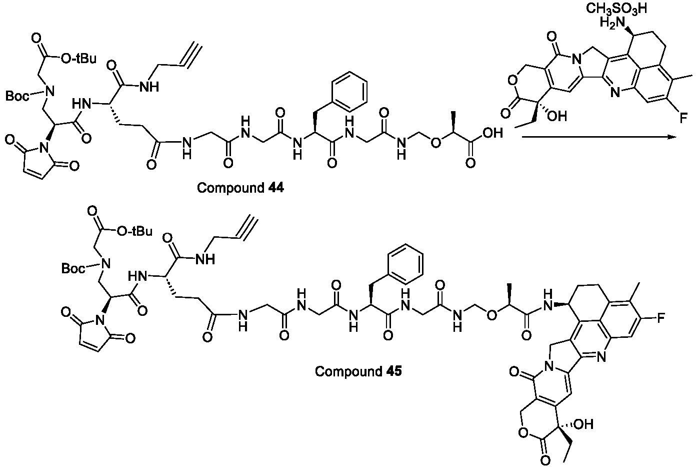

In a 50 mL single-neck flask, compound 44 (500 mg, 0.51 mmol) was completely dissolved in 10 mL of DMF, then PyBOP (396 mg, 0.76 mmol), HOBt (103 mg, 0.76 mmol) and Exatecan mesylate (270 mg, 0.51 mmol) were added in sequence in condition of an ice water bath, followed by addition of DIEA (252 µL, 1.52 mmol), and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain 500 mg of compound 45 as a white solid; LC-MS m/z (M+H)⁺:1402.2.

### Example 37: Synthesis of compound P2-A

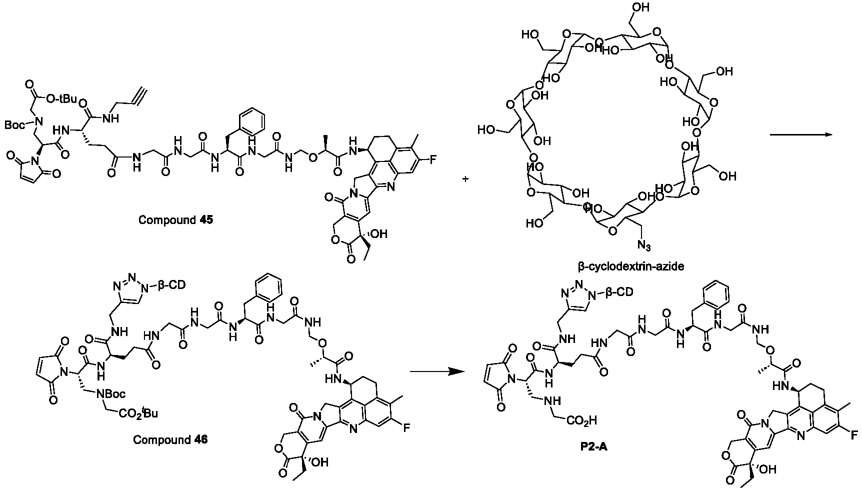

In a 25 mL single-neck flask, compound 45 (50 mg, 0.035 mmol) and β-cyclodextrin-N₃ (41 mg, 0.035 mmol) were completely dissolved in 5 mL of DMF, followed by addition of cuprous iodide (2 mg, 0.007 mmol) and TEA (10 µL, 0.07 mmol). The resulting solution was heated to 50 °C to react for 10 h, and the reaction process was monitored by HPLC. After the reaction was completed, the reaction solution was purified by preparative high-performance liquid phase chromatography to obtain a preparation solution of the product. The preparation solution was lyophilized to obtain 59 mg of compound 46; TOF-MS m/z(M+Na)⁺:2583.0.

In a 25 mL single-neck flask, compound 46 (50 mg, 0.019 mmol) was dissolved completely in 5 mL of nitromethane, followed by addition of zinc bromide (88 mg, 0.39 mmol), and the reaction was allowed at room temperature for 1 h. The reaction process was monitored by HPLC. After the reaction was completed, the reaction solution was concentrated at 45 °C under reduced-pressure by a water pump to obtain a crude product. The crude product was purified by preparative high-performance liquid phase chromatography to obtain a preparation solution of the product. The preparation solution was lyophilized and weighed to obtain 30 mg of compound P2-A; TOF-MS m/z(M+H)⁺:2404.9.

### Example 38: Synthesis of compound P2-B

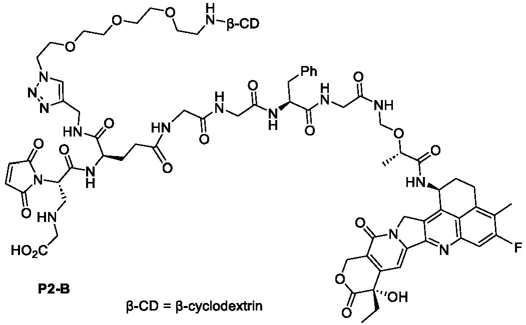

24 mg of compound P1-B was prepared according to the synthesis route of Example 37; TOF-MS m/z(M+H)⁺:2580.0.

### Example 39: Synthesis of compound 49

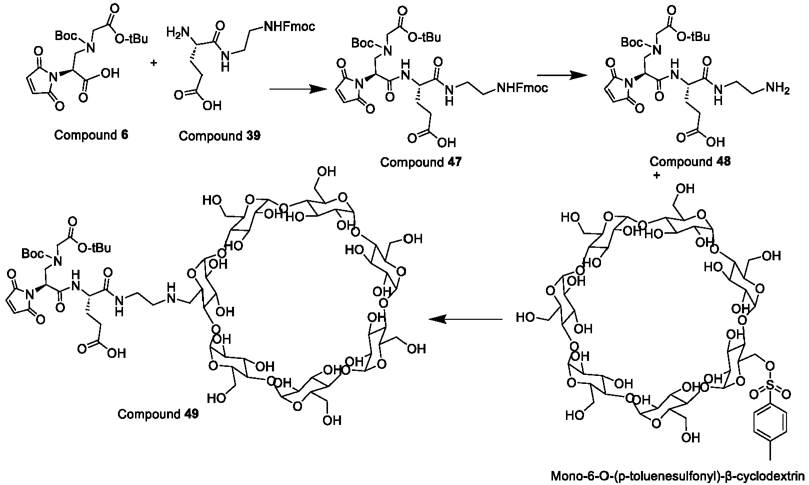

In a 50 mL single-neck flask, compound 39 (5.0 g, 12.1 mmol) and compound 6 (4.83 g, 12.1 mmol) were mixed and completely dissolved in 25 mL of DMF, and EEDQ (6.0 g, 24.3 mmol) was then added in condition of an ice water bath. After the addition was completed, the reaction was allowed at room temperature for 2 h. After the reaction was found to be completed from the monitoring of HPLC, the reaction solution was purified by preparative high-performance liquid phase chromatography, then lyophilized to obtain 5.1 g of the product; LC-MS m/z (M+H)⁺:792.3.

In a 50 mL single-neck flask, compound 47 (0.3 g, 0.378 mmol) was completely dissolved in 5 mL of DMF, followed by addition of 0.3 mL of diethylamine, and the resulting solution was stirred at room temperature for 1 h. After the reaction was found to be completed from the monitoring of TLC, the reaction solution was concentrated under reduced-pressure to remove DMF and diethylamine to obtain a crude product, which was directly put into the next reaction.

The crude product was dissolved in 5 mL of DMF, followed by addition of mono-6-O-(*p*-toluenesulfonyl)-*β*-cyclodextrin (488 mg, 0.378 mmol), and the reaction was allowed at room temperature for 2 h in the presence of N₂. After the reaction was found to be completed from the monitoring of HPLC, the reaction solution was purified by preparative high-performance liquid phase chromatography, and lyophilized to obtain 601 mg of compound 49; TOF-MS m/z (M+H)⁺:1686.9.

### Example 40: Synthesis of compound P2-C

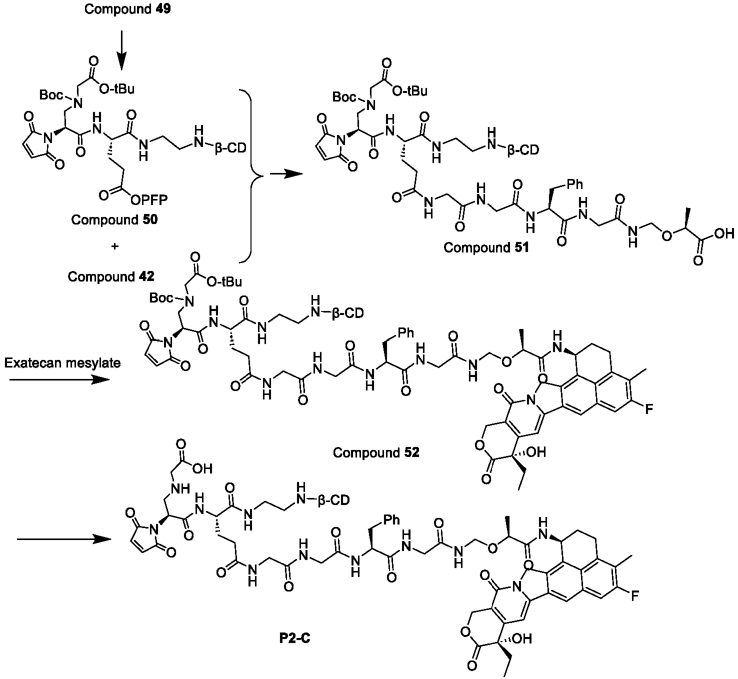

In a 25 mL single-neck flask, compound 49 (1.05 g, 0.62 mmol) was completely dissolved in 10 mL of DMF. DCC (653 mg, 3.1 mmol) and pentafluorophenol (119 mg, 0.63 mmol) were added in condition of an ice water bath, and the reaction was allowed at room temperature for 2 h. After the reaction was found to be completed from the monitoring of TLC, the reaction solution was filtered to obtain the filtrate;

DIEA (0.22 mL, 1.24 mmol) was added into the above filtrate in condition of an ice water bath, followed by addition of compound 42 (272 mg, 0.62 mmol), and the reaction was allowed at room temperature for 1 h. After the reaction was found to be completed from the monitoring of HPLC, the reaction solution was purified by preparative high-performance liquid phase chromatography to obtain a preparation solution. The preparation solution was lyophilized to obtain 588 mg of compound 51 as a white solid; TOF-MS m/z (M+H)⁺:2105.8.

In a 50 mL single-neck flask, compound 51 (500 mg, 0.24 mmol) was completely dissolved in 10 mL of DMF, then PyBOP (188 mg, 0.36 mmol), HOBt (49 mg, 0.36 mmol) and Exatecan mesylate (140 mg, 0.26 mmol) were added in sequence in condition of an ice water bath, followed by addition of DIEA (86 µL, 0.48 mmol), and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain 403 mg of compound 52 as a solid; TOF-MS m/z (M+Na)⁺:2544.0.

In a 25 mL single-neck flask, compound 52 (60 mg, 0.024 mmol) was dissolved completely in 5 mL of nitromethane, followed by addition of zinc bromide (109 mg, 0.48 mmol), and the reaction was allowed at room temperature for 1 h. After the reaction was found to be completed from the monitoring of HPLC, the reaction solution was concentrated at 45 °C under reduced-pressure by a water pumpto obtain a crude product. The crude product was purified by preparative high-performance liquid phase chromatography to obtain a preparation solution of the product. The preparation solution was lyophilized and weighed to obtain 29 mg of compound P2-C; TOF-MS m/z(M+H)⁺:2365.9.

### Example 41: Synthesis of compound P2-D

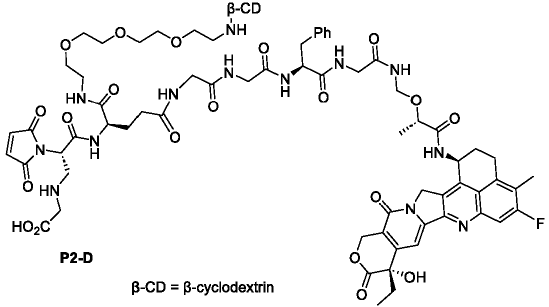

24 mg of compound P2-D was prepared according to the synthesis route of Examples 39 and 40; TOF-MS m/z(M+H)⁺:2500.3.

### Example 42: Synthesis of compound P2-E

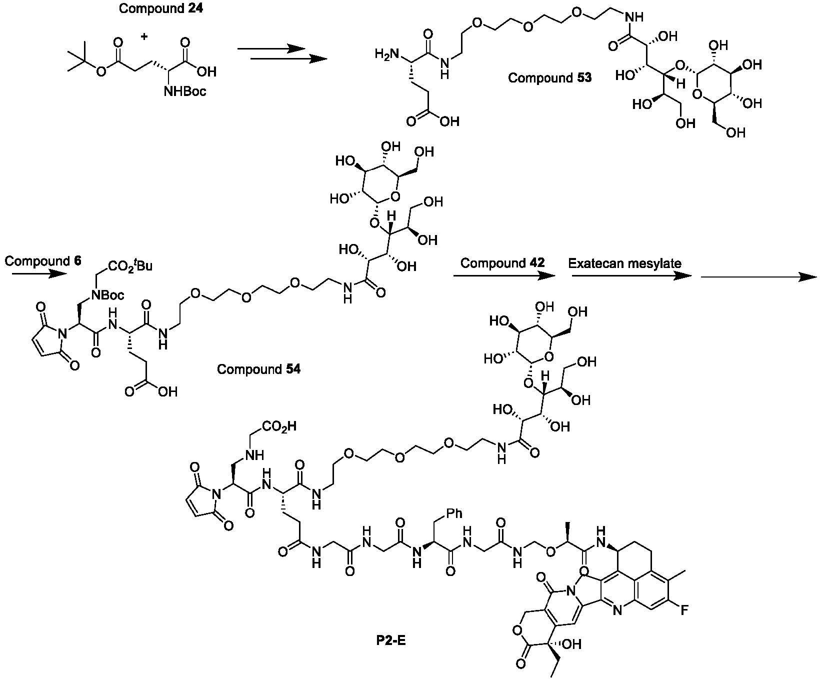

Compound 53 was prepared according to the synthesis route of Example 29;

Compound 54 was prepared according to the synthesis route of Example 31; and

33 mg of compound P2-E was prepared according to the synthesis route of Example 40; TOF-MS m/z(M+Na)⁺:1244.8.

### Example 43: Synthesis of compound P2-F

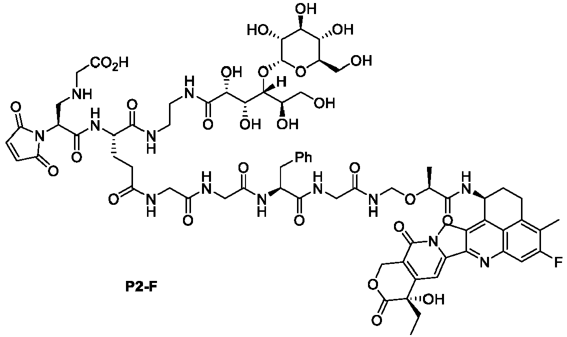

16 mg of compound P2-F was prepared according to the synthesis route of Examples 39 and 40; TOF-MS m/z(M+H)⁺:1590.7.

### Example 44: Synthesis of compound P2-G

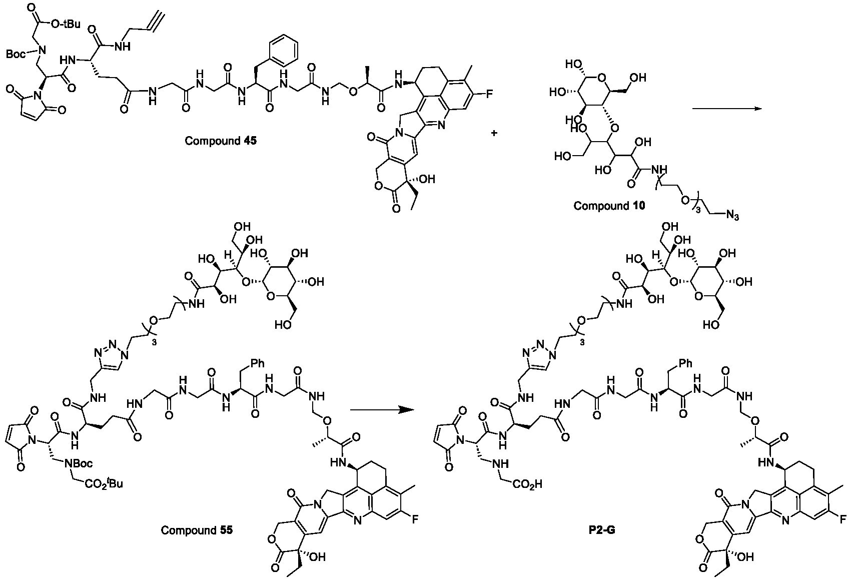

In a 25 mL single-neck flask, compound 45 (50 mg, 0.035 mmol) and compound 10 (20 mg, 0.035 mmol) were mixed and completely dissolved in 5 mL of DMF, followed by addition of cuprous iodide (2 mg, 0.007 mmol) and TEA (10 µL, 0.07 mmol), and the resulting solution was heated to 50 °C to react for 10 h. The reaction process was monitored by HPLC. After the reaction was completed, the reaction solution was purified by preparative high-performance preparation liquid phase chromatography to obtain a preparation solution of the product. The preparation solution was lyophilized and weighed to obtain 43 mg of the product; TOF-MS m/z(M+Na)⁺: 1981.9.

In a 25 mL single-neck flask, compound 55 (40 mg, 0.02 mmol) was dissolved completely in 3 mL of nitromethane, followed by addition of zinc bromide (92 mg, 0.4 mmol, 20 eq), and the reaction was allowed at room temperature for 1 h. The reaction process was monitored by HPLC. After the reaction was completed, the reaction solution was concentrated at 45 °C under reduced-pressure by a water pump to obtain a crude product. The crude product was purified by preparative high-performance liquid phase chromatography to obtain a preparation solution of the product. The preparation solution was lyophilized and weighed to obtain 25 mg of product P2-G; TOF-MS m/z(M+H)⁺:1803.7.

### Example 45: Synthesis of compound P2-H

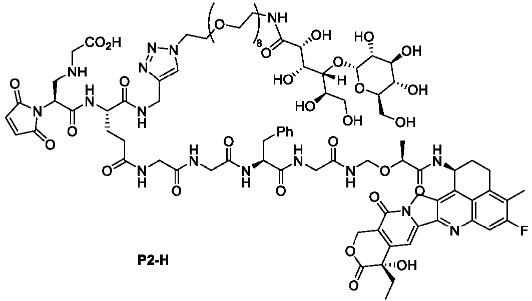

16 mg of compound P2-H was prepared according to the synthesis route of Example 44; TOF-MS m/z(M+H)⁺:2023.9.

### Example 46: Synthesis of compound P2-I

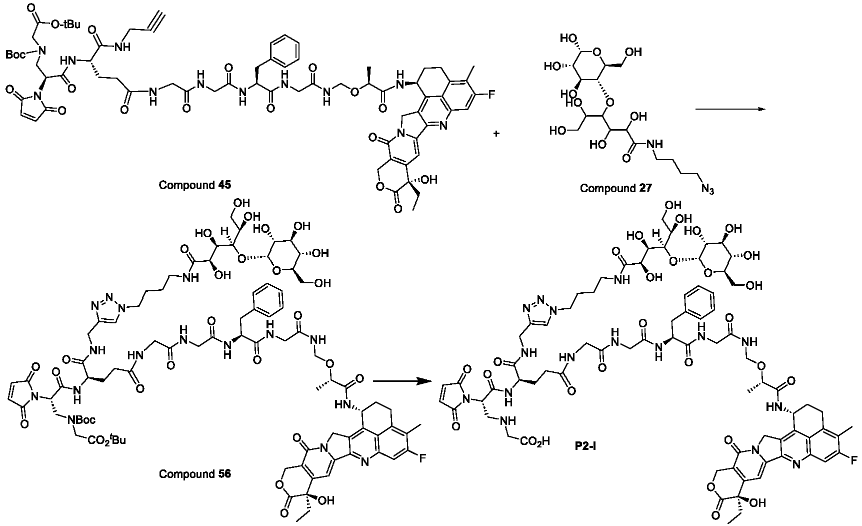

In a 25 mL single-neck flask, compound 45 (50 mg, 0.035 mmol) and compound 27 (16 mg, 0.035 mmol) were mixed and completely dissolved in 5 mL of DMF, followed by addition of cuprous iodide (2 mg, 0.007 mmol) and TEA (10 µL, 0.07 mmol), and the resulting solution was heated to 50 °C to react for 10 h. The reaction process was monitored by HPLC. After the reaction was completed, the reaction solution was purified by preparative high-performance liquid phase chromatography to obtain a preparation solution of the product. The preparation solution was lyophilized and weighed to obtain 43 mg of the product; TOF-MS m/z(M+H)⁺: 1955.9.

In a 25 mL single-neck flask, compound 56 (40 mg, 0.021 mmol) was dissolved completely in 5 mL of nitromethane, followed by addition of zinc bromide (97 mg, 0.42 mmol), and the reaction was allowed at room temperature for 1 h. The reaction process was monitored by HPLC. After the reaction was completed, the reaction solution was concentrated at 45 °C under reduced-pressure by a water pump to obtain a crude product. The crude product was purified by preparative high-performance liquid phase chromatography to obtain a preparation solution of the product. The preparation solution was lyophilized and weighed to obtain 25 mg of compound P2-I; TOF-MS m/z(M+H)⁺: 1699.7.

### Example 47: Synthesis of compound P2-J

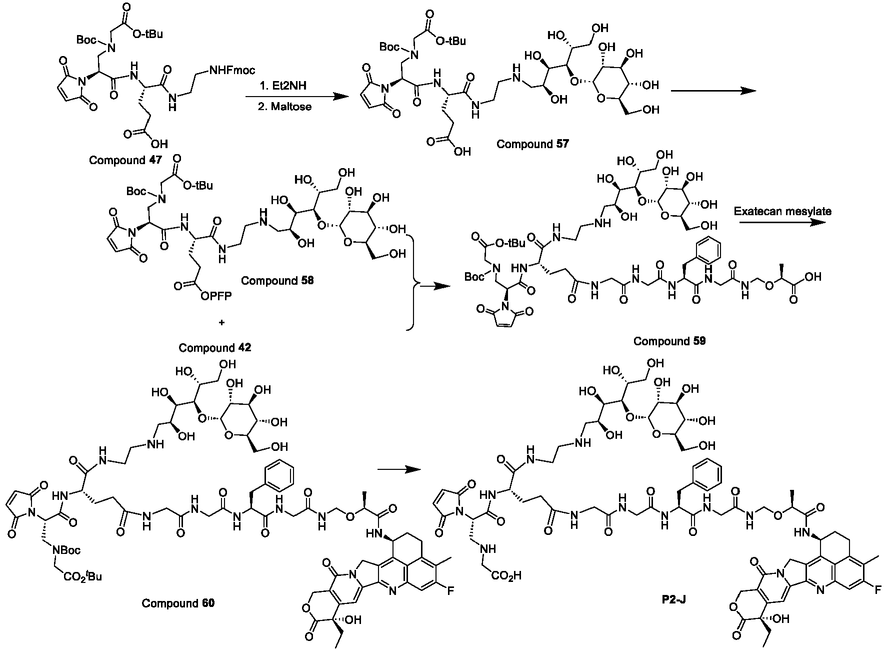

In a 50 mL single-neck flask, compound 47 (3.0 g, 3.78 mmol) was completely dissolved in 15 mL of DMF, followed by addition of 3 mL of diethylamine, and the resulting solution was stirred at room temperature for 1 h. After the reaction was found to be completed from the monitoring of TLC, concentration under reduced-pressure was performed on the reaction solution to remove DMF and diethylamine to obtain a crude product. The crude product was dissolved in 20 mL of methanol, maltose (1.29 g, 3.78 mmol) was then added, and the reaction was allowed at room temperature for 2 h in the presence of N₂. NaBH₄ (285 mg, 7.56 mmol) was added sin batch in condition of an ice water bath to react for 30 min. After the reaction was found to be completed from the monitoring of TLC, 20 mL of water was added, the resulting reaction solution was extracted three times with ethyl acetate (30 mL×3), the organics were combined, washed three times with saturated NaCl solution, dried with anhydrous Na₂SO₄, filtered, and the filtrate was concentrated to obtain a crude product. The crude product was purified by preparative high-performance liquid phase chromatography, lyophilized and weighed to obtain 1.4 g of a solid; LC-MS m/z (M+H)⁺:896.3.

In a 25 mL single-neck flask, compound 57 (677 mg, 0.75 mmol) was completely dissolved in 15 mL of DMF, DCC (780 mg, 3.78 mmol) and pentafluorophenol (153 mg, 0.83 mmol) were added in condition of an ice water bath, and the reaction was allowed at room temperature for 2 h. After the reaction was found to be completed from the monitoring of TLC, the rection solution was filtered to obtain a filtrate; DIEA (187 µL, 1.13 mmol) was added into the above filtrate in condition of an ice water bath, followed by addition of compound 42 (0.75 mmol), and the reaction was allowed at room temperature for 1 h. After the reaction was found to be completed from the monitoring of HPLC, the reaction solution was purified by preparative high-performance liquid phase chromatography to obtain a preparation solution. The preparation solution was lyophilized to obtain 350 mg of compound 59 as a white solid; LC-MS m/z (M +H)⁺:1315.6.

In a 50 mL single-neck flask, compound 59 (200 mg, 0.15 mmol) was completely dissolved in 10 mL of DMF, PyBOP (118 mg, 0.22 mmol), HOBt (31 mg, 0.22 mmol) and Exatecan mesylate (80 mg, 0.15 mmol) were added in sequence in condition of an ice water bath, followed by addition of DIEA (75 µL, 0.45 mmol), and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain 150 mg of compound 60 as a white solid; TOF -MS m/z (M +H)⁺: 1732.7.

In a 25 mL single-neck flask, compound 60 (50 mg, 0.028 mmol) was dissolved completely in 5 mL of nitromethane, followed by addition of zinc bromide (130 mg, 0.57 mmol), and the reaction was allowed at room temperature for 1 h. The reaction process was monitored by HPLC. After the reaction was completed, the reaction solution was concentrated at 45 °C under reduced-pressure by a water pump to obtain a crude product. The crude product was purified by preparative high-performance liquid phase chromatography to obtain a preparation solution of the product. The preparation solution was lyophilized and weighed to obtain 30 mg of the product; TOF -MS m/z (M +H)⁺: 1576.6.

### Example 48: Synthesis of compound P2-K

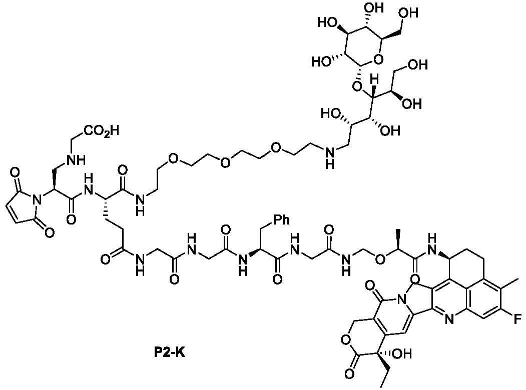

28 mg of compound P2-K was prepared according to the synthesis route of Example 47; TOF-MS m/z(M+H)⁺:1708.7.

### Example 49: Synthesis of compound P2-L

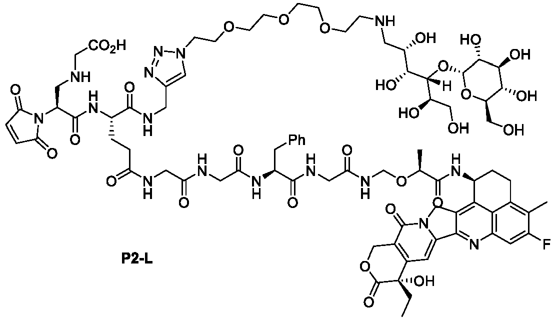

33 mg of compound P2-L was prepared according to the synthesis route of Example 47; TOF-MS m/z(M+H)⁺:1789.8.

### Example 50: Synthesis of compound P2-M

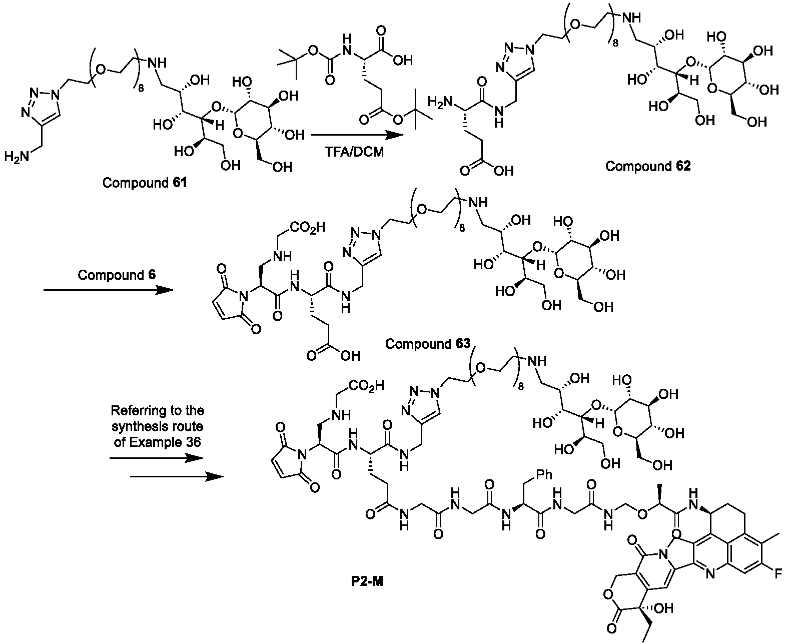

77 mg of compound 61 was prepared according to the synthesis route of compound 30; LC-MS m/z (M+H)⁺:820.9.

15 mg of compound P2-M was prepared according to the synthesis route of Example 40; TOF-MS m/z (M+H)⁺:2010.0.

### Example 51: Synthesis of compound P2-N

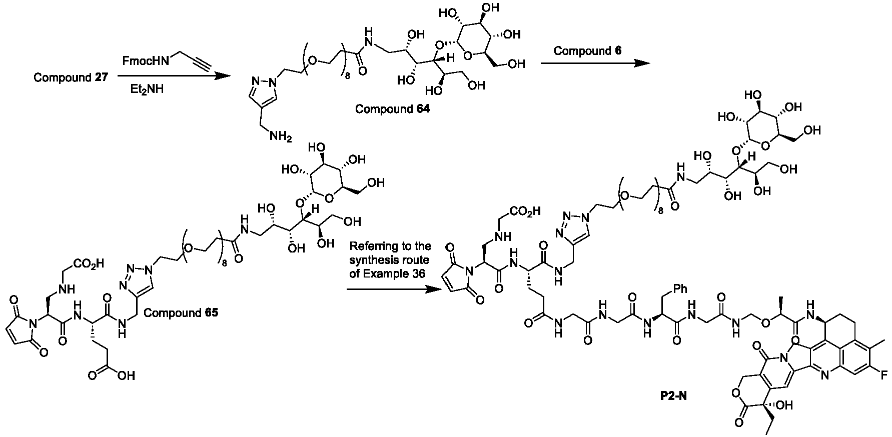

62 mg of compound 64 was prepared according to the synthesis route of compound 30; LC-MS m/z (M+H)⁺:496.5.

23 mg of compound P2-N was prepared according to the synthesis route of Example 40; TOF-MS m/z (M+Na)⁺:1707.7.

### Example 52: Synthesis of compound P2-O

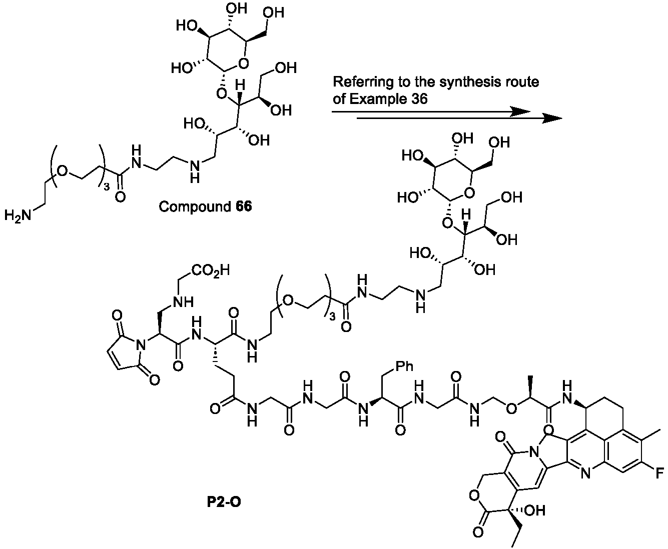

71 mg of compound 66 was prepared according to the synthesis route of compound 32; LC-MS m/z (M+H)⁺:590.6.

34 mg of compound P2-O was prepared according to the synthesis route of Example 40; TOF-MS m/z (M+Na)⁺:1801.9.

### Example 53: Synthesis of compound P2-P

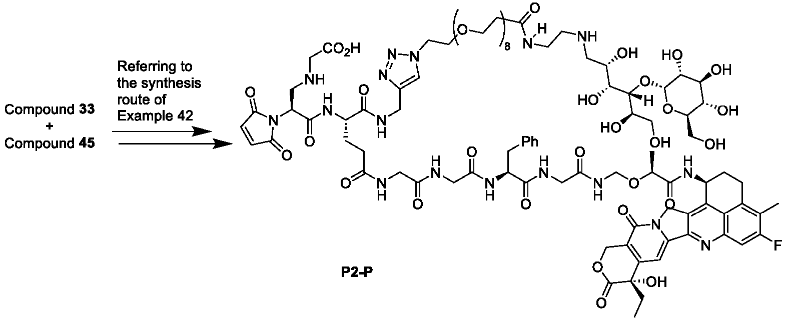

20 mg of compound P2-P was prepared according to the synthesis route of Example 46; TOF-MS m/z (M+H)⁺:2081.1.

### Example 54: Synthesis of compound P2-Q

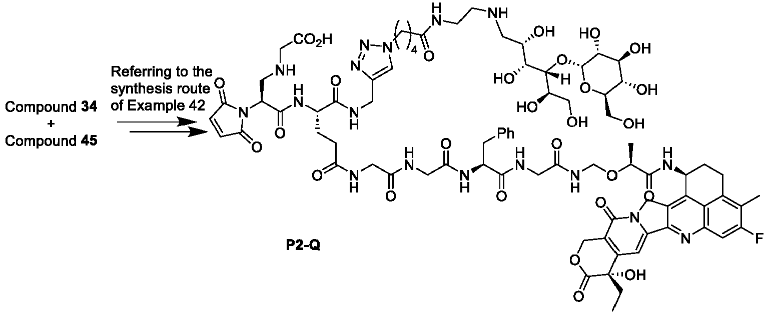

29 mg of compound P2-Q was prepared according to the synthesis route of Example 46; TOF-MS m/z (M+H)⁺:1756.7.

### Example 55: Synthesis of compound P2-R

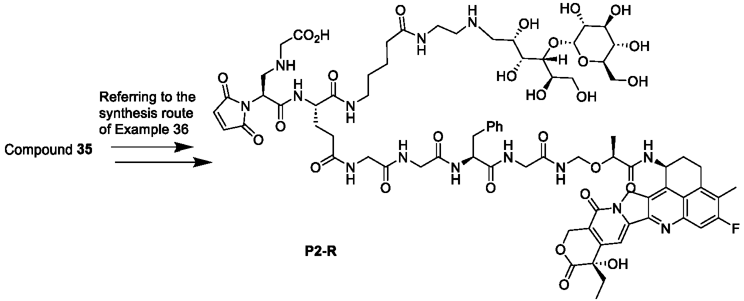

18 mg of compound P2-R was prepared according to the synthesis route of Example 40; TOF-MS m/z (M+H)⁺:1675.9.

### Example 56: Synthesis of compound P2-S

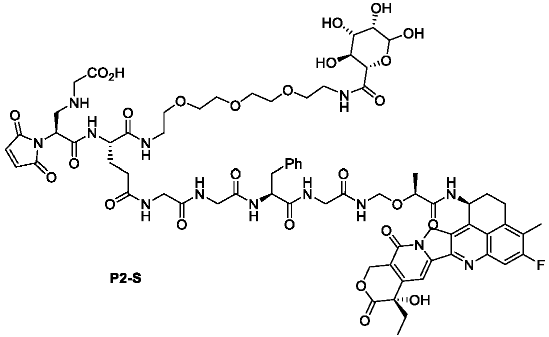

33 mg of compound P2-S was prepared according to the synthesis route of Example 40; TOF-MS m/z (M+H)⁺:1558.9.

### Example 57: Synthesis of compound P2-T

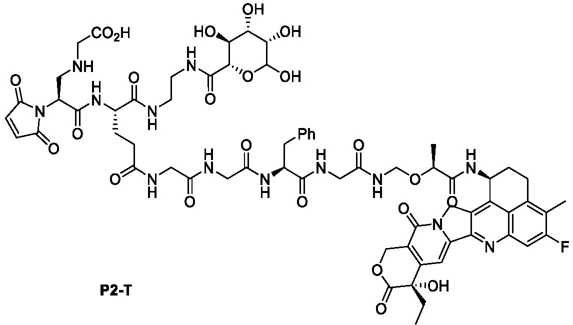

42 mg of compound P2-T was prepared according to the synthesis route of Example 40; TOF-MS m/z (M+H)⁺:1426.8.

### Example 58: Synthesis of compound P2-U

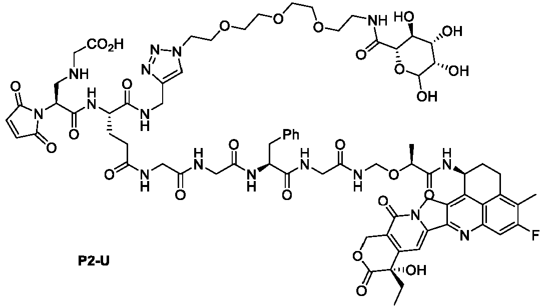

30 mg of compound P2-U was prepared according to the synthesis route of Example 46; TOF-MS m/z (M+H)⁺:1640.1.

### Example 59: Synthesis of compound P2-V

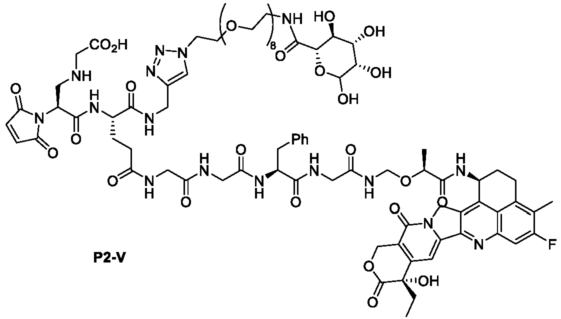

18 mg of compound P2-V was prepared according to the synthesis route of Example 46; TOF-MS m/z (M+H)⁺:1860.0.

### Example 60: Synthesis of compound P2-W

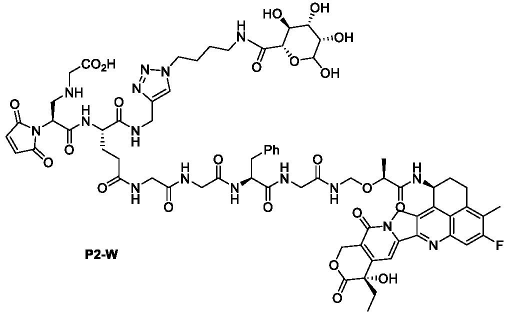

21 mg of compound P2-W was prepared according to the synthesis route of Example 46; TOF-MS m/z (M+H)⁺:1535.8.

### Example 61: Synthesis of compound P2-X2

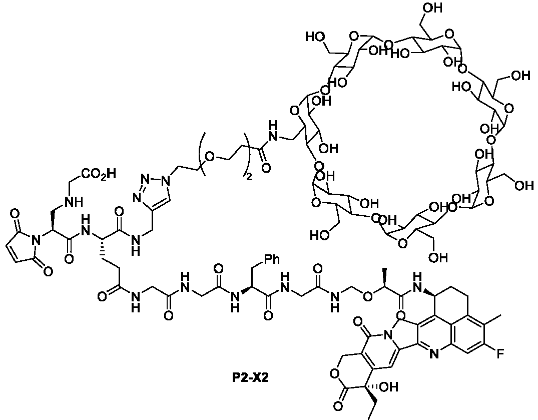

11 mg of compound P2-X2 was prepared according to the synthesis route of Example 37; TOF-MS m/z (M+H)⁺:2563.9238.

### Example 62: Synthesis of compound P2-Y8

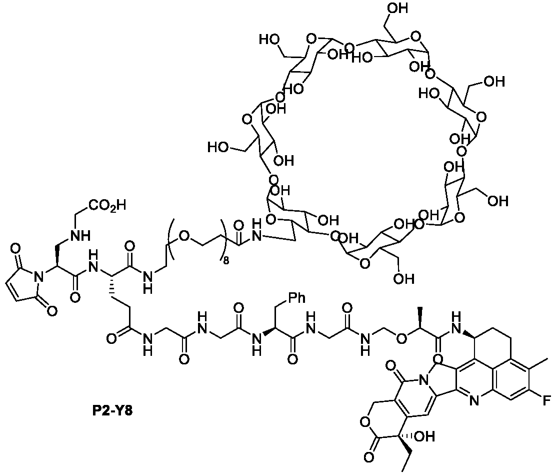

12 mg of compound P2-Y8 was prepared according to the synthesis route of Example 40; TOF-MS m/z (M+H)⁺:2747.0488.

### Example 63: Synthesis of compound P2-Z

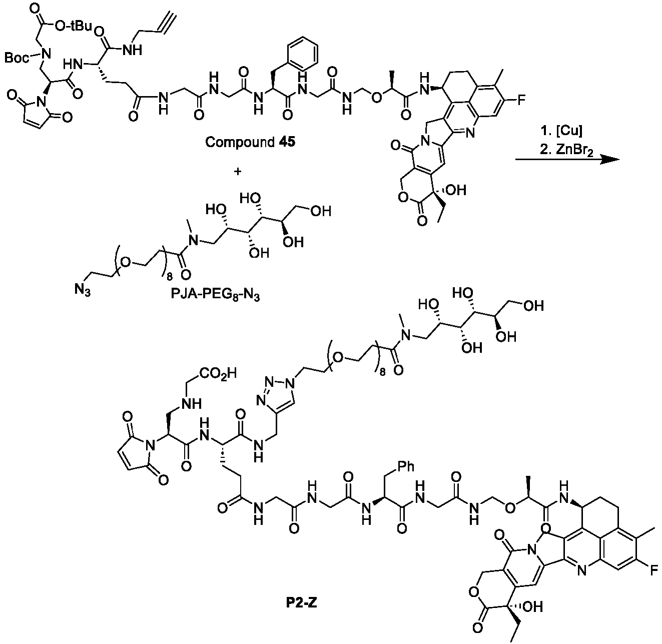

Compound 45 (70 mg, 0.05 mmol) and PJA-PEG8-N3 (32 mg, 0.05 mmol) were mixed and completely dissolved in 5 mL of DMF, followed by addition of cuprous iodide (1 mg, 0.005 mmol) and triethylamine (14 µL, 0.1 mmol), and the resulting reaction solution was heated to 50 °C to react overnight. The reaction process was monitored by HPLC. After the reaction was completed, the reaction solution was purified by preparative high-performance liquid phase chromatography to obtain a preparation solution of the product. The preparation solution was lyophilized to obtain 69 mg of the product.

In a 25 mL single-neck flask, the above compound (69 mg) was dissolved completely in 5 mL of nitromethane, followed by addition of zinc bromide (230 mg, 1 mmol), and the reaction was allowed at room temperature for 1 h. The reaction process was monitored by HPLC. After the reaction was completed, the reaction solution was concentrated at 45 °C under reduced-pressure by a water pumpto obtain a crude product. The crude product was purified by preparative high-performance liquid phase chromatography to obtain a preparation solution of the product. The preparation solution was lyophilized and weighed to obtain 44 mg of compound P2-Z; TOF-MS m/z (M+H)⁺:1889.8077.

### Example 64: Synthesis of compound P-1

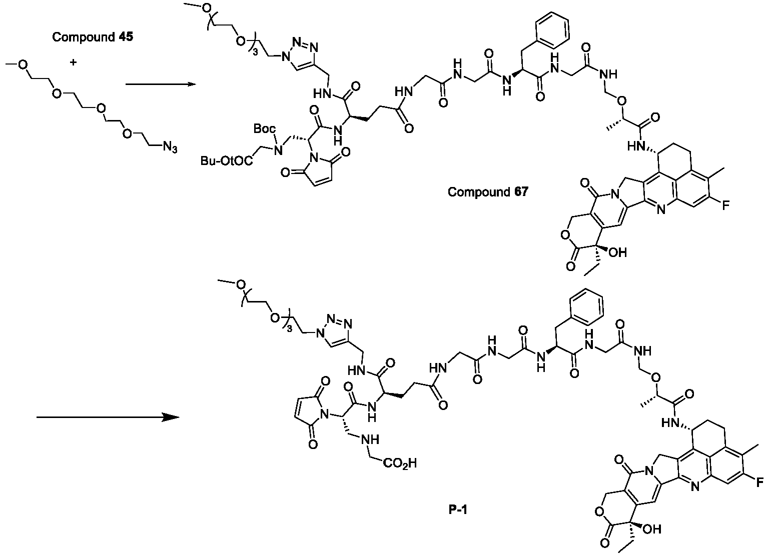

Compound 45 (50 mg, 0.035 mmol) and 1-methoxy-11-azido-3,6,9-trioxane (8.3 mg, 0.035 mmol) were mixed and completely dissolved in 5 mL of DMF, followed by addition of cuprous iodide (1.4 mg, 0.007 mmol) and triethylamine (10 µL, 0.07 mmol), and the resulting reaction solution was heated to 50 °C to react for 10 h. The reaction process was monitored by HPLC. After the reaction was completed, the reaction solution was purified by preparative high-performance liquid phase chromatography to obtain a preparation solution of the product. The preparation solution was lyophilized to obtain 41 mg of the product; TOF-MS m/z (M+H)⁺:1634.7.

In a 25 mL single-neck flask, compound 67 (40 mg, 0.024 mmol) was dissolved completely in 5 mL of nitromethane, followed by addition of zinc bromide (110 mg, 0.48 mmol), and the reaction was allowed at room temperature for 1 h. The reaction process was monitored by HPLC. After the reaction was completed, the reaction solution was concentrated at 45 °C under reduced-pressure by a water pump to obtain a crude product. The crude product was purified by preparative high-performance liquid phase chromatography to obtain a preparation solution of the product. The preparation solution was lyophilized and weighed to obtain 25 mg of compound P-1; TOF-MS m/z (M+H)⁺:1478.6.

### Example 65: Synthesis of compound P-2

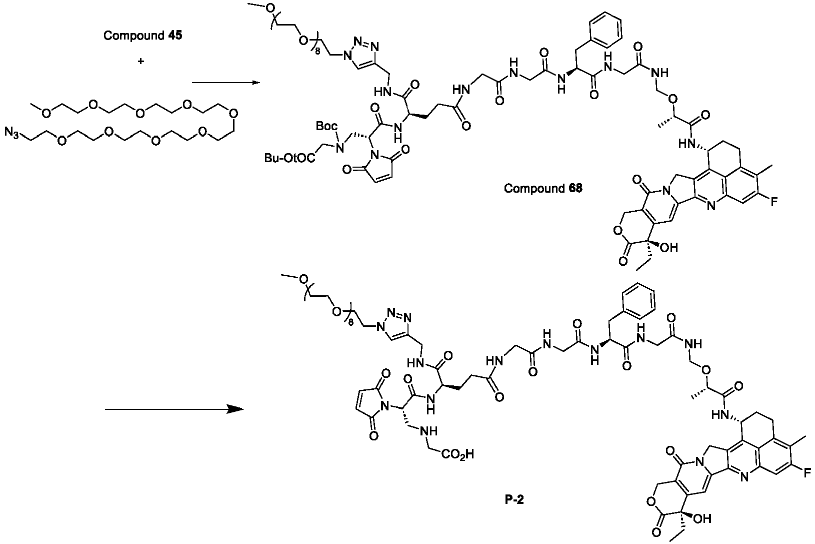

Compound 45 (50 mg, 0.035 mmol) and 28-azido-2,5,8,11,14,17,20,23,26-nonaoxaoctacosane (16.1 mg, 0.035 mmol) were mixed and completely dissolved in 5 mL of DMF, followed by addition of cuprous iodide (1.4 mg, 0.007 mmol) and triethylamine (10 µL, 0.07 mmol). The resulting reaction solution was heated to 50 °C to react for 10 h, and the reaction process was monitored by HPLC. After the reaction was completed, the reaction solution was purified by preparative high-performance liquid phase chromatography to obtain a preparation solution of the product. The preparation solution was lyophilized to obtain 45 mg of the product; TOF-MS m/z (M+H)⁺:1853.8.

In a 25 mL single-neck flask, compound 68 (40 mg, 0.021 mmol) was dissolved completely in 5 mL of nitromethane, followed by addition of zinc bromide (97 mg, 0.43 mmol), and the reaction was allowed at room temperature for 1 h. The reaction process was monitored by HPLC. After the reaction was completed, the reaction solution was concentrated at 45 °C under reduced-pressure by a water pump to obtain a crude product. The crude product was purified by preparative high-performance liquid phase chromatography to obtain a preparation solution of the product. The preparation solution was lyophilized and weighed to obtain 24 mg of compound P-2; TOF-MS m/z (M+H)⁺:1698.7.

### Example 66: Synthesis of compound P-3

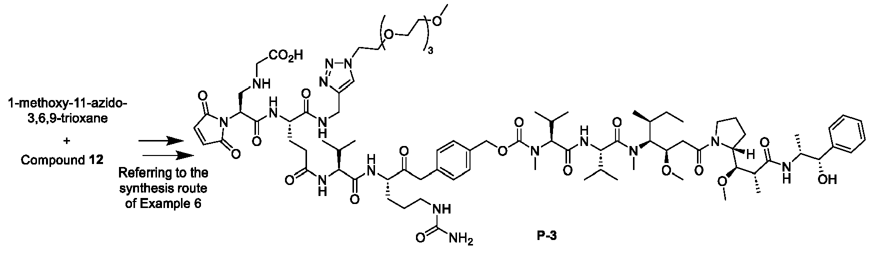

21 mg of compound P-3 was prepared according to the synthesis route of Example 6; TOF-MS m/z (M+H)⁺:1746.1.

### Example 67: Synthesis of compound P-4

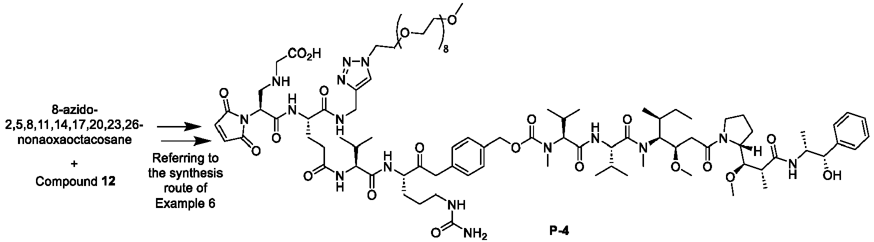

26 mg of compound P-4 was prepared according to the synthesis route of Example 6; TOF-MS m/z (M+H)⁺:1966.3.

### Example 68: Synthesis of compound 69

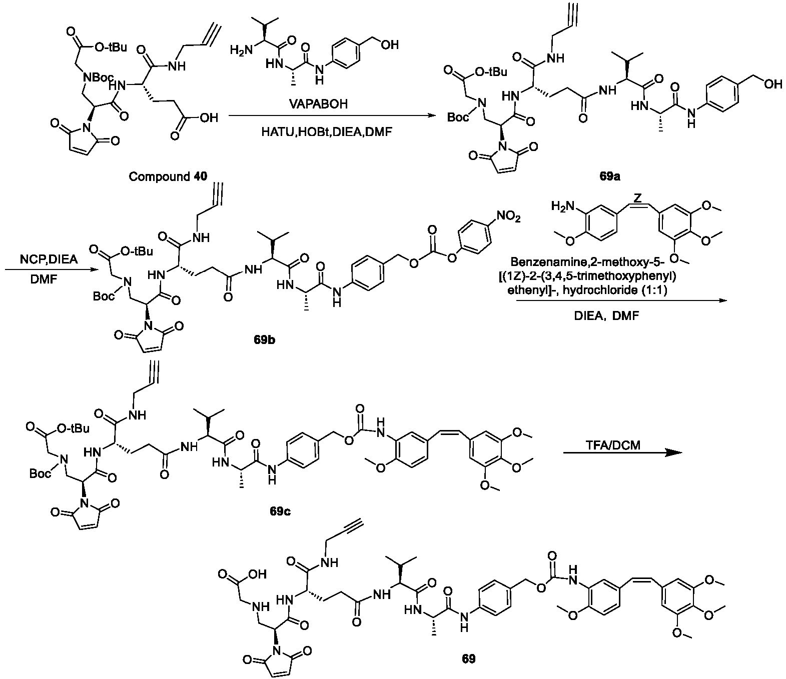

### Step 1: Synthesis of compound 69a

In a 100 mL single-neck flask, compound 40 (10 g, 17.7 mmol) and compound VAPAB-OH (5.19 g, 17.7 mmol) were mixed and dissolved in 30 mL of DMF, followed by addition of HATU (8.08 g, 21.2 mmol), HOBt (2.87 g, 21.2 mmol) and DIEA (4.38 mL, 26.5 mmol), and the reaction was allowed at room temperature for 2 h. After the reaction was found to be completed from the monitoring of HPLC, the reaction solution was purified by preparative liquid phase chromatography to obtain a preparation solution. The preparation solution was lyophilized to obtain compound 69a (9.3 g) with a yield of 62.6%, LCMS: [M+H]⁺=840.4.

### Step 2: Synthesis of compound 69b

In a 100 mL single-neck flask, compound 69a (9 g, 10.7 mmol) and compound NPC (16.2 g, 53.5 mmol) were mixed and dissolved in 30 mL of DMF, followed by addition of DIEA (3.24 mL, 32.1 mmol), and the reaction was allowed at room temperature for 3 h. After the reaction was found to be completed from the monitoring of TLC, the reaction solution was purified by preparative liquid phase chromatography to obtain a preparation solution. The preparation solution was lyophilized to obtain compound 69b (8.23 g) with a yield of 76.5%, LCMS: [M+H]⁺=1005.4.

### Step 3: Synthesis of compound 69c

In a 25 mL single-neck flask, compound 69b (100 mg, 0.099 mmol), benzenamine 2-methoxy-5-[(1Z)-2-(3,4,5-trimethoxyphenyl)ethenyl]-, and hydrochloride (1:1) (31.3 mg, 0.099 mmol) were mixed and dissolved in 5 mL of DMF, followed by addition of DIEA (32.8 µL, 0.199 mmol), and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain compound 69c (75.8 mg) with a yield of 64.5%, LCMS: [M+H]⁺=1181.5.

### Step 4: Synthesis of compound 69

In a 25 mL single-neck flask, compound 69c (75 mg, 0.063 mmol) was dissolved in 2 mL of DCM, followed by addition of 4 mL of TFA, and the reaction was allowed at room temperature for 2 h. After the reaction was found to be completed from the monitoring of HPLC, the solvent was removed at 45 °C under reduced-pressure by a water pump to obtain a residue. The residue was purified by preparative high-performance liquid phase chromatography to obtain a preparation solution. The preparation solution was lyophilized to obtain compound 69 (49.6 mg) with a yield of 76.3%, LCMS: [M+H]⁺= 1025.4.

### Example 69: Synthesis of compound P3-A

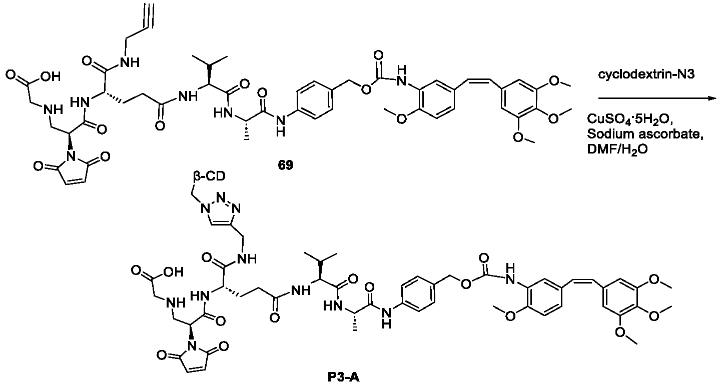

### Step 1: Synthesis of compound P3-A

In a 25 mL single-neck flask, compound 69 (25 mg, 0.024 mmol) and cyclodextrin-N₃ (28.2 mg, 0.024 mmol) were mixed and dissolved in 5 mL of DMF and 2.5 mL of water, followed by addition of CuSO₄·5H₂O (6.7 mg, 0.026 mmol) and sodium ascorbate (5.3 mg, 0.026 mmol), and the reaction was allowed at room temperature for 1 h. After the reaction was found to be completed from the monitoring of HPLC, the reaction solution was purified by preparative high-performance liquid phase chromatography to obtain a preparation solution. The preparation solution was lyophilized to obtain compound P3-A (40.6 mg), TOF: [M+Na]⁺=2206.7.

### Example 70: Synthesis of compound P3-B

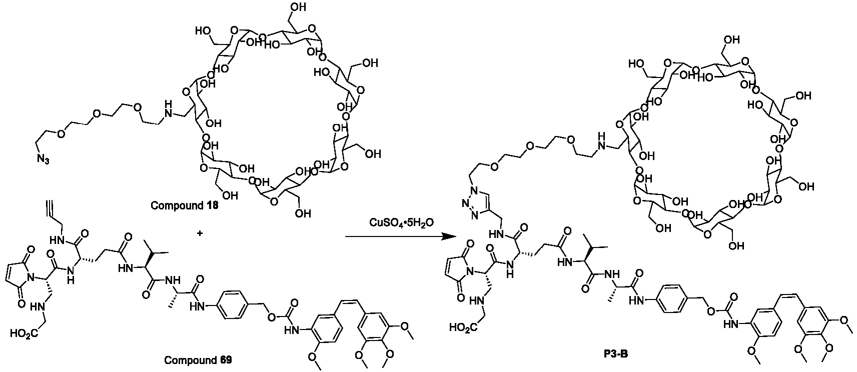

### Step 1: Synthesis of compound P3-B

In a 25 mL single-neck flask, compound 69 (25 mg, 0.024 mmol) and compound 18 (27.2 mg, 0.024 mmol) were mixed and dissolved in 5 mL of DMF and 2.5 mL of water, followed by addition of CuSO₄·5H₂O (6.7 mg, 0.026 mmol) and sodium ascorbate (5.3 mg, 0.026 mmol), and the reaction was allowed at room temperature for 1 h. After the reaction was found to be completed from the monitoring of HPLC, the reaction solution was purified by preparative high-performance liquid phase chromatography to obtain a preparation solution. The preparation solution was lyophilized to obtain compound P3-B (34.5 mg), TOF: [M+H]⁺=2360.1.

### Example 71: Synthesis of compound P3-C

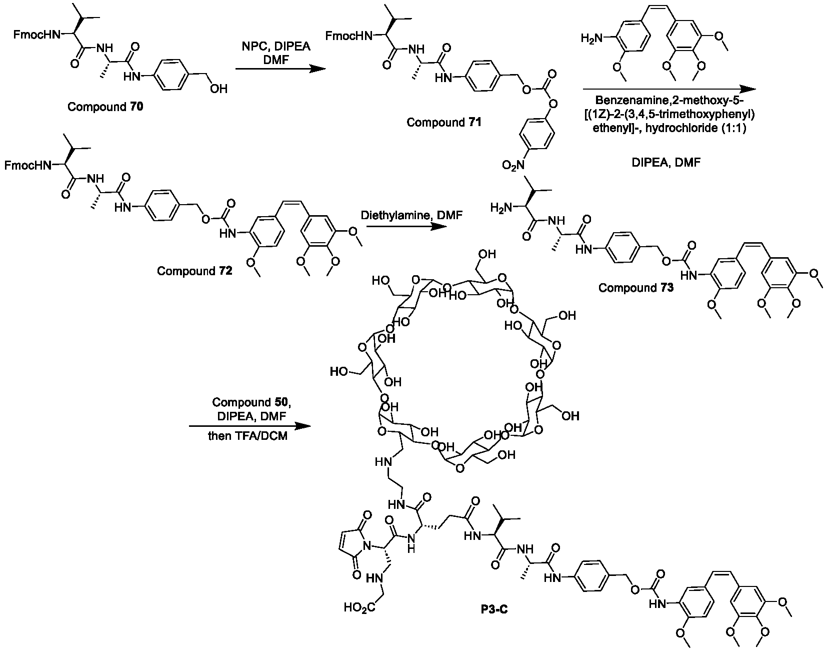

### Step 1: Synthesis of compound 71

In a 100 mL single-neck flask, compound 70 (5 g, 9.7 mmol) and compound NCP (14.7 g, 48.5 mmol) were mixed and dissolved in 30 mL of DMF, followed by addition of DIEA (4.9 mL, 48.5 mmol), and the reaction was allowed at room temperature for 3 h. The reaction end point was monitored by TLC. After the reaction was completed, the reaction solution was purified by preparative liquid phase chromatography to obtain a preparation solution. The preparation solution was lyophilized to obtain compound 71 (7.66 g), LCMS: [M+H]⁺=516.3.

### Step 2: Synthesis of compound 72

In a 25 mL single-neck flask, compound 71 (4 g, 7.76 mmol), benzenamine, 2-methoxy-5-[(1Z)-2-(3,4,5-trimethoxyphenyl)ethenyl]-, and hydrochloride (1:1) (2.45 g, 7.76 mmol) were mixed and dissolved in 15 mL of DMF, followed by addition of DIEA (2.56 mL, 15.5 mmol), and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain compound 72 (4.1 g), LCMS: [M+H]⁺=857.5.

### Step 3: Synthesis of compound 73

In a 50 mL single-neck flask, compound 72 (4 g) was dissolved in 27 mL of DMF, followed by addition of diethylamine (3 mL), and the reaction was allowed at room temperature for 4 h. After the reaction was found to be completed from the monitoring of HPLC, the reaction solution was concentrated under reduced pressure by an oil pump. The obtained yellow oil was purified by preparative high-performance liquid phase chromatography to obtain a preparation solution. The preparation solution was lyophilized to obtain compound 73 (2.8 g), LCMS: [M+H]⁺=635.5.

### Step 4: Synthesis of compound P3-C

In a 25 mL single-neck flask, compound 73 (80 mg, 0.126 mmol) was dissolved in 5 mL of DMF, followed by addition of DIPEA (42 µL,0.252 mmol) and compound 50 (0.126 mmol), and the reaction was allowed at room temperature for 1 h. After the reaction was found to be completed from the monitoring of HPLC, the reaction solution was concentrated at 45 °C under reduced-pressure by a water pump to remove the solvent, the resulting residue was directly put into the next reaction.

In a 25 mL single-neck flask, the above crude compound was dissolved in 2 mL of DCM, followed by addition of 4 mL of TFA, and the reaction was allowed at room temperature for 0.5 h. After the reaction was found to be completed from the monitoring of HPLC, the reaction solution was concentrated at 45 °C under reduced-pressure by a water pump to remove the solvent to obtain a residue. The residue was purified by preparative high-performance liquid phase chromatography to obtain a preparation solution. The preparation solution was lyophilized to obtain compound P3-C (37.7 mg), TOF: [M+H]⁺= 2146.8.

### Example 72: Synthesis of compound P3-D

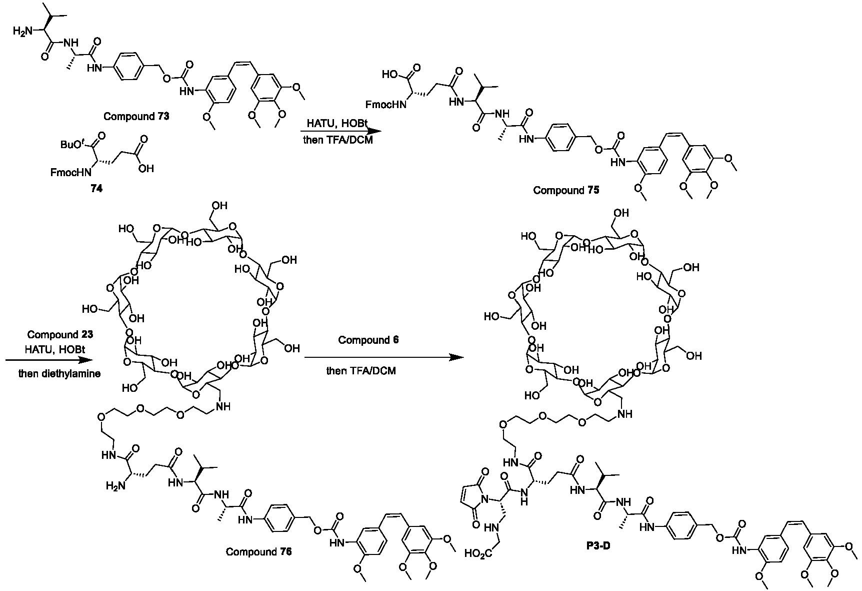

### Step 1: Synthesis of compound 75

In a 25 mL single-neck flask, compound 73 (200 mg, 0.315 mmol) and 10 mL of DMF were added, then compound 74 (134 mg, 0.315 mmol) was added at room temperature, HATU (240 mg, 0.62 mmol), HOBt (84 mg, 0.62 mmol) and DIPEA (0.12 mL) were added in sequence under the cooling of an ice water bath, and the resulting reaction solution was slowly brought naturally to room temperature to have a reaction for 3 h. After the reaction was found to be completed from the monitoring of TLC, rotary evaporation under reduced pressure was performed to remove the solvent. The concentrated residual was a pale yellow oily rude product. The crude product was directly put into the next reaction without purification.

The above compound was dissolved in 3 mL of dichloromethane, the resulting solution was cooled to 0 °C in condition of an ice water bath, TFA(1.5 mL) was slowly added dropwise. The resulting reaction solution was slowly brought naturally to room temperature to react for 5 h, and the reaction was monitored by HPLC. After the reaction was completed, the reaction solution was concentrated at 45 °C under reduced-pressure to obtain a yellow oily crude product compound. The residue was purified by preparative high-performance liquid phase chromatography to obtain a preparation solution. The preparation solution was lyophilized to obtain compound 75 (162.0 mg), LC-MS: [M+H]⁺= 986.6.

### Step 2: Synthesis of compound 76

In a 25 mL single-neck flask, compound 75 (80 mg, 0.081 mmol) and 10 mL of DMF were added, then compound 23 (106 mg, 0.081 mmol) was added at room temperature, HATU (94 mg, 0.0.243 mmol), HOBt (16.5 mg, 0.122 mmol) and DIPEA (14 µL) were added in sequence under the cooling of an ice water bath to have a reaction for 2 h. After the reaction was found to be completed from the monitoring of HPLC, the reaction solution was purified by preparative high-performance liquid phase chromatography to obtain a preparation solution. The preparation solution was lyophilized to obtain an intermediate compound which was directly put into the next reaction.

The above intermediate was dissolved in 3 mL of DMF, diethylamine (0.3 mL) was added dropwise at room temperature to have a reaction for 6 h, and the reaction was monitored by HPLC. After the reaction was completed, the reaction solution was purified by preparative high-performance liquid phase chromatography to obtain a preparation solution. The preparation solution was lyophilized to obtain compound 76 (123.0 mg), TOF: [M+H]⁺=2055.1.

### Step 3: Synthesis of compound P3-D

In a 10 mL PE tube, compound 76 (100 mg, 0.042 mmol) and 3 mL of DMF were added, then compound 6 (17 mg, 0.042 mmol) was added at room temperature, HATU (34 mg, 0.084 mmol), HOBt (12.5 mg, 0.084 mmol) and DIPEA (22 µL) were added in sequence under the cooling of an ice water bath, and the reaction was allowed at room temperature for 1 h. After the reaction was found to be completed from the monitoring of HPLC, rotary evaporation under reduced pressure was performed using an oil pump to remove the solvent, the residual was concentrated to obtain a pale yellow oily rude product. The crude product was directly put into the next reaction without purification.

The above crude product was dissolved in 2 mL of dichloromethane, the resulting solution was cooled to 0 °C in condition of an ice water bath, TFA (2 mL) was slowly added dropwise and then the solution was slowly brought naturally to room temperature to react for 2 h. The reaction was monitored by HPLC. After the reaction was completed, the reaction solution was concentrated at 45 °C under reduced-pressure to obtain a yellow oily crude product compound; the residue was purified by preparative high-performance liquid phase chromatography to obtain a preparation solution. The preparation solution was lyophilized to obtain compound P3-D (52.0 mg), TOF: [M+Na]⁺= 2301.1.

### Example 73: Synthesis of compound P3-E

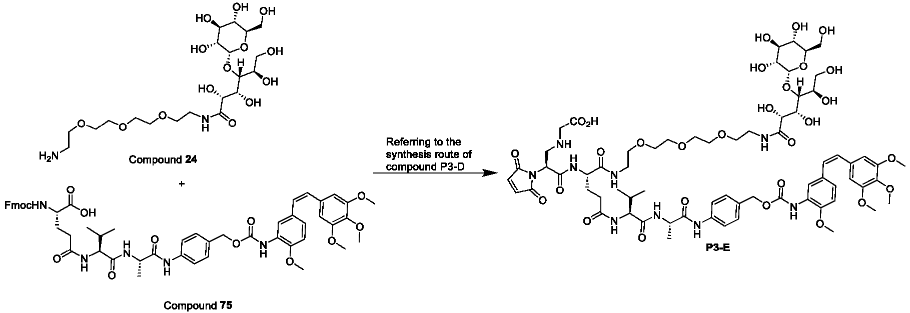

Taking of compound 75 and compound 24 as starting raw materials, compound P3-E (44 mg) was prepared according to the synthesis route of compound P3-D, LC-MS: [M+H]⁺= 1502.8.

### Example 74: Synthesis of compound P3-F

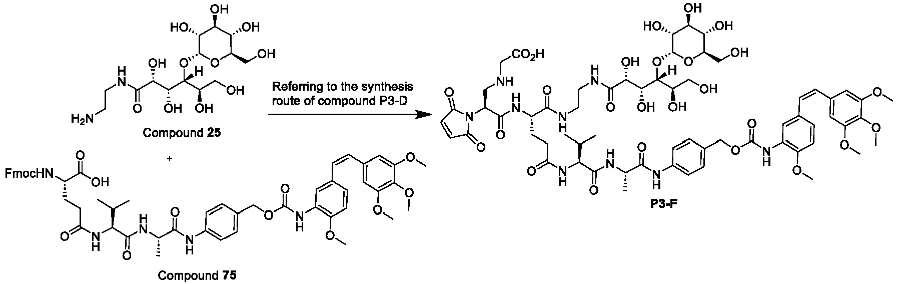

Taking of compound 75 and compound 25 as starting raw materials, compound P3-F (26 mg) was prepared according to the synthesis route of compound P3-D, LC-MS: [M+H]⁺= 1370.5.

### Example 75: Synthesis of compound P3-G

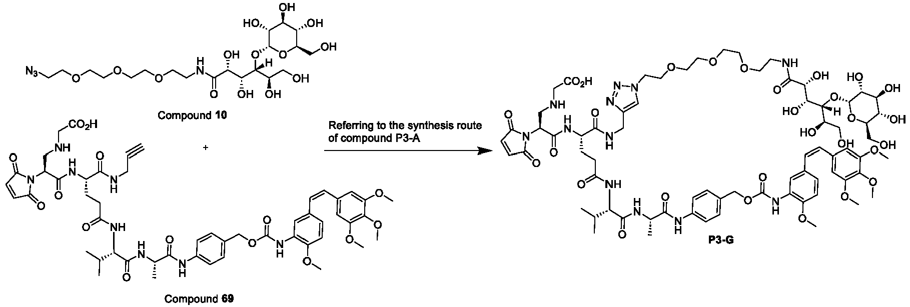

Taking of compound 69 and compound 10 as starting raw materials, compound P3-G (71 mg) was prepared according to the synthesis route of compound P3-A, TOF: [M+H]⁺= 1583.6.

### Example 76: Synthesis of compound P3-H

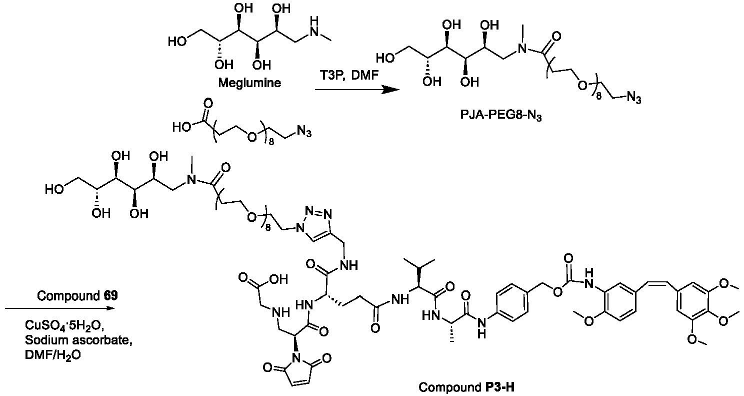

### Step 1: Synthesis of compound PJA-PEG8-N3

In a 25 mL single-neck flask, meglumine (50 mg, 0.256 mmol), COOH-PEG-8-N3 (150 mg, 0.256 mmol) and 5 mL of DMF were added, then T3P (244 mg, 0.384 mmol, 50% in total) was added at room temperature, and the reaction was allowed at room temperature for 5 h. After the reaction was found to be completed from the monitoring of HPLC, the reaction solution was purified by preparative high-performance liquid phase chromatography to obtain a preparation solution. The preparation solution was lyophilized to obtain compound PJA-PEG8-N3 (35 mg), LC-MS: [M+H]⁺=645.7.

### Step 2: Synthesis of compound P3-H

In a 25 mL single-neck flask, compound 69 (25 mg, 0.024 mmol) and PJA-PEG₈₋N₃ (15.7 mg, 0.024 mmol) were mixed and dissolved in 5 mL of DMF and 2.5 mL of water, followed by addition of CuSO₄·5H₂O (6.7 mg, 0.026 mmol) and sodium ascorbate (5.3 mg, 0.026 mmol), and the reaction was allowed at room temperature for 1 h. After the reaction was found to be completed from the monitoring of HPLC, the reaction solution was purified by preparative high-performance liquid phase chromatography to obtain a preparation solution. The preparation solution was lyophilized to obtain compound 2 (35 mg) with a yield of 78.6%, TOF: [M+H]⁺=1669.7.

### Example 77: Synthesis of compound P3-I

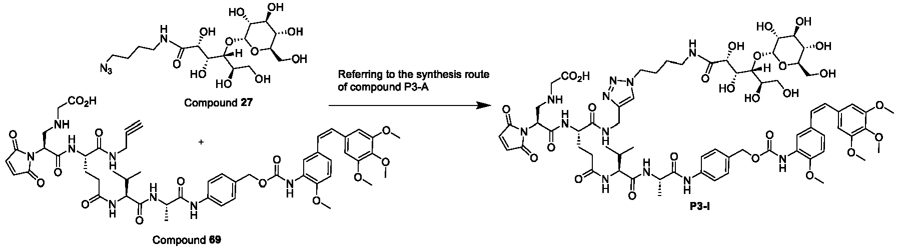

Taking of compound 69 and compound 27 as starting raw materials, compound P3-I (28 mg) was prepared according to the synthesis route of compound P3-A, LC-MS: [M+H]⁺= 1479.8.

### Example 78: Synthesis of compound P3-J

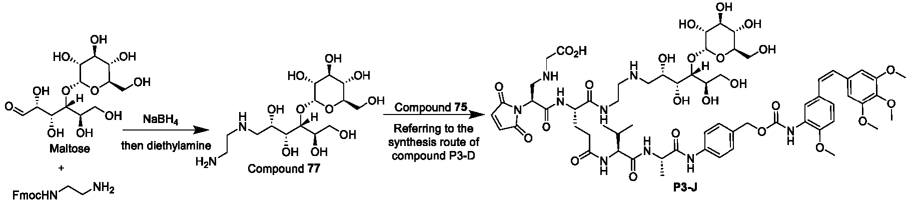

### Step 1: Synthesis of compound 77

In a 25 mL single-neck flask, maltose (2 g, 5.85 mmol) and Fmoc ethylenediamine (1.7 g, 6 mmol) were mixed and dissolved in 10 mL of anhydrous tetrahydrofuran, then the resulting solution was cooled to 0 °C in condition of an ice water bath, followed by addition of sodium borohydride (240 mg, 6 mmol), and the resulting reaction solution was slowly brought naturally to room temperature to have a reaction for 1 h. After the reaction was found to be completed from the monitoring of HPLC, 10 mL of acetone was added with cooling in ice-water bath to quench the reaction. Filtering was performed with diatomaceous earth. The filter cake was rinsed with 200 mL of methanol, and the reaction solution was concentrated under reduced-pressure and directly put into the next reaction.

In a 25 mL single-neck flask, the above crude compound was dissolved in 8 mL of DMF, then diethylamine (2 mL) was added dropwise at room temperature, and the reaction was allowed at room temperature for 5 h. After the reaction was found to be completed from the monitoring of HPLC,, the reaction solution was purified by preparative high-performance liquid phase chromatography to obtain a preparation solution. The preparation solution was lyophilized to obtain compound 77 (1.1 g), LC-MS: [M+H]⁺=387.4.

### Step 2: Synthesis of compound P3-J

Taking of compound 75 and compound 77 as starting raw materials, compound P3-J (45 mg) was prepared according to the synthesis route of compound P3-D, LC-MS: [M+H]⁺= 1356.9.

### Example 79: Synthesis of compound P3-K

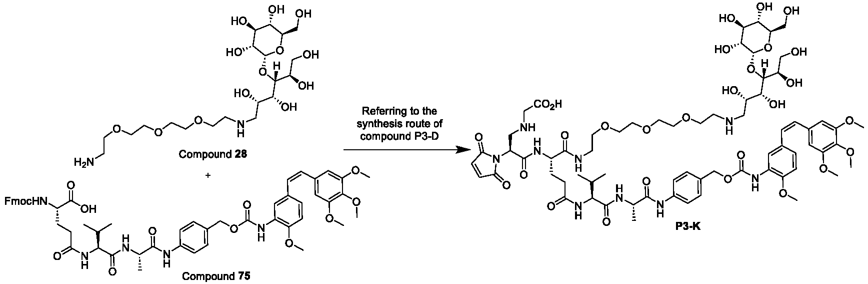

Taking of compound 75 and compound 28 as starting raw materials, compound P3-K (39 mg) was prepared according to the synthesis route of compound P3-D, LC-MS: [M+H]⁺= 1488.4.

### Example 80: Synthesis of compound P3-L

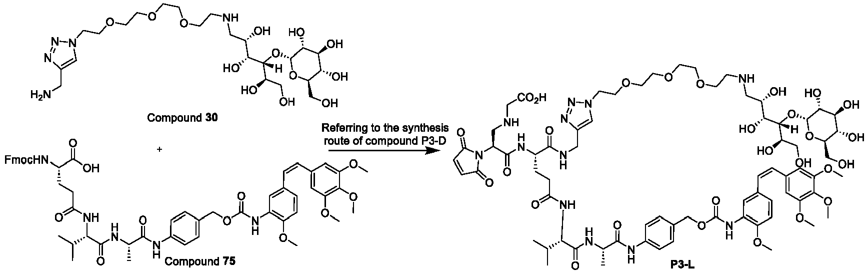

Taking of compound 75 and compound 30-PEG3 as starting raw materials, compound P3-L (22 mg) was prepared according to the synthesis route of compound P3-D, LC-MS: [M+H]⁺= 1569.6.

### Example 81: Synthesis of compound P3-M

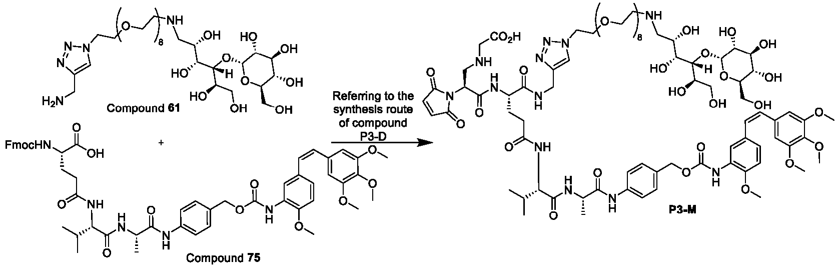

Taking of compound 75 and compound 61 as starting raw materials, compound P3-M (29 mg) was prepared according to the synthesis route of compound P3-D, TOF: [M+H]⁺= 1790.1.

### Example 82: Synthesis of compound P3-N

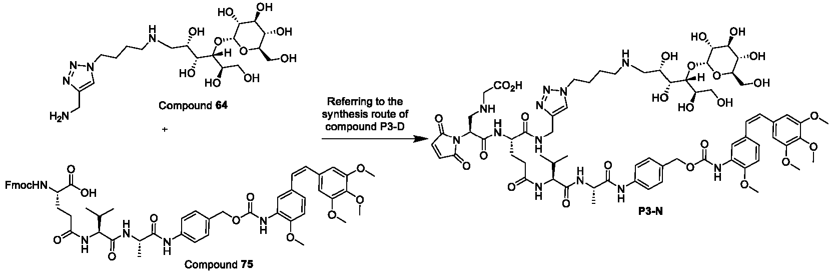

Taking of compound 75 and compound 64 as starting raw materials, compound P3-N (24 mg) was prepared according to the synthesis route of compound P3-D, TOF: [M+H]⁺= 1465.7.

### Example 83: Synthesis of compound P3-O

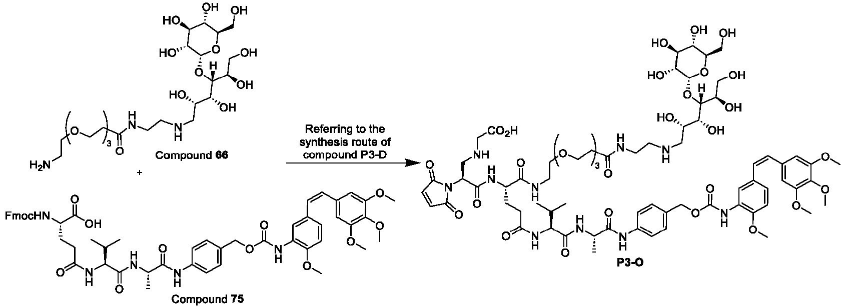

Taking of compound 75 and compound 66 as starting raw materials, compound P3-O (31 mg) was prepared according to the synthesis route of compound P3-D, TOF: [M+H]⁺= 1759.6.

### Example 84: Synthesis of compound P3-P

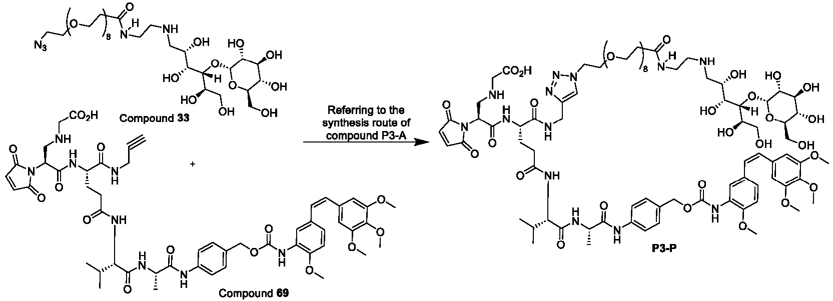

Taking of compound 69 and compound 33 as starting raw materials, compound P3-P (41 mg) was prepared according to the synthesis route of compound P3-A, TOF: [M+H]⁺= 1861.1.

### Example 85: Synthesis of compound P3-Q

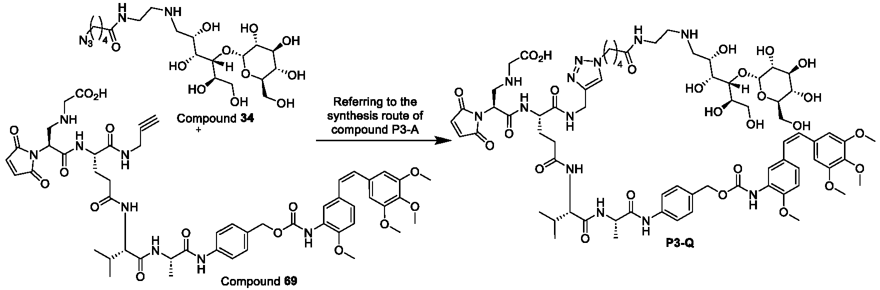

Taking of compound 69 and compound 34 as starting raw materials, compound P3-Q (36 mg) was prepared according to the synthesis route of compound P3-A, TOF: [M+H]⁺= 1861.1.

### Example 86: Synthesis of compound P3-R

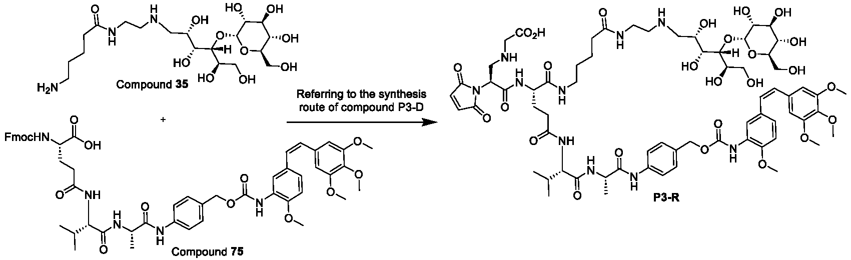

Taking of compound 75 and compound 35 as starting raw materials, compound P3-R (18 mg) was prepared according to the synthesis route of compound P3-D, TOF: [M+H]⁺= 1455.9.

### Example 87: Synthesis of compound P3-S

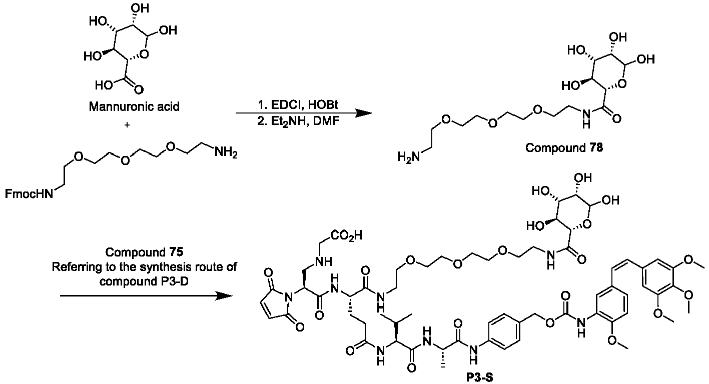

### Step 1: Synthesis of compound 78

In a 100 mL single-neck flask, mannuronic acid (5 g, 25.8 mmol) and propargylamine (11.1' g, 25.8 mmol) were mixed and completely dissolved in 50 mL of DMF, the resulting solution was cooled to 0 °C in condition of an ice water bath, EDCI (9.9 g, 51.6 mmol), HOBt (7 g, 51.6 mmol) and DIEA (12.7 mL, 77.4 mmol) were then added, and the resulting solution was brought naturally to room temperature to react for 2 h. After the reaction was found to be completed from the monitoring of TLC, the reaction solution was poured into 100 mL of water, extracted three times with ethyl acetate (100 mL*3); the organics were combined, washed twice with saturated NaCl solution, and dried with anhydrous Na₂SO₄ to obtain a crude compound, which was directly put into the next reaction.

In a 250 mL single-neck flask, the above crude compound was added and dissolved in 50 mL of DMF, followed by addition of 10 mL of diethylamine, and the reaction was allowed at room temperature for 2 h. After the reaction was found to be completed from the monitoring of TLC, 500 mL of methyl tert-ether was added to the reaction solution to obtain white solid precipitate. The precipitate was then separated from the solution by filtration and dried to obtain compound 78 (3.96 g), LC-MS: [M-H]⁺=369.5.

### Step 2: Synthesis of compound P3-S

Taking of compound 75 and compound 78 as starting raw materials, compound P3-S (24 mg) was prepared according to the synthesis route of compound P3-D, TOF: [M+H]⁺= 1338.4.

### Example 88: Synthesis of compound P3-T

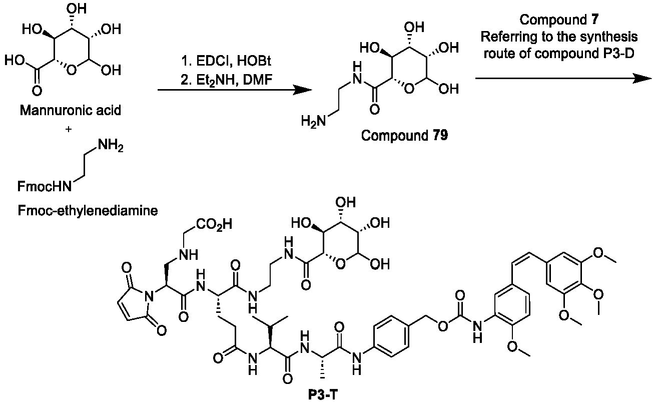

### Step 1: Synthesis of compound 79

13.2 g of compound 79 was prepared according to the synthesis route of compound 78, LC-MS: [M-H]⁺=237.5.

### Step 2: Synthesis of compound P3-T

Taking of compound 75 and compound 79 as starting raw materials, compound P3-T (44 mg) was prepared according to the synthesis route of compound P3-D, TOF: [M+H]⁺= 1206.6.

### Example 89: Synthesis of compound P3-U

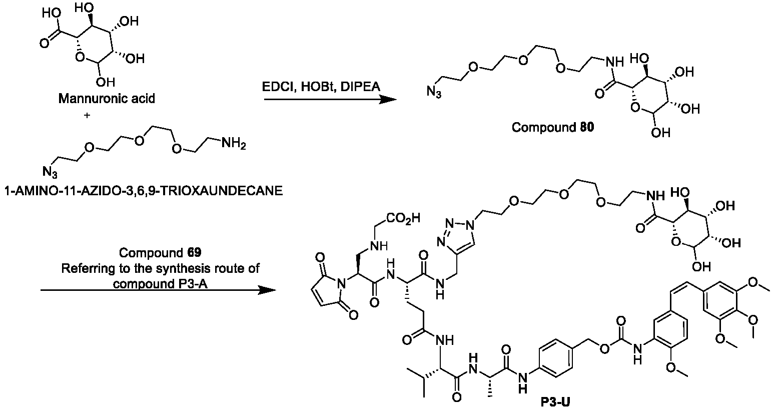

### Step 1: Synthesis of compound 80

In a 50 mL single-neck flask, mannuronic acid (1 g, 5.2 mmol) and propargylamine (2.2 g, 5.2 mmol) were mixed and completely dissolved in 10 mL of DMF, the resulting solution was cooled to 0 °C in condition of an ice water bath, EDCI (2.0 g, 10.3 mmol), HOBt (1.4 g, 10.3 mmol) and DIEA (2.6 mL, 15.5 mmol) were added, and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain compound 80 (0.97 g), LC-MS: [M+H]⁺=395.6.

### Step 2: Synthesis of compound P3-U

Taking of compound 69 and compound 80 as starting raw materials, compound P3-U (16 mg) was prepared according to the synthesis route of compound P3-A, TOF: [M+H]⁺= 1419.8.

### Example 90: Synthesis of compound P3-V

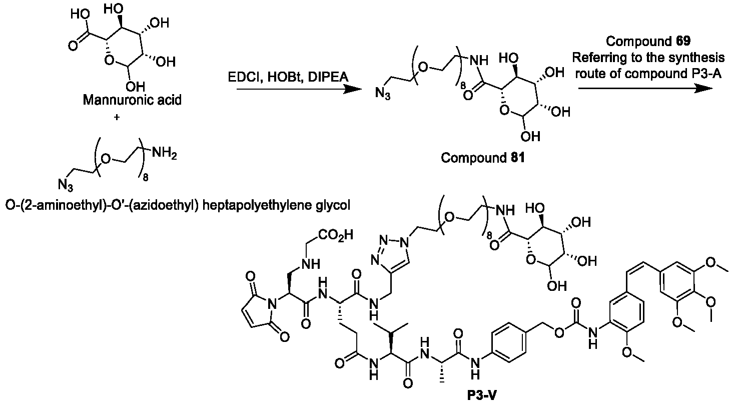

### Step 1: Synthesis of compound 81

Compound 81 (0.49 g) was prepared according to the synthesis route of compound 80, LC-MS: [M+H]⁺=615.4.

### Step 2: Synthesis of compound P3-V

Taking of compound 69 and compound 81 as starting raw materials, compound P3-V (30 mg) was prepared according to the synthesis route of compound P3-A, TOF: [M+H]⁺= 1639.9.

### Example 91: Synthesis of compound P3-W

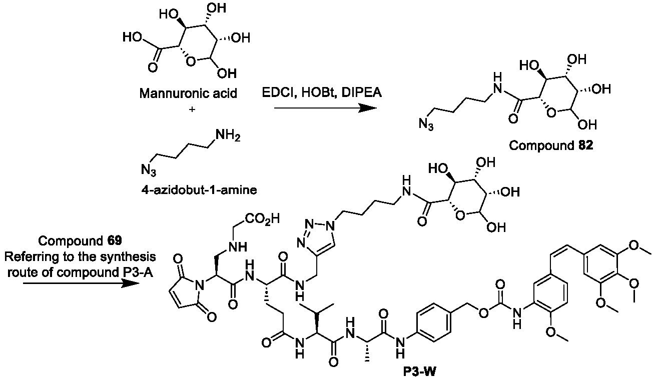

### Step 1: Synthesis of compound 82

Compound 82 (2.17 g) was prepared according to the synthesis route of compound 80, LC-MS: [M+H]⁺=291.1.

### Step 2: Synthesis of compound P3-W

Taking of compound 69 and compound 82 as starting raw materials, compound P3-W (35 mg) was prepared according to the synthesis route of compound P3-A, TOF: [M+H]⁺= 1315.8.

### Example 92: Synthesis of compound P3-X2

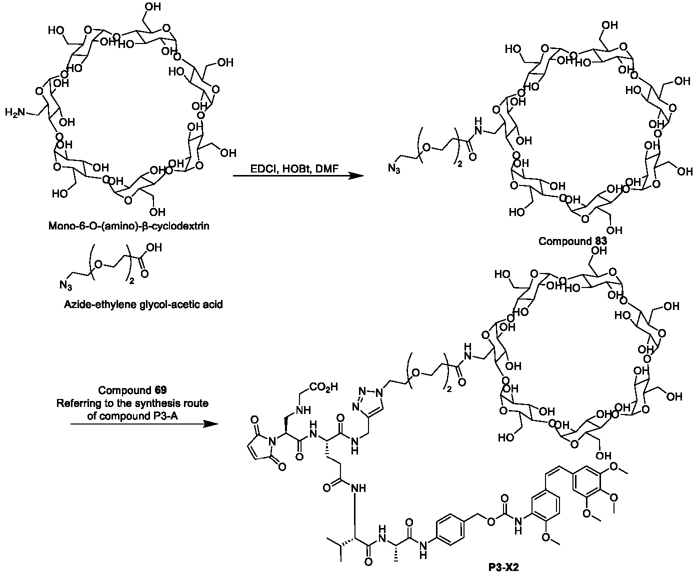

### Step 1: Synthesis of compound 83

In a 25 mL single-neck flask, mono-6-O-(amino)-β-cyclodextrin (1 g, 0.88 mmol) and azide-ethylene glycol-acetic acid (180 mg, 0.88 mmol) were mixed and completely dissolved in 5 mL of DMF. The resulting solution was cooled to about 0 °C in condition of an ice water bath. EDCI (386 mg, 2 mmol), HOBt (276 mg, 2 mmol) and DIEA (0.66 mL, 4 mmol) were then added, and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain compound 83 (0.66 g), LC-MS: [M+H]⁺=1319.6.

### Step 2: Synthesis of compound P3-X2

Taking of compound 69 and compound 83 as starting raw materials, compound P3-X2 (17 mg) was prepared according to the synthesis route of compound P3-A, TOF: [M+Na]⁺= 2365.9.

### Example 93: Synthesis of compound P3-Y8

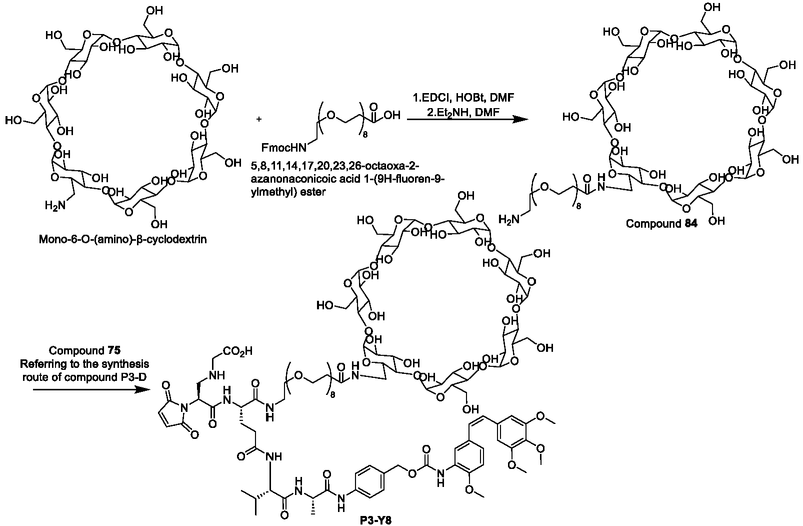

### Step 1: Synthesis of compound 84

In a 25 mL single-neck flask, mono-6-O-(amino)-β-cyclodextrin (1 g, 0.88 mmol), 5,8,11,14,17,20,23,26-octaoxa-2-azanonaconicoic acid 1-(9H-fluoren-9-ylmethyl) ester (585.1 mg, 0.88 mmol) was completely dissolved in 5 mL of DMF. The resulting solution was cooled to about 0 °C in condition of an ice water bath, EDCI (388 mg, 2 mmol), HOBt (275 mg, 2 mmol) and DIEA (0.66 mL, 4 mmol) were added, and the reaction was allowed at room temperature for 2 h. After the reaction was found to be completed from the monitoring of HPLC, the reaction solution was concentrated under reduced pressure by an oil pump and directly put into the next reaction.

In a 25 mL single-neck flask, the above crude compound was added and dissolved in 10 mL of DMF, followed by addition of 2 mL of diethylamine, and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain compound 84 (0.87 g), TOF: [M+H]⁺=1557.5.

### Step 2: Synthesis of compound P3-Y8

Taking of compound 75 and compound 84 as starting raw materials, compound P3-Y8 (21 mg) was prepared according to the synthesis route of compound P3-D, TOF: [M+Na]⁺= 2549.1.

### Example 94: Synthesis of compound P3-Z4

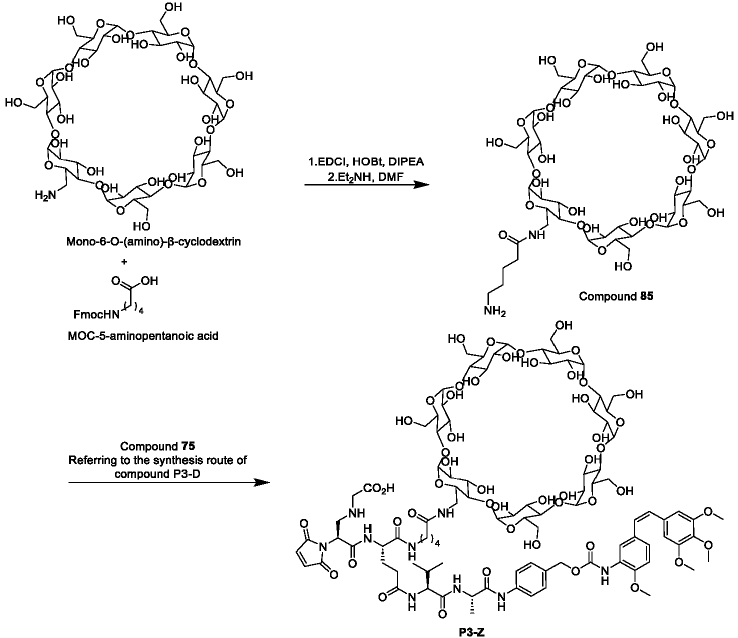

### Step 1: Synthesis of compound 85

Compound 85 (0.91 g) was prepared according to the synthesis route of compound 84, LC-MS: [M+H]⁺=1233.3.

### Step 2: Synthesis of compound P3-Y8

Taking of compound 75 and compound 85 as starting raw materials, compound P3-Z4 (33 mg) was prepared according to the synthesis route of compound P3-D, TOF: [M+H]⁺= 2203.1.

### Example 95: Synthesis of compound 86

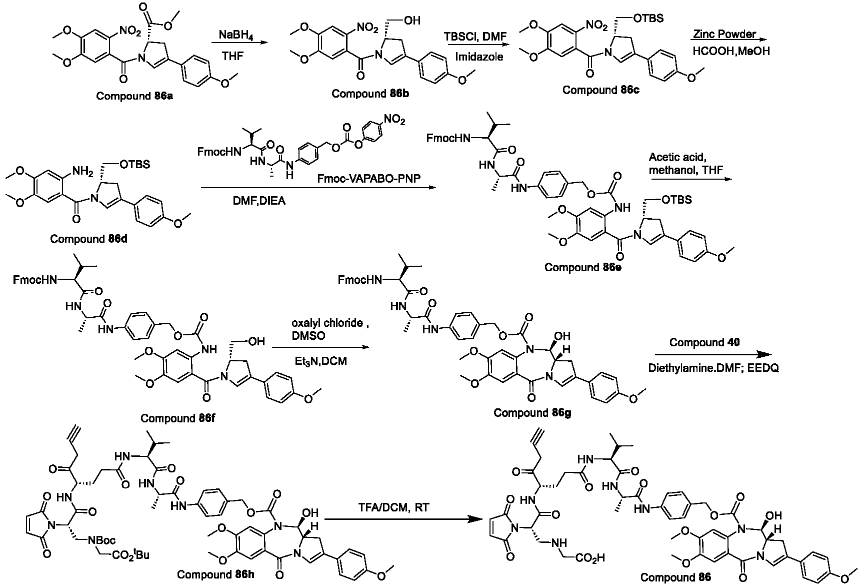

### Step 1: Synthesis of compound 86b

Referring to the synthesis of "compound 26" in patent application CN102933236A to obtain compound 86a. In a 250 mL three-necked flask, compound 86a (3 g, 6.7 mmol) and 50 mL of anhydrous THF were added in the presence of N₂, maintain the temperature of 0 °C with an ice water bath, and solid sodium borohydride (384 mg, 10.2 mmol) was added. The reaction solution reacted at 0 °C for 30 min, then brought naturally to room temperature to react for 2 h. After the reaction was found to be completed from the monitoring of TLC, the reaction solution was cooled by ice water, 50 mL of water was slowly added to quench the reaction, and 1 N dilute HCl was added until no bubbles were observed. The resulting solution was extracted with ethyl acetate (60 mL*3); the organics were combined, washed twice with saturated NaCl solution, dried with anhydrous Na₂SO₄, filtered, and concentrated to obtain a crude product. The rude product was purified by column chromatography (DCM/MeOH=20:1-5:1) to obtain compound 86b (2.08 g, yellow solid) with a yield of 74.3%, LC-MS: [M+H]⁺=415.1.

### Step 2: Synthesis of compound 86c

In a 100 mL single-neck flask, compound 86b (2 g, 4.8 mmol), TBSCl (1.32 g, 8.8 mmol), and imidazole (1.2 g, 17.6 mmol) were mixed and dissolved in 15 mL of anhydrous DMF, and the reaction was allowed at room temperature for 3 h. After the reaction was found to be completed from the monitoring of TLC, the reaction solution was poured into 30 mL of water, extracted with DCM (25 mL*3); the organics were combined, washed with water and salt water, dried with anhydrous Na₂SO₄ and concentrated by evaporation under reduced pressure to obtain a crude product. The crude product was subject to column chromatography (PE/EA=5:1-1:2) to obtain compound 86c (2.28 g, yellow solid) with a yield of 89.3%, LC-MS: [M+H]⁺=529.2.

### Step 3: Synthesis of compound 86d

In a 100 mL single-neck flask, compound 3f (2.2 g, 4.16 mmol) and 30 mL of 5% formic acid/methanol solution were mixed and cooled to below 5 °C in an ice bath, Zinc powder (5.44 g, 83.2 mmol) was slowly added with stirring, and the reaction was allowed at room temperature for 1 h. After the reaction was found to be completed from the monitoring of TLC, the reaction solution was filtered when the reaction solution was hot. The filter cake was washed with a small amount of methanol. The filtrated was adjusted the pH to 7 with saturated sodium bicarbonate solution, and then concentrated under reduced pressure at 45 °C to remove the solvent to obtain a brown oil. 60 mL of DCM was added and the organic layer was separated, washed one time with saturated salt solution, dried with anhydrous Na₂SO₄, filtered, and concentrated at 45 °C under reduced-pressure to obtain a rude product. The rude product was purified by column chromatography (PE: EA=1:1-1:3) to obtain compound 86d (1.87 g, pale yellow solid) with a yield of 90.1%, LC-MS: [M+H]⁺=500.2.

### Step 4: Synthesis of compound 86e

In a 50 mL single-neck flask, compound 3g (1.8 g, 3.6 mmol) and Fmoc-VA-PABO-PNP (2.45 g, 3.6 mmol) were mixed and completely dissolved in 15 mL of DMF, followed by addition of DIEA (893 µL, 5.4 mmol), and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain compound 86e (2.94 g, pale yellow solid) with a yield of 78.3%, LC-MS: [M+H]⁺=1040.5.

### Step 5: Synthesis of compound 86f

In a 50 mL single-neck flask, compound 86e (2.9 g, 2.78 mmol) was dissolved in 8 mL of THF and 8 mL of water, followed by addition of 20 mL of glacial acetic acid, and the reaction was allowed at room temperature. After the reaction was found to be completed from the monitoring of HPLC, the reaction solution was slowly added dropwise into 400 mL of saturated sodium bicarbonate solution, extracted with ethyl acetate (100 mL*3), the organics were combined, washed with water and salt water, dried with anhydrous Na₂SO₄ and concentrated by evaporation under reduced pressure to obtain a crude product. The crude product was subject to column chromatography (DCM/MeOH=10:1-5:1) to obtain compound 86f (1.94 g, pale yellow solid) with a yield of 75%, LC-MS: [M+H]⁺=926.3.

### Step 6: Synthesis of compound 86g

In a 100 mL three-necked flask, compound 86f (1.9 g, 2.0 mmol) was dissolved in 40 mL of anhydrous DCM, a Dess-Martin high iodine reagent (0.93 g, 2.2 mmol) was added in the presence of N₂, and the reaction was allowed at room temperature for 4 h. After the reaction was found to be completed from the monitoring of TLC, the reaction solution was filtered, and the filtrated was washed with saturated sodium bicarbonate solution, water and saturated NaCl solution, dried with anhydrous Na₂SO₄, and concentrated to obtain a rude product. The crude product was purified by preparative high-performance liquid phase chromatography to obtain a preparation solution of the product. The preparation solution was lyophilized to obtain compound 86 g (1.61 g, a kind of white solid) with a yield of 85%, LC-MS: [M+H]⁺=924.4.

### Step 7: Synthesis of compound 86h

In a 50 mL single-neck flask, compound 86g (1.5 g,1.62 mmol) was completely dissolved in 15 mL of DMF, followed by addition of 3 mL of diethylamine, and the reaction was allowed at room temperature for 1 h. After the reaction was found to be completed from the monitoring of TLC, the reaction solution was poured into 100 mL of methyl tert-ether to obtain solid precipitate. The precipitate was separated from the solution by filtration to obtain a deprotected product. The deprotected product was dissolved in 15 mL of DMF, compound 40 (0.92 g,1.62 mmol) and EEDQ (0.48 g,1.94 mmol) were added, and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain compound 86 h (1.30 g, a kind of white solid), with a yield of 64.2%, LC-MS: [M+H]⁺=1248.5.

### Step 8: Synthesis of compound 86

In a 50 mL single-neck flask, compound 86h (500 mg, 0.4 mmol) was dissolved in 5 mL of DCM, followed by addition of trifluoroacetic acid (5 mL), and the reaction was allowed at room temperature for 1 h. After the reaction was found to be completed from the monitoring of HPLC, the reaction solution was concentrated at 45 °C under reduced-pressure to remove the solvent to obtain a residue. The residue was purified by preparative high-performance liquid phase chromatography to obtain a preparation solution. The preparation solution was lyophilized to obtain compound 86 (294 mg) with a yield of 67.4%, LC-MS: [M+H]⁺=1092.4.

### Example 96: Synthesis of compound P4-A

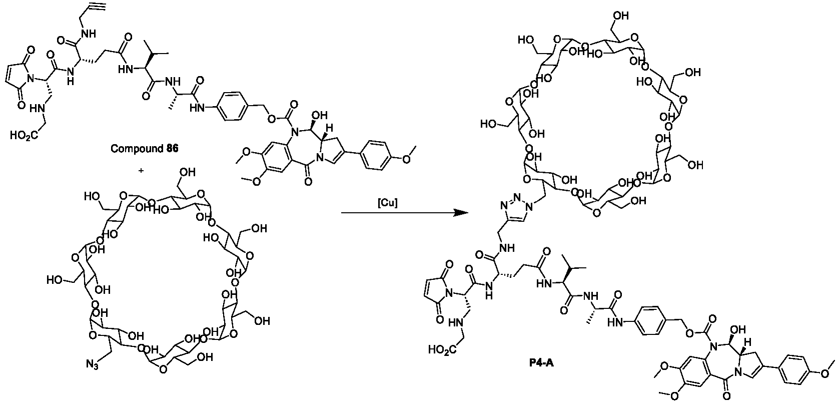

### Step 1: Synthesis of compound P4-A

In a 25 mL single-neck flask, compound 86 (25 mg, 0.023 mmol) and cyclodextrin-N₃ (26.5 mg, 0.023 mmol) were mixed and dissolved in 5 mL of DMF and 2.5 mL of water, followed by addition of CuSO₄·5H₂O (6.7 mg, 0.026 mmol) and sodium ascorbate (5.3 mg, 0.026 mmol). The reaction was allowed at room temperature for 1 h. After the reaction was found to be completed from the monitoring of HPLC, the reaction solution was purified by preparative high-performance liquid phase chromatography to obtain a preparation solution. The preparation solution was lyophilized to obtain compound P4-A (36.9 mg) with a yield of 71.6%, TOF: [M+H]⁺=2251.8.

### Example 97: Synthesis of compound P4-B

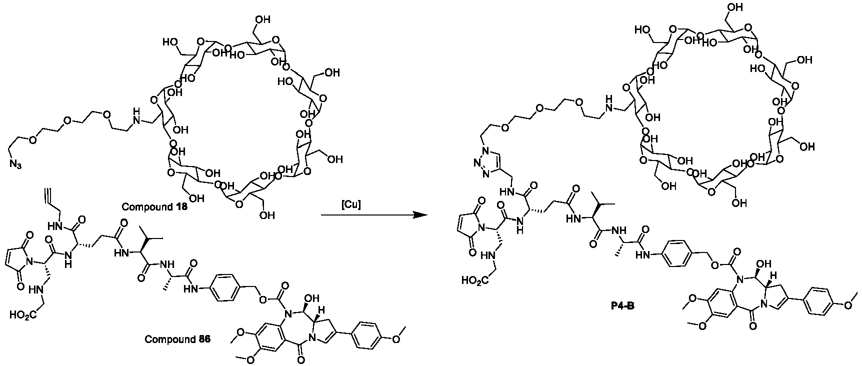

### Step 1: Synthesis of compound P4-B

In a 25 mL single-neck flask, compound 86 (25 mg, 0.023 mmol) and compound 18 (30.7 mg, 0.023 mmol) were mixed and dissolved in 5 mL of DMF and 2.5 mL of water, followed by addition of CuSO₄·5H₂O (6.6 mg, 0.026 mmol) and sodium ascorbate (5.3 mg, 0.026 mmol). The reaction was allowed at room temperature for 1 h. After the reaction was found to be completed from the monitoring of HPLC, the reaction solution was purified by preparative high-performance liquid phase chromatography to obtain a preparation solution. The preparation solution was lyophilized to obtain compound P4-B (37.2 mg) with a yield of 66.6%, TOF: [M+H]⁺=2427.2.

### Example 98: Synthesis of compound P4-C

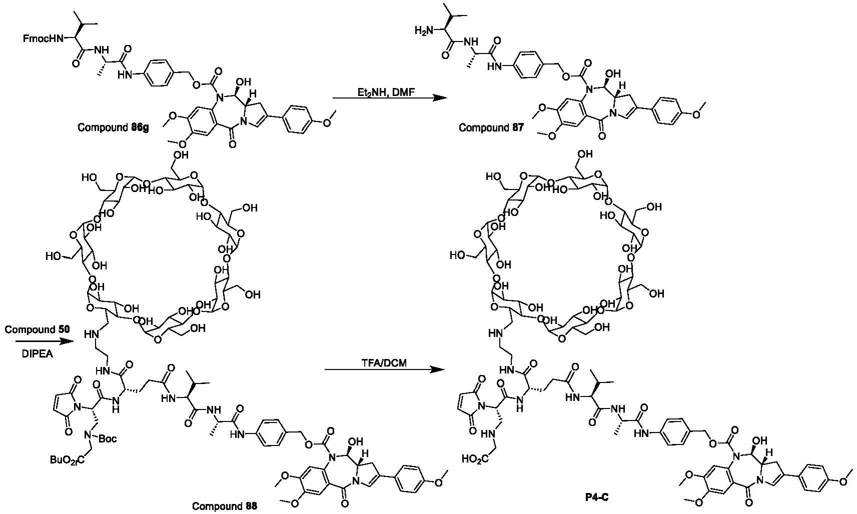

### Step 1: Synthesis of compound 88

In a 50 mL single-neck flask, compound 86 g (2 g, 2.16 mmol) was dissolved in 16 mL of DMF, followed by addition of diethylamine (4 mL), and the reaction was allowed at room temperature for 8 h. After the reaction was found to be completed from the monitoring of HPLC, the reaction solution was concentrated under reduced pressure by an oil pump, and the obtained yellow oil was concentrated by an oil pump to remove the solvent to obtain compound 88, which was directly put into the next reaction.

The above crude compound was dissolved in 10 mL of DMF, followed by DIPEA (714 µL, 4.32 mmol), then compound 50 (4 g, 2.16 mmol) was added at room temperature, and the reaction was allowed at room temperature for 3 h. After the reaction was found to be completed from the monitoring of HPLC, the reaction solution was purified by preparative high-performance liquid phase chromatography to obtain a preparation solution. The preparation solution was lyophilized to obtain compound 88 (3.9 g), TOF: [M+H]⁺= 2370.3.

### Step 2: Synthesis of compound P4-C

In a 25 mL single-neck flask, compound 88 (1 g, 0.42 mmol) and 4 mL of DCM were mixed and dissolved completely, followed by addition of 4 mL TFA, and the reaction was allowed at room temperature for 0.5 h. After the reaction was found to be completed from the monitoring of HPLC, the reaction solution was concentrated at 45 °C by a water pump to remove the solvent to obtain a residue. The residue was purified by preparative high-performance liquid phase chromatography to obtain a preparation solution. The preparation solution was lyophilized to obtain compound P4-C (577 mg), TOF: [M+H]⁺= 2214.0.

### Example 99: Synthesis of compound P4-D

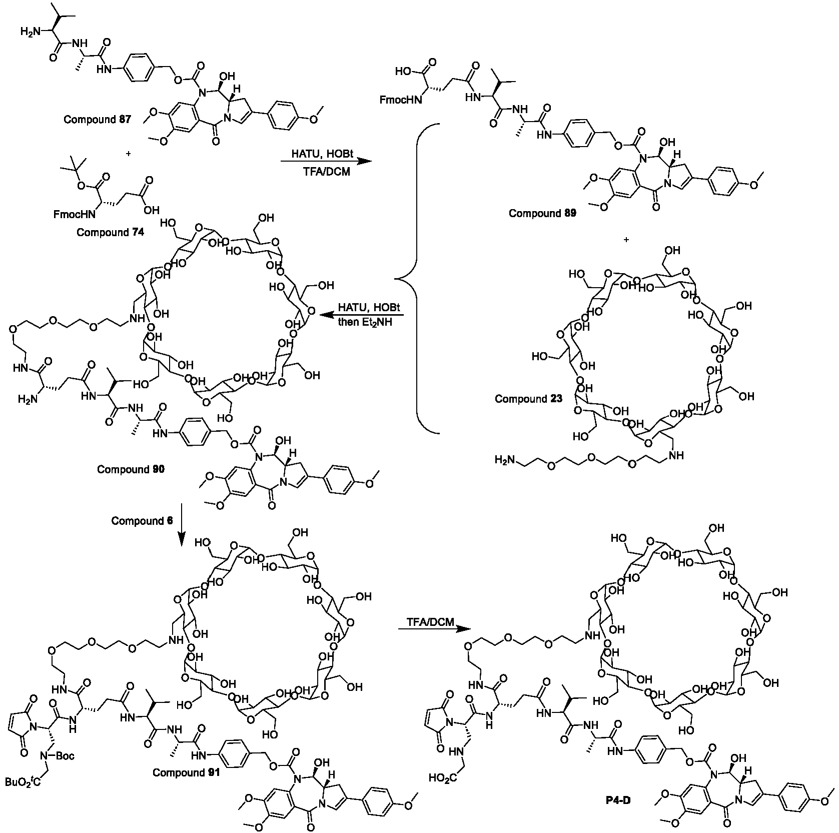

### Step 1: Synthesis of compound 89

In a 25 mL single-neck flask, compound 87 (300 mg, 0.43 mmol) and 10 mL of DMF were added, then compound 74 (183 mg, 0.43 mmol) was added at room temperature. HATU (333 mg, 0.86 mmol), HOBt (118 mg, 0.86 mmol) and DIPEA (0.22 mL) were added in sequence under the cooling of an ice water bath, and the resulting solution was slowly brought naturally to room temperature to react for 5 h. After the reaction was found to be completed from the monitoring of TLC, rotary evaporation under reduced pressure was performed to remove the solvent. The residual was concentrated to obtain a pale yellow oily rude product. The crude product was directly put into the next reaction without purification.

The above compound was dissolved in 3 mL of dichloromethane, the resulting solution was cooled to 0 °C in condition of an ice water bath, TFA (3 mL) was slowly added dropwise, and the resulting reaction solution was slowly brought naturally to room temperature to react for 0.5 h. After the reaction was found to be completed from the monitoring of HPLC, the reaction solution was concentrated at 45 °C under reduced-pressure to obtain a yellow oily crude compound. The residue was purified by preparative high-performance liquid phase chromatography to obtain a preparation solution. The preparation solution was lyophilized to obtain compound 89 (266 mg), LC-MS: [M+H]⁺= 1053.5.

### Step 2: Synthesis of compound 90

In a 25 mL single-neck flask, compound 89 (200 mg, 0.19 mmol) and 10 mL of DMF were added, and compound 23 (484 mg, 0.19 mmol) was then added at room temperature. HATU (156 mg, 0.4 mmol), HOBt (82 mg, 0.6 mmol) and DIPEA (100 µL) were added in sequence under the cooling of an ice water bath to have a reaction for 3 h. After the reaction was found to be completed from the monitoring of HPLC, the reaction solution was purified by preparative high-performance liquid phase chromatography to obtain a preparation solution. The preparation solution was lyophilized to obtain an intermediate compound directly put into the next reaction.

The above intermediate was dissolved in 3 mL of DMF, then diethylamine (0.6 mL) was added dropwise at room temperature, and the reaction was allowed at room temperature for 5 h. The reaction was monitored by HPLC. After the reaction was completed, the reaction solution was purified by preparative high-performance liquid phase chromatography to obtain a preparation solution. The preparation solution was lyophilized to obtain compound 90 (301 mg), TOF: [M+H]⁺=2122.1.

### Step 3: Synthesis of compound 91

In a 25 mL single-neck flask, compound 90 (200 mg, 0.094 mmol) and 5 mL of DMF were added, then compound 6 (38 mg, 0.042 mmol) was added at room temperature. HATU (76 mg, 0.188 mmol), HOBt (29 mg, 0.188 mmol) and DIPEA (64 µL) were added in sequence under the cooling of an ice water bath, and the reaction was allowed at room temperature for 2 h. After the reaction was found to be completed from the monitoring of HPLC, the reaction solution was purified by preparative high-performance liquid phase chromatography to obtain a preparation solution. The preparation solution was lyophilized to obtain compound 91 (198 mg), TOF: [M+H]⁺=2502.2.

### Step 4: Synthesis of compound P4-D

Compound 91 (100 mg, 0.04 mmol) was dissolved in 2 mL of dichloromethane, and the resulting solution was cooled to 0 °C in condition of an ice water bath. TFA (2 mL) was slowly added dropwise, and the resulting solution was slowly brought naturally to room temperature to react for 0.5 h. The reaction was monitored by HPLC. After the reaction was completed, the reaction solution was concentrated at 45 °C under reduced-pressure to obtain a yellow oily crude compound. The residue was purified by preparative high-performance liquid phase chromatography to obtain a preparation solution. The preparation solution was lyophilized to obtain compound P4-D (62.0 mg), TOF: [M+Na]⁺= 2368.0.

### Example 100: Synthesis of compound P4-E

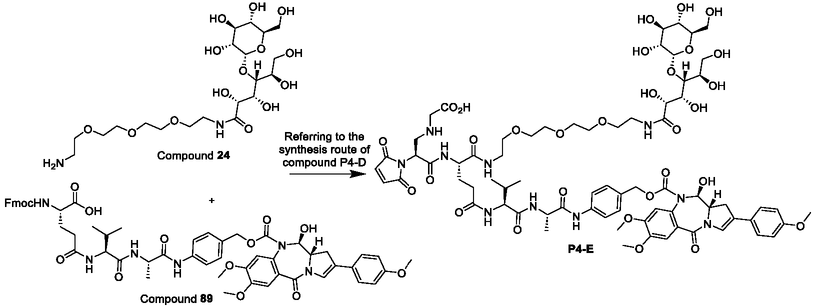

Taking of compound 89 and compound 24 as starting raw materials, compound P4-E (37 mg) was prepared according to the synthesis route of compound P4-D, LC-MS: [M+H]⁺= 1569.6.

### Example 101: Synthesis of compound P4-F

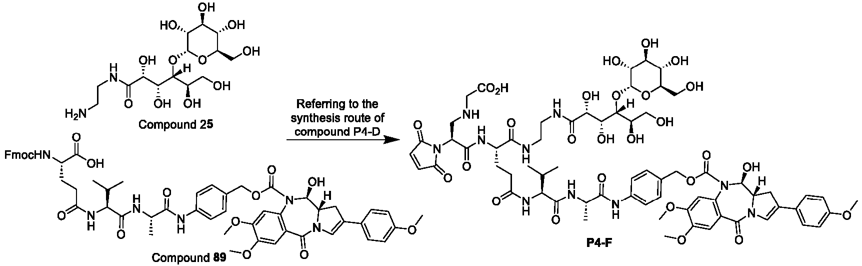

Taking of compound 89 and compound 25 as starting raw materials, compound P4-F (41 mg) was prepared according to the synthesis route of compound P4-D, LC-MS: [M+H]⁺= 1437.5.

### Example 102: Synthesis of compound P4-G

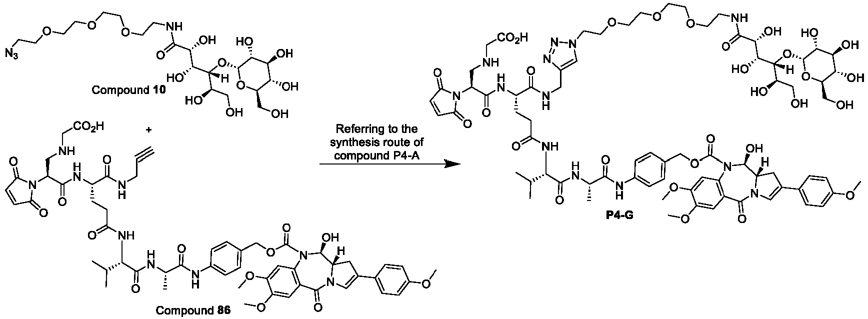

Taking of compound 86 and compound 10 as starting raw materials, compound P4-G (25 mg) was prepared according to the synthesis route of compound P4-A, LC-MS: [M+H]⁺= 1650.6.

### Example 103: Synthesis of compound P4-H

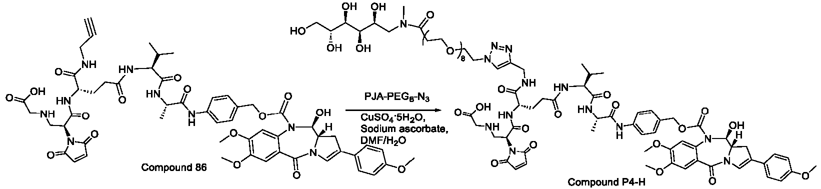

Taking of compound 86 and compound PJA-PEG8-N3 as starting raw materials, compound P4-H (34 mg) was prepared according to the synthesis route of compound P4-A, LC-MS: [M+H]⁺= 1736.8.

### Example 104: Synthesis of compound P4-I

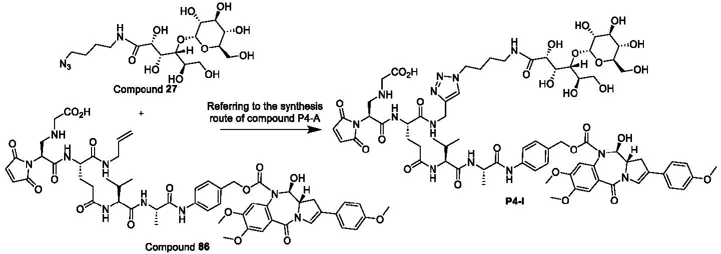

Taking of compound 86 and compound 27 as starting raw materials, compound P4-I (55 mg) was prepared according to the synthesis route of compound P4-A, LC-MS: [M+H]⁺= 1546.9.

### Example 105: Synthesis of compound P4-J

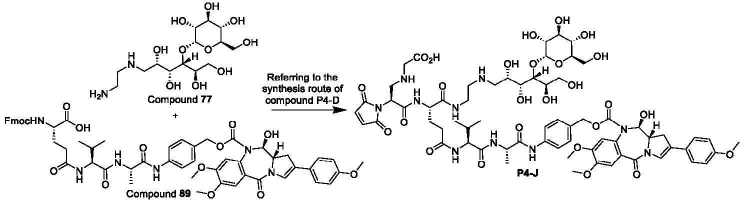

Taking of compound 89 and compound 77 as starting raw materials, compound P4-J (31 mg) was prepared according to the synthesis route of compound P4-D, LC-MS: [M+H]⁺= 1423.7.

### Example 106: Synthesis of compound P4-K

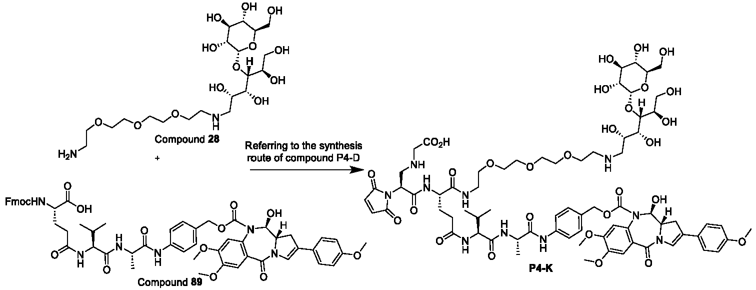

Taking of compound 89 and compound 28 as starting raw materials, compound P4-K (24 mg) was prepared according to the synthesis route of compound P4-D, LC-MS: [M+H]+= 1555.7.

### Example 107: Synthesis of compound P4-L

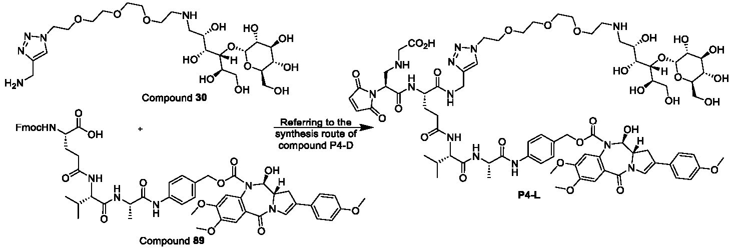

Taking of compound 89 and compound 30 as starting raw materials, compound P4-L (24 mg) was prepared according to the synthesis route of compound P4-D, LC-MS: [M+H]⁺= 1636.6.

### Example 108: Synthesis of compound P4-M

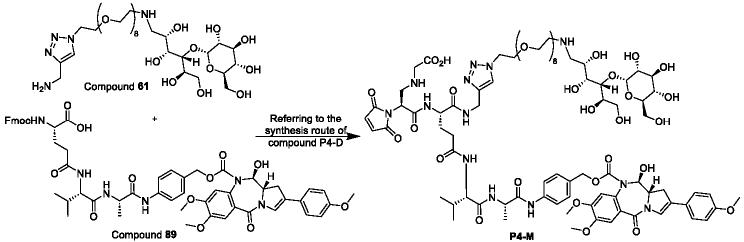

Taking of compound 89 and compound 61 as starting raw materials, compound P4-M (20 mg) was prepared according to the synthesis route of compound P4-D, LC-MS: [M+H]⁺= 1857.0.

### Example 109: Synthesis of compound P4-N

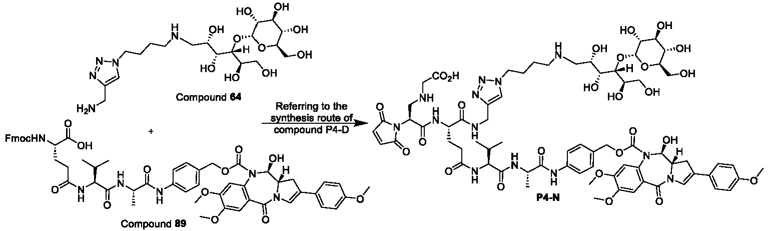

Taking of compound 89 and compound 64 as starting raw materials, compound P4-N (17 mg) was prepared according to the synthesis route of compound P4-D, LC-MS: [M+H]⁺= 1532.7.

### Example 110: Synthesis of compound P4-O

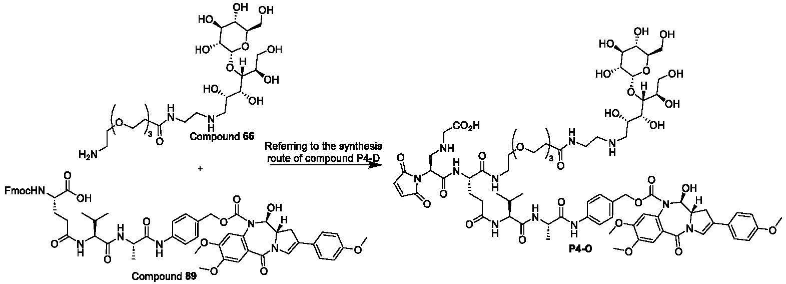

Taking of compound 89 and compound 66 as starting raw materials, compound P4-O (32 mg) was prepared according to the synthesis route of compound P4-D, LC-MS: [M+H]⁺= 1626.8.

### Example 111: Synthesis of compound P4-P

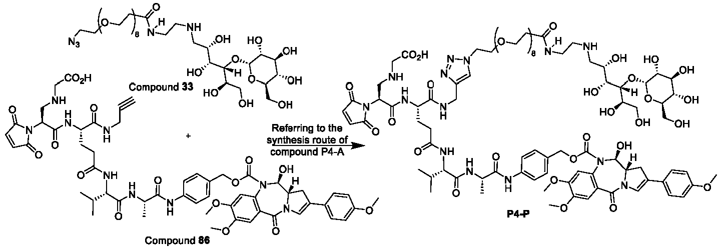

Taking of compound 86 and compound 33 as starting raw materials, compound P4-P (42 mg) was prepared according to the synthesis route of compound P4-A, LC-MS: [M+H]⁺= 1928.0.

### Example 112: Synthesis of compound P4-Q

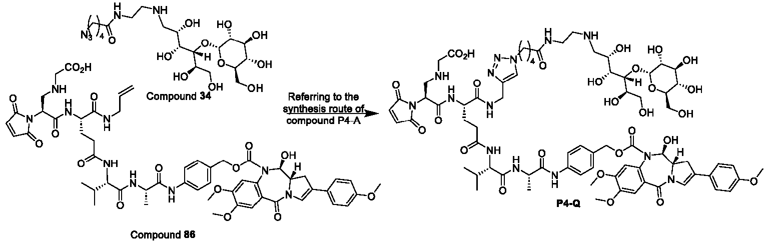

Taking of compound 86 and compound 34 as starting raw materials, compound P4-Q (35 mg) was prepared according to the synthesis route of compound P4-A, LC-MS: [M+H]⁺= 1603.6.

### Example 113: Synthesis of compound P4-R

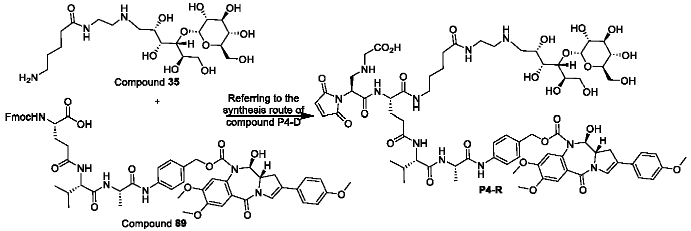

Taking of compound 89 and compound 35 as starting raw materials, compound P4-R (31 mg) was prepared according to the synthesis route of compound P4-D, LC-MS: [M+H]⁺= 1522.8.

### Example 114: Synthesis of compound P4-S

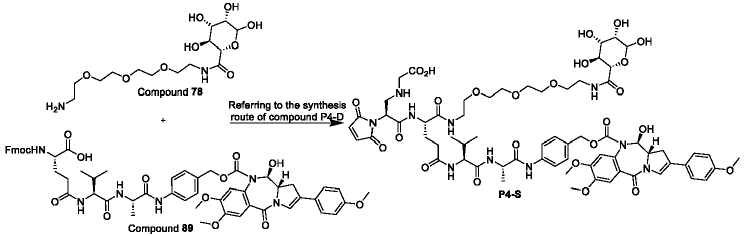

Taking of compound 89 and compound 78 as starting raw materials, compound P4-S (21 mg) was prepared according to the synthesis route of compound P4-D, LC-MS: [M+H]⁺= 1405.5.

### Example 115: Synthesis of compound P4-T

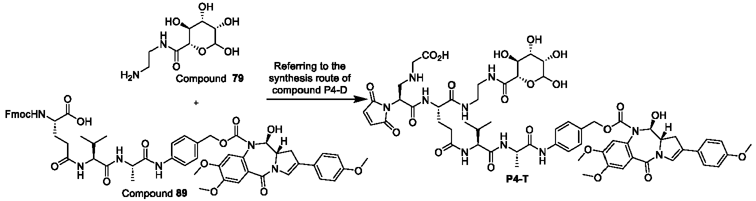

Taking of compound 89 and compound 79 as starting raw materials, compound P4-T (31 mg) was prepared according to the synthesis route of compound P4-D, LC-MS: [M+H]⁺= 1273.4.

### Example 116: Synthesis of compound P4-U

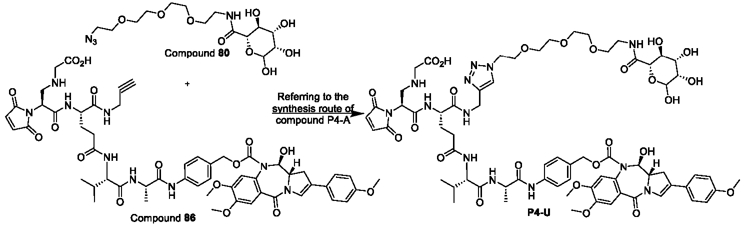

Taking of compound 86 and compound 80 as starting raw materials, compound P4-U (47 mg) was prepared according to the synthesis route of compound P4-A, LC-MS: [M+H]⁺= 1486.7.

### Example 117: Synthesis of compound P4-V

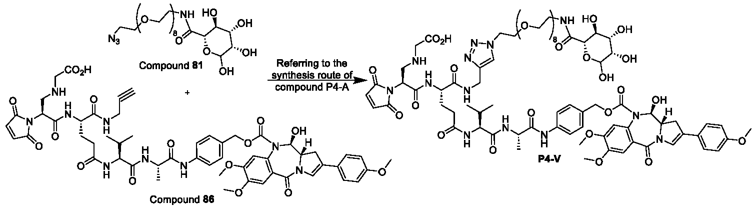

Taking of compound 86 and compound 81 as starting raw materials, compound P4-V (40 mg) was prepared according to the synthesis route of compound P4-A, LC-MS: [M+H]⁺= 1706.7.

### Example 118: Synthesis of compound P4-W

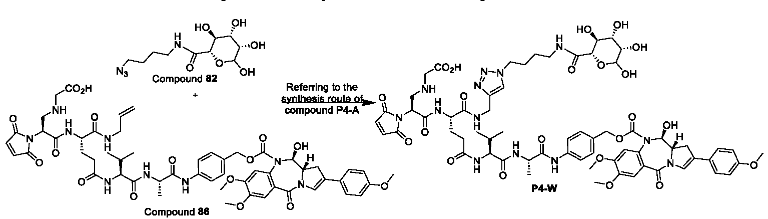

Taking of compound 86 and compound 82 as starting raw materials, compound P4-W (37 mg) was prepared according to the synthesis route of compound P4-A, LC-MS: [M+H]⁺= 1382.9.

### Example 119: Synthesis of compound P4-X2

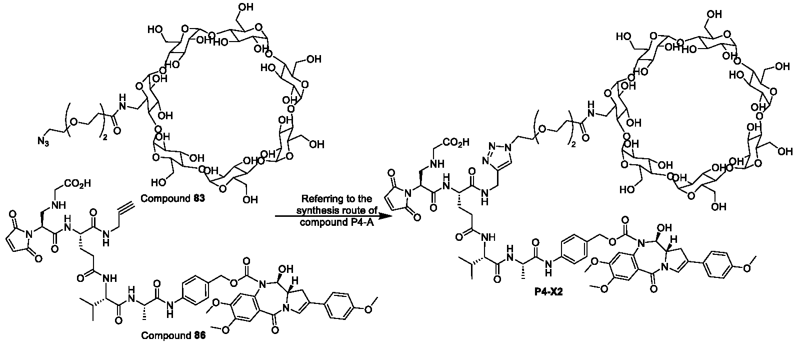

Taking of compound 86 and compound 83 as starting raw materials, compound P4-X2 (22 mg) was prepared according to the synthesis route of compound P4-A, TOF: [M+H]⁺= 2411.2.

### Example 120: Synthesis of compound P4-Y8

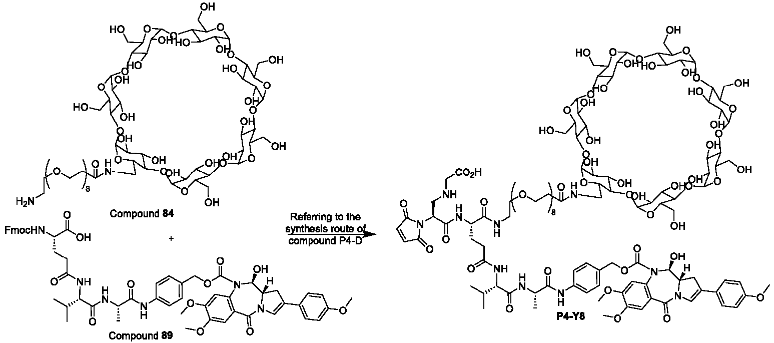

Taking of compound 89 and compound 84 as starting raw materials, compound P4-Y8 (29 mg) was prepared according to the synthesis route of compound P4-D, TOF: [M+H]⁺= 2594.5.

### Example 121: Synthesis of compound P4-Z4

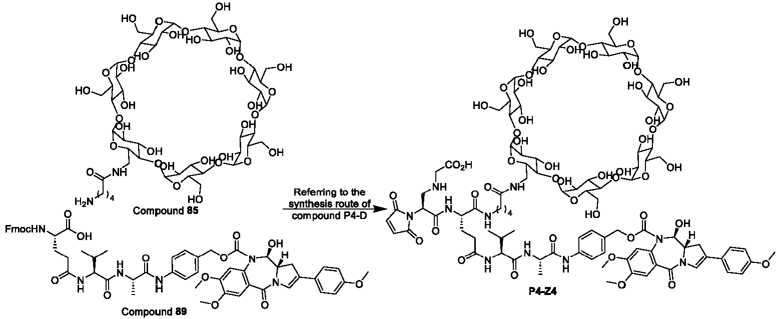

Taking of compound 89 and compound 85 as starting raw materials, compound P4-Z4 (28 mg) was prepared according to the synthesis route of compound P4-D, TOF: [M+Na]⁺= 2292.1.

Antibodies were prepared according to conventional preparation methods for antibody, for example, by vector construction, followed by transfection into eukaryotic cells for purification and expression.

The sequences of the anti-Trop-2 antibody are shown below:
Light chain (SEQ ID NO: 1) 
DIQMTQSPSSLSASVGDRVTITCRASQDINKYLAWYQQKPGKVPKLLIYS
TSTLQSGVPSRFSGSGSGTDFTLTISSLQPEDVATYYCLQYDDLFTFGQGTKLEI
KRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNR
GEC
Heavy chain (SEQ ID NO: 2)

QVQLVQSGAEVKKPGASVKLSCKASGYTFTSFDINWVRQAPEQRLEWM
GWIFPGDGNTKYSQKFQGRATITRDTSASTAYMELSSLRSEDTAVYYCVRGEA
LYYFDYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPS
NTKVDKRVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRW

QQGNVFSCSVMHEALHNHYTQKSLSLSPG

The sequences of the anti-CD33 antibody are shown below:
Light chain (SEQ ID NO: 3) 
DIQLTQSPSTLSASVGDRVTITCRASESLDNYGIRFLTWFQQKPGKAPKLL
MYAASNQGSGVPSRFSGSGSGTEFTLTISSLQPDDFATYYCQQTKEVPWSFGQ
GTKVEVKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSPV
CTKSFNRGEC
Heavy chain (SEQ ID NO: 4)

EVQLVQSGAEVKKPGSSVKVSCKASGYTITDSNIHWVRQAPGQSLEWIG
YIYPYNGGTDYNQKFKNRATLTVDNPTNTAYMELSSLRSEDTAFYYCVNGNP
WLAYWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNT
KVDKRVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDW
LNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQ
GNVFSCSVMHEALHNHYTQKSLSLSPG

In the following examples, the anti-CD33 antibody was used to prepare the ligand-drug conjugates. The ligand-drug conjugates containing the anti-TROP-2 antibody were prepared according to the preparation methods disclosed in these examples.

### Example 122: Preparation of ligand-drug conjugate:

### General conjugation method

After the preliminary purification, the antibody molecule L with a monomer rate greater than 95% was moved to a phosphate buffer solution using an ultrafiltration centrifuge tube at a concentration of 10 mg/mL. TCEP 20 -fold the number of antibody moles was added and reacted at room temperature for 4 h to open the interchain disulfide bonds of the antibody. A linker-drug compound (payload) 20-fold the number of antibody moles was added and reacted at room temperature for 2 h. After the action was completed, the reaction solution was moved to PBS using an ultrafiltration centrifuge tube with a molecular weight cutoff of 30 KDa, and the un-conjugated payload was removed. The ADC sample after the solution replacement was filtered using a 0.22 µm sterilizing filter for later use.

### Example 123:

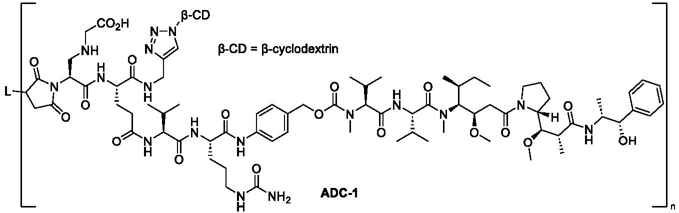

ADC-1 was synthesized according to the general preparation method of ligand-drug conjugates.

### Example 124:

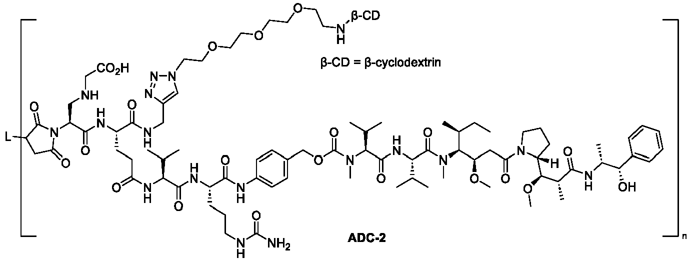

ADC-2 was synthesized according to the general preparation method of ligand-drug conjugates.

### Example 125:

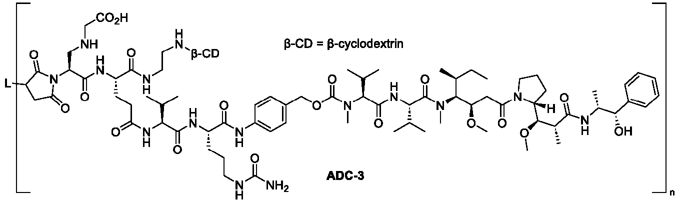

ADC-3 was synthesized according to the general preparation method of ligand-drug conjugates.

### Example 126:

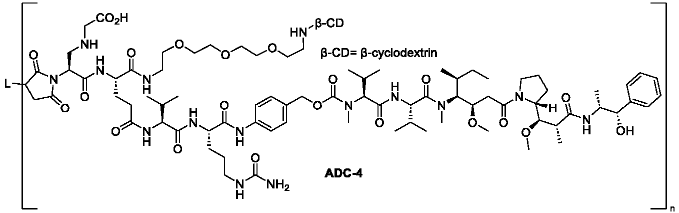

ADC-4 was synthesized according to the general preparation method of ligand-drug conjugates.

### Example 127:

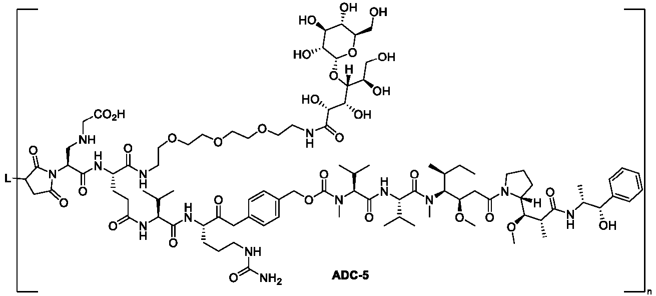

ADC-5 was synthesized according to the general preparation method of ligand-drug conjugates.

### Example 128:

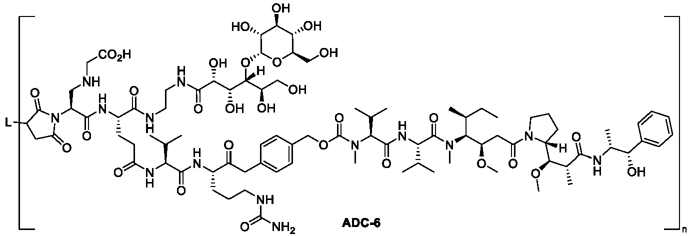

ADC-6 was synthesized according to the general preparation method of ligand-drug conjugates.

### Example 129:

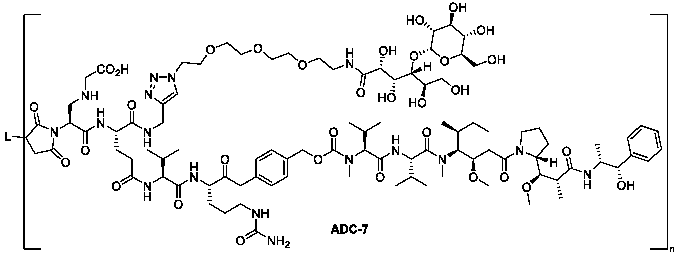

ADC-7 was synthesized according to the general preparation method of ligand-drug conjugates.

### Example 130:

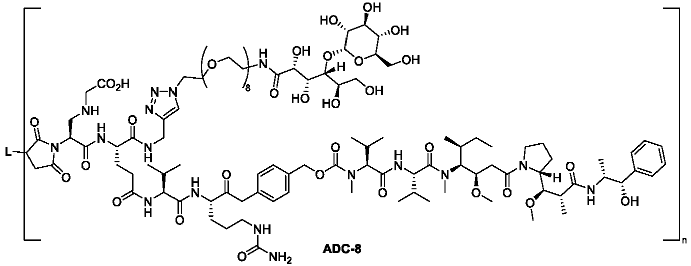

ADC-8 was synthesized according to the general preparation method of ligand-drug conjugates.

### Example 131:

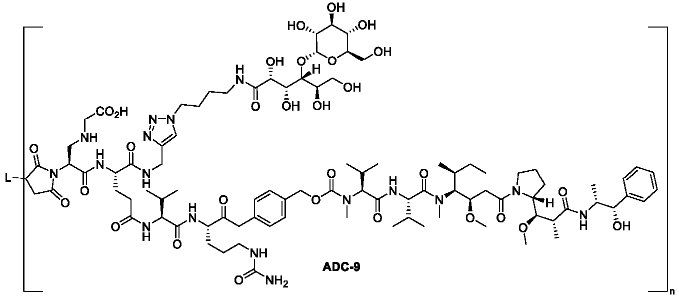

ADC-9 was synthesized according to the general preparation method of ligand-drug conjugates.

### Example 132:

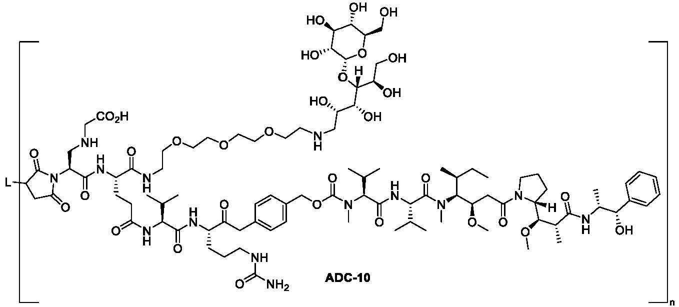

ADC-10 was synthesized according to the general preparation method of ligand-drug conjugates.

### Example 133:

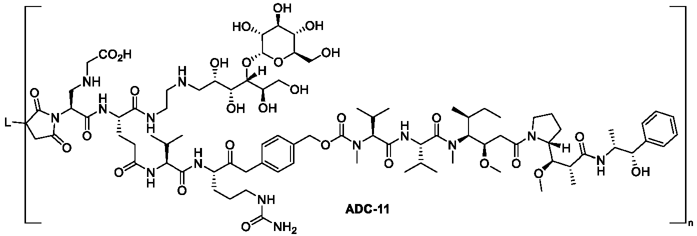

ADC-11 was synthesized according to the general preparation method of ligand-drug conjugates.

### Example 134:

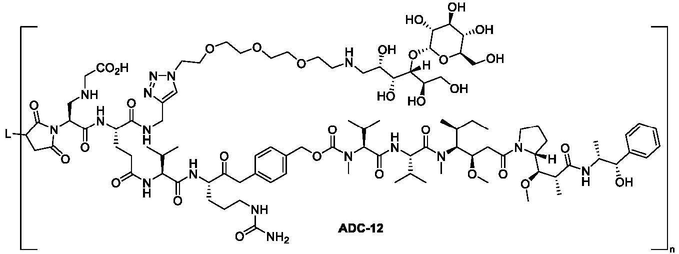

ADC-12 was synthesized according to the general preparation method of ligand-drug conjugates.

### Example 135:

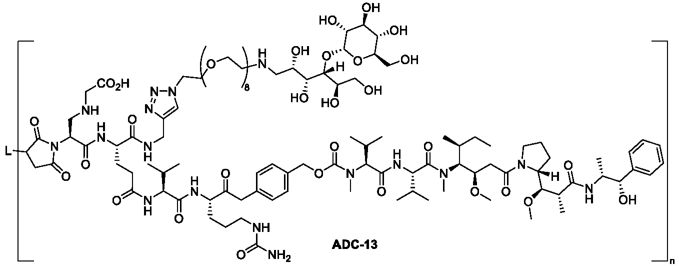

ADC-13 was synthesized according to the general preparation method of ligand-drug conjugates.

### Example 136:

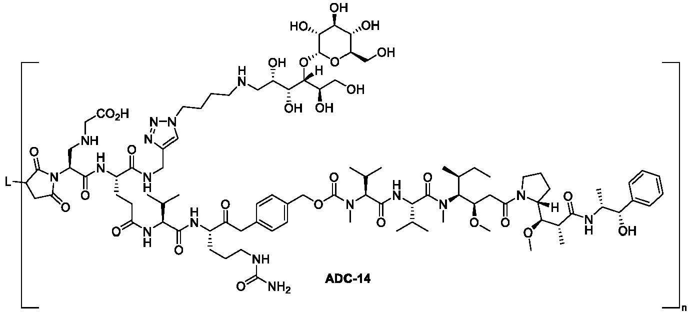

ADC-14 was synthesized according to the general preparation method of ligand-drug conjugates.

### Example 137:

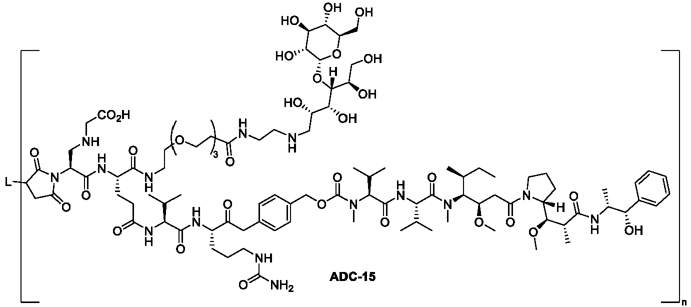

ADC-15 was synthesized according to the general preparation method of ligand-drug conjugates.

### Example 138:

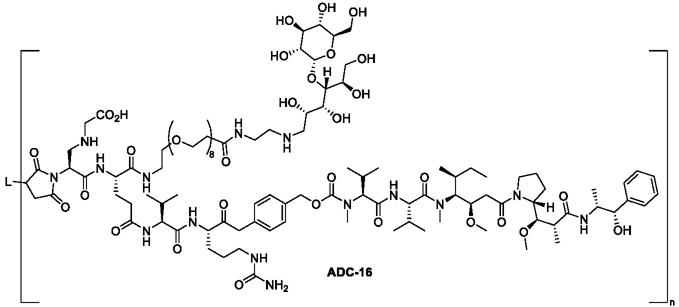

ADC-16 was synthesized according to the general preparation method of ligand-drug conjugates.

### Example 139:

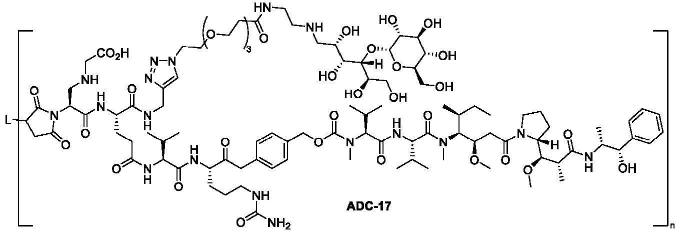

ADC-17 was synthesized according to the general preparation method of ligand-drug conjugates.

### Example 140:

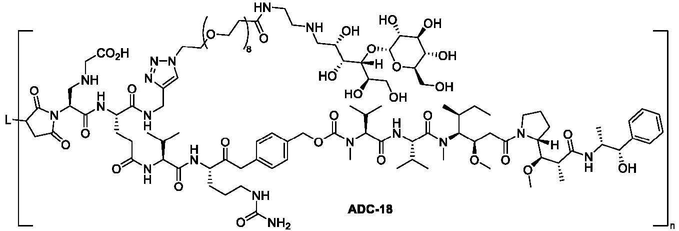

ADC-18 was synthesized according to the general preparation method of ligand-drug conjugates.

### Example 141:

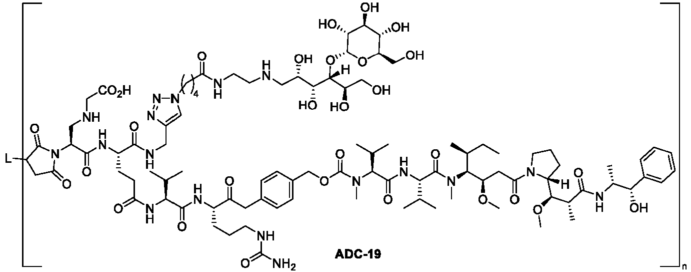

ADC-19 was synthesized according to the general preparation method of ligand-drug conjugates.

### Example 142:

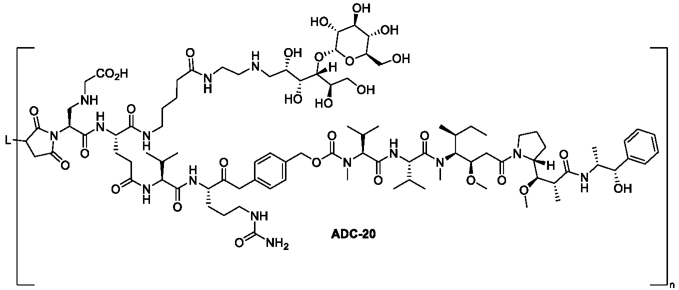

ADC-20 was synthesized according to the general preparation method of ligand-drug conjugates.

### Example 143:

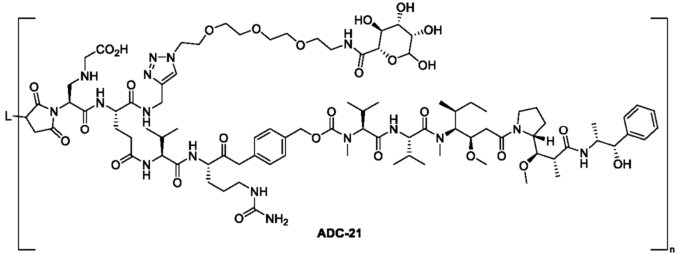

ADC-21 was synthesized according to the general preparation method of ligand-drug conjugates.

### Example 144:

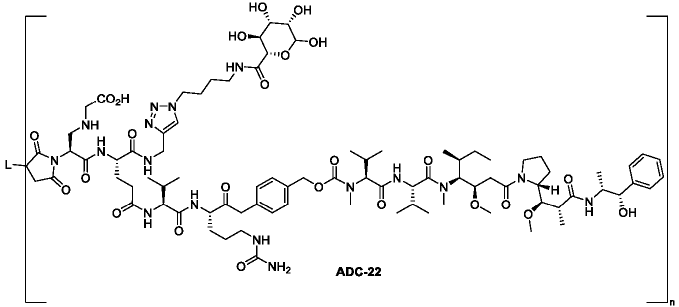

ADC-22 was synthesized according to the general preparation method of ligand-drug conjugates.

### Example 145:

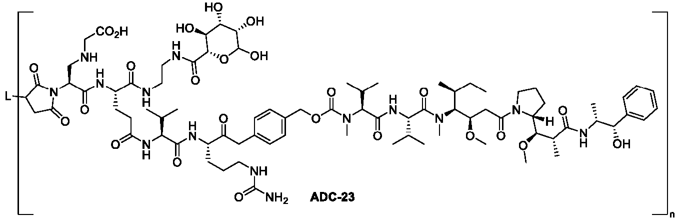

ADC-23 was synthesized according to the general preparation method of ligand-drug conjugates.

### Example 146:

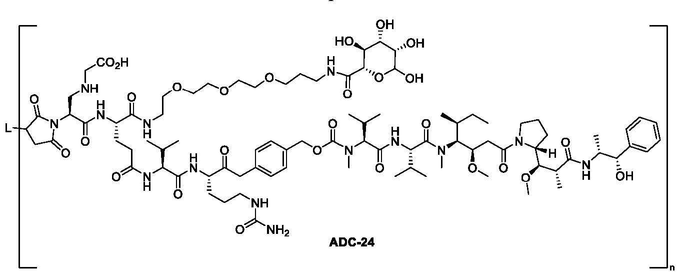

ADC-24 was synthesized according to the general preparation method of ligand-drug conjugates.

### Example 147:

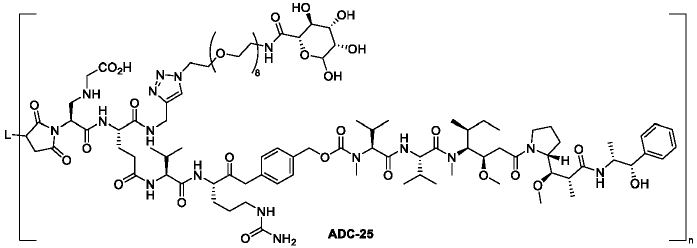

ADC-25 was synthesized according to the general preparation method of ligand-drug conjugates.

### Example 148:

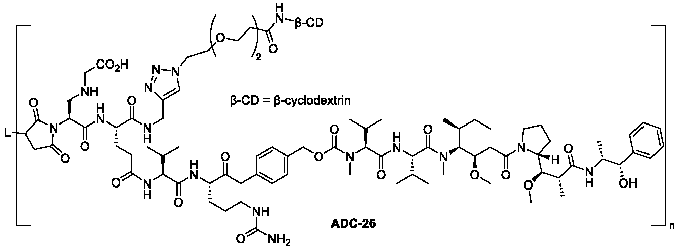

ADC-26 was synthesized according to the general preparation method of ligand-drug conjugates.

### Example 149:

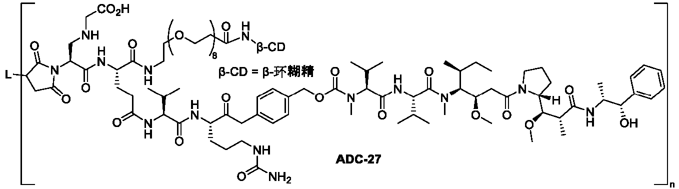

ADC-27 was synthesized according to the general preparation method of ligand-drug conjugates.

### Example 150:

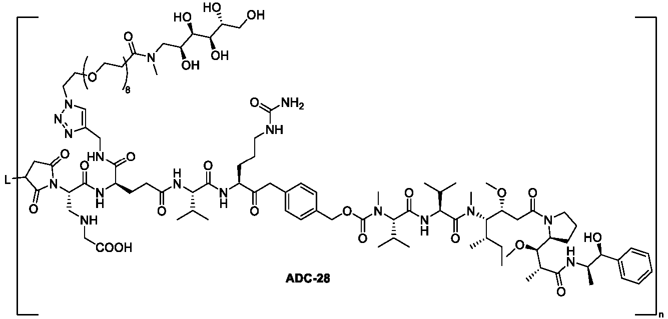

ADC-28 was synthesized according to the general preparation method of ligand-drug conjugates.

### Example 151:

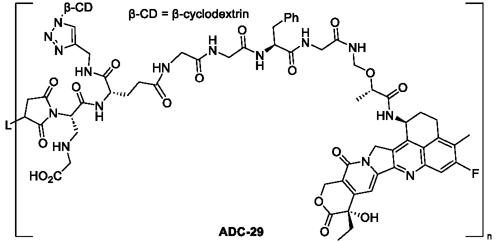

ADC-29 was synthesized according to the general preparation method of ligand-drug conjugates.

### Example 152:

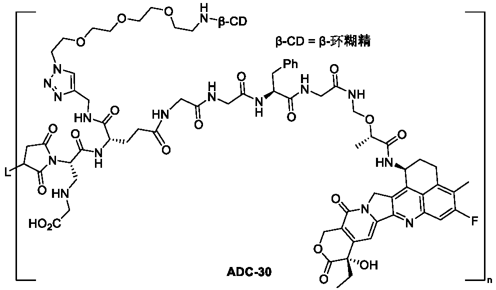

ADC-30 was synthesized according to the general preparation method of ligand-drug conjugates.

### Example 153:

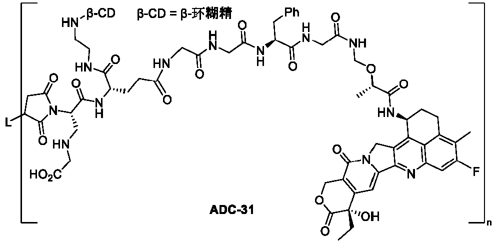

ADC-31 was synthesized according to the general preparation method of ligand-drug conjugates.

### Example 154:

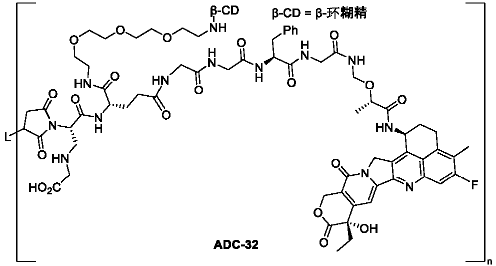

ADC-32 was synthesized according to the general preparation method of ligand-drug conjugates.

### Example 155:

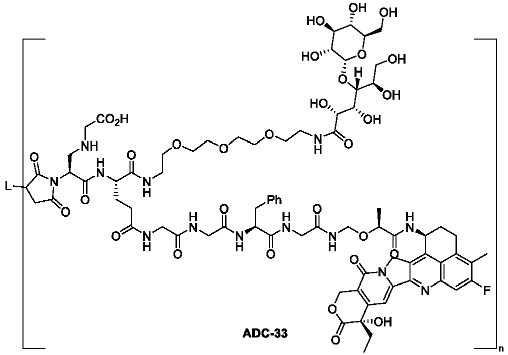

ADC-33 was synthesized according to the general preparation method of ligand-drug conjugates.

### Example 156:

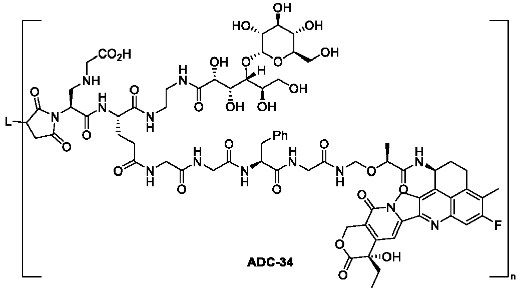

ADC-34 was synthesized according to the general preparation method of ligand-drug conjugates.

### Example 157:

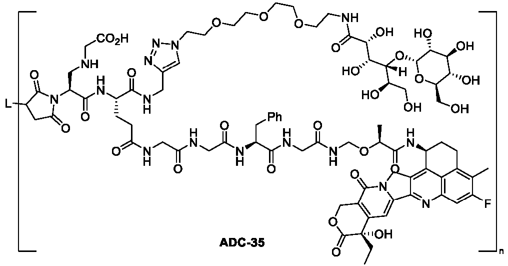

ADC-35 was synthesized according to the general preparation method of ligand-drug conjugates.

### Example 158:

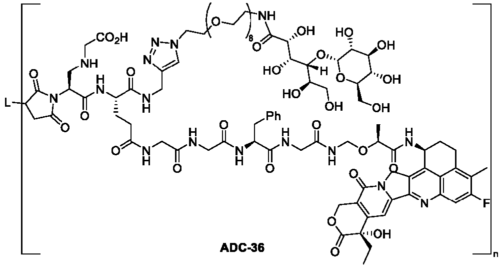

ADC-36 was synthesized according to the general preparation method of ligand-drug conjugates.

### Example 159:

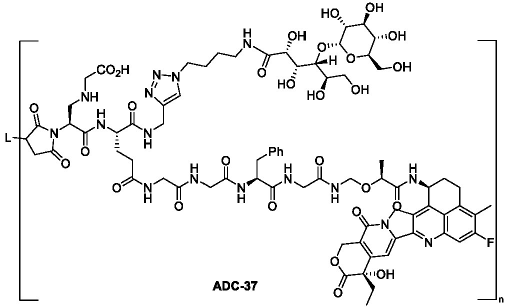

ADC-37 was synthesized according to the general preparation method of ligand-drug conjugates.

### Example 160:

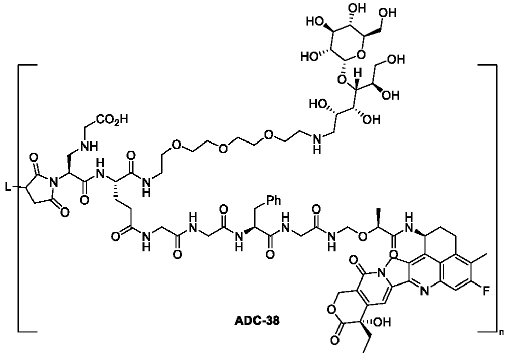

ADC-38 was synthesized according to the general preparation method of ligand-drug conjugates.

### Example 161:

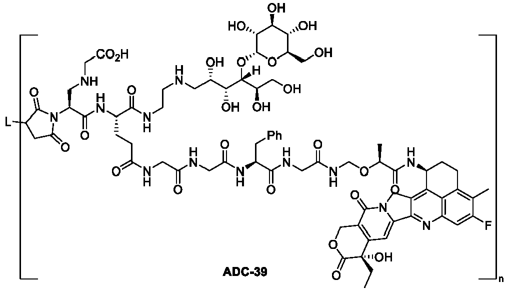

ADC-39 was synthesized according to the general preparation method of ligand-drug conjugates.

### Example 162:

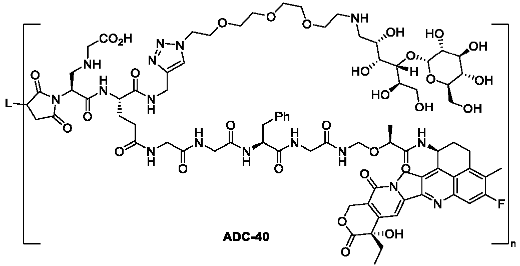

ADC-40 was synthesized according to the general preparation method of ligand-drug conjugates.

### Example 163:

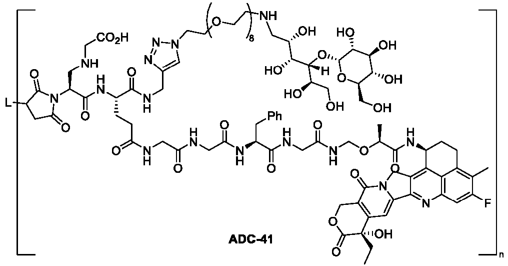

ADC-41 was synthesized according to the general preparation method of ligand-drug conjugates.

### Example 164:

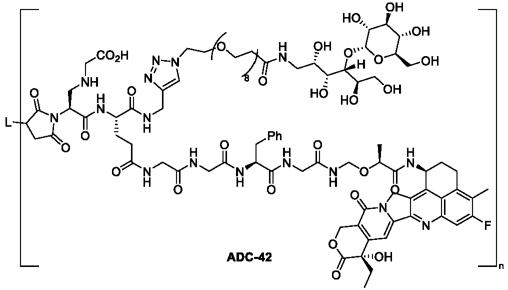

ADC-42 was synthesized according to the general preparation method of ligand-drug conjugates.

### Example 165:

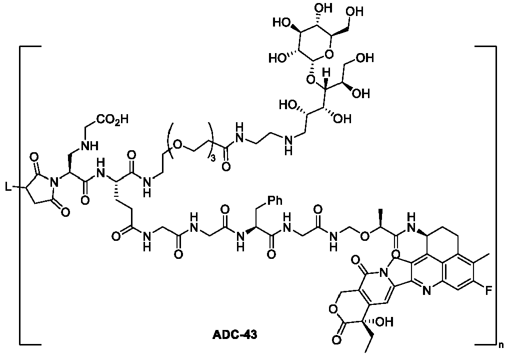

ADC-43 was synthesized according to the general preparation method of ligand-drug conjugates.

### Example 166:

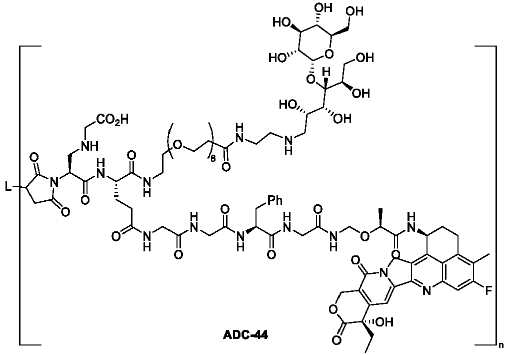

ADC-44 was synthesized according to the general preparation method of ligand-drug conjugates.

### Example 167:

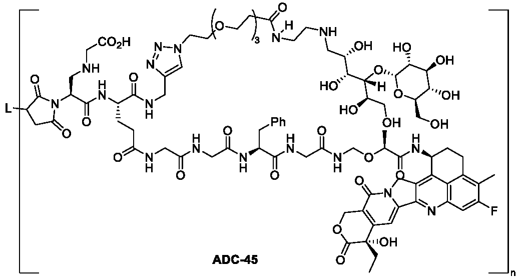

ADC-45 was synthesized according to the general preparation method of ligand-drug conjugates.

### Example 168:

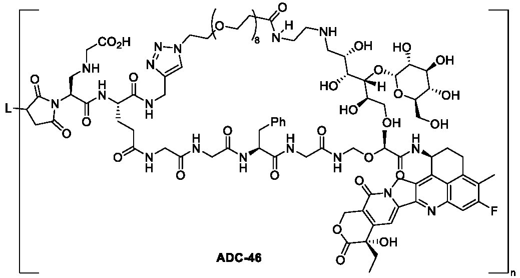

ADC-46 was synthesized according to the general preparation method of ligand-drug conjugates.

### Example 169:

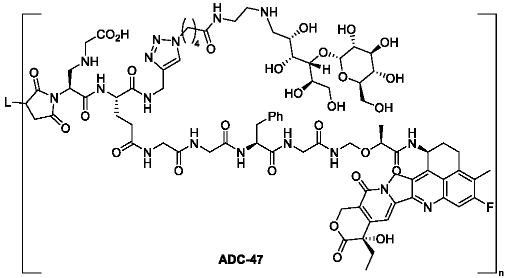

ADC-47 was synthesized according to the general preparation method of ligand-drug conjugates.

### Example 170:

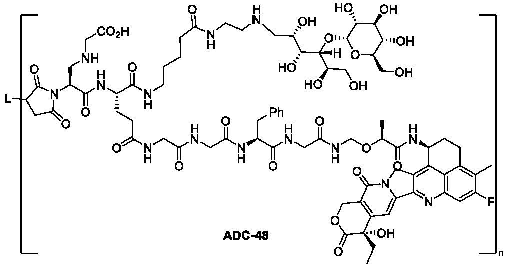

ADC-48 was synthesized according to the general preparation method of ligand-drug conjugates.

### Example 171:

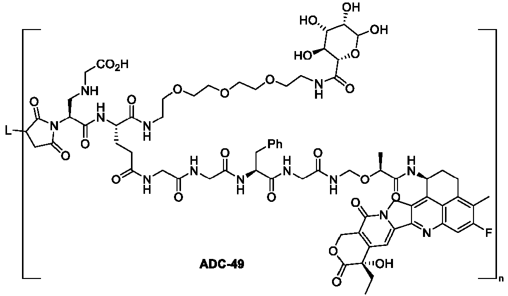

ADC-49 was synthesized according to the general preparation method of ligand-drug conjugates.

### Example 172:

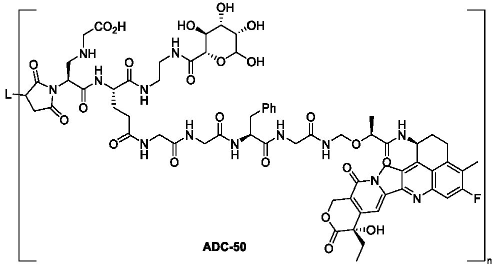

ADC-50 was synthesized according to the general preparation method of ligand-drug conjugates.

### Example 173:

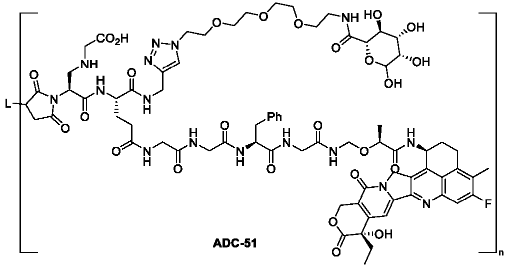

ADC-51 was synthesized according to the general preparation method of ligand-drug conjugates.

### Example 174:

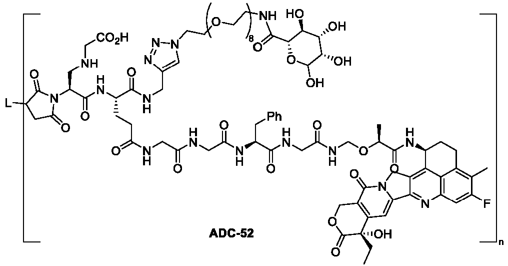

ADC-52 was synthesized according to the general preparation method of ligand-drug conjugates.

### Example 175:

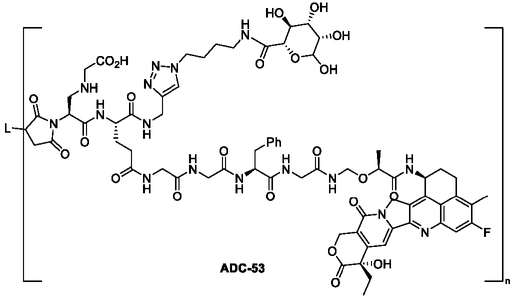

ADC-53 was synthesized according to the general preparation method of ligand-drug conjugates.

### Example 176:

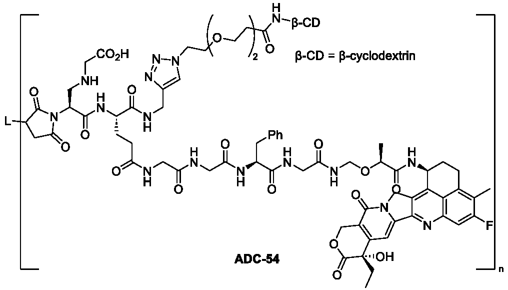

ADC-54 was synthesized according to the general preparation method of ligand-drug conjugates.

### Example 177:

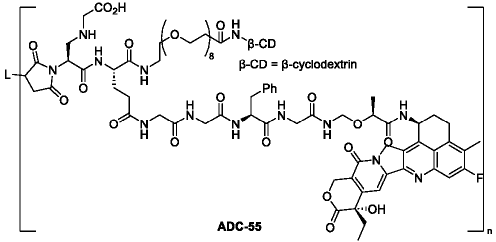

ADC-55 was synthesized according to the general preparation method of ligand-drug conjugates.

### Example 178:

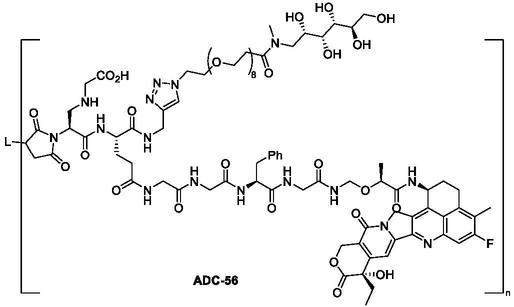

ADC-56 was synthesized according to the general preparation method of ligand-drug conjugates.

### Example 179:

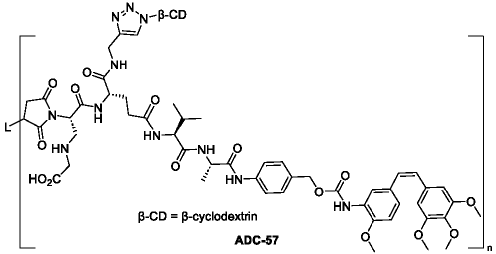

ADC-57 was synthesized according to the general preparation method of ligand-drug conjugates.

### Example 180:

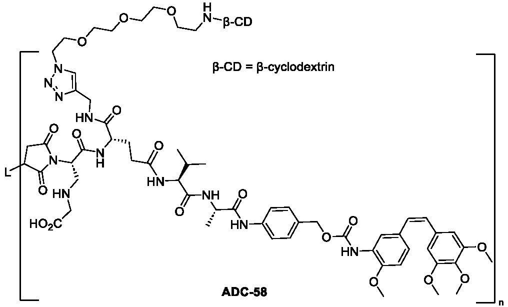

ADC-58 was synthesized according to the general preparation method of ligand-drug conjugates.

### Example 181:

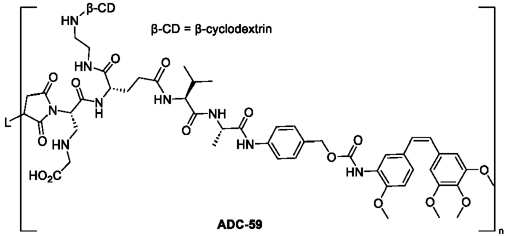

ADC-59 was synthesized according to the general preparation method of ligand-drug conjugates.

### Example 182:

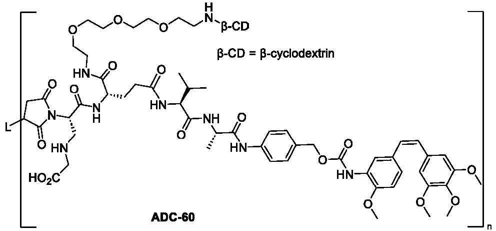

ADC-60 was synthesized according to the general preparation method of ligand-drug conjugates.

### Example 183:

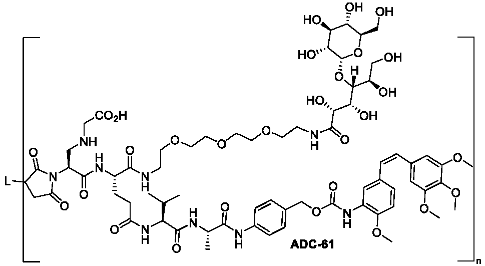

ADC-61 was synthesized according to the general preparation method of ligand-drug conjugates.

### Example 184:

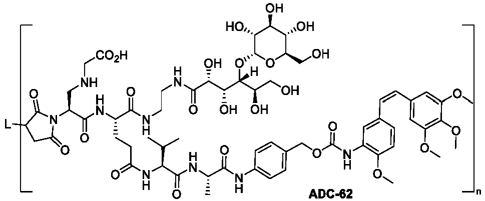

ADC-62 was synthesized according to the general preparation method of ligand-drug conjugates.

### Example 185:

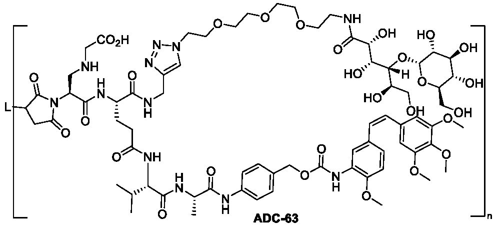

ADC-63 was synthesized according to the general preparation method of ligand-drug conjugates.

### Example 186:

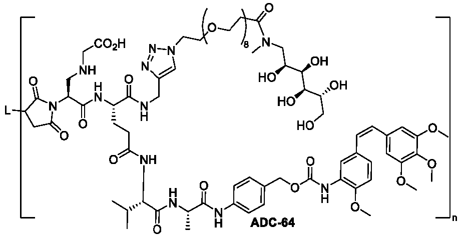

ADC-64 was synthesized according to the general preparation method of ligand-drug conjugates.

### Example 187:

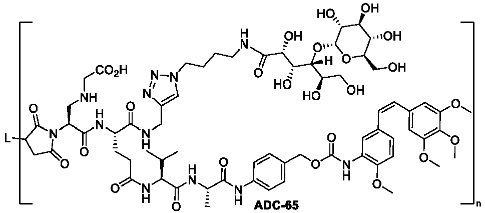

ADC-65 was synthesized according to the general preparation method of ligand-drug conjugates.

### Example 188:

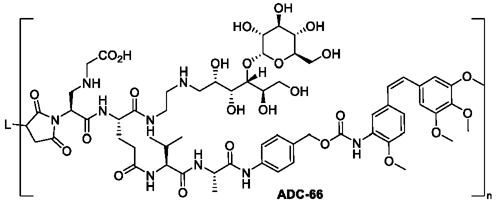

ADC-66 was synthesized according to the general preparation method of ligand-drug conjugates.

### Example 189:

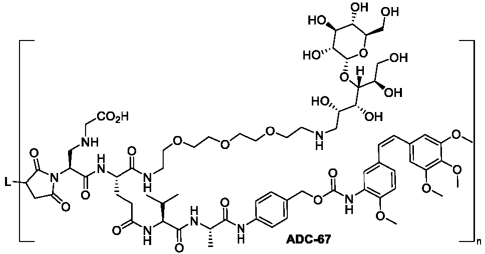

ADC-68 was synthesized according to the general preparation method of ligand-drug conjugates.

### Example 190:

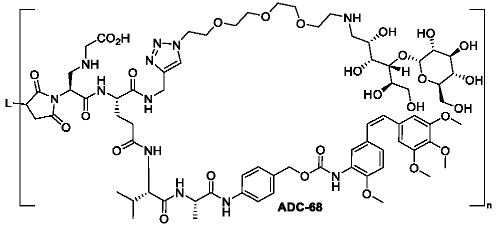

ADC-68 was synthesized according to the general preparation method of ligand-drug conjugates.

### Example 191:

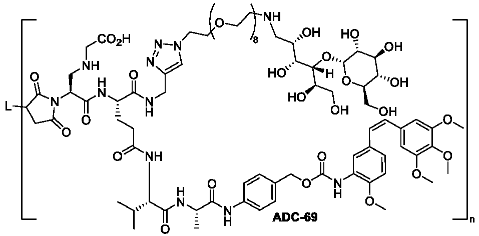

ADC-69 was synthesized according to the general preparation method of ligand-drug conjugates.

### Example 192:

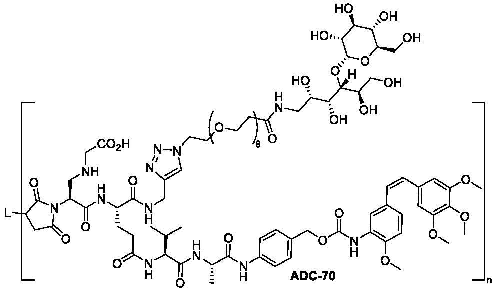

ADC-70 was synthesized according to the general preparation method of ligand-drug conjugates.

### Example 193:

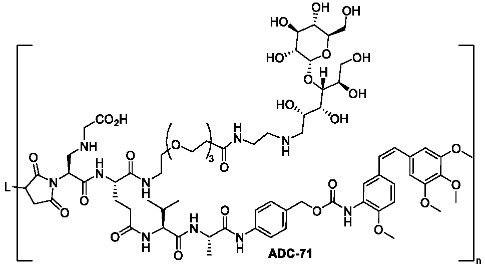

ADC-71 was synthesized according to the general preparation method of ligand-drug conjugates.

### Example 194:

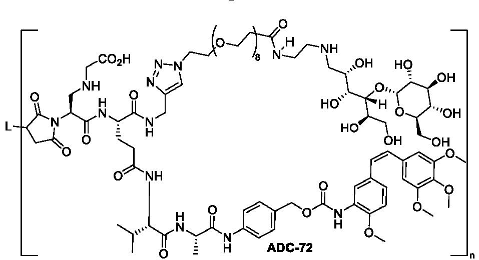

ADC-72 was synthesized according to the general preparation method of ligand-drug conjugates.

### Example 195:

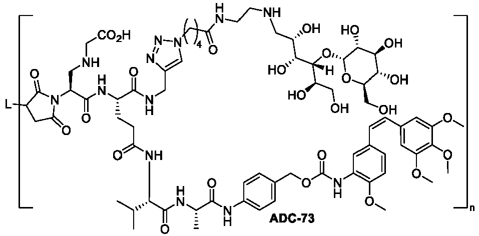

ADC-73 was synthesized according to the general preparation method of ligand-drug conjugates.

### Example 196:

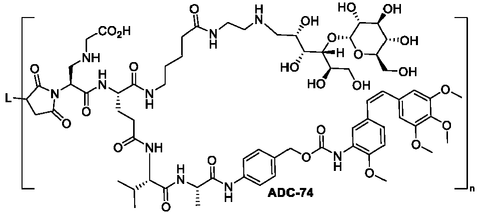

ADC-74 was synthesized according to the general preparation method of ligand-drug conjugates.

### Example 197:

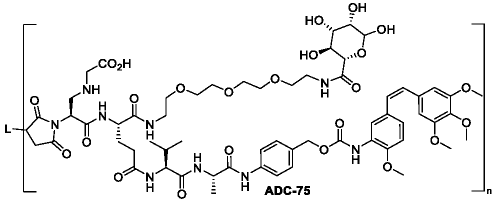

ADC-75 was synthesized according to the general preparation method of ligand-drug conjugates.

### Example 198:

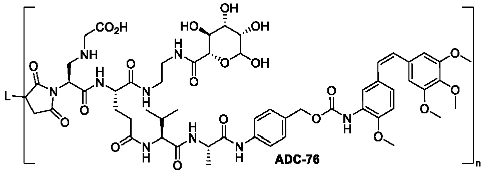

ADC-76 was synthesized according to the general preparation method of ligand-drug conjugates.

### Example 199:

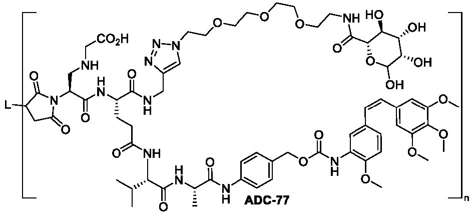

ADC-77 was synthesized according to the general preparation method of ligand-drug conjugates.

### Example 200:

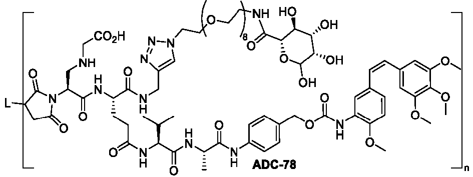

ADC-78 was synthesized according to the general preparation method of ligand-drug conjugates.

### Example 201:

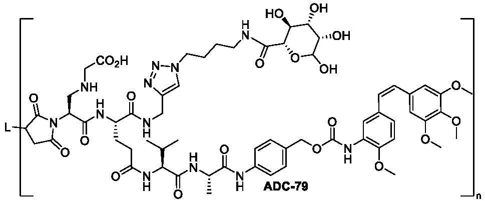

ADC-79 was synthesized according to the general preparation method of ligand-drug conjugates.

### Example 202:

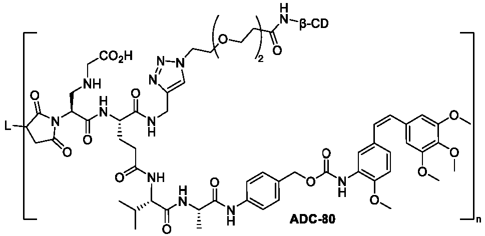

ADC-80 was synthesized according to the general preparation method of ligand-drug conjugates.

### Example 203:

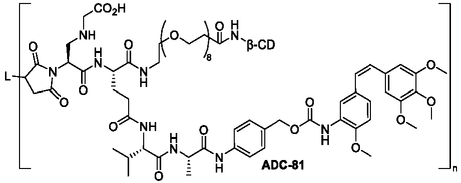

ADC-81 was synthesized according to the general preparation method of ligand-drug conjugates.

### Example 204:

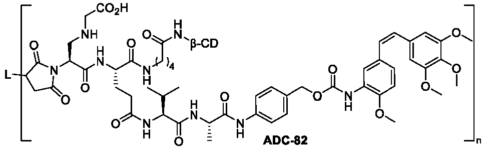

ADC-82 was synthesized according to the general preparation method of ligand-drug conjugates.

### Example 205:

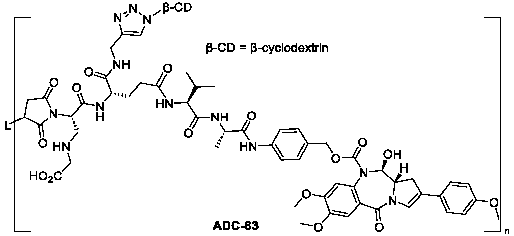

ADC-83 was synthesized according to the general preparation method of ligand-drug conjugates.

### Example 206:

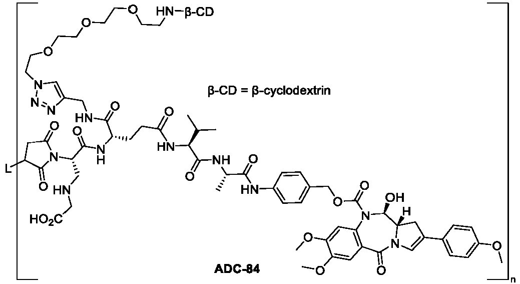

ADC-84 was synthesized according to the general preparation method of ligand-drug conjugates.

### Example 207:

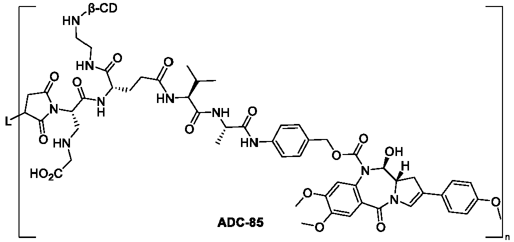

ADC-85 was synthesized according to the general preparation method of ligand-drug conjugates.

### Example 208:

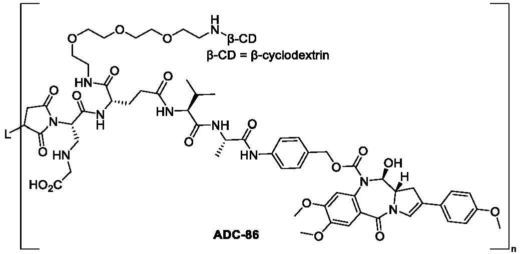

ADC-86 was synthesized according to the general preparation method of ligand-drug conjugates.

### Example 209:

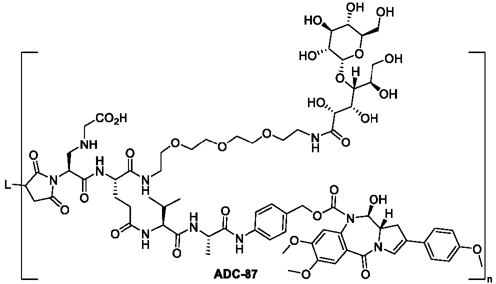

ADC-87 was synthesized according to the general preparation method of ligand-drug conjugates.

### Example 210:

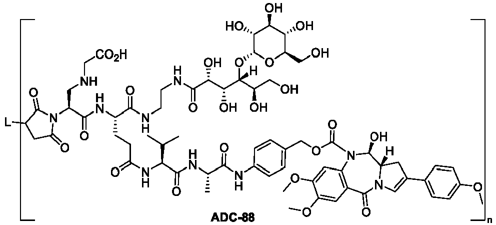

ADC-88 was synthesized according to the general preparation method of ligand-drug conjugates.

### Example 211:

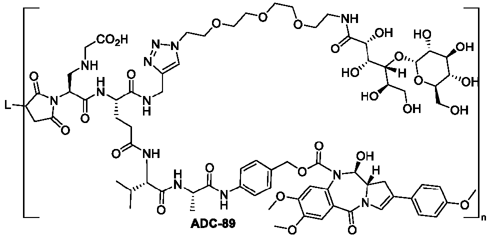

ADC-89 was synthesized according to the general preparation method of ligand-drug conjugates.

### Example 212:

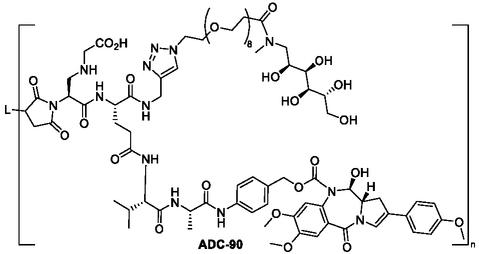

ADC-90 was synthesized according to the general preparation method of ligand-drug conjugates.

### Example 213:

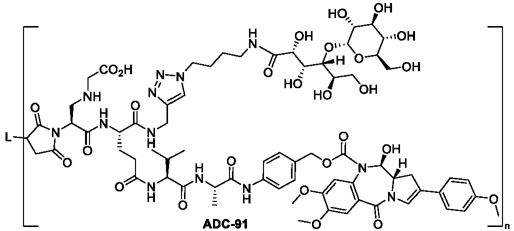

ADC-91 was synthesized according to the general preparation method of ligand-drug conjugates.

### Example 214:

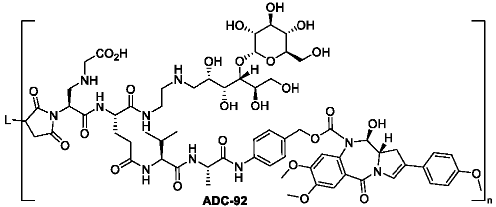

ADC-92 was synthesized according to the general preparation method of ligand-drug conjugates.

### Example 215:

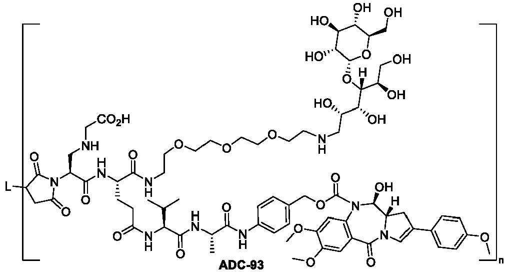

ADC-93 was synthesized according to the general preparation method of ligand-drug conjugates.

### Example 216:

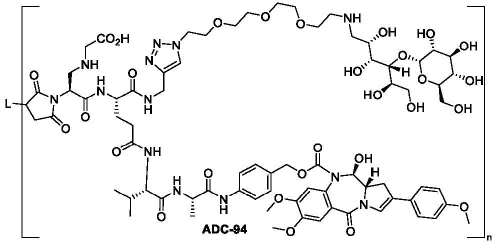

ADC-94 was synthesized according to the general preparation method of ligand-drug conjugates.

### Example 217:

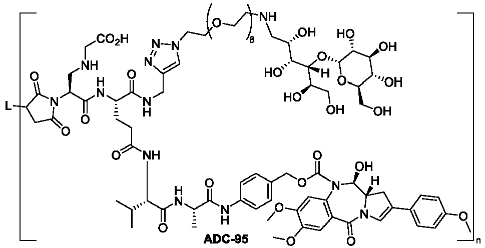

ADC-95 was synthesized according to the general preparation method of ligand-drug conjugates.

### Example 218:

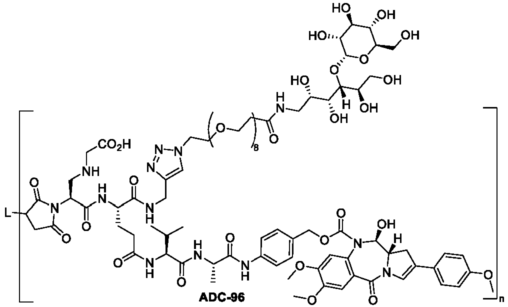

ADC-96 was synthesized according to the general preparation method of ligand-drug conjugates.

### Example 219:

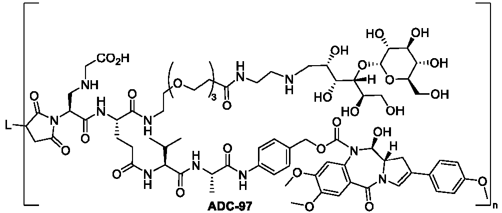

ADC-97 was synthesized according to the general preparation method of ligand-drug conjugates.

### Example 220:

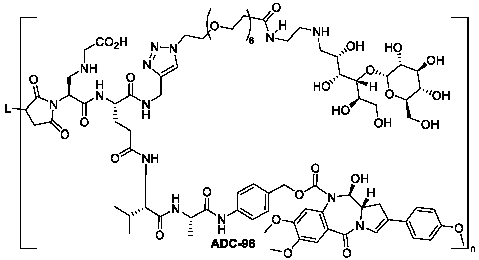

ADC-98 was synthesized according to the general preparation method of ligand-drug conjugates.

### Example 221:

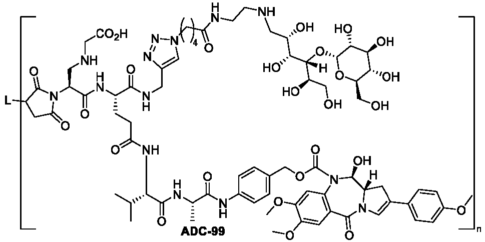

ADC-99 was synthesized according to the general preparation method of ligand-drug conjugates.

### Example 222:

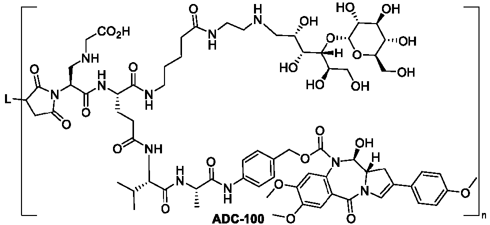

ADC-100 was synthesized according to the general preparation method of ligand-drug conjugates.

### Example 223:

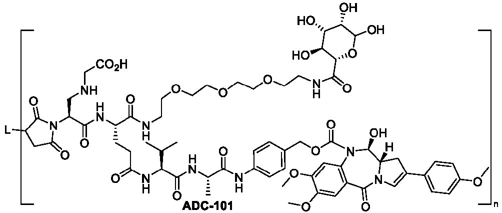

ADC-101 was synthesized according to the general preparation method of ligand-drug conjugates.

### Example 224:

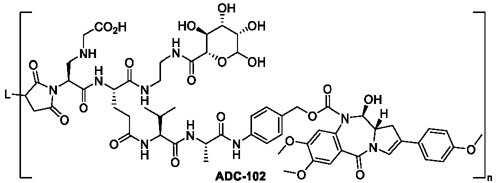

ADC-102 was synthesized according to the general preparation method of ligand-drug conjugates.

### Example 225:

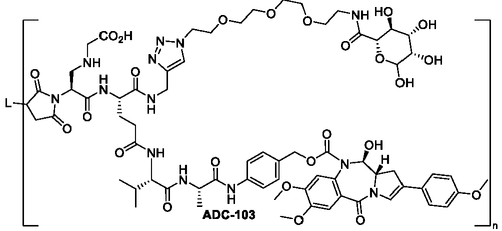

ADC-103 was synthesized according to the general preparation method of ligand-drug conjugates.

### Example 226:

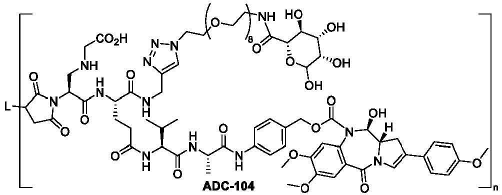

ADC-104 was synthesized according to the general preparation method of ligand-drug conjugates.

### Example 227:

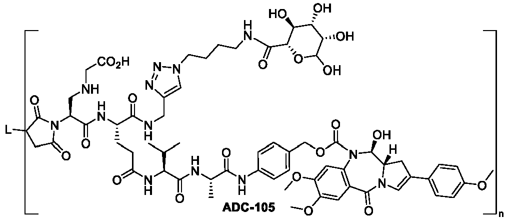

ADC-105 was synthesized according to the general preparation method of ligand-drug conjugates.

### Example 228:

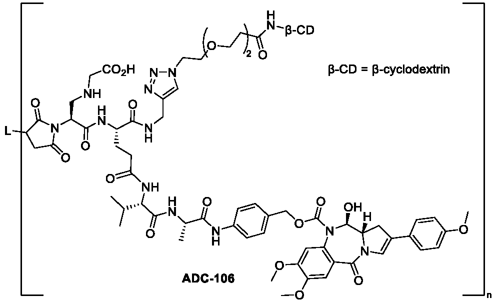

ADC-106 was synthesized according to the general preparation method of ligand-drug conjugates.

### Example 229:

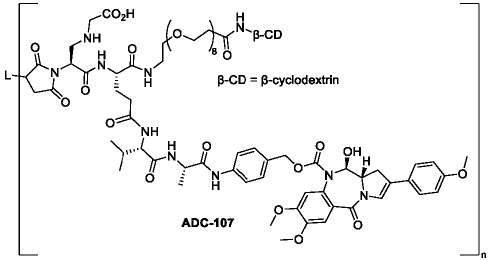

ADC-107 was synthesized according to the general preparation method of ligand-drug conjugates.

### Example 230:

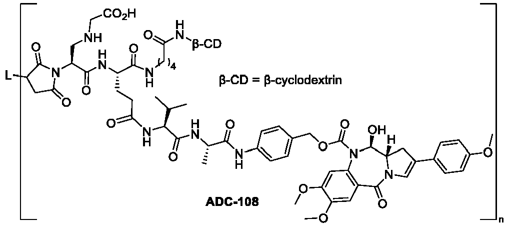

ADC-108 was synthesized according to the general preparation method of ligand-drug conjugates.

### Example 231: Control ADC-109

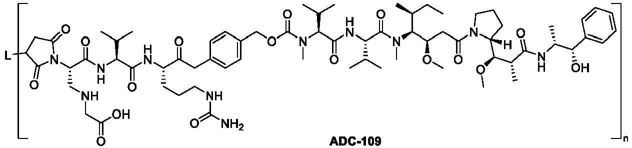

The payload of ADC-109 was prepared according to the synthesis route of in Example 11 of the patent application "WO2018233571A1", and the ligand-drug conjugate was prepared according to the general method of the present invention.

### Example 232: Control ADC-110

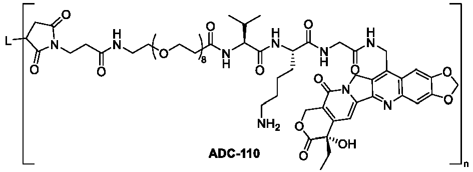

The payload of ADC-110 was prepared according to the synthesis route in Example 4-3 on page 148 of the patent application "WO2019195665A1", and the ligand-drug conjugate was prepared according to the general method of the present invention.

### Example 233: Control ADC-111

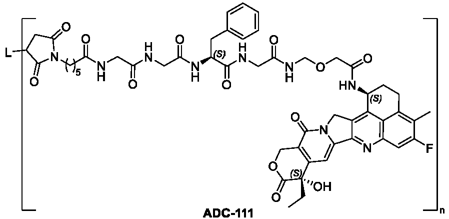

The payload of ADC-111 was prepared according to the synthesis route of the compound of Formula (14) of the patent application "WO2019044946A1", and the ligand-drug conjugate was prepared according to the general method of the present invention.

### Example 234: Determination of monomer rate of ADCs

### 1) Determination of monomer rate using SEC-HPLC:

Chromatographic column: Biocore SEC-300 5 µm, 4.6×300 mm; manufacturer: NanoChrom, Product code: B213-050030-04630S; mobile phase: 50 mM PB+300 mM NaCl+200 mM Arg+5% IPA, pH=6.5

**Table 1 Method parameters**

| Parameter | Setting | Parameter | Setting |
|---|---|---|---|
| Flow rate | 0.3 mL/min | Sample load | 20 µg |
| Wavelength | 214 nm and 280 nm | Max. pressure | 150 bar/15 MPa/2175 PSI |
| Column temperature | 30 °C | Gradient | Isocratic |
| Sample tray temperature | Room temperature | Run time | 20 min |

### 2) Results:

**Table 2 Monomer rates of ADCs synthesized in some examples**

| Description | Degradation % | Aggregate % | Monomer % |
|---|---|---|---|
| ADC-29 | 0.000 | 8.448 | 91.552 |
| ADC-56 | 0.000 | 5.215 | 94.785 |
| ADC-111 | 0.000 | 0.793 | 99.207 |

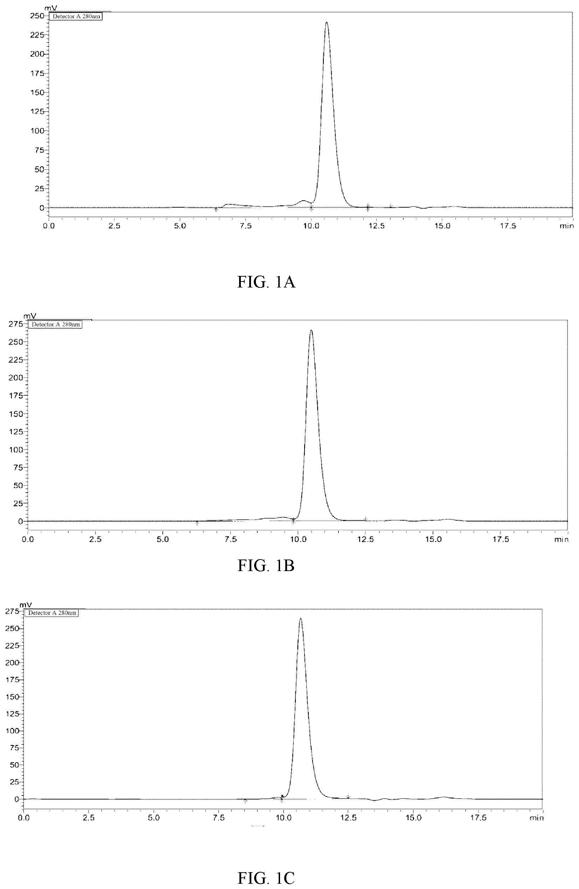

FIGS. 1A-1C are SEC-HPLC spectra of ADC-29, ADC-56, and ADC-111, respectively, showing the aggregation of ADCs.

### 3) Conclusion:

The ADCs of the present invention have the characteristics of low degradation and low aggregation and have an excellent property of high monomer rate.

### Example 235: Determination of DAR

### 1) Method: The RP-HPLC was used to determine the DAR:

Chromatographic column: Proteomix RP-1000 4.6×100mm 5µm 1000A; manufacturer: Sepax Product code: 465950-4610

**Table 3 Method parameters**

| Parameter | Setting | | | |
|---|---|---|---|---|
| Mobile phase | A: 0.1%TFA in water; B: 0.1%TFA in acetonitrile | | | |
| Flow rate | 0.5 mL/min | | | |
| Wavelength | 214 nm and 280 nm | | | |
| Column temperature | 65 °C | | | |
| Sample tray temperature | Room temperature | | | |
| Sample load | 25 ug | | | |
| Max. pressure | 100bar/10MPa/1450PSI | | | |
| Gradient | Time (min) | Flow rate (mL/min) | Mobile phase A (%) | Mobile phase B (%) |
| | 0.0 | 0.5 | 75 | 25 |
| | 3 | 0.5 | 75 | 25 |
| | 28 | 0.5 | 50 | 50 |
| | 30 | 0.5 | 5 | 95 |
| | 32 | 0.5 | 5 | 95 |
| | 33 | 0.5 | 75 | 25 |
| | 40 | 0.5 | 75 | 25 |

### 2) Results:

**Table 4 DAR of ADCs synthesized in some examples**

| Sample | RP-DAR |
|---|---|
| ADC-29 | 8.32 |
| ADC-56 | 8.01 |
| ADC-111 | 8.66 |

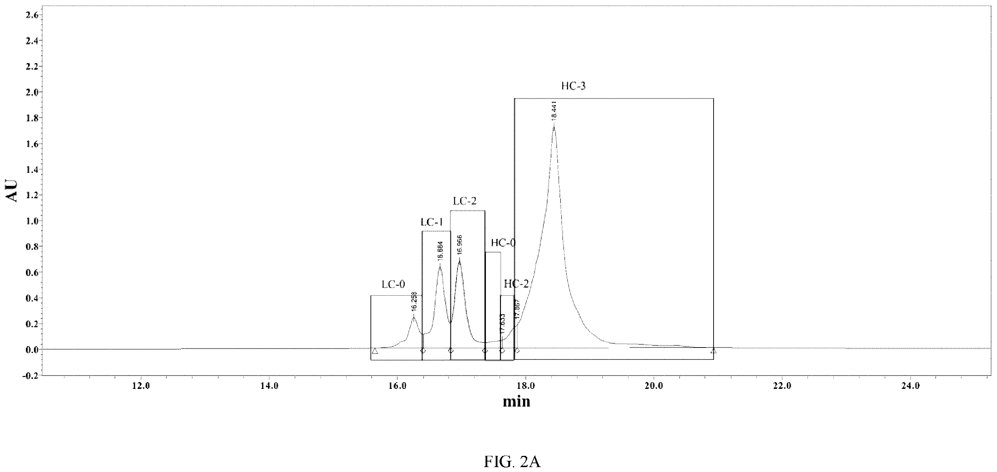

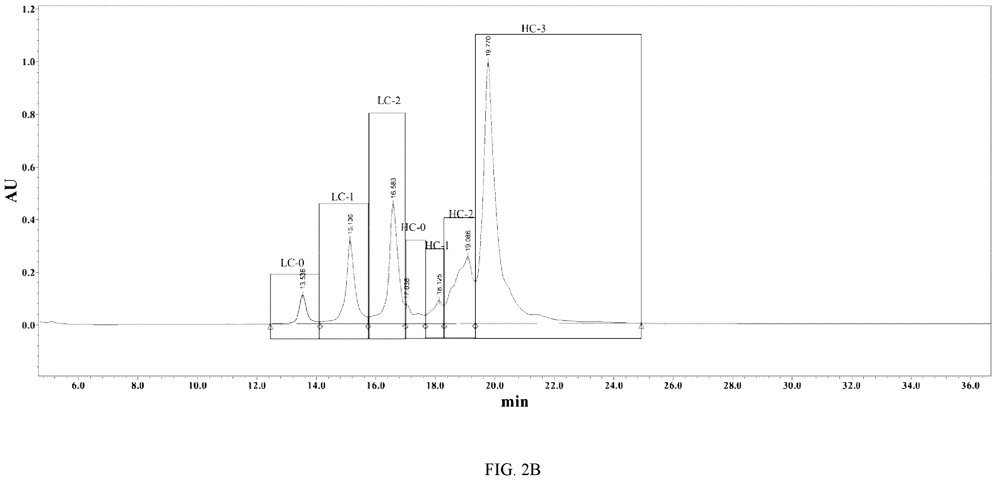

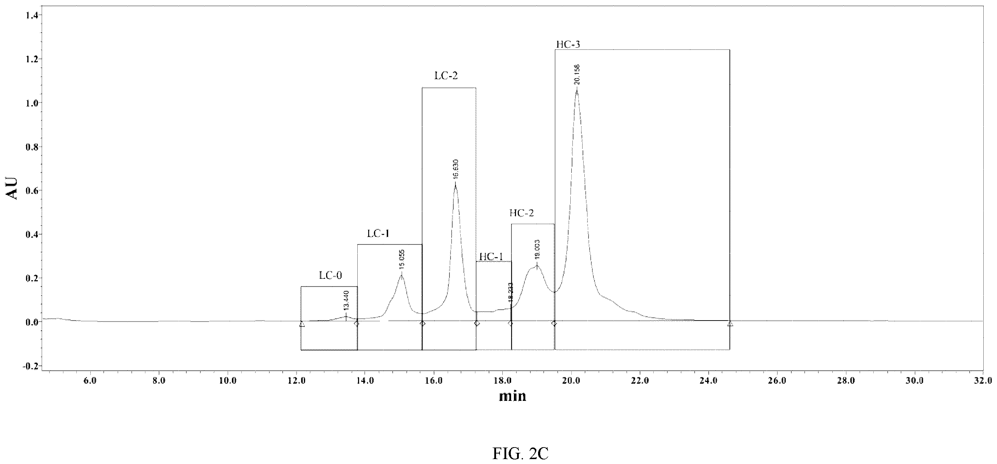

FIGS. 2A-2C are the RP-HPLC spectra of ADC-29, ADC-56, and ADC111 respectively, showing the DAR of ADCs.

### 3) Conclusion:

The ADCs of the present invention have the excellent property of high DAR, and can significantly increase the drug concentration at the target location under the same dosage of ADCs.

### Example 236: Plasma stability

### 1) Operation

A certain amount of ADC sample was added to human plasma with human IgG removed. Three tubes of each ADC was set up. Incubation was performed in a 37°C water bath. After incubation for 7 days, 14 days, and 21 days, the ADC samples were taken out, 100 uL of Protein A resin (MabSelect SuReTM LX Lot: #10221479GE, washed with PBS) was added to each tube. The adsorption was carried out by shaking in a vertical mixer for 2 h, and washing and elution steps were then carried out to obtain incubated ADC samples. The ADC samples incubated for a specific time were tested by RP-HPLC.

### 2) Results:

**Table 5 Plasma stability data of ADCs synthesized in some examples**

| Description | 37°C SEC (aggregate %/monomer %/degradation %) | | | | | | | | |
|---|---|---|---|---|---|---|---|---|---|
| | Day 7 | | | Day 14 | | | Day 21 | | |
| CD33-mAb | 0.452 | 99.326 | 0.222 | 0.431 | 99.158 | 0.411 | 0.405 | 98.841 | 0.754 |
| ADC-1 | 7.608 | 91.994 | 0.398 | 7.913 | 91.201 | 0.886 | 7.866 | 91.395 | 0.739 |
| ADC-28 | 9.796 | 90.100 | 0.104 | 10.902 | 88.188 | 0.910 | 11.437 | 88.032 | 0.531 |
| ADC-109 | 38.204 | 61.591 | 0.205 | 40.880 | 58.939 | 0.181 | 44.817 | 55.008 | 0.175 |

### 3) Conclusion

Compared with the control ADC-109, the hydrophilicity-enhanced ADCs of the present invention, such as ADC-1 and ADC-28, had a higher monomer rate and less aggregation in plasma after 21 days of incubation in plasma at 37°C, proving that the ADCs of the present invention have better hydrophilicity and stability.

### Example 237: In vitro efficacy testing of ADCs

### 1) Method:

In this example, various human tumor cell lines (A431 (human epidermal carcinoma cell), HL-60 (human promyelocytic acute leukemia cell), TF-1 (human blood leukemia cell), HEL92.1.7 (human erythroleukemia cell), MV4-11 (human myelomonocytic leukemia cell) and MOLM-13 (human acute myeloid leukemia cell)) were used as experimental models to evaluate the in vitro efficacy of ADCs. A certain number of tumor cell lines were inoculated in 96-well plates, and gradiently diluted test antibodies and corresponding ADCs were added to the cells and 5 days later, the cell viability was tested using Alamar Blue or MTS. The inhibitory effects of the test antibodies and ADCs against tumor cell lines were evaluated by calculating IC₅₀. The antibody drug with a starting concentration of 500 nM was diluted 7 folds, with a total of 8 concentration points, and the treatment was carried out for 5 days. The final algorithm was based on survival rate = (experimental group - blank) / (control - blank) × 100%, and then Graph Pad Prism was used to fit the curve and calculate the median inhibitory concentration (IC₅₀).

### 2) Results:

**Table 6 In vitro efficacy results of the tested ADCs:**

| Groups | IC₅₀ (nM) | | | | | |
|---|---|---|---|---|---|---|
| | A431 | HL60 | TF-1 | HEL92.1.7 | MV4-11 | MOLM-13 |
| ADC-1 | 134.5 | 0.442 | 0.02 | >500 | 104 | 1.564 |
| ADC-28 | 87.26 | 0.003 | 0.006 | 83.9 | 56.51 | 0.005 |
| ADC-109 Control | 316.1 | 0.006 | 0.022 | 104.1 | 187.4 | 0.005 |
| ADC-29 | 74.12 | 17.58 | 0.029 | 19.19 | 1.945 | 0.023 |
| ADC-42 | 51.83 | 9.108 | 3.427 | 18.5 | 0.996 | 0.035 |
| ADC-56 | 150.5 | 32.98 | 4.321 | 26.09 | 19.95 | 0.007 |
| ADC-111 Control | 302.2 | 137.75 | 0.054 | 162.64 | 12.73 | 0.266 |

### 3) Conclusion:

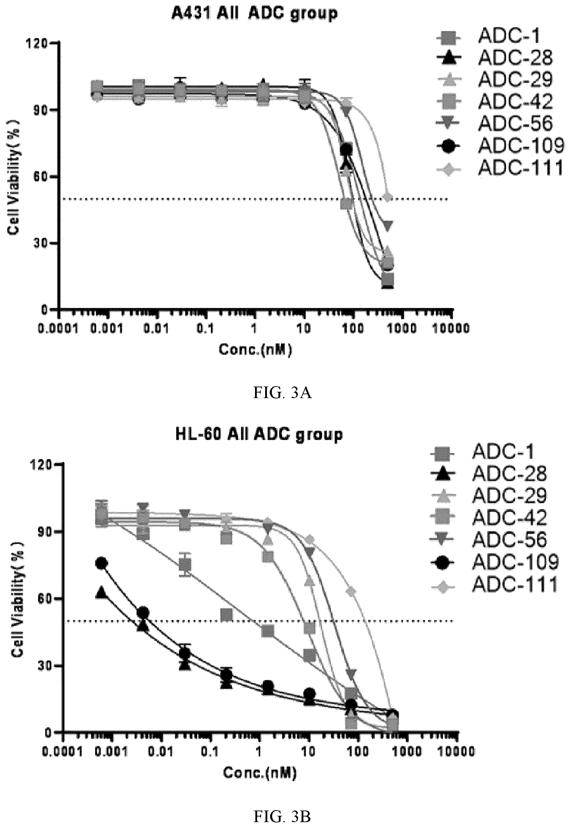

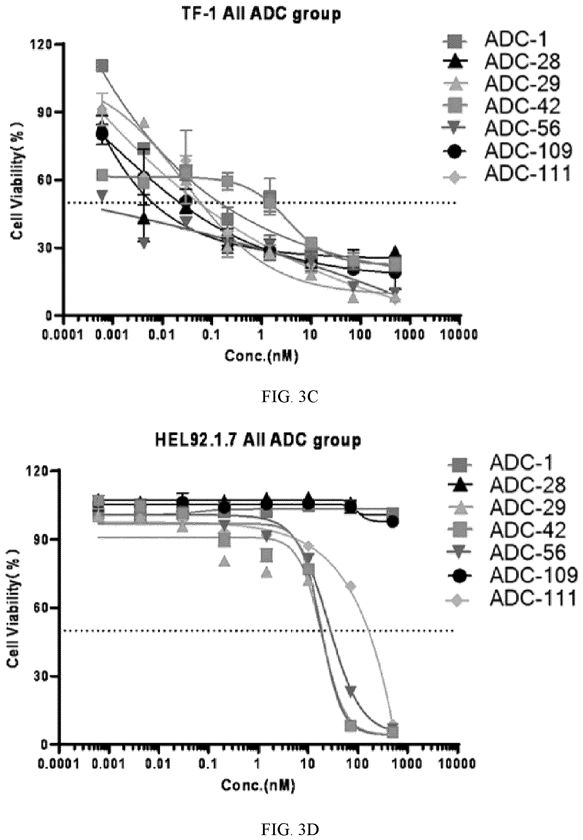

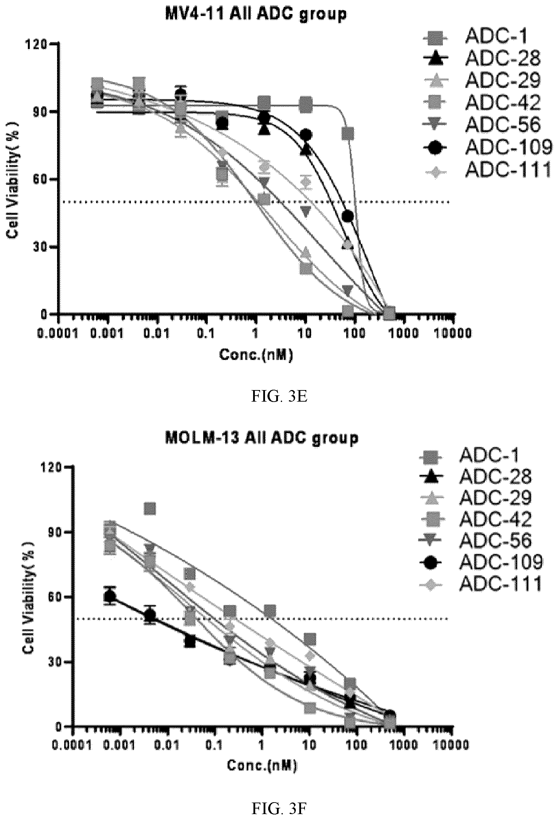

The results are shown in Table 6 and FIGS. 3A-3F. In the in vitro efficacy tests of various human tumor cell lines (A431, HL-60, TF-1, HEL92.1.7, MV4-11 and MOLM-13), the ADCs of the present invention show excellent activity.

### Example 238: In vivo efficacy test of ADCs

The present invention established a subcutaneous transplant tumor model of HEL92.1. 7 BALB/c Nude mice to evaluate the in vivo efficacy of ADCs.

### 1) Method:

5×10⁶ HEL92.1.7 cells (0.1 mL/mouse) were subcutaneously injected into the right scapula of 5-6 week-old BALB/c Nude mice. When the average tumor size of the mice grew to about 230-270 mm³, the mice were randomly divided into a vehicle control group (Vehicle), a monoclonal antibody (CD33-mAb) treatment group (10 mg/kg), and an ADC treatment group (5 mg/kg, 10 mg/kg), with 5 mice in each group, and were administrated (D0). The mice in each group were administrated with a tail vein injection of 10 mL/kg body weight.

### 2) Results:

**Table 7 Drug efficacy of groups in the subcutaneous transplant tumor model of HEL92.1.7 BALB/c-Nude mice**

| Group information | | Tumor volume (mm³)* | | | | |
|---|---|---|---|---|---|---|
| | | Day 0 | Day 3 | Day 7 | Day 10 | Day 14 |
| 1 | Vehicle; 1 dosage/week | 269.92 | 449.01 | 755.49 | 1222.66 | 1544.77 |
| 2 | mAb(1 dosage/week;10 mg/kg) | 242.24 | 381.55 | 552.42 | 934.47 | 1152.12 |
| 3 | ADC-29 (1 dosage/week 5 mg/kg) | 232.44 | 79.09 | 77.51 | 35.77 | 15.71 |
| 4 | ADC-29 (1 dosage/week 10 mg/kg) | 246.19 | 53.37 | 23.70 | 0.00 | 0.00 |
| 5 | ADC-56 (1 dosage/week 5 mg/kg) | 233.57 | 75.19 | 27.80 | 8.82 | 0.00 |
| 6 | ADC-56 (1 dosage/week 10 mg/kg) | 235.81 | 41.11 | 16.64 | 3.14 | 0.00 |

| | | | | | | |
|---|---|---|---|---|---|---|
| *The average transplant tumor volume of mice in each test group. | | | | | | |

**Table 8 Body weights of mice in groups in the subcutaneous transplant tumor model of HEL92.1.7 BALB/c-Nude mice**

| Group information | | Body weight (g)* | | | | |
|---|---|---|---|---|---|---|
| | | Day 0 | Day 3 | Day 7 | Day 10 | Day 14 |
| 1 | Vehicle; 1 dosage/week | 22.12 | 23.52 | 23.71 | 24.60 | 25.17 |
| 2 | mAb (1 dosage/week;10 mg/kg) | 22.08 | 22.05 | 22.36 | 23.54 | 23.76 |
| 3 | ADC-29 (1 dosage/week 5 mg/kg) | 22.27 | 22.44 | 21.81 | 22.67 | 23.39 |
| 4 | ADC-29 (1 dosage/week 10 mg/kg) | 22.36 | 22.78 | 22.74 | 23.27 | 22.84 |
| 5 | ADC-56 (1 dosage/week 5 mg/kg) | 22.22 | 22.08 | 22.65 | 23.30 | 23.66 |
| 6 | ADC-56 (1 dosage/week 10 mg/kg) | 22.13 | 21.96 | 21.67 | 22.06 | 22.78 |

| | | | | | | |
|---|---|---|---|---|---|---|
| *The average body weight of mice in each test group. | | | | | | |

### 3) Conclusion:

As shown in Table 7, compared with the blank control and monoclonal antibody treatment groups, the ADCs of the present invention can significantly inhibit the growth of tumors at different doses, and show dose-dependence to a certain extent.

As shown in Table 8, during the administration process at different doses, the body weight of the test animals in each group was normal without significant changes, indicating that the ADCs of the present invention have no obvious toxic or side effects.

Although specific embodiments of the invention have been described in detail, those skilled in the art will understand that: according to all the teachings that have been disclosed, various modifications and changes can be made to the details, and these modifications and changes are all within the scope of the invention. The full scope of the invention was given by the appended claims and any equivalents thereof.

## Claims

1. A ligand-drug conjugate of formula I, formula II or formula III, or a pharmaceutically acceptable salt or solvate thereof: 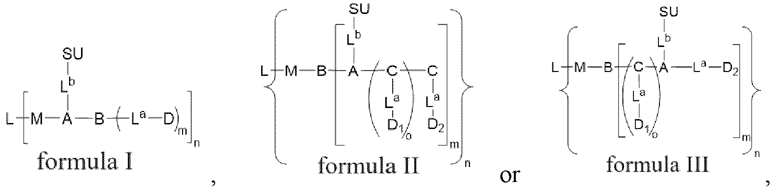 wherein
L is selected from ligands;
M is selected from any linker units or bonds;
A is selected from any linking scaffolds;
B is present or absent, and when present, is selected from any linking structures or bonds;
C is present or absent, and when present, is selected from any branching units or bonds; D, D₁, D₂ are the same or different, and are each independently selected from drugs; SU is selected from sugars or derivatives thereof;
L^{a} and L^{b} are the same or different, and are each independently selected from any linking units or bonds;
m is selected from integers of 1 to 5;
n is selected from integers of 1 to 10;
o is selected from integers of 1 to 10.

2. The ligand-drug conjugate or a pharmaceutically acceptable salt or solvate thereof according to claim 1, wherein the ligand L is selected from an antibody, a functional antibody fragment and a protein with a targeting effect.

3. The ligand-drug conjugate or a pharmaceutically acceptable salt or solvate thereof according to any one of claim 1 or 2, wherein the ligand L is selected from antibodies, including but not limited to chimeric antibodies, humanized antibodies, fully human antibodies or mouse antibodies.

4. The ligand-drug conjugate or a pharmaceutically acceptable salt or solvate thereof according to any one of claims 1-3, wherein the linker unit M is non-restrictively selected from structures or bonds represented by the following formulas; when the structure contains a cycloalkyl or a heterocyclyl, the linker unit M may also be selected from a derived structure thereof in which the cycloalkyl or heterocyclyl is in open form, 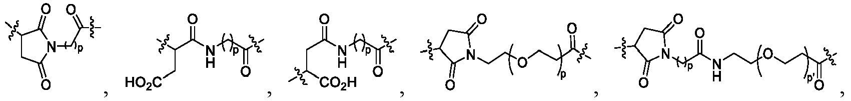 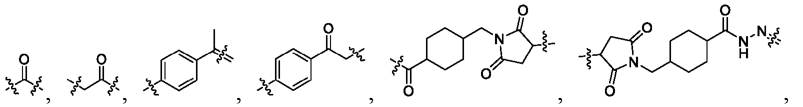 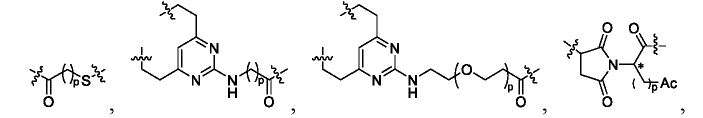 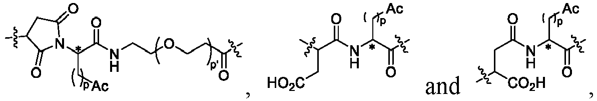 wherein
the position indicated by a wavy line is non-restrictively connected to the ligand, the linking scaffold A, the linking structure B or the branching unit C;
carbon at the position indicated by * is a chiral carbon and has an R or S configuration; p and p' are each independently selected from integers of 1 to 10;
Ac is non-restrictively selected from a residue of a natural or non-natural amino acid, a polyethylene glycol segment with 1-20 repeating units, a phosphate group, a carboxylic acid group, a sulfonic acid group, a sulfinic acid group or the following structure, 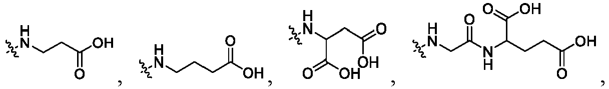 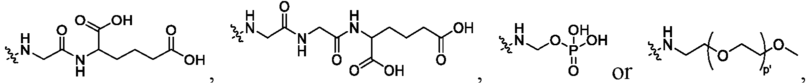
wherein the position indicated by a wavy line is connected to the carbon atom at the position indicated by *.

5. The ligand-drug conjugate or a pharmaceutically acceptable salt or solvate thereof according to any one of claims 1-4, wherein the linker unit M is non-restrictively selected from structures represented by the following formulas, or stereoisomers thereof or derived structures thereof in which rings in succinimide groups are in open form, 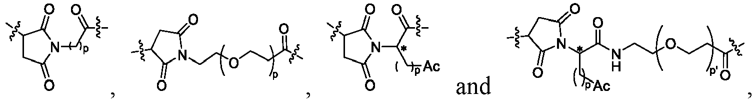 wherein
the position indicated by a wavy line is non-restrictively connected to the ligand, the linking scaffold A, the linking structure B or the branching unit C;
carbon at the position indicated by * is a chiral carbon and has an R or S configuration; p and p' are each independently selected from integers of 1 to 10;
Ac is non-restrictively selected from a residue of a natural or non-natural amino acid, a polyethylene glycol segment with 1-20 repeating units, a phosphate group, a carboxylic acid group, a sulfonic acid group, a sulfinic acid group or the following structure, 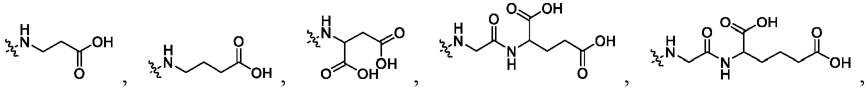 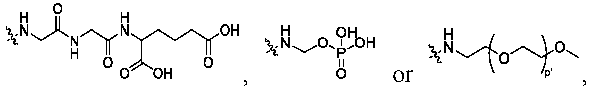
wherein the position indicated by a wavy line is connected to the carbon atom at the position indicated by *.

6. The ligand-drug conjugate or a pharmaceutically acceptable salt or solvate thereof according to any one of claims 1-5, wherein the linker unit M is non-restrictively selected from structures represented by the following formulas, or derived structures thereof in which rings in succinimide groups are in open form, 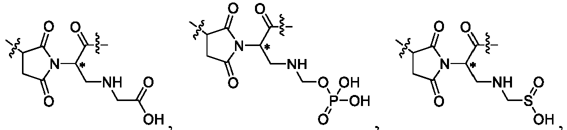 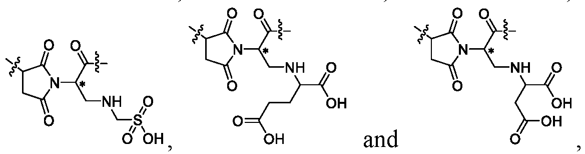
preferably 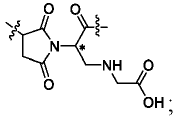
wherein
carbon at the position indicated by * is a chiral carbon and has an R or S configuration;
the position indicated by a wavy line is non-restrictively connected to the ligand, the linking scaffold A, the linking structure B or the branching unit C.

7. The ligand-drug conjugate or a pharmaceutically acceptable salt or solvate thereof according to any one of claims 1-6, wherein the linking scaffold A is non-restrictively selected from one or more of natural or non-natural amino acids or from the following structures, 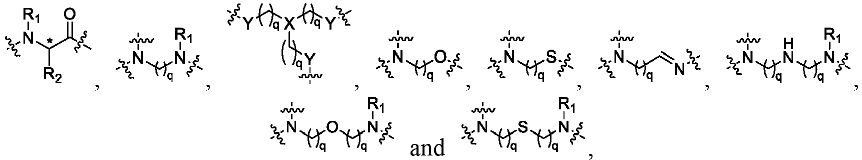 wherein
X is selected from N, CH, C3-C8 cycloalkyl, 3- to 8-membered heterocyclyl, aryl, substituted aryl, and heteroaryl;
Y is selected from -NH-, -O-, -S-, -CO-, -CO₂-, -CONH-, -NHCO-, -SO-, -SO₂-, -OSO₂-, and -OP=O(OH)O-;
q is selected from integers of 1 to 10;
the position indicated by a wavy line is non-restrictively connected to the linker unit M, the linking unit L^{a} or L^{b}, the linking structure B or the branching unit C;
R₁ is selected from hydrogen atom, deuterium atom, halogen, C1-C6 alkyl, C1-C6 substituted alkyl, C3-C8 cycloalkyl C1-C6 alkyl, C1-C6 alkoxy C1-C6 alkyl, carboxyl,
3- to 8-membered heterocyclyl, aryl, substituted aryl and heteroaryl;
R₂ is non-restrictively selected from: 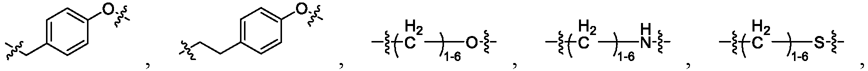 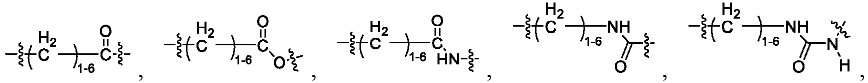 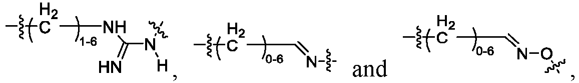
wherein a wavy line on the left indicates the position where R₂ is connected to the chiral carbon indicated by *, and a wavy line on the right indicates one of any three connection sites of the linking scaffold A.

8. The ligand-drug conjugate or a pharmaceutically acceptable salt or solvate thereof according to any one of claims 1-6, wherein the linking scaffold A is non-restrictively selected from the following structures or stereoisomers thereof, 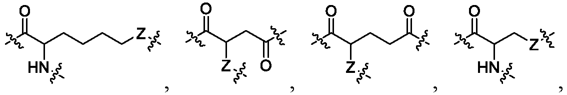 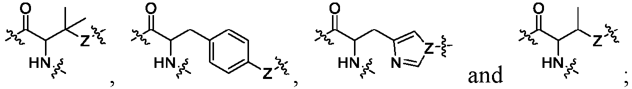 wherein
Z is selected from -NH-, -O- and -S-;
the position indicated by a wavy line is non-restrictively connected to the linker unit M, the linking unit L^{a} or L^{b}, the linking structure B or the branching unit C;
preferably, the linking scaffold A is 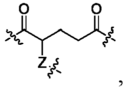
wherein, Z is -NH-.

9. The ligand-drug conjugate or a pharmaceutically acceptable salt or solvate thereof according to any one of claims 1-8, wherein the B is non-restrictively selected from any linking structures or bonds.

10. The ligand-drug conjugate or a pharmaceutically acceptable salt or solvate thereof according to any one of claims 1-9, wherein the C is present or absent, and when present, is non-restrictively selected from one or more of natural or non-natural amino acids.

11. The ligand-drug conjugate or a pharmaceutically acceptable salt or solvate thereof according to any one of claims 1-10, wherein the drugs D, D₁, and D₂ are the same or different, and are each independently and non-restrictively selected from an anti-tumor drug, an autoimmune disease drug, an anti-infective disease drug (such as an antiviral drug), a radioactive isotope, a chromogenic molecule, or a pharmaceutically acceptable salt or solvate thereof.

12. The ligand-drug conjugate or a pharmaceutically acceptable salt or solvate thereof according to any one of claims 1-11, wherein the drugs D, D₁, and D₂ are the same or different, and are independently and non-restrictively selected from anti-tumor drugs, including but not limited to DNA damaging agents, RNA damaging agents, enzyme inhibitors, or microtubule inhibitors.

13. The ligand-drug conjugate or a pharmaceutically acceptable salt or solvate thereof according to any one of claims 1-12, wherein the SU is non-restrictively selected from a natural or non-natural monosaccharide, disaccharide, polysaccharide and derivatives thereof.

14. The ligand-drug conjugate or a pharmaceutically acceptable salt or solvate thereof according to any one of claims 1-13, wherein the natural or non-natural monosaccharide, disaccharide, polysaccharide or derivatives thereof are non-restrictively selected from the following structures, 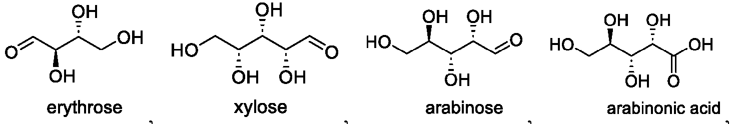 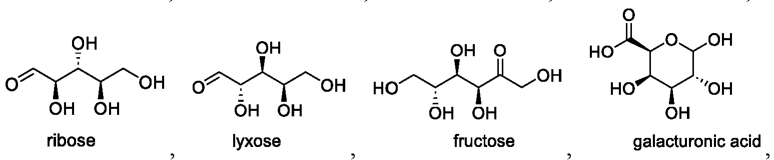 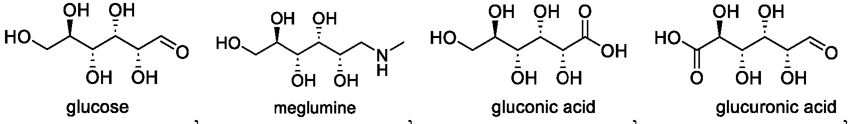 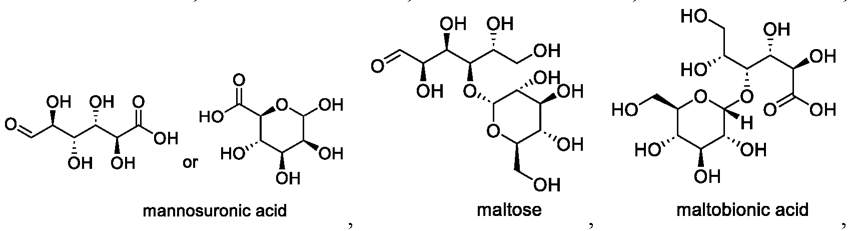 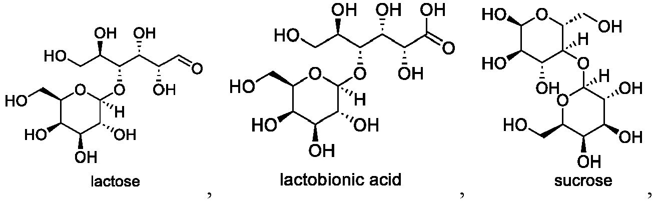 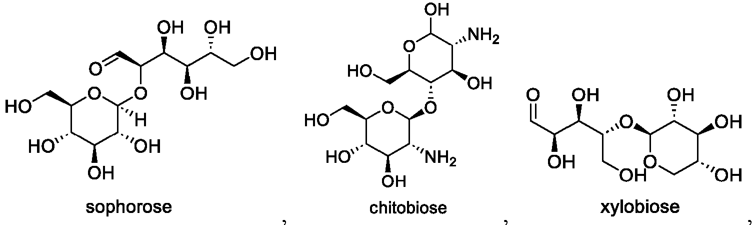 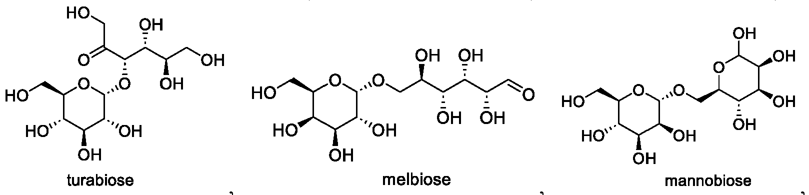 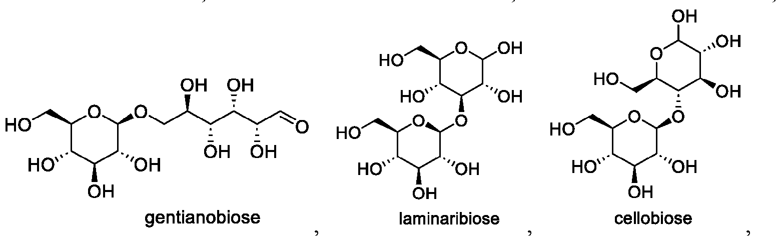 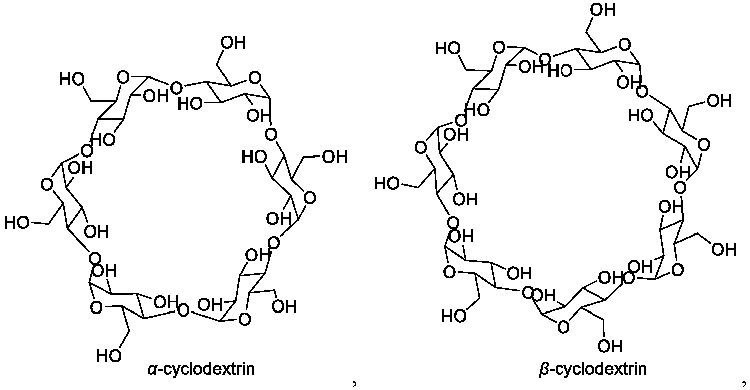 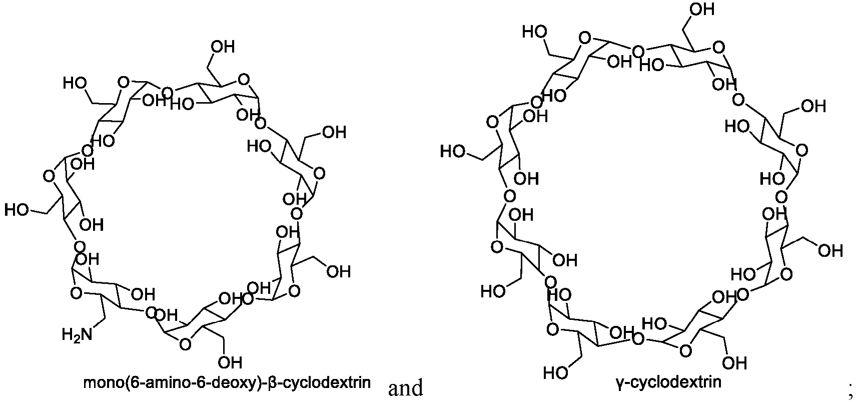 preferably, the SU is selected from methylglucamine, maltose, maltobionic acid, mannuronic acid, β-cyclodextrin and mono(6-amino-6-deoxy)-β-cyclodextrin.

15. The ligand-drug conjugate or a pharmaceutically acceptable salt or solvate thereof according to any one of claims 1-14, wherein the SU is non-restrictively linked to L^{b} in a covalent manner.

16. The ligand-drug conjugate or a pharmaceutically acceptable salt or solvate thereof according to any one of claims 1-15, wherein the linking units L^{a} and L^{b} are the same or different, and are each independently and non-restrictively selected from one or more of a chemically unstable linking unit, an enzyme-catalyzed cleavage linking unit and a non-cleavable linking unit.

17. The ligand-drug conjugate or a pharmaceutically acceptable salt or solvate thereof according to any one of claims 1-16, wherein L^{a} is selected from a chemically unstable linking unit and an enzyme-catalyzed cleavage linking unit.

18. The ligand-drug conjugate or a pharmaceutically acceptable salt or solvate thereof according to any one of claims 1-17, wherein the linking unit L^{a} is non-restrictively selected from the following structures or stereoisomers thereof: 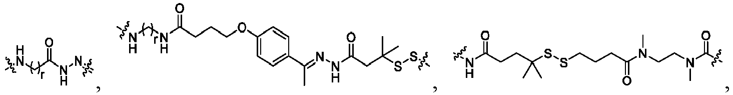 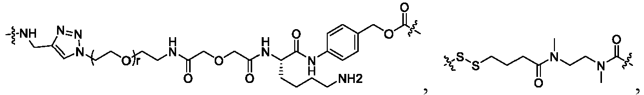 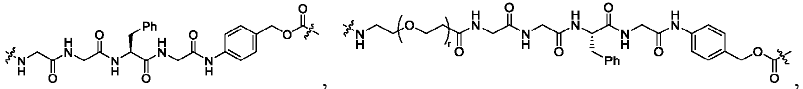 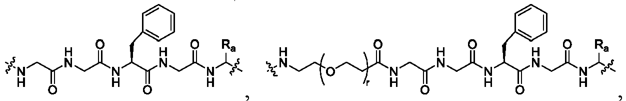 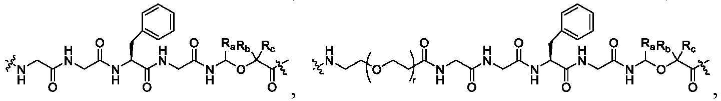 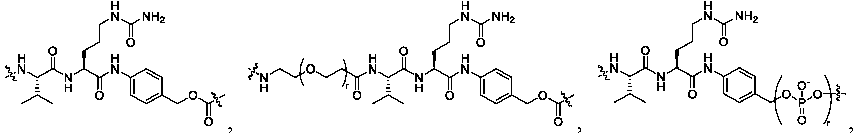 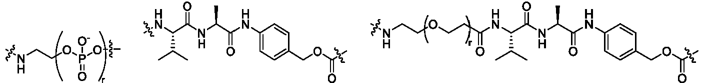 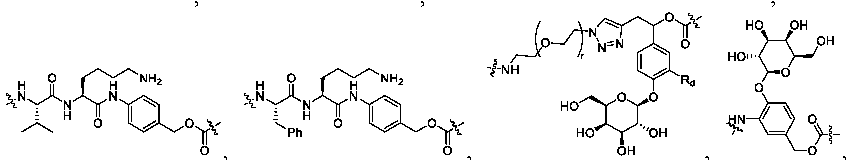 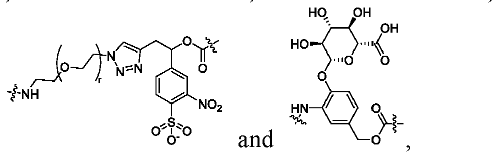 wherein
Rₐ, R_{b}, and R_{c} are the same or different, and are each independently selected from hydrogen atom, deuterium atom, halogen, alkyl, substituted alkyl, deuterated alkyl, cycloalkyl, cycloalkylalkyl, alkoxyalkyl, heterocyclyl, aryl, substituted aryl or heteroaryl;
or, R_{b}, R_{c} and carbon atoms connected to R_{b} and R_{c} constitute a C3-C8 cycloalkyl or a 3- to 8-membered heterocyclyl;
R_{d} is selected from H, NO₂, HO-SO₃- and -OSO₃;
r is selected from integers of 1 to 10;
the position indicated by a wavy line on the left is connected to the linking scaffold A, the linking structure B or the branching unit C;
the position indicated by a wavy line on the right is connected to the drug D, D₁ or D₂.

19. The ligand-drug conjugate or a pharmaceutically acceptable salt or solvate thereof according to any one of claims 1-18, wherein the linking units L^{a}-D, L^{a}-D₁ and L^{a}-D₂ are the same or different and are non-restrictively selected from the following structures or stereoisomers thereof:
(1) Auristatins 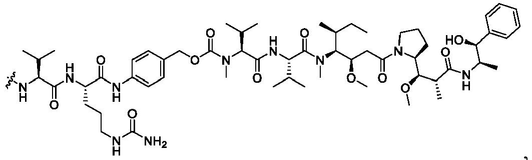 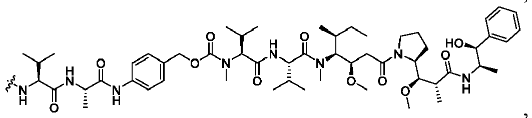 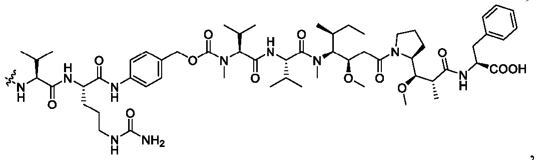 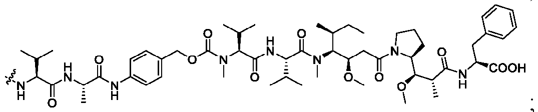
(2) Maytansinoids 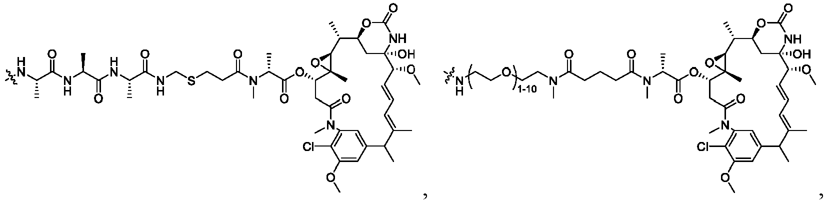 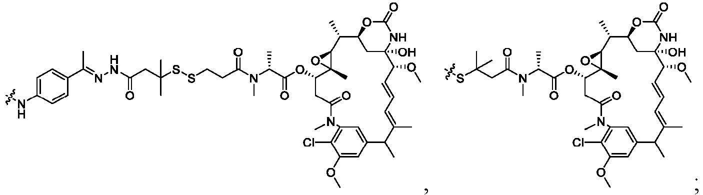
(3) Benzodiazepines 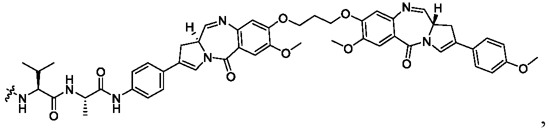 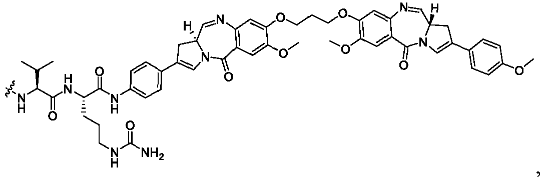 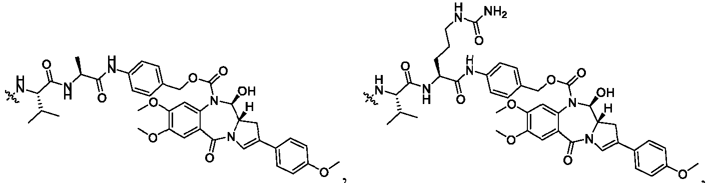 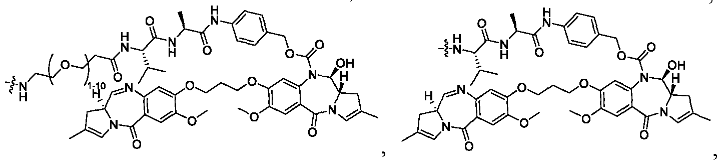 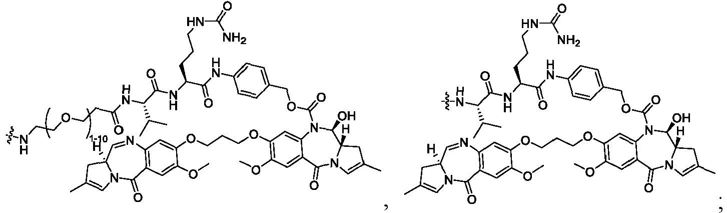
(4) Camptothecin analogues 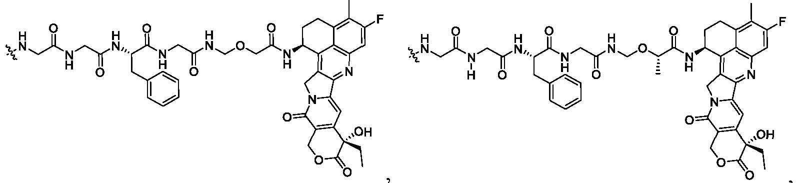 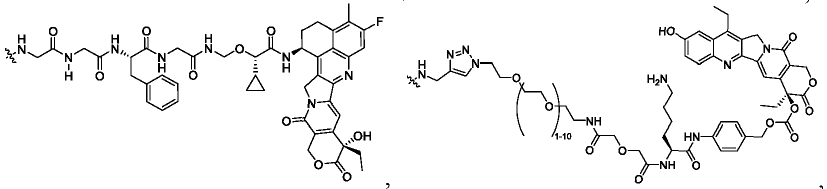 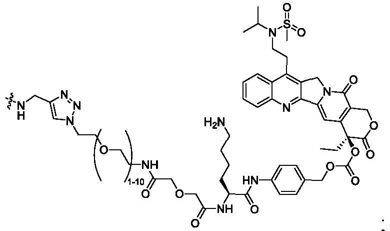
(5) Tubulysins 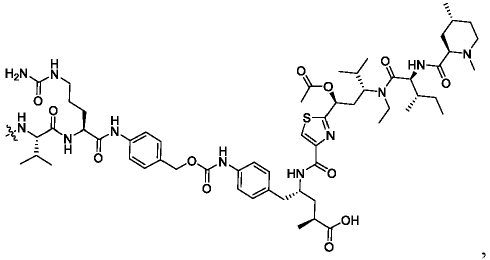 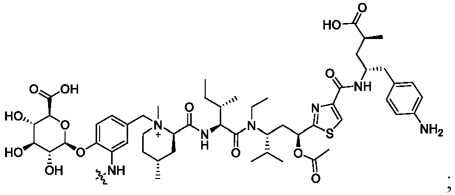
(6) Adriamycins 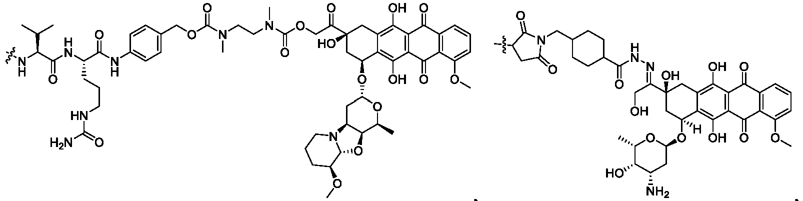 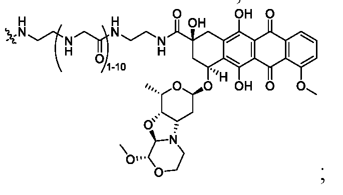
(7) Calicheamicins 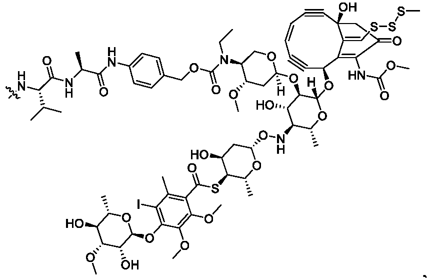 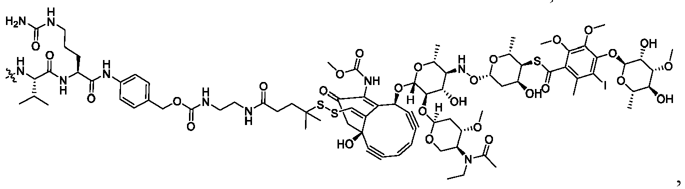 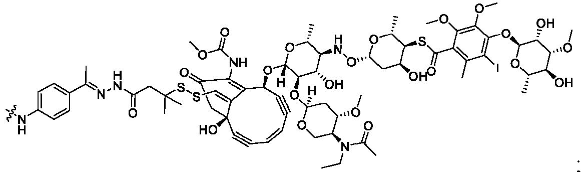
(8) Duocarmycins 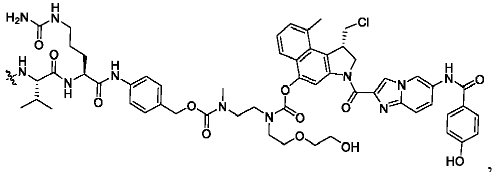 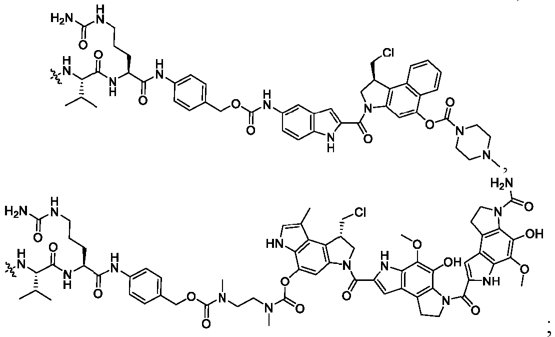
(9) Other antitumor drugs 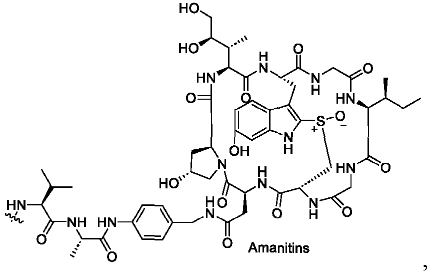 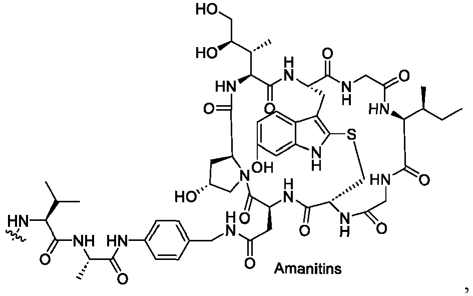 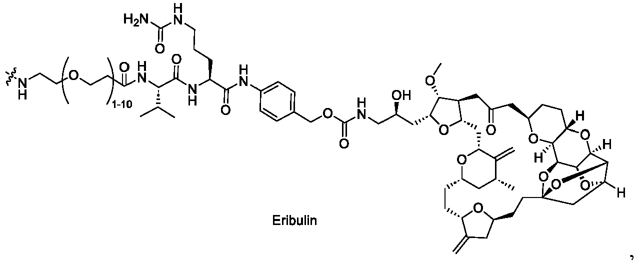 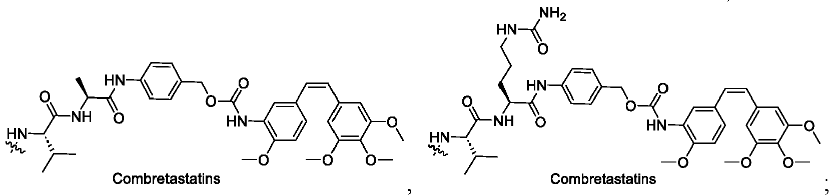 wherein
the position indicated by a wavy line is connected to the linking scaffold A, the linking structure B or the branching unit C;
preferably, the L^{a}-D, L^{a}-D₁ or L^{a}-D₂ is selected from:
Auristatins, for example, 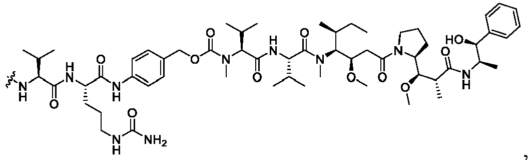
Benzodiazepines, for example, 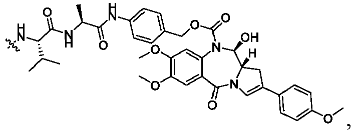
Camptothecin analogues, for example, 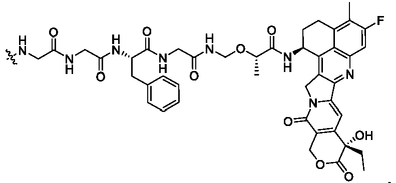
Combretastatins, for example,

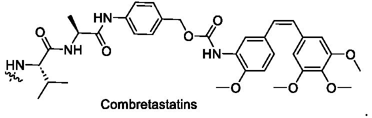

20. The ligand-drug conjugate or a pharmaceutically acceptable salt or solvate thereof according to any one of claims 1-19, wherein the linking unit L^{b} is non-restrictively selected from the following structures or stereoisomers thereof: 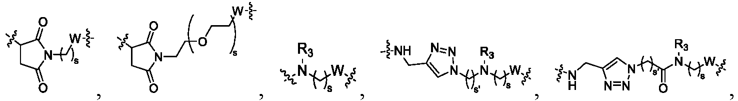 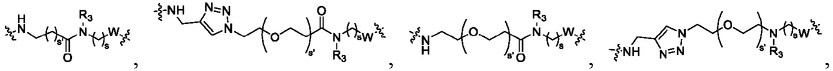 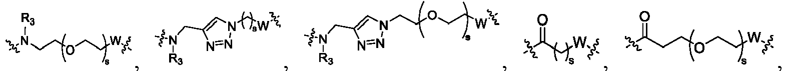 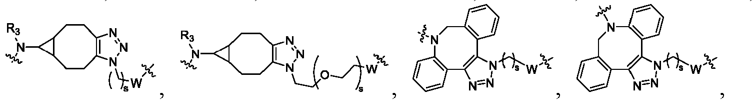 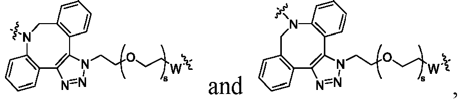 wherein
R₃ is selected from hydrogen atom, deuterium atom, halogen, C1-C6 alkyl, deuterated C1-C6 alkyl, halogenated C1-C6 alkyl, C3-C8 cycloalkyl C1-C6 alkyl,C1-C6 alkoxy C1-C6 alkyl, heterocyclyl, aryl, substituted aryl, and heteroaryl;
W is selected from -NR₄-, -O-, -S-, -CO- and -CONR₅-;
R₄ or R₅ is independently selected from hydrogen atom, deuterium atom, halogen,C1-C6 alkyl, deuterated C1-C6 alkyl, halogenated C1-C6 alkyl, C3-C8 cycloalkyl C1-C6 alkyl,C1-C6 alkoxy C1-C6 alkyl, heterocyclyl, aryl, substituted aryl, and heteroaryl;
s is selected from integers of 1 to 10;
s' is selected from integers of 1 to 10;
the positions indicated by a wavy line on the left and a wavy line on the right are non-restrictively connected to the SU or to the linking scaffold A.

21. A linker-drug compound of formula IV, V or VI or a pharmaceutically acceptable salt or solvate thereof: 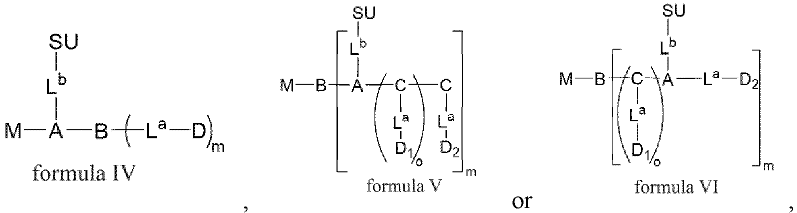 wherein
M is selected from any linker units;
A is selected from any linking scaffolds;
B is present or absent, and when present, is selected from any linking structures or bonds;
C is present or absent, and when present, is selected from any branching units or bonds; D, D₁, D₂ are the same or different, and are each independently selected from drugs; SU is selected from sugars or derivatives thereof;
L^{a} and L^{b} are the same or different, and are each independently selected from any linking units or bonds;
m is selected from integers of 1 to 5;
o is selected from integers of 1 to 10.

22. The linker-drug compound or a pharmaceutically acceptable salt or solvate thereof according to claim 21, wherein the linker unit M is non-restrictively selected from structures represented by the following formulas; when the structure contains a cycloalkyl or a heterocyclyl, the linker unit M may also be selected from a derived structure thereof in which the cycloalkyl or a heterocyclyl is in open form, 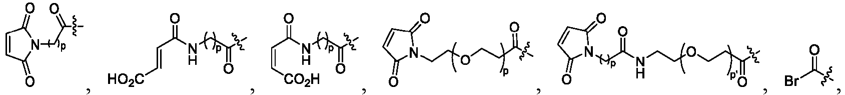 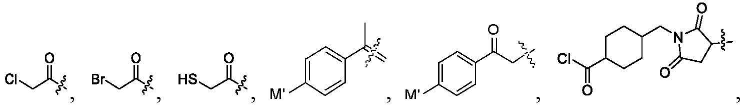 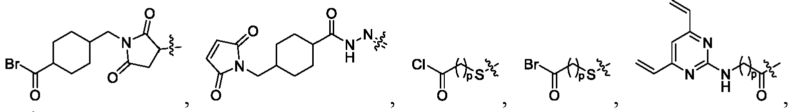 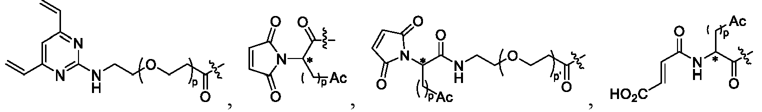 and 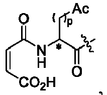 wherein
the position indicated by a wavy line is non-restrictively connected to the linking scaffold A, the linking structure B or the branching unit C;
M' is non-restrictively selected from halogen, OTf and OTs;
carbon at the position indicated by * is a chiral carbon and has an R or S configuration; p and p' are each independently selected from integers of 1 to 10;
Ac is non-restrictively selected from a residue of a natural or non-natural amino acid, a polyethylene glycol segment with 1-20 repeating units, a phosphate group, a carboxylic acid group, a sulfonic acid group, a sulfinic acid group or the following structure, 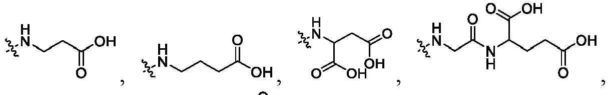 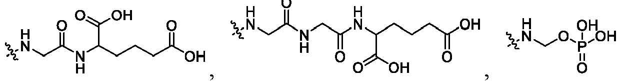
or 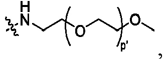
wherein the position indicated by a wavy line is connected to the carbon atom at the position indicated by *.

23. The linker-drug compound or a pharmaceutically acceptable salt or solvate thereof according to any one of claim 21 or 22, wherein the linker unit M is non-restrictively selected from structures represented by the following formulas, or stereoisomers thereof or derived structures thereof in which rings in succinimide groups are in open form, 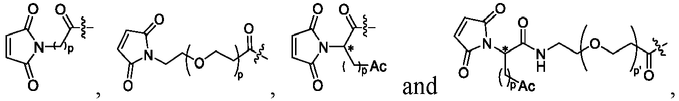 wherein
the position indicated by a wavy line is non-restrictively connected to the linking scaffold A, the linking structure B or the branching unit C;
carbon at the position indicated by * is a chiral carbon and has an R or S configuration; p and p' are each independently selected from integers of 1 to 10;
Ac is non-restrictively selected from a residue of a natural or non-natural amino acid, a polyethylene glycol segment with 1-20 repeating units, a phosphate group, a carboxylic acid group, a sulfonic acid group, a sulfinic acid group or the following structure, 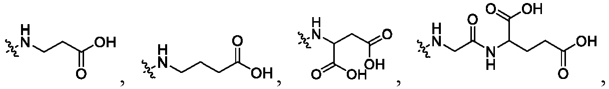 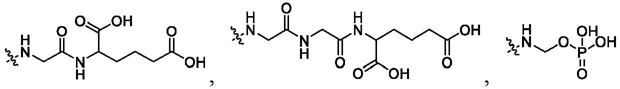
or 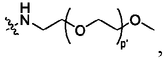
wherein the position indicated by a wavy line is connected to the carbon atom at the position indicated by *.

24. The linker-drug compound or a pharmaceutically acceptable salt or solvate thereof according to any one of claims 21-23, wherein the linker unit M is non-restrictively selected from structures represented by the following formulas, or derived structures thereof in which rings in succinimide groups are in open form, 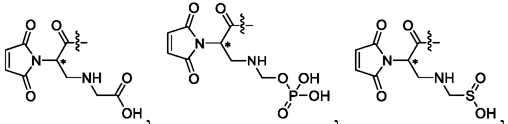 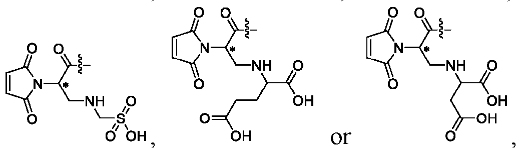
preferably 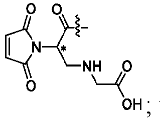
wherein carbon at the position indicated by * is a chiral carbon and has an R or S configuration; the position indicated by a wavy line is non-restrictively connected to the linking scaffold A, the linking structure B or the branching unit C.

25. The linker-drug compound or a pharmaceutically acceptable salt or solvate thereof according to any one of claims 21-24, wherein the linking scaffold A is selected from one or more of natural or non-natural amino acids or from the following structures: 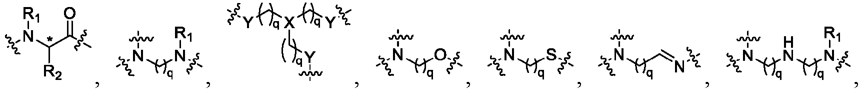 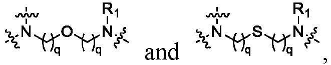 wherein
X is selected from N, CH, C3-C8 cycloalkyl, 3- to 8-membered heterocyclyl, aryl, substituted aryl, and heteroaryl;
Y is selected from -NH-, -O-, -S-, -CO-, -CO₂-, -CONH-, -NHCO-, -SO-, -SO₂-, -OSO₂-, and -OP=O(OH)O-;
q is selected from integers of 1 to 10;
the position indicated by a wavy line is non-restrictively connected to the linker unit M, the linking unit L^{a} or L^{b}, the linking structure B or the branching unit C;
R₁ is selected from hydrogen atom, deuterium atom, halogen, C1-C6 alkyl, C1-C6 substituted alkyl, C3-C8 cycloalkyl C1-C6 alkyl, C1-C6 alkoxy C1-C6 alkyl, carboxyl, 3- to 8-membered heterocyclyl, aryl, substituted aryl and heteroaryl;
R₂ is non-restrictively selected from: 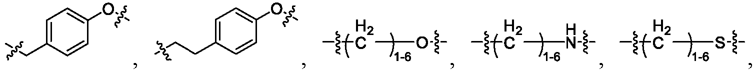 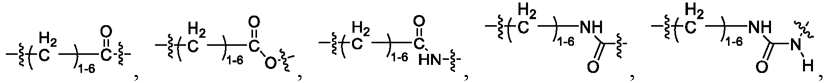 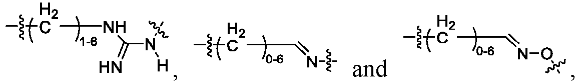
wherein a wavy line on the left indicates the position where R₂ is connected to the chiral carbon indicated by *, and a wavy line on the right indicates one of any three connection sites of the linking scaffold A.

26. The linker-drug compound or a pharmaceutically acceptable salt or solvate thereof according to any one of claims 21-25, wherein the linking scaffold A is non-restrictively selected from the following structures or stereoisomers thereof, 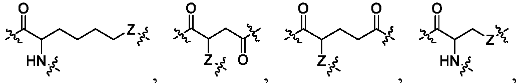 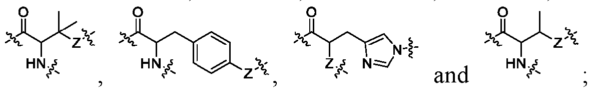 wherein
Z is selected from -NH-, -O- and -S-;
the position indicated by a wavy line is non-restrictively connected to the linker unit M, the linking unit L^{a} or L^{b}, the linking structure B or the branching unit C;
preferably, the linking scaffold A is 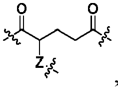
wherein Z is -NH-.

27. The linker-drug compound or a pharmaceutically acceptable salt or solvate thereof according to any one of claims 21-26, wherein the B is non-restrictively selected from any linking structures or bonds.

28. The linker-drug compound or a pharmaceutically acceptable salt or solvate thereof according to any one of claims 21-27, wherein the C is present or absent, and when present, is non-restrictively selected from one or more of natural or non-natural amino acids.

29. The linker-drug compound or a pharmaceutically acceptable salt or solvate thereof according to any one of claims 21-28, wherein the drugs D, D₁, and D₂ are the same or different, and are each independently and non-restrictively selected from an anti-tumor drug, an autoimmune disease drug, an anti-infective disease drug (such as an antiviral drug), a radioactive isotope, a chromogenic molecule, or a pharmaceutically acceptable salt or solvate thereof.

30. The linker-drug compound or a pharmaceutically acceptable salt or solvate thereof according to any one of claims 21-29, wherein the drugs D, D₁, and D₂ are the same or different, and are independently and non-restrictively selected from anti-tumor drugs, including but not limited to DNA damaging agents, RNA damaging agents, enzyme inhibitors, or microtubule inhibitors.

31. The linker-drug compound or a pharmaceutically acceptable salt or solvate thereof according to any one of claims 21-30, wherein the SU is non-restrictively selected from a natural or non-natural monosaccharide, disaccharide, polysaccharide and derivatives thereof.

32. The linker-drug compound or a pharmaceutically acceptable salt or solvate thereof according to any one of claims 21-31, wherein the natural or non-natural monosaccharide, disaccharide, polysaccharide or derivatives thereof are non-restrictively selected from the following structures, 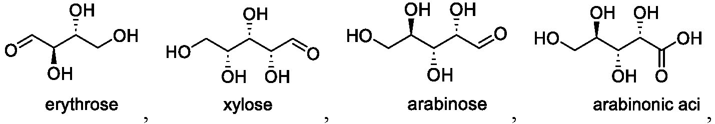 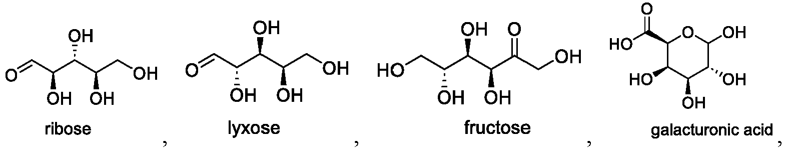 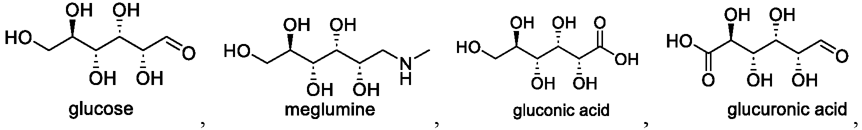 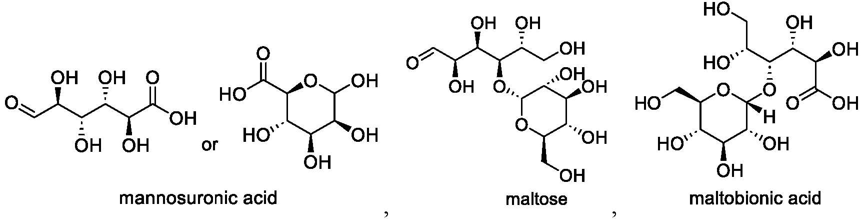 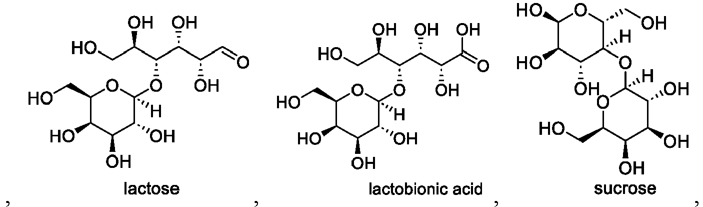 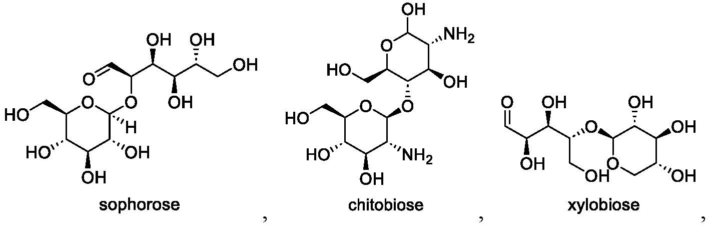 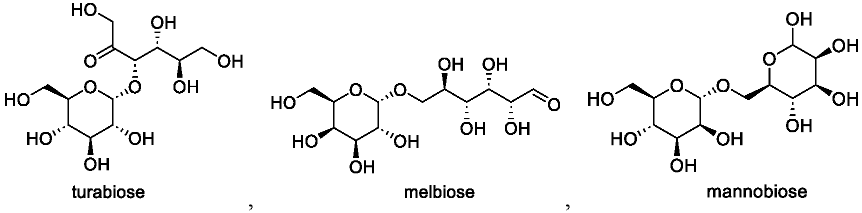 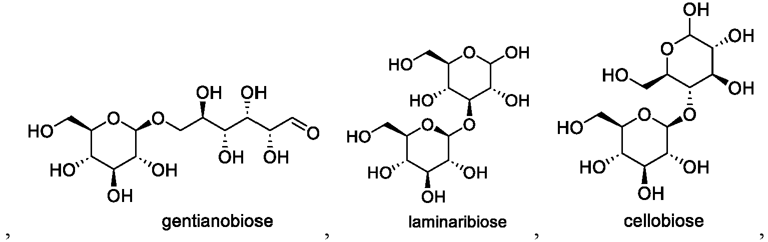 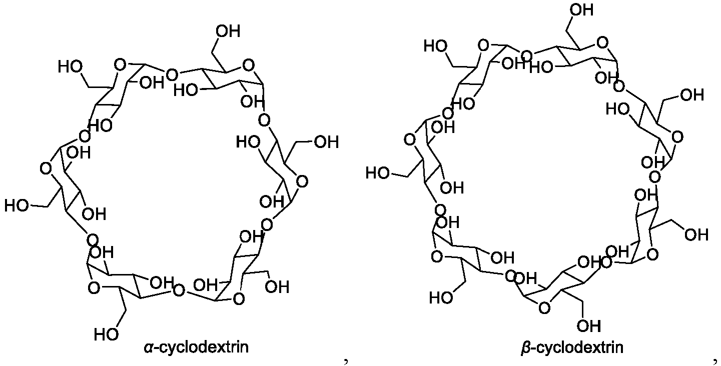 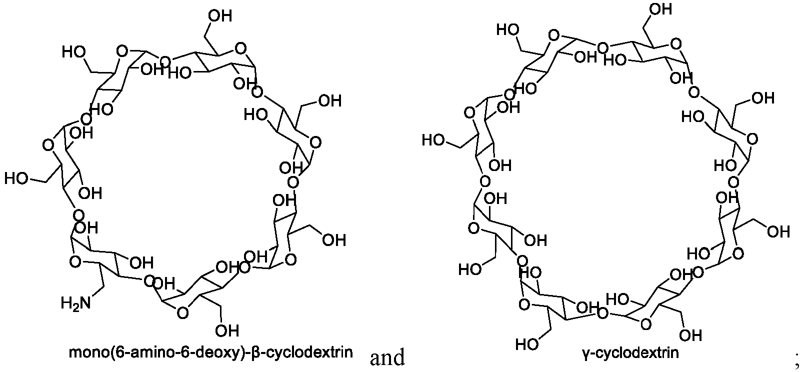 preferably, the SU is selected from methylglucamine, maltose, maltobionic acid, mannuronic acid, β-cyclodextrin and mono(6-amino-6-deoxy)-β-cyclodextrin.

33. The linker-drug compound or a pharmaceutically acceptable salt or solvate thereof according to any one of claims 21-32, wherein the SU is non-restrictively linked to L^{b} in a covalent manner.

34. The linker-drug compound or a pharmaceutically acceptable salt or solvate thereof according to any one of claims 21-33, wherein the linking units L^{a} and L^{b} are the same or different, and are each independently and non-restrictively selected from one or more of a chemically unstable linking unit, an enzyme-catalyzed cleavage linking unit and a non-cleavable linking unit.

35. The linker-drug compound or a pharmaceutically acceptable salt or solvate thereof according to any one of claims 21-34, wherein L^{a} is selected from a chemically unstable linking unit and an enzyme-catalyzed cleavage linking unit.

36. The linker-drug compound or a pharmaceutically acceptable salt or solvate thereof according to any one of claims 21-35, wherein the linking unit L^{a} is non-restrictively selected from the following structures or stereoisomers thereof, 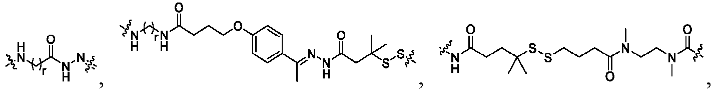 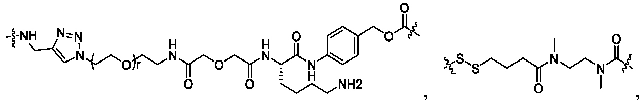 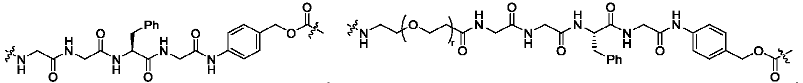 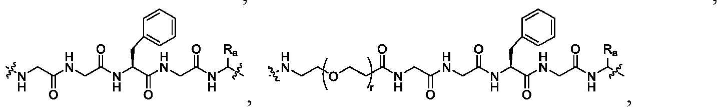 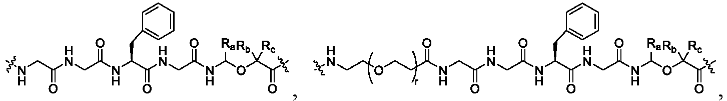 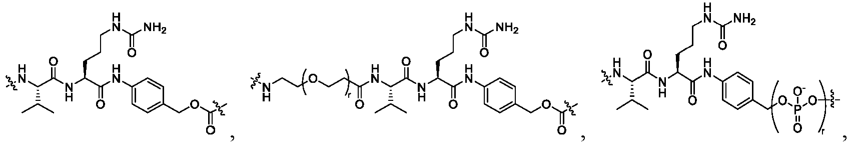 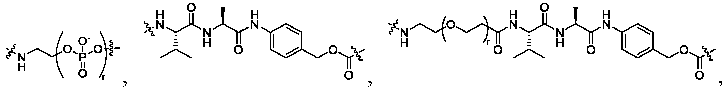 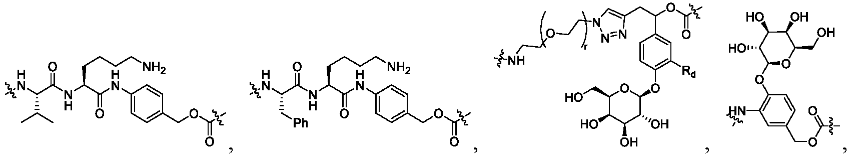 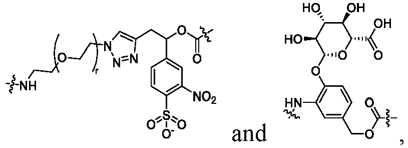 wherein
Rₐ, R_{b}, and R_{c} are the same or different, and are each independently selected from hydrogen atom, deuterium atom, halogen, alkyl, substituted alkyl, deuterated alkyl, cycloalkyl, cycloalkylalkyl, alkoxyalkyl, heterocyclyl, aryl, substituted aryl or heteroaryl;
or, R_{b}, R_{c} and carbon atoms connected to R_{b} and R_{c} constitute a C3-C8 cycloalkyl or a 3- to 8-membered heterocyclyl;
R_{d} is selected from H, NO₂, HO-SO₃ and -OSO₃;
r is selected from integers of 1 to 10;
the position indicated by a wavy line on the left is connected to the linking scaffold A, the linking structure B or the branching unit C;
the position indicated by a wavy line on the right is connected to the drug D, D₁ or D₂.

37. The linker-drug compound or a pharmaceutically acceptable salt or solvate thereof according to any one of claims 21-36, wherein the L^{a}-D, L^{a}-D₁ and L^{a}-D₂ are the same or different and are non-restrictively selected from the following structures or stereoisomers thereof:
(1) Auristatins 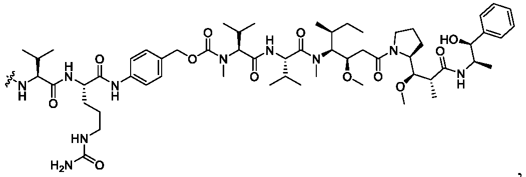 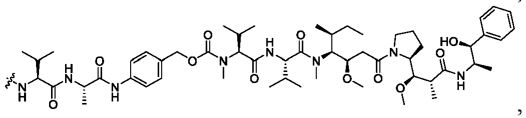 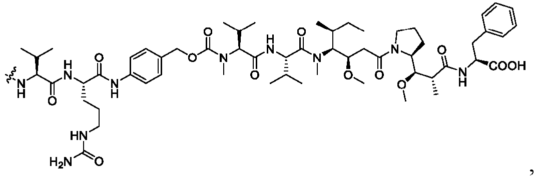 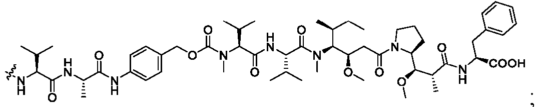
(2) Maytansinoids 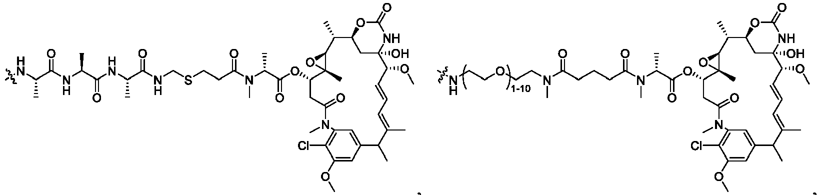 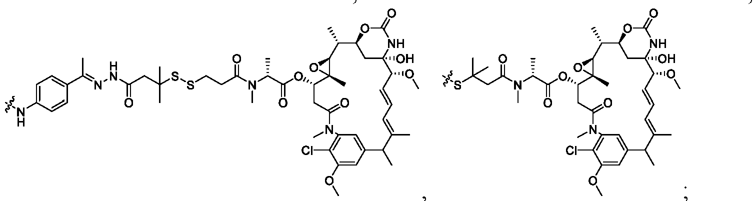
(3) Benzodiazepines 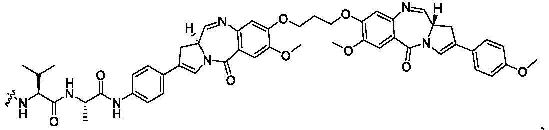 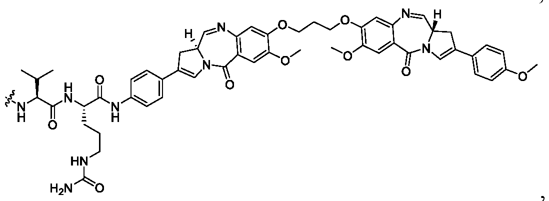 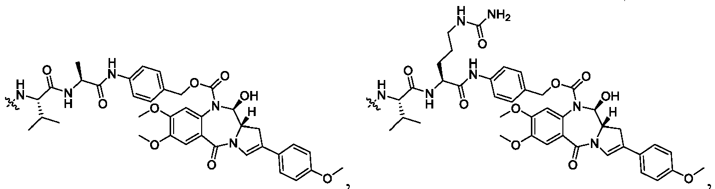 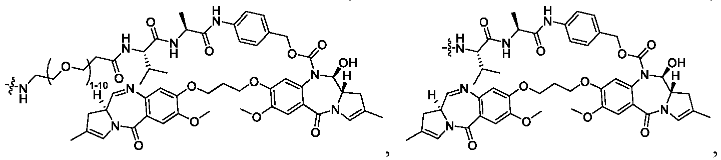 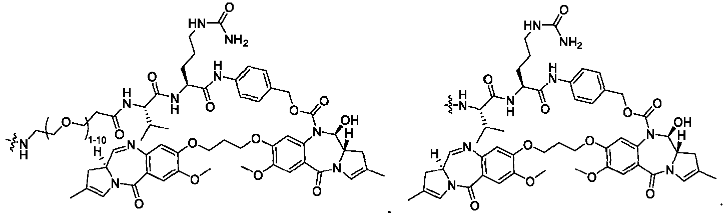
(4) Camptothecin analogues 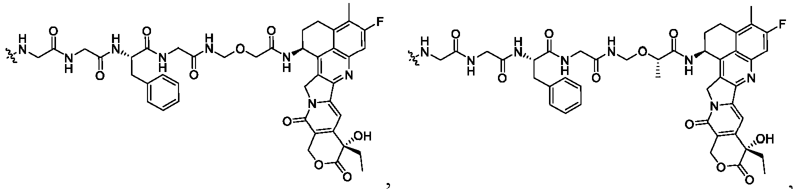 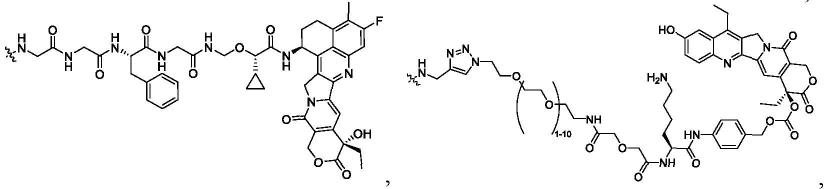 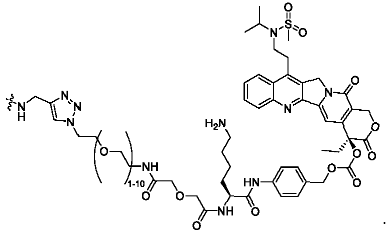
(5) Tubulysins 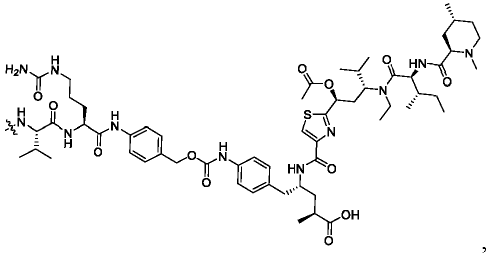 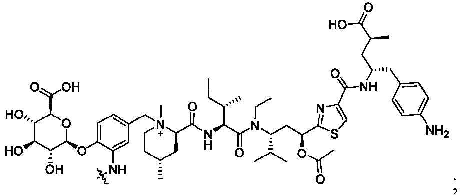
(6) Adriamycins 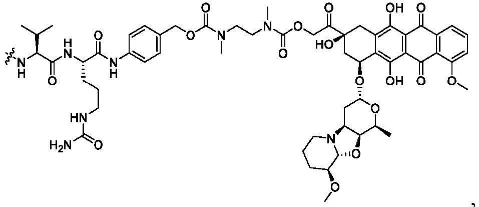 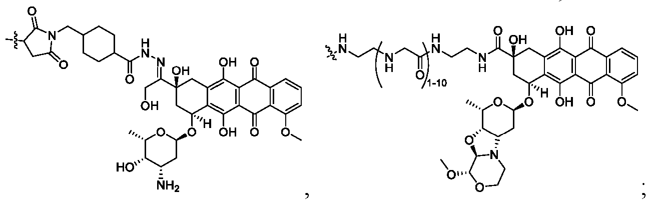
(7) Calicheamicins 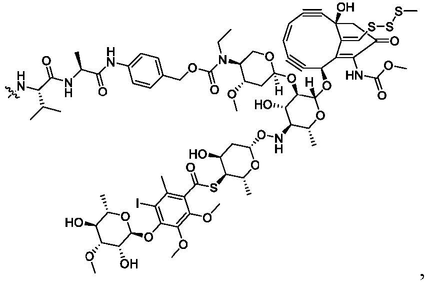 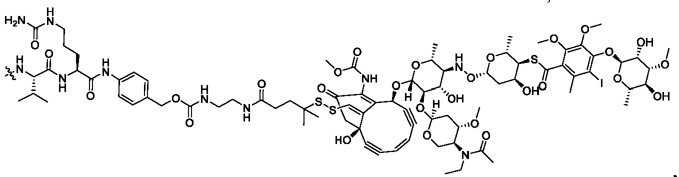 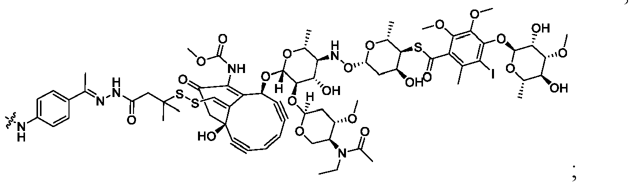
(8) Duocarmycins 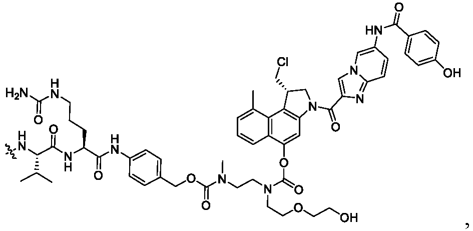 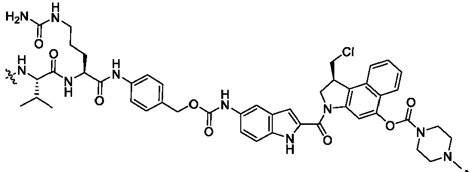 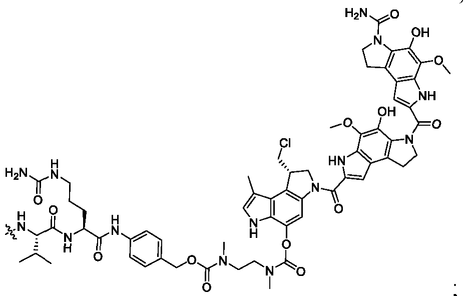
(9) Other antitumor drugs 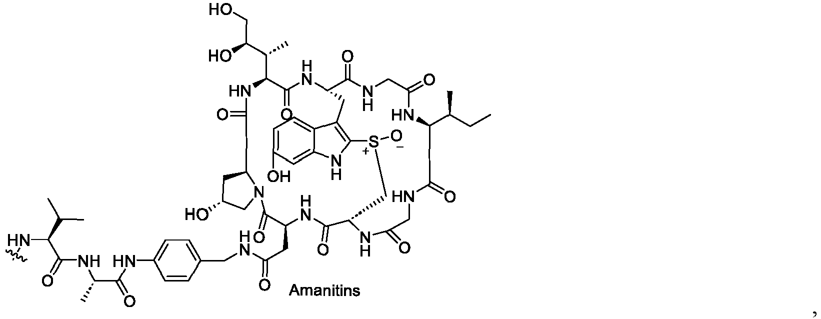 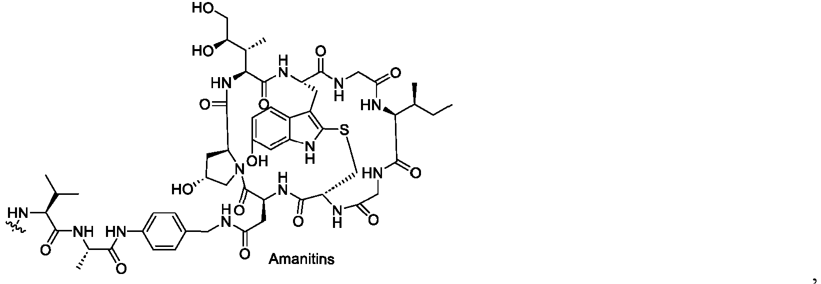 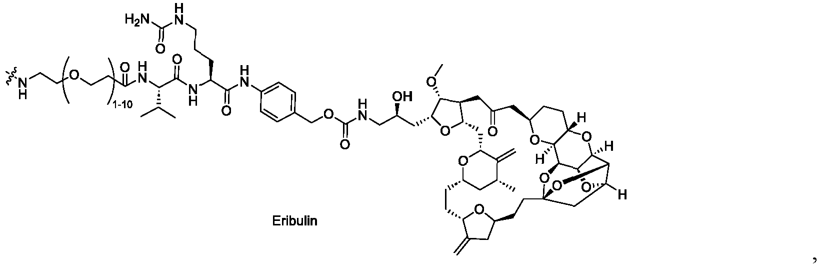 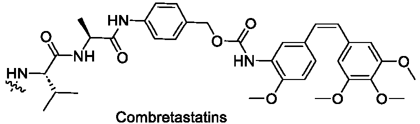 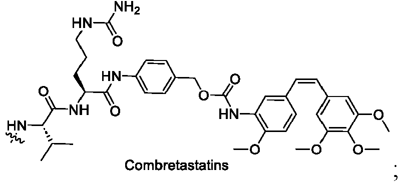 wherein
the position indicated by a wavy line is connected to the linking scaffold A, the linking structure B or the branching unit C;
preferably, the L^{a}-D, L^{a}-D₁ or L^{a}-D₂ is selected from:
Auristatins, for example, 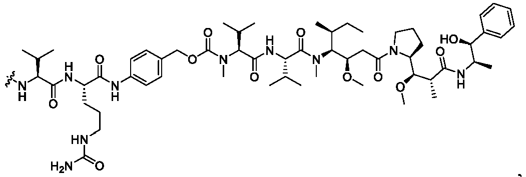
Benzodiazepines, for example, 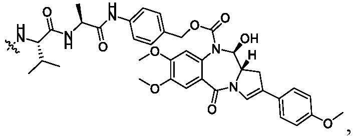
Camptothecin analogues, for example, 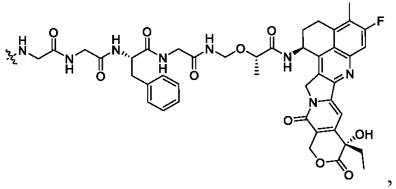
Combretastatins, for example,

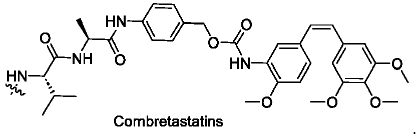

38. The ligand-drug conjugate or a pharmaceutically acceptable salt or solvate thereof according to any one of claims 21-37, wherein the linking unit L^{b} is non-restrictively selected from the following structures or stereoisomers thereof: 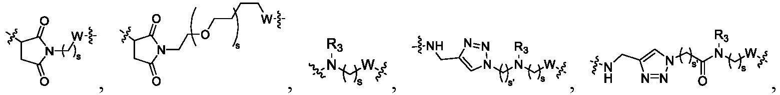 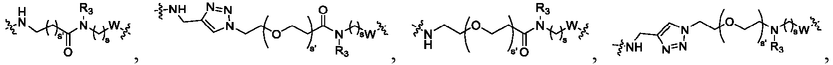 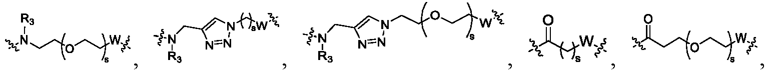 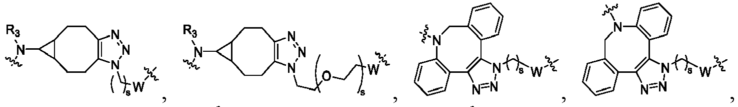 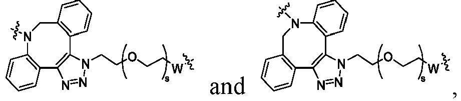 wherein
R₃ is selected from hydrogen atom, deuterium atom, halogen, C1-C6 alkyl, deuterated C1-C6 alkyl, halogenated C1-C6 alkyl, C3-C8 cycloalkyl C1-C6 alkyl,C1-C6 alkoxy C1-C6 alkyl, heterocyclyl, aryl, substituted aryl, and heteroaryl;
W is selected from -NR₄-, -O-, -S-, -CO- and -CONR₅-;
R₄ or R₅ is independently selected from hydrogen atom, deuterium atom, halogen,C1-C6 alkyl, deuterated C1-C6 alkyl, halogenated C1-C6 alkyl, C3-C8 cycloalkyl C1-C6 alkyl, C1-C6 alkoxy C1-C6 alkyl, heterocyclyl, aryl, substituted aryl, and heteroaryl;
s is selected from integers of 1 to 10;
s' is selected from integers of 1 to 10;
the positions indicated by a wavy line on the left and a wavy line on the right are non-restrictively connected to the SU or to the linking scaffold A.

39. The linker-drug compound or a pharmaceutically acceptable salt or solvate thereof according to any one of claims 21-38, wherein the L^{b}-SU is non-restrictively selected from the following structures: 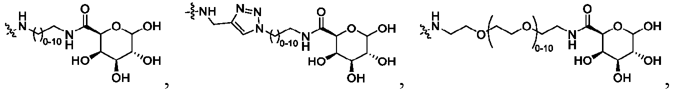 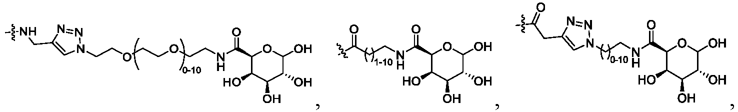 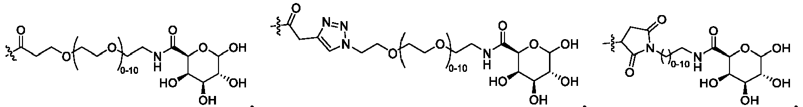 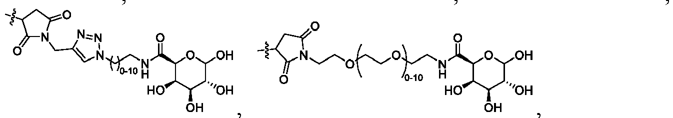 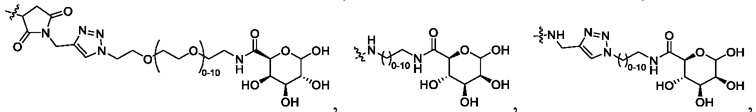 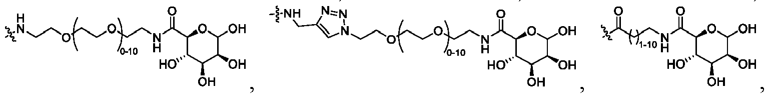 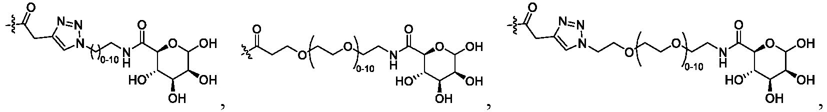 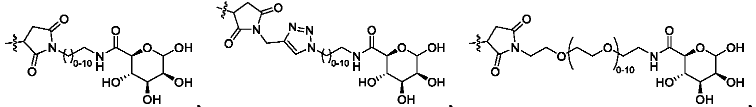 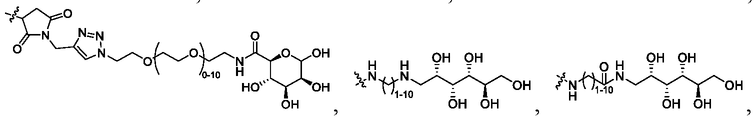 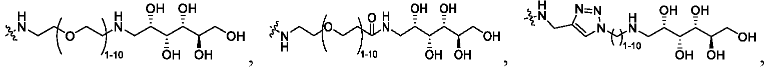 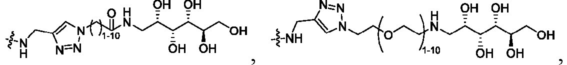 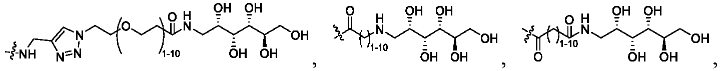 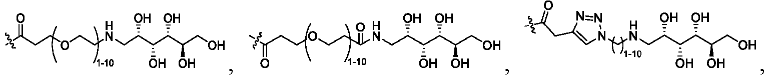 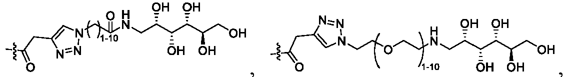 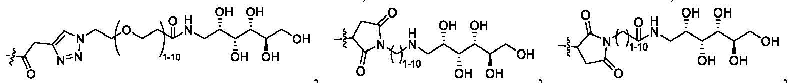 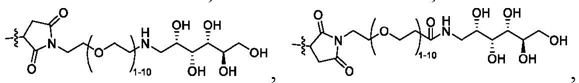 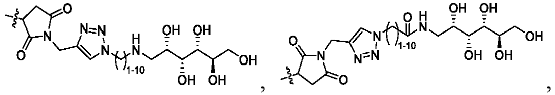 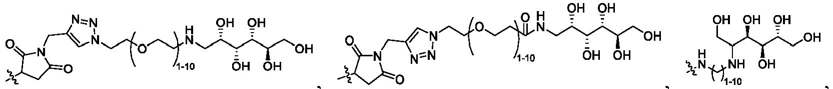 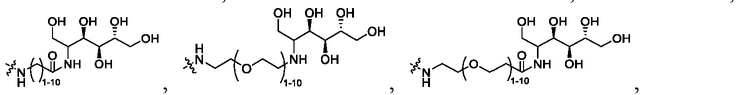 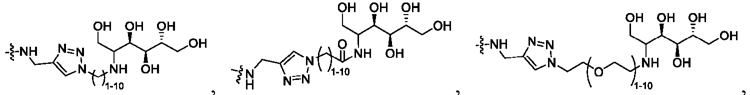 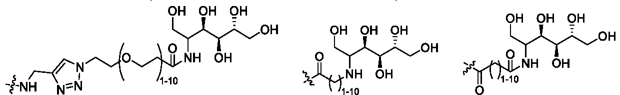 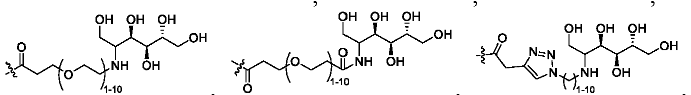 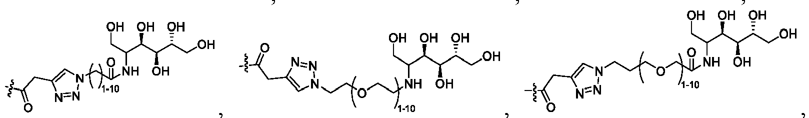 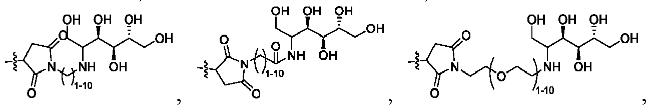 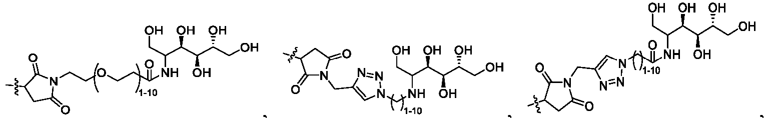 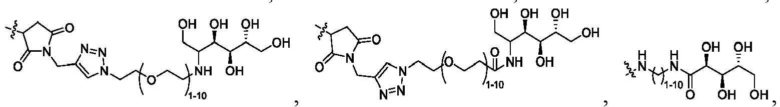 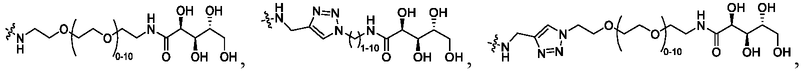 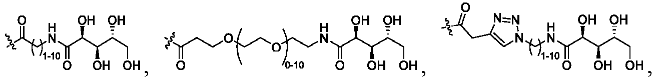 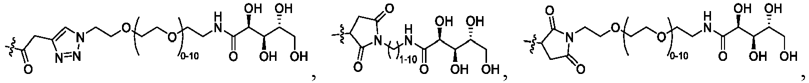 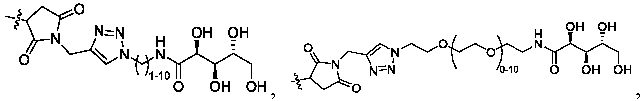 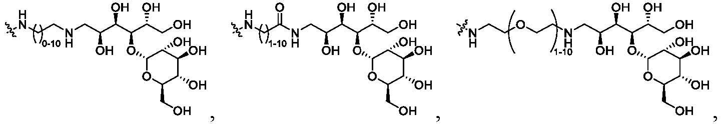 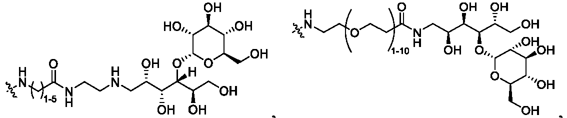 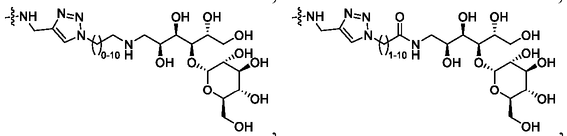 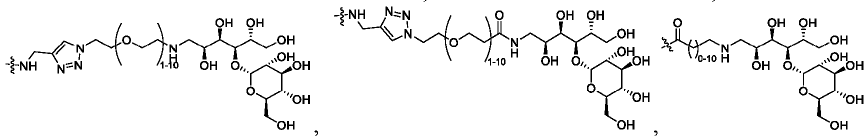 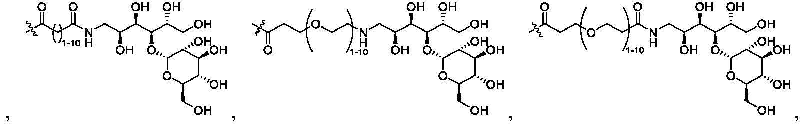 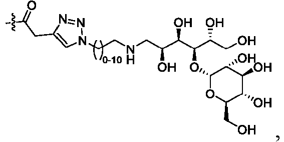 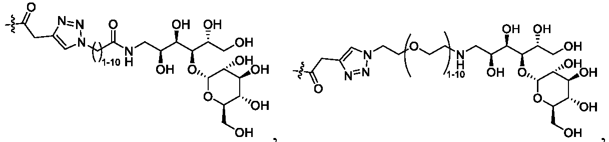 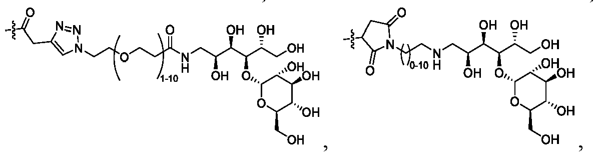 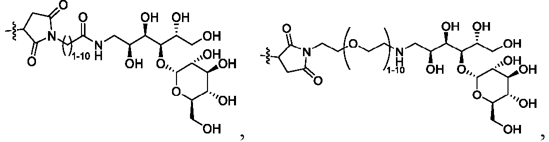 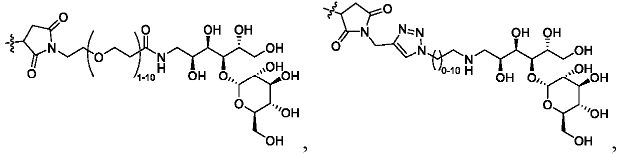 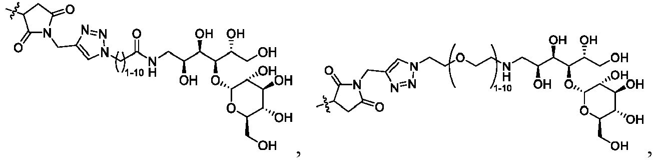 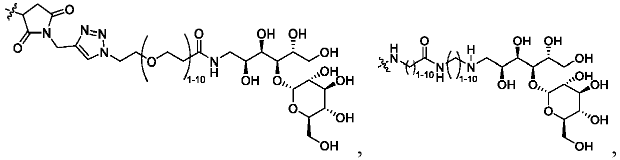 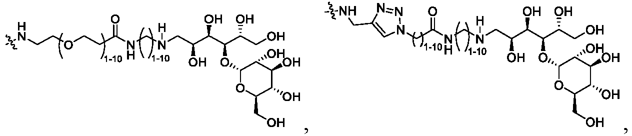 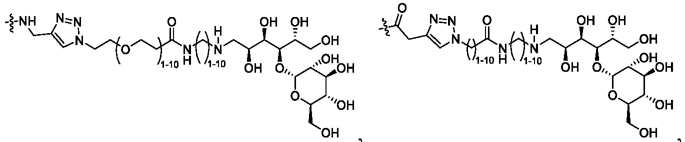 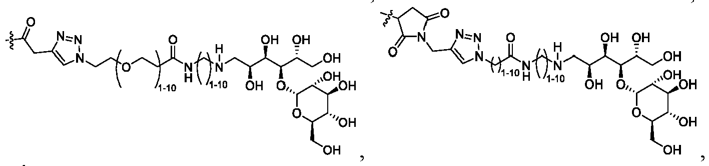 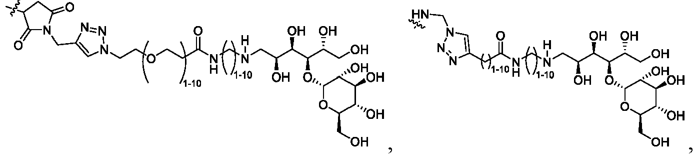 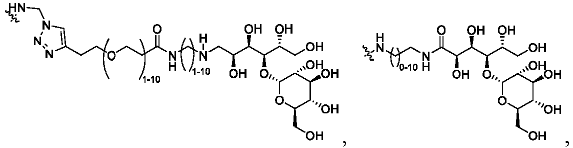 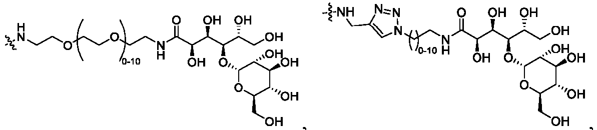 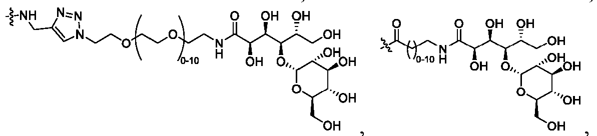 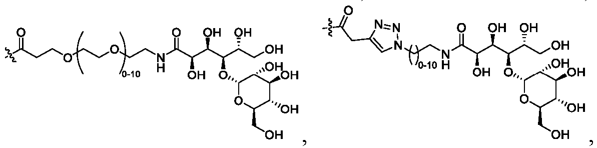 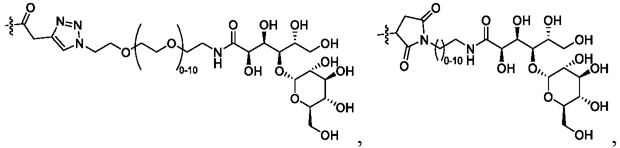 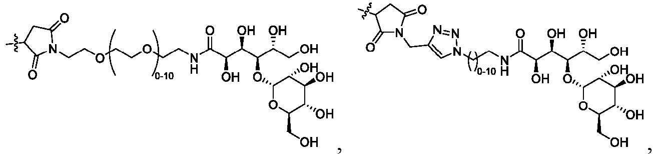 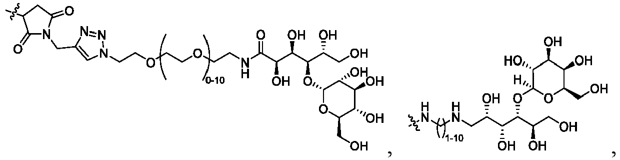 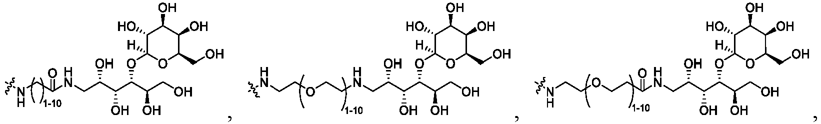 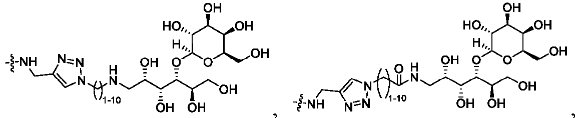 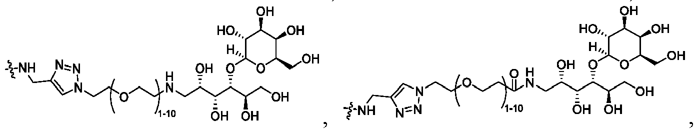 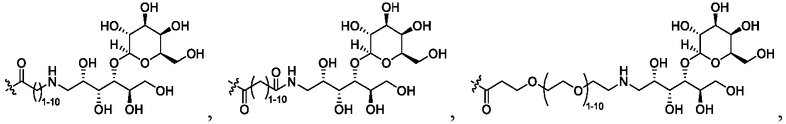 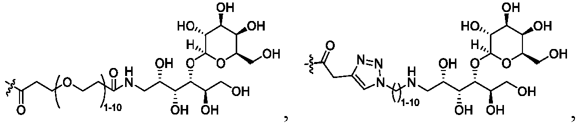 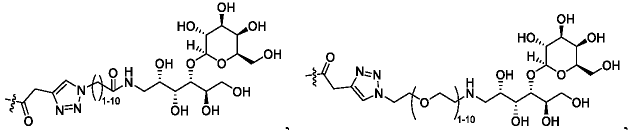 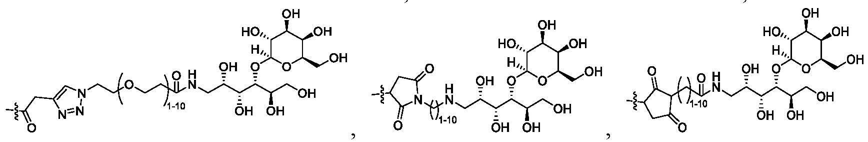 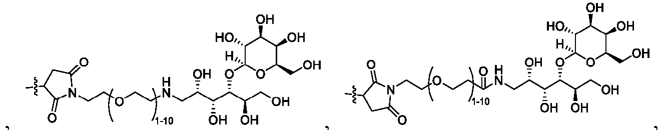 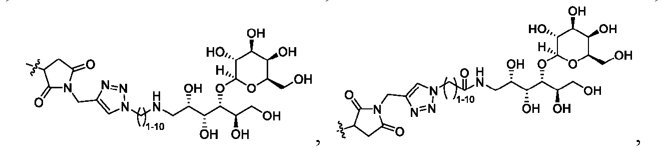 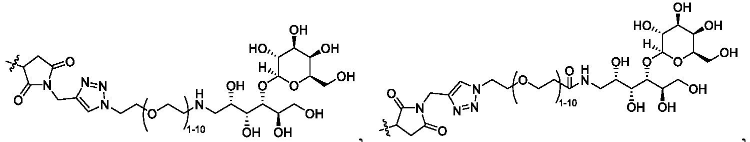 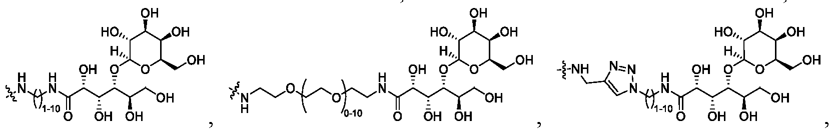 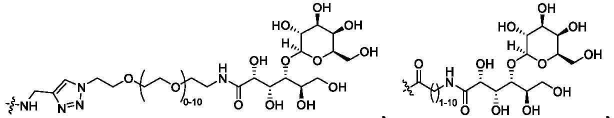 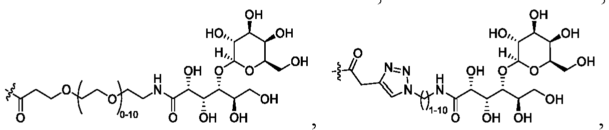 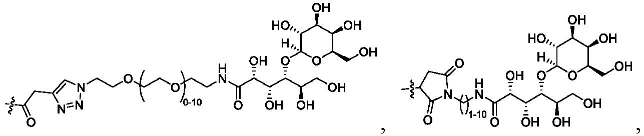 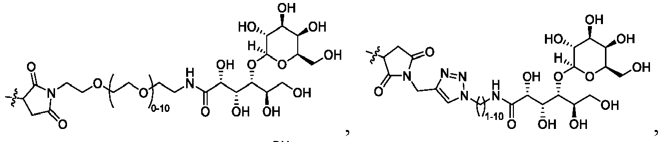 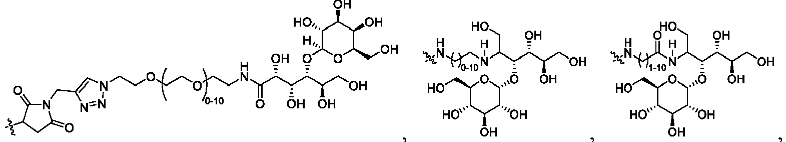 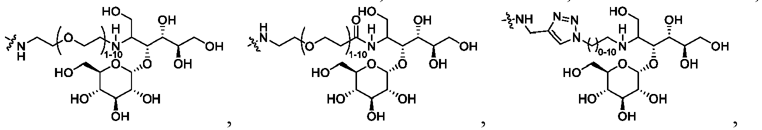 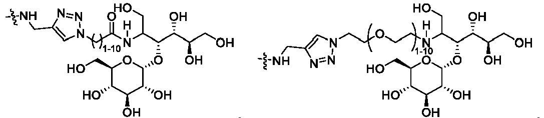 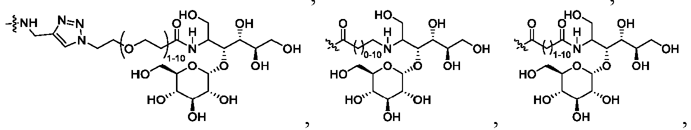 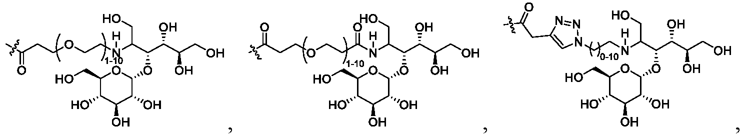 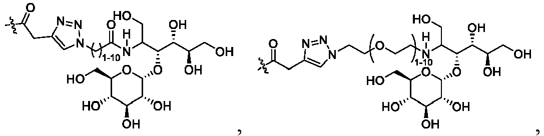 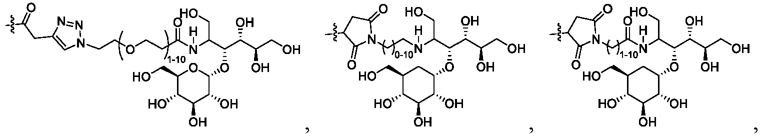 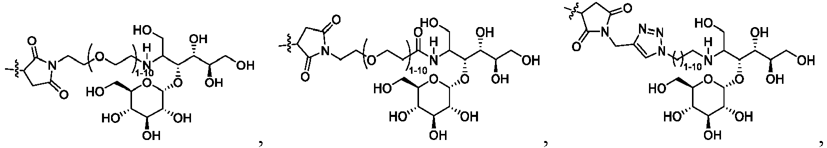 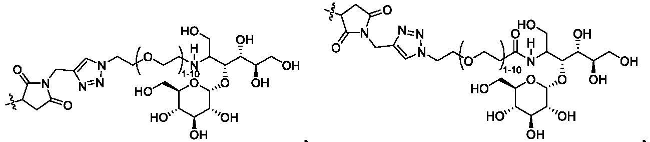 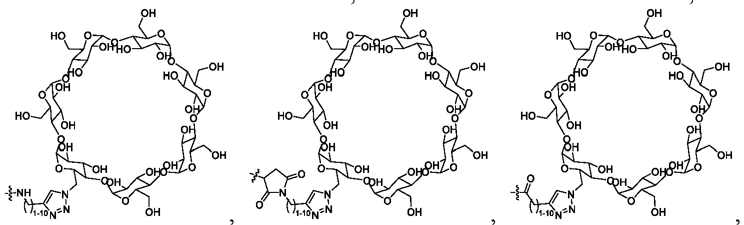 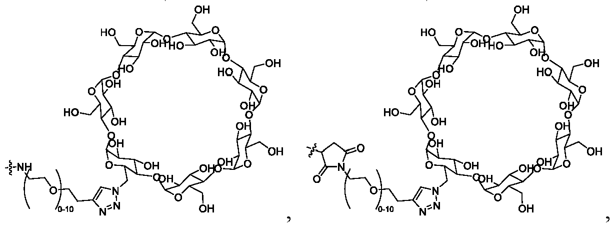 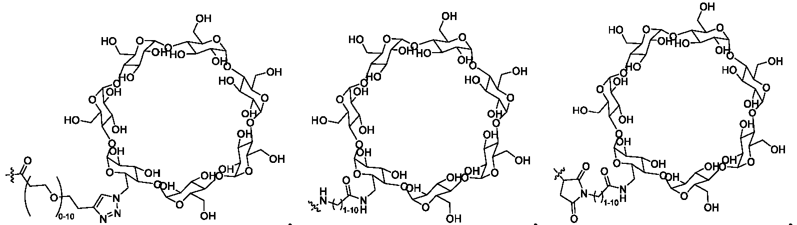 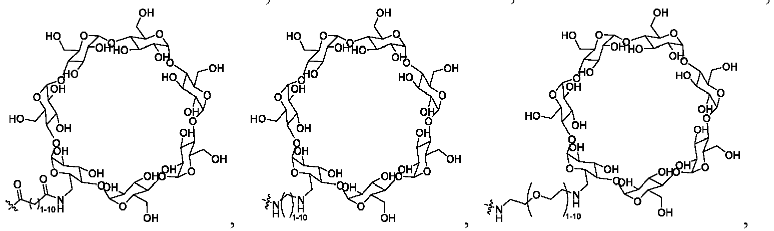 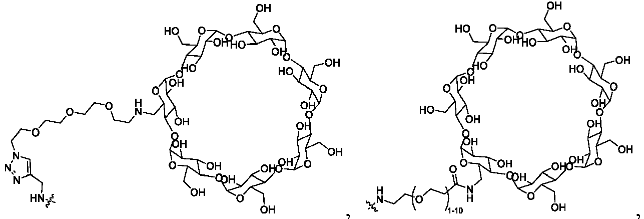 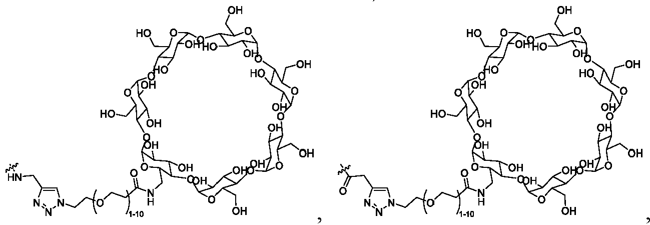 and

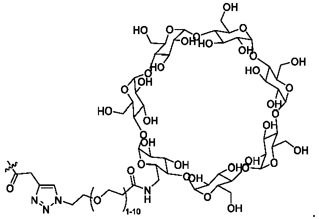

40. The linker-drug compound or a pharmaceutically acceptable salt or solvate thereof according to any one of claims 21-39, wherein the linker-drug compound is non-restrictively selected from the following structures: 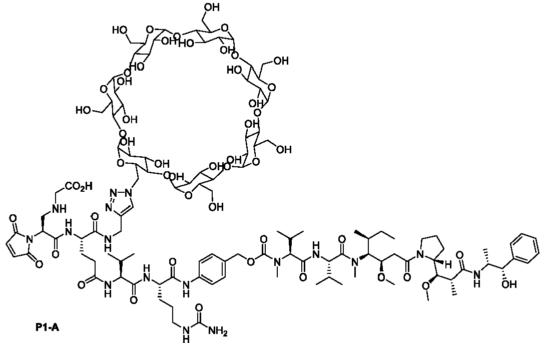 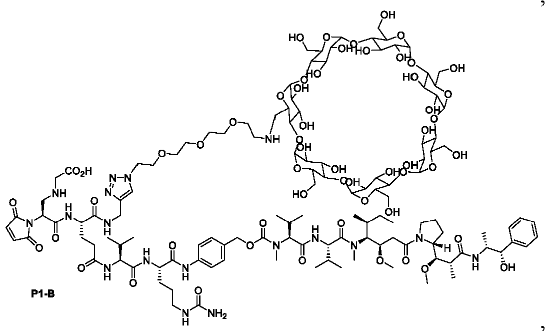 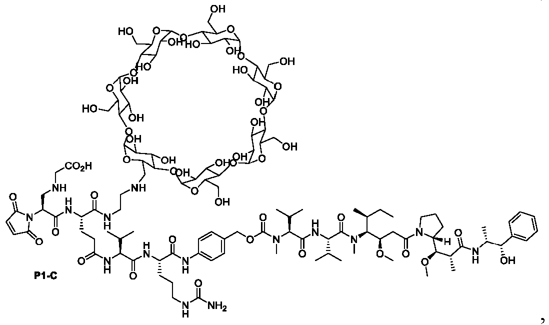 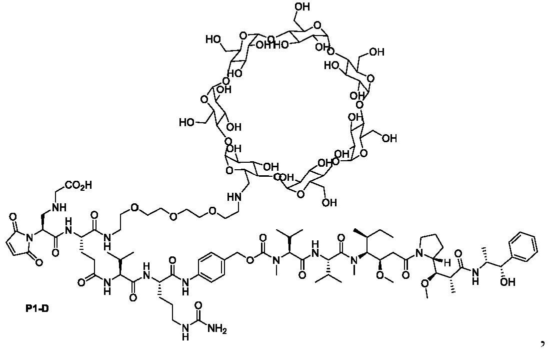 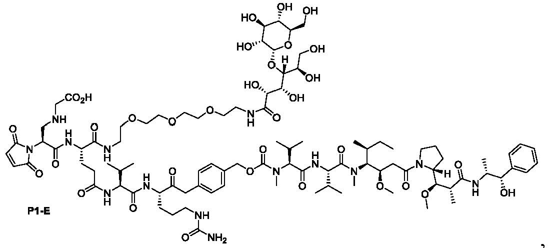 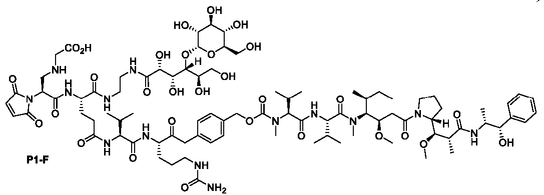 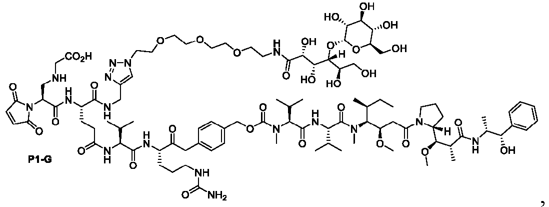 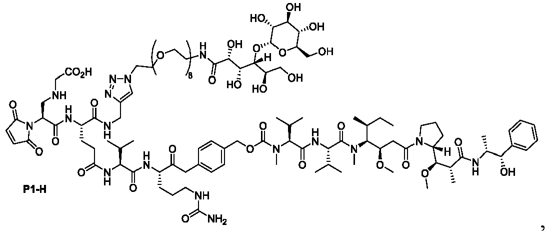 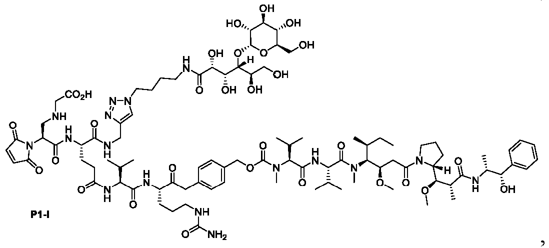 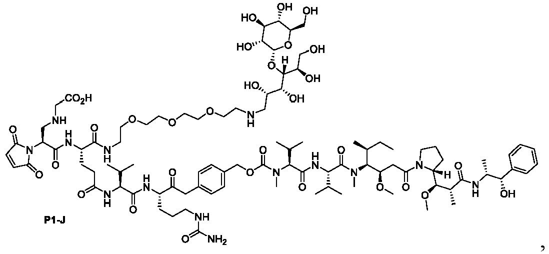 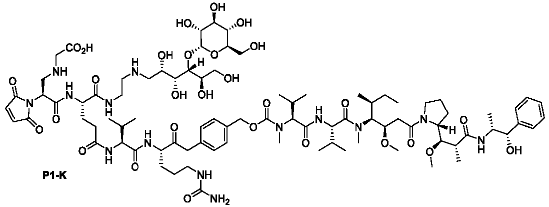 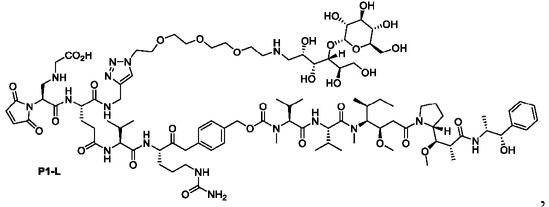 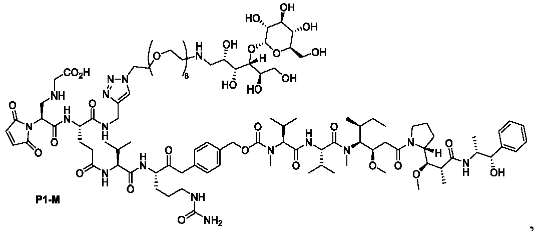 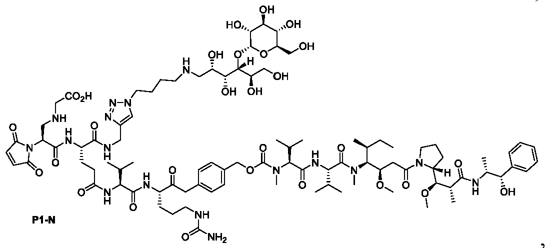 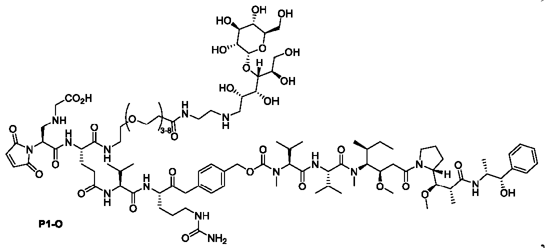 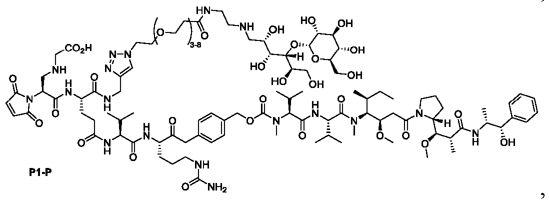 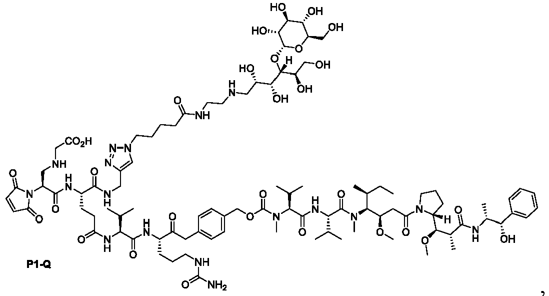 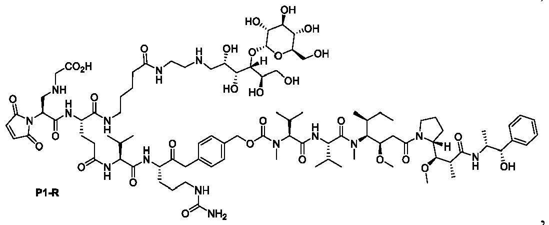 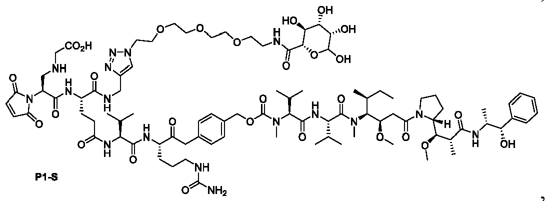 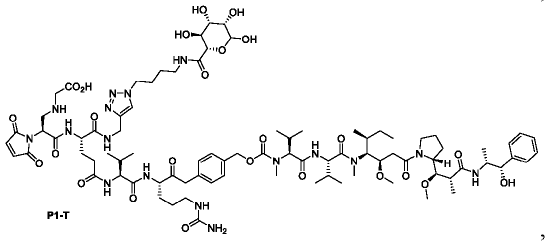 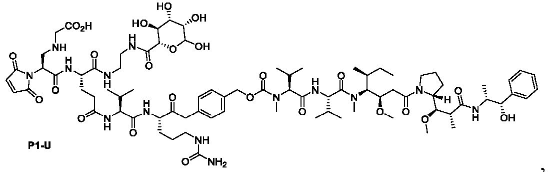 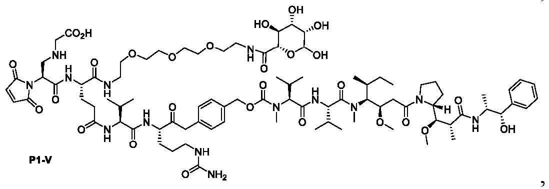 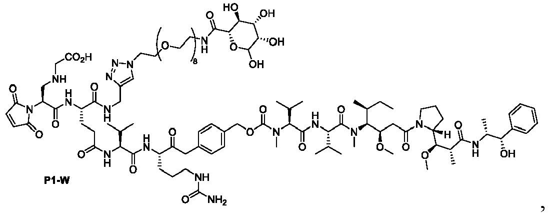 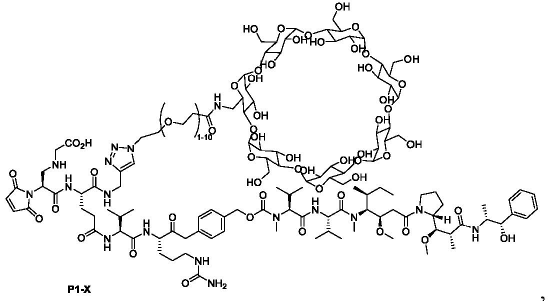 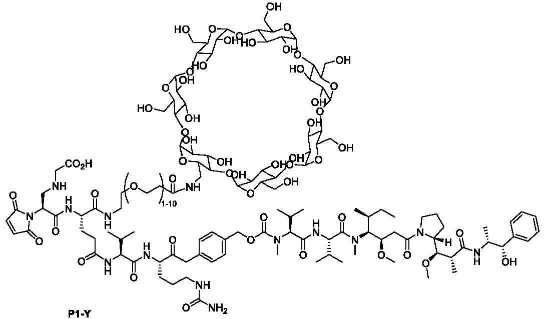 and

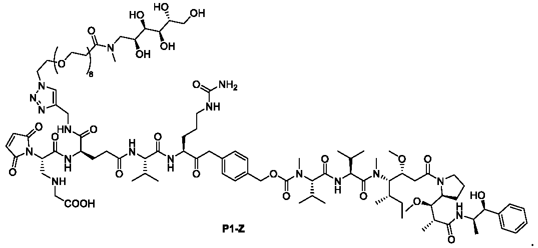

41. The linker-drug compound or a pharmaceutically acceptable salt or solvate thereof according to any one of claims 21-39, wherein the linker-drug compound is non-restrictively selected from the following structures:

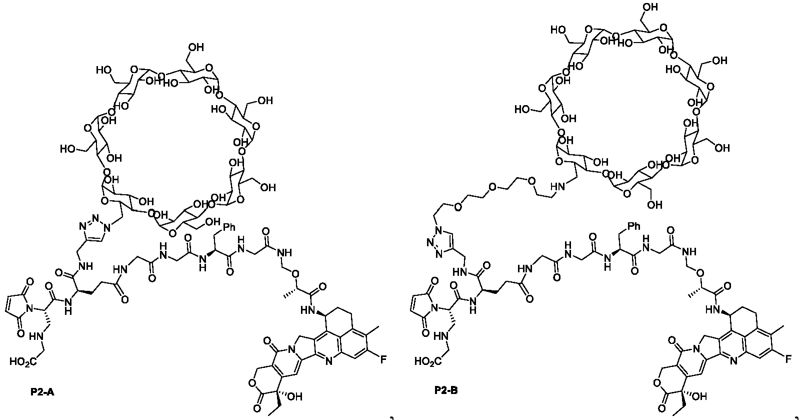

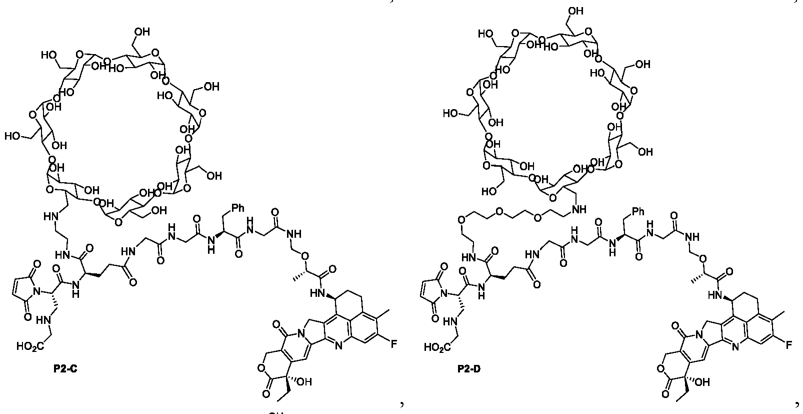

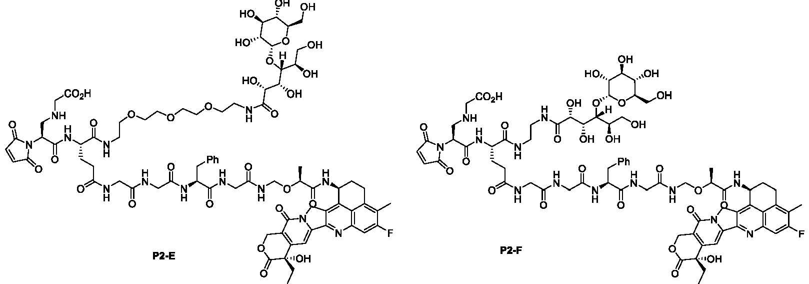

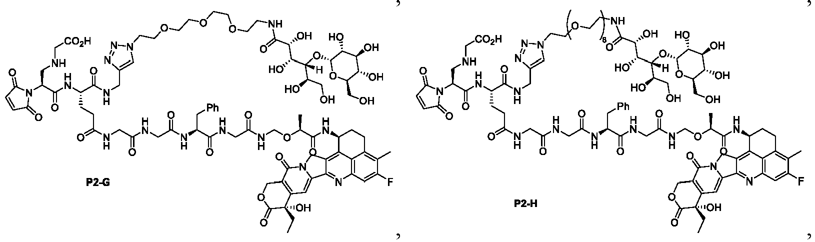

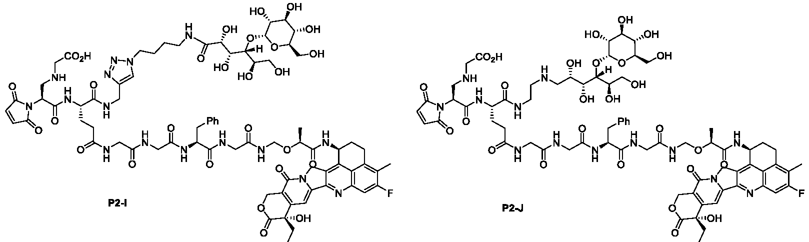

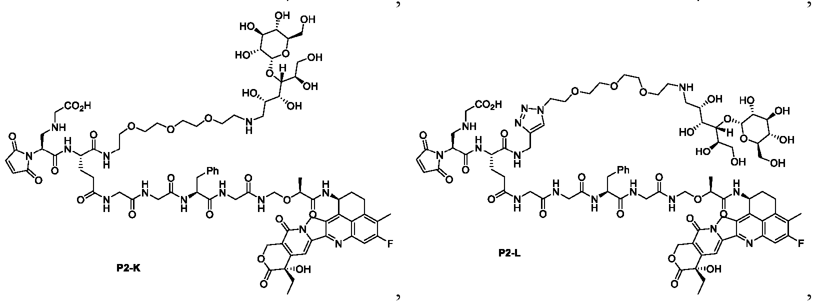

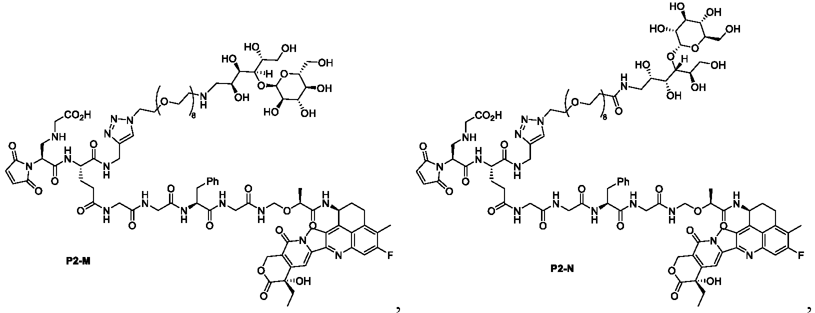

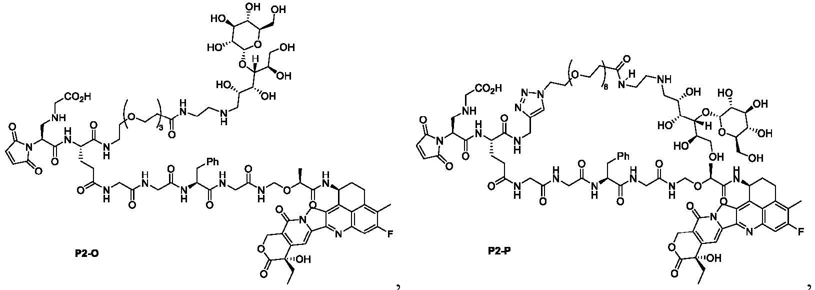

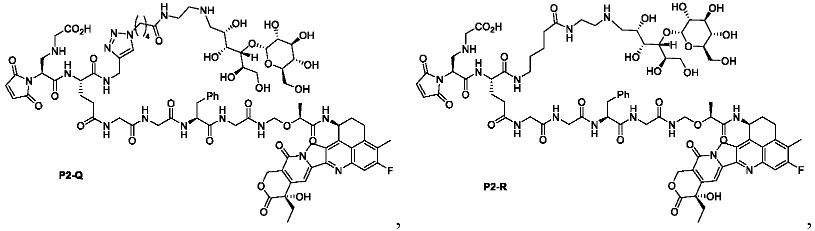

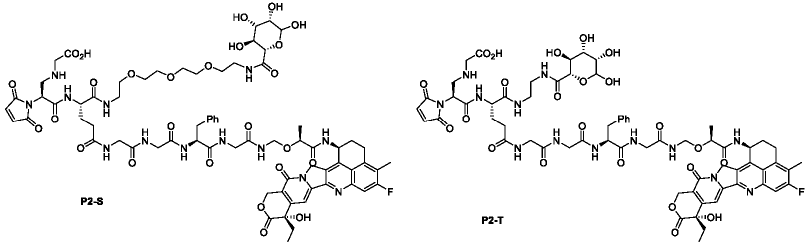

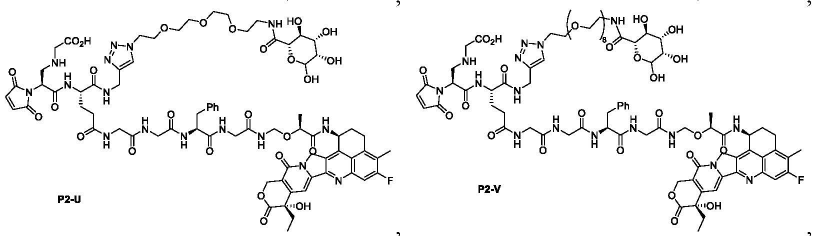

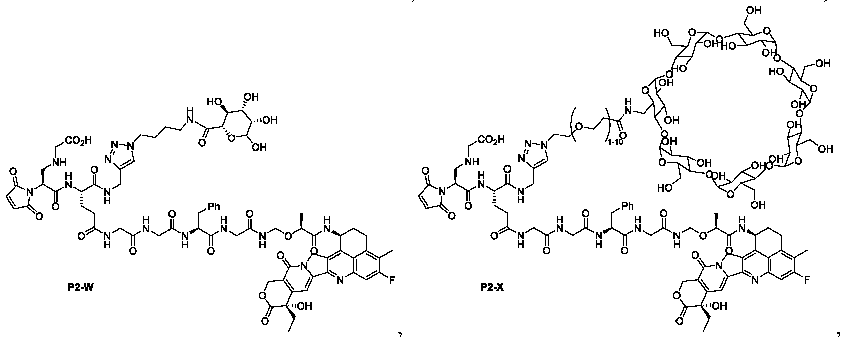

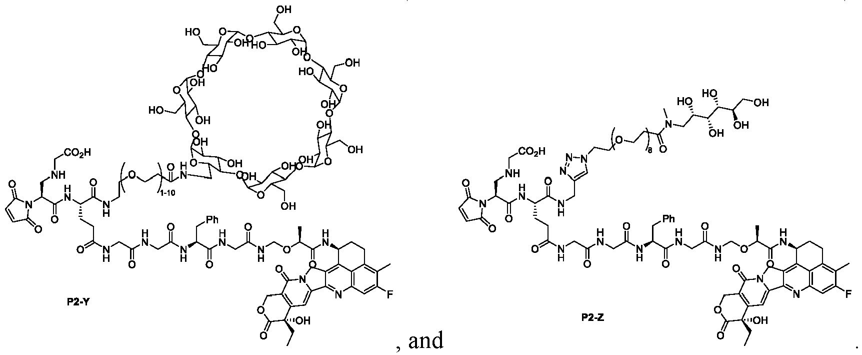

42. The linker-drug compound or a pharmaceutically acceptable salt or solvate thereof according to any one of claims 21-39, wherein the linker-drug compound is non-restrictively selected from the following structures:

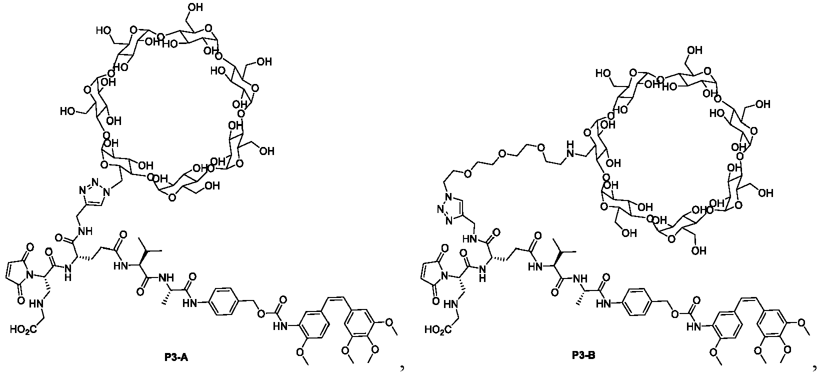

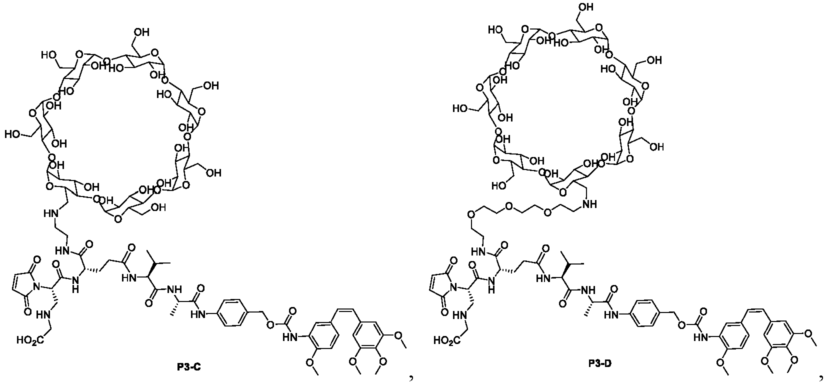

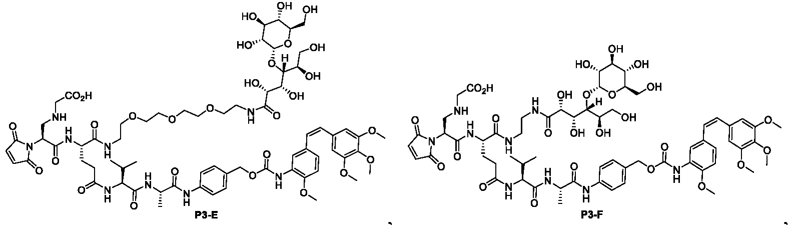

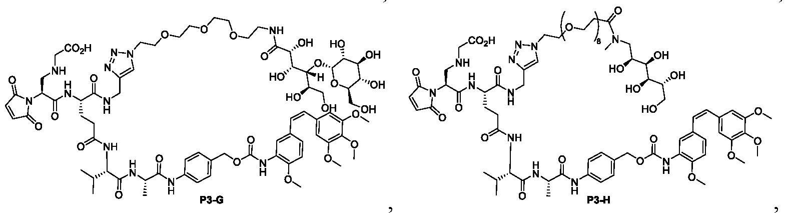

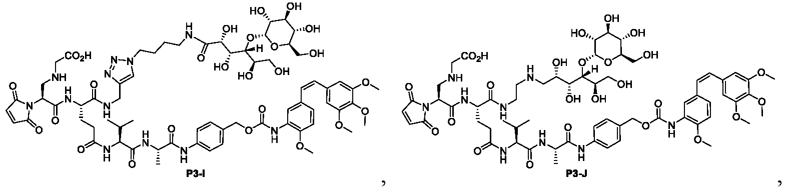

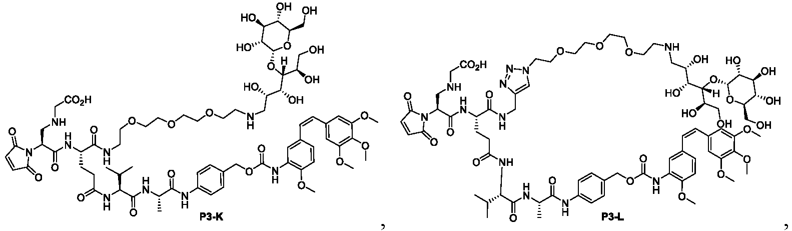

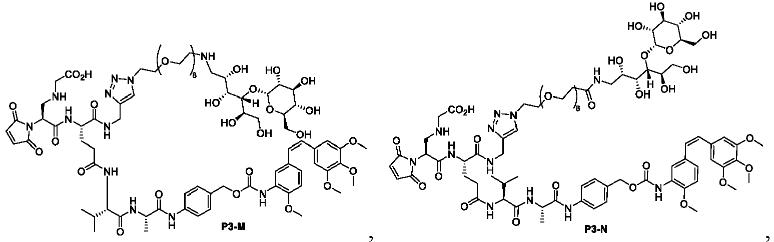

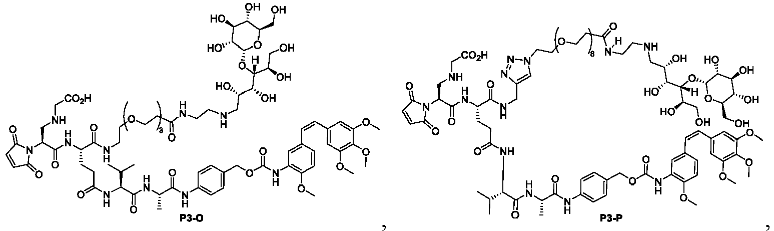

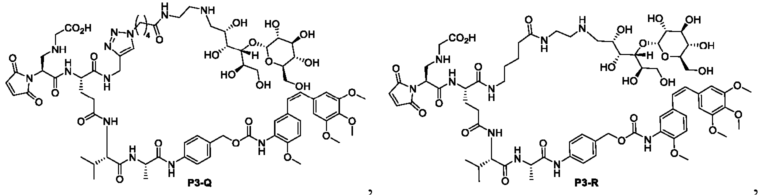

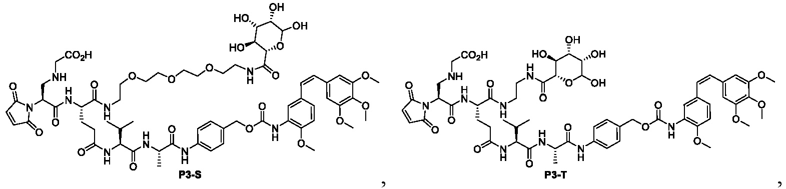

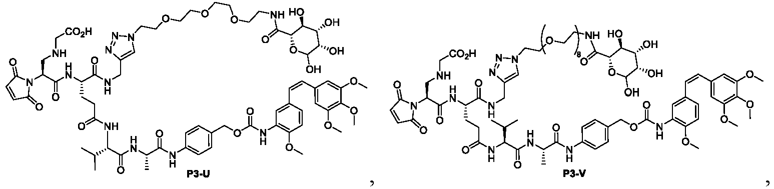

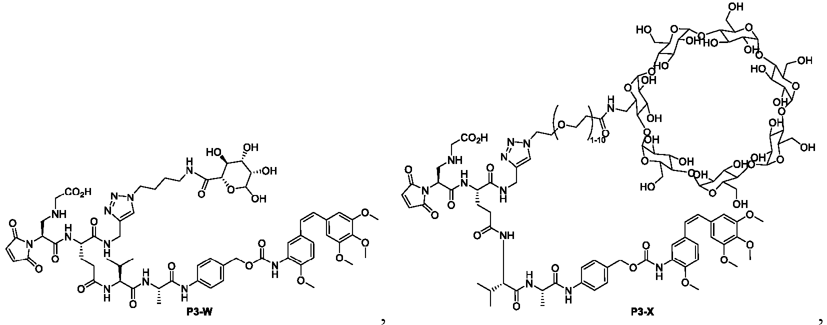

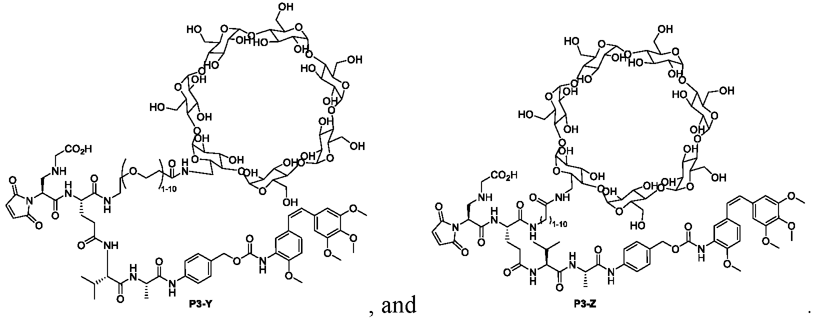

43. The linker-drug compound or a pharmaceutically acceptable salt or solvate thereof according to any one of claims 21-39, wherein the linker-drug compound is non-restrictively selected from the following structures:

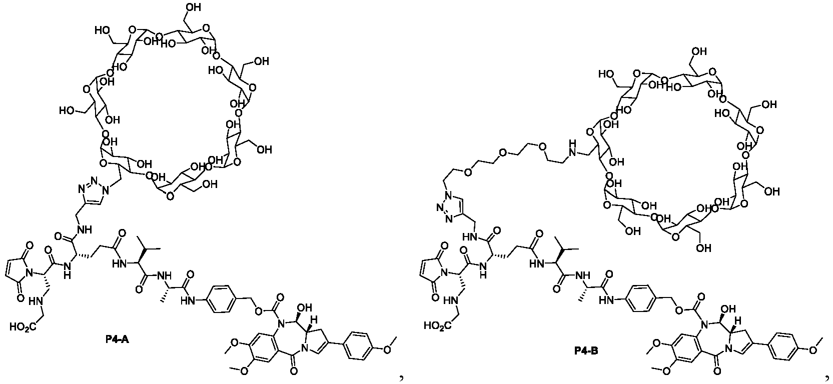

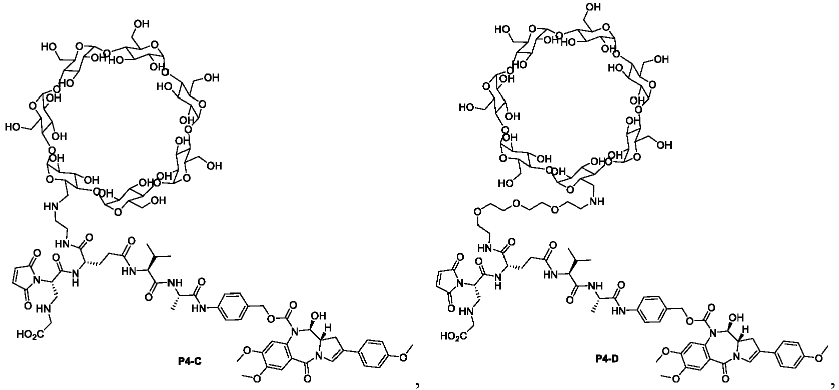

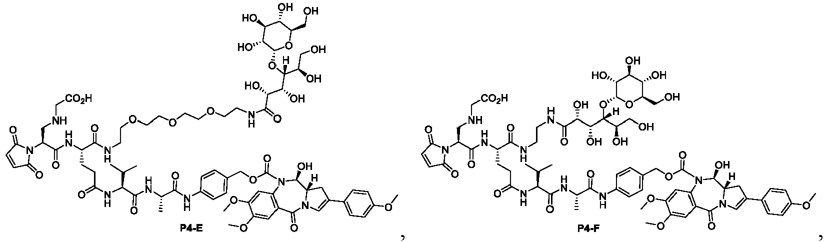

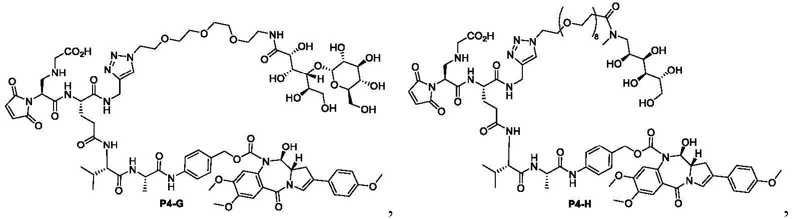

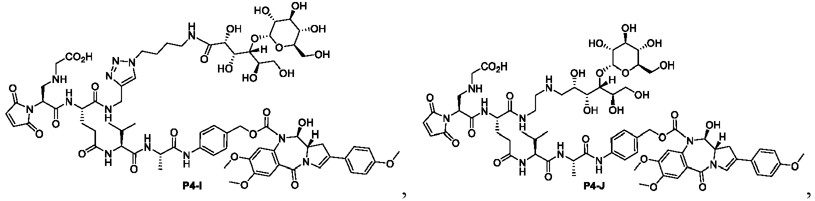

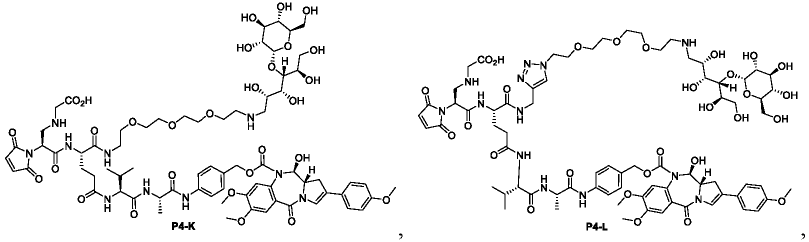

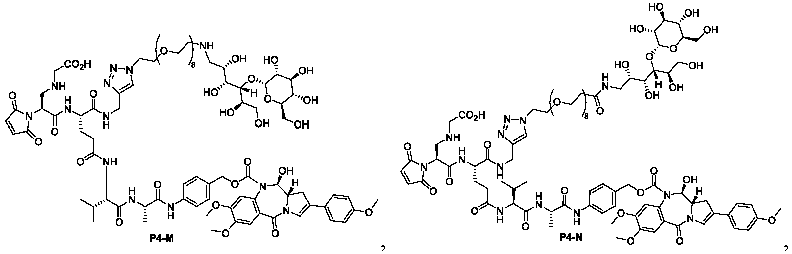

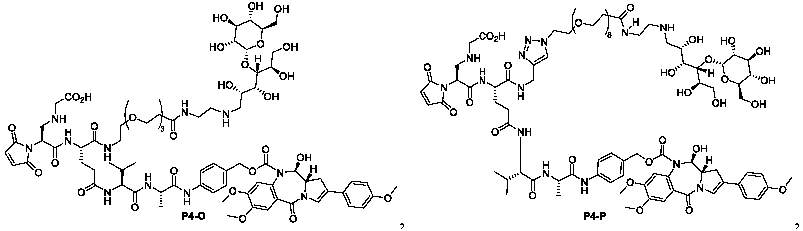

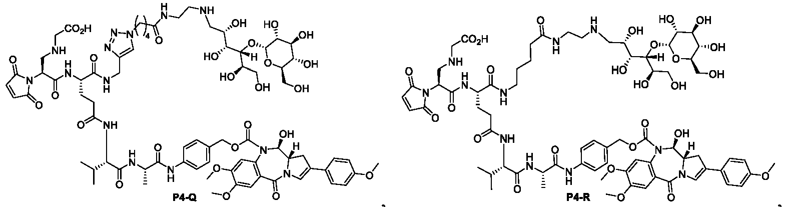

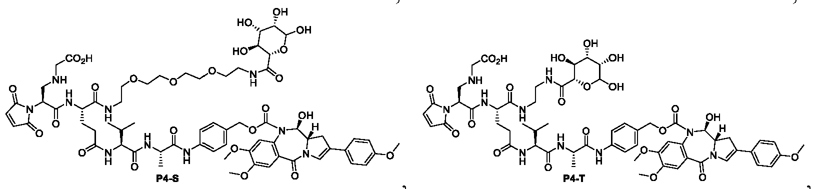

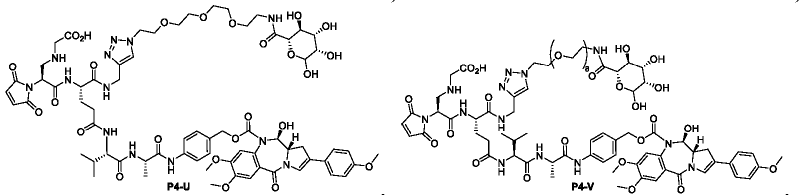

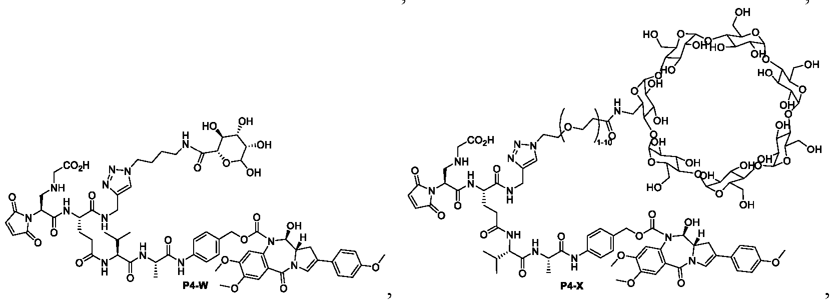

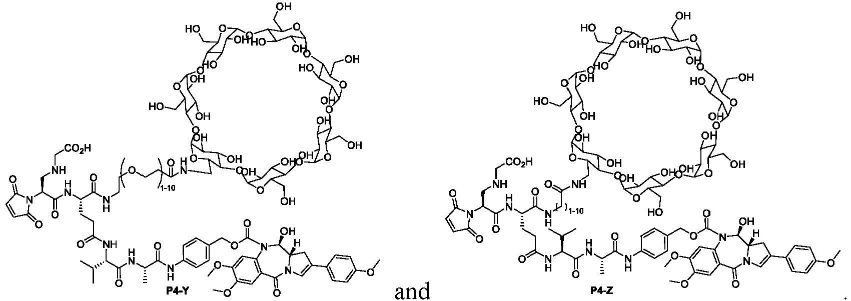

44. A preparation method for a ligand-drug conjugate of formula I, formula II or formula III, or a pharmaceutically acceptable salt or solvate thereof according to any one of claims 1-20, wherein comprises the following steps: 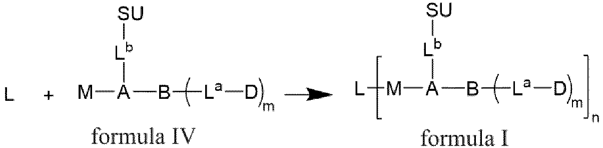 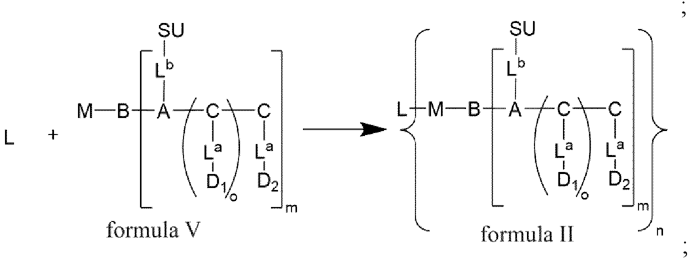 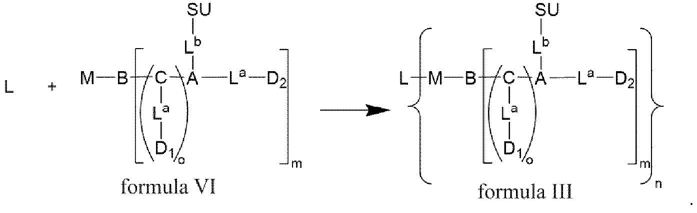
allowing a modified ligand to react with a compound of formula IV, V or VI to obtain a ligand-drug conjugate of formula I, II or III, or a pharmaceutically acceptable salt or solvate thereof,
wherein
L is selected from ligands;
M is selected from any linker units;
A is selected from any linking scaffolds;
B is present or absent, and when present, is selected from any linking structures or bonds;
C is present or absent, and when present, is selected from any branching units or bonds; D, D₁, D₂ are the same or different, and are each independently selected from drugs; the SU is selected from sugars or derivatives thereof;
L^{a} and L^{b} are the same or different, and are each independently selected from any linking units or bonds;
m is selected from integers of 1 to 5;
n is selected from integers of 1 to 10;
o is selected from integers of 1 to 10.

45. The ligand-drug conjugate or a pharmaceutically acceptable salt or solvate thereof according to any one of claims 1-20, or a ligand-drug conjugate containing the linker-drug compound or a pharmaceutically acceptable salt or solvate of the ligand-drug conjugate according to any one of claims 21-43, wherein the ligand-drug conjugate or the pharmaceutically acceptable salt or solvate thereof is non-restrictively selected from the following structures or stereoisomers thereof: 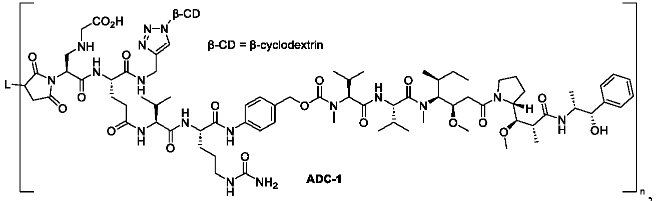 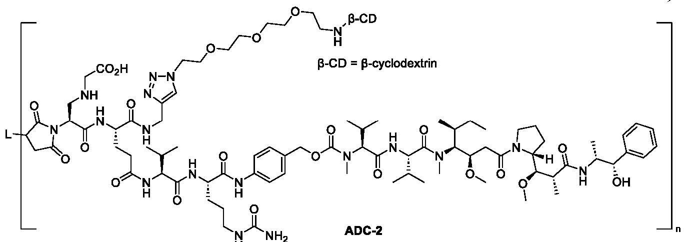 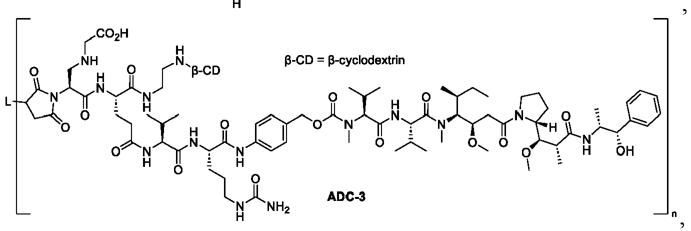 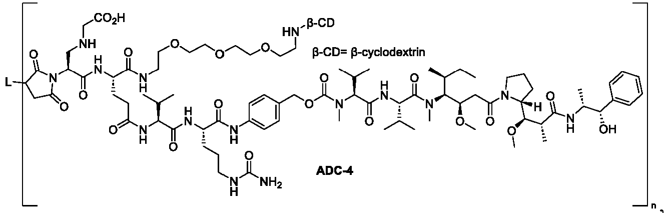 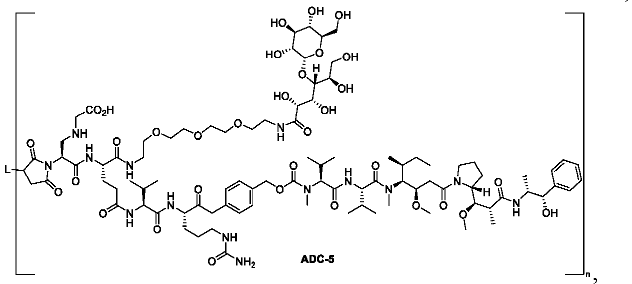 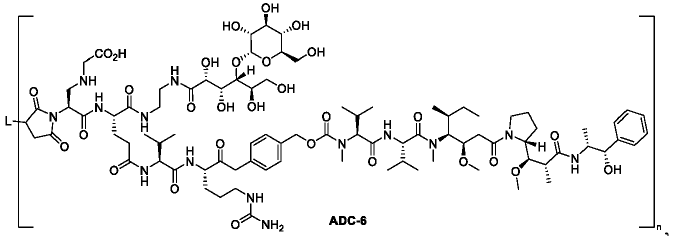 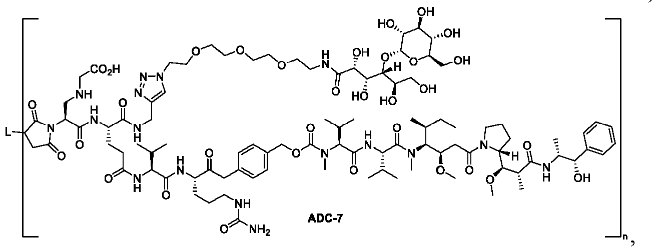 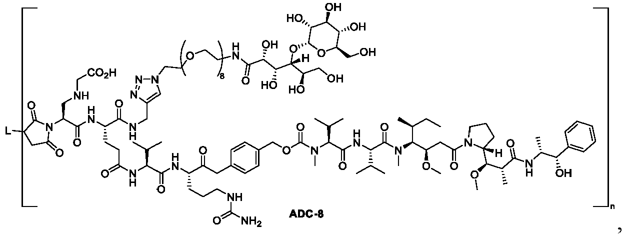 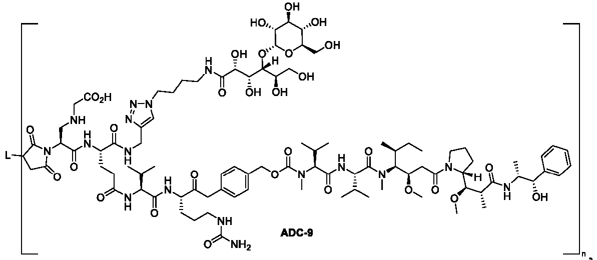 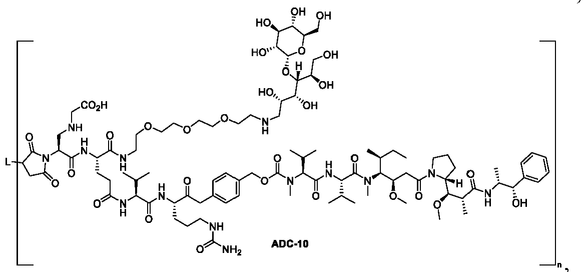 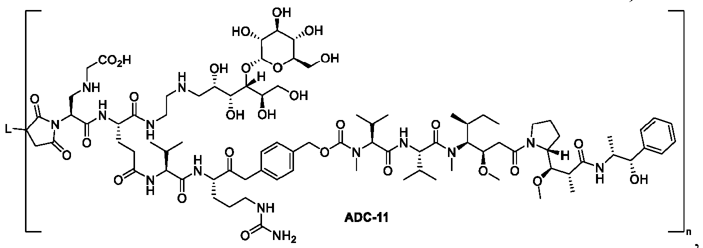 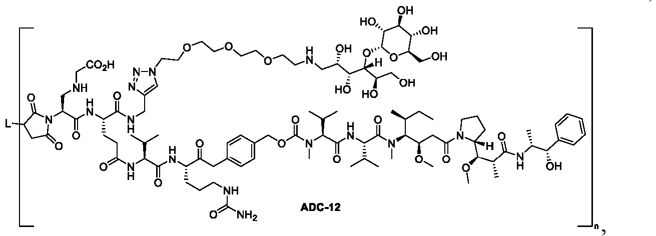 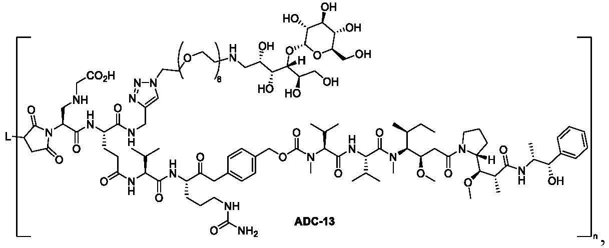 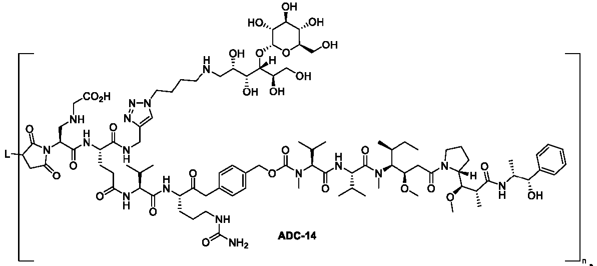 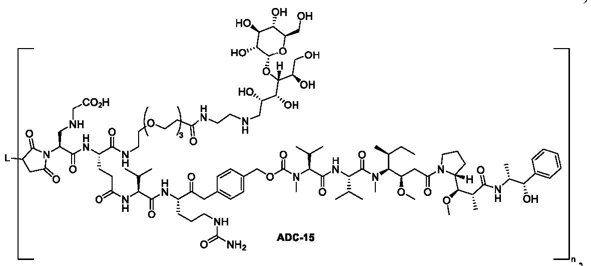 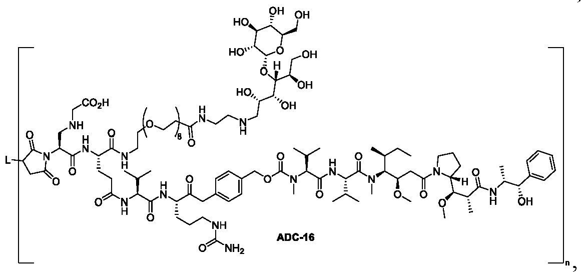 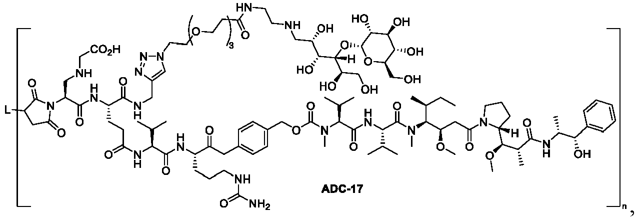 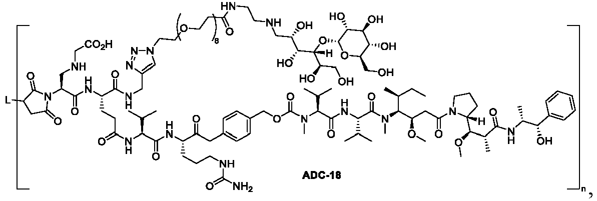 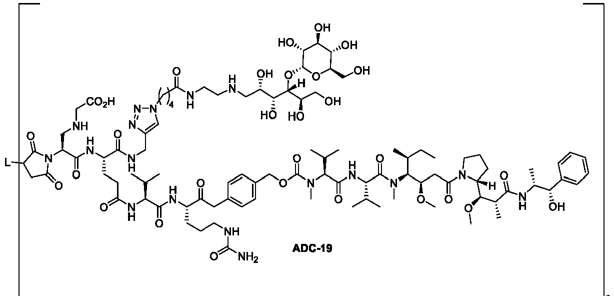 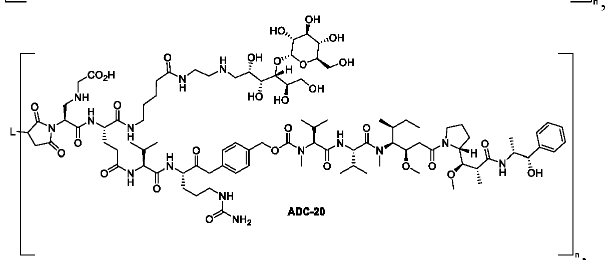 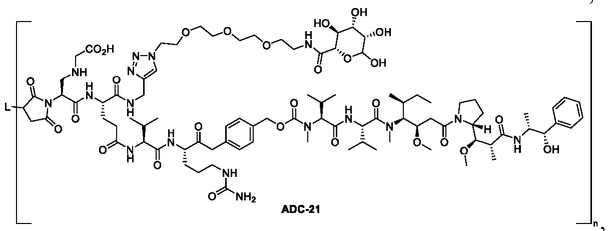 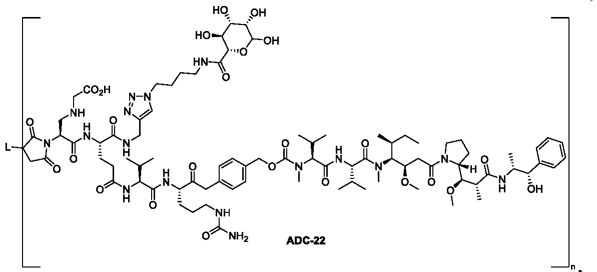 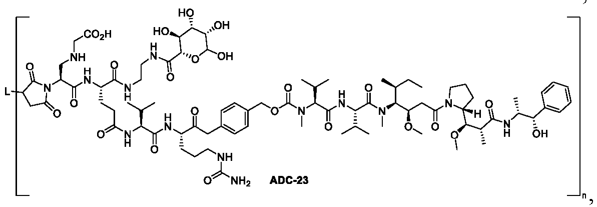 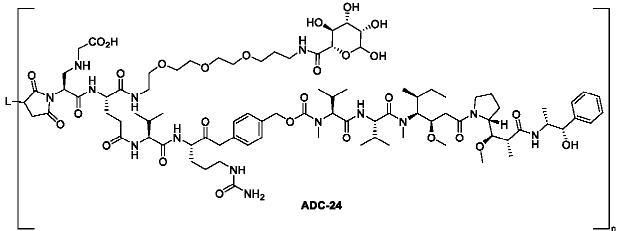 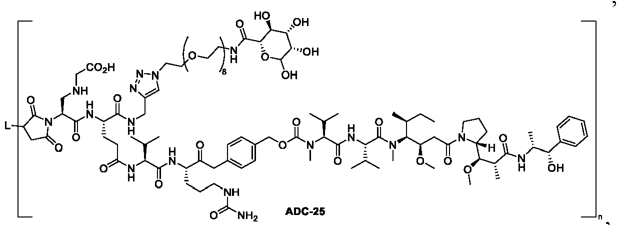 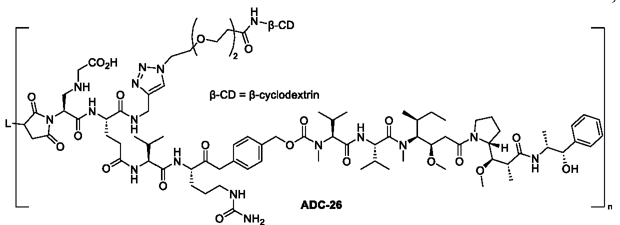 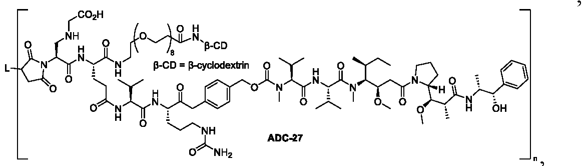 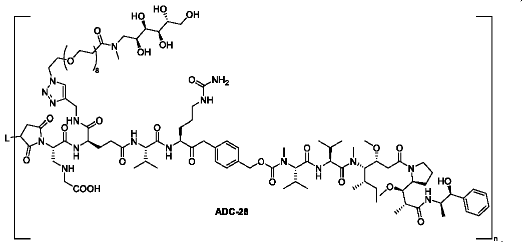 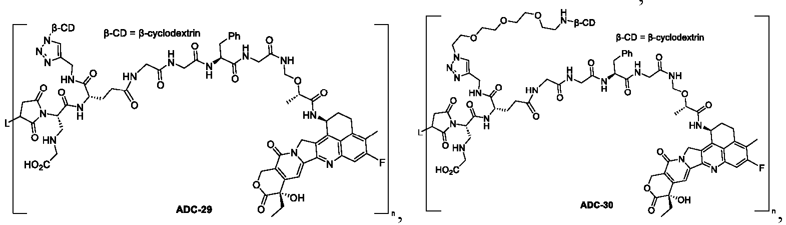 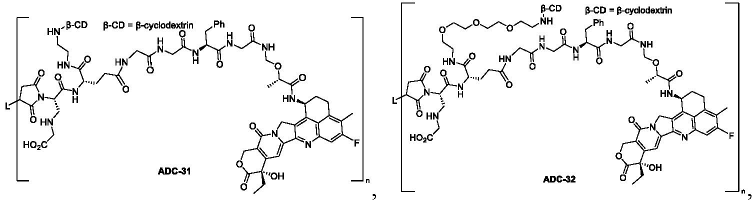 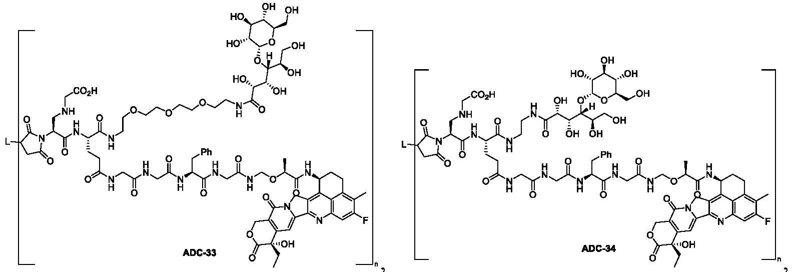 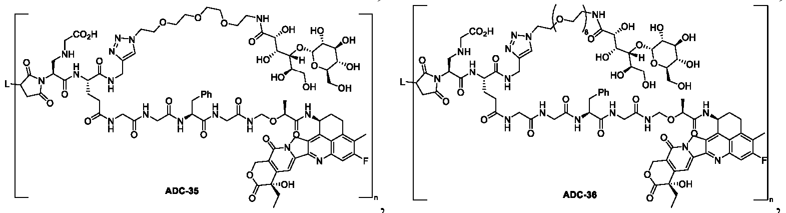 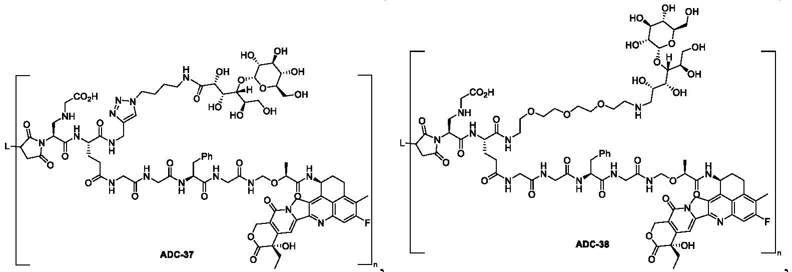 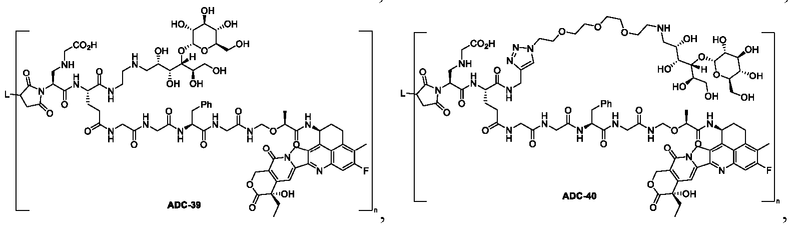 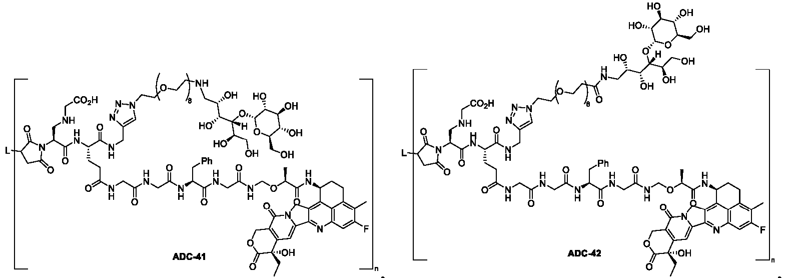 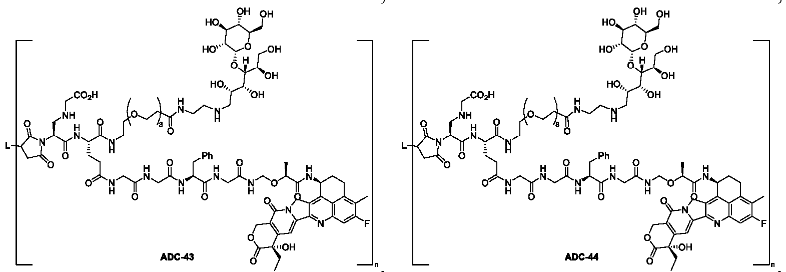 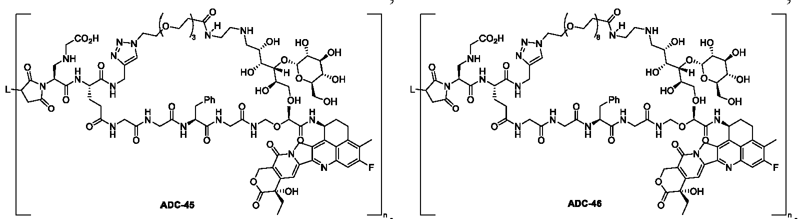 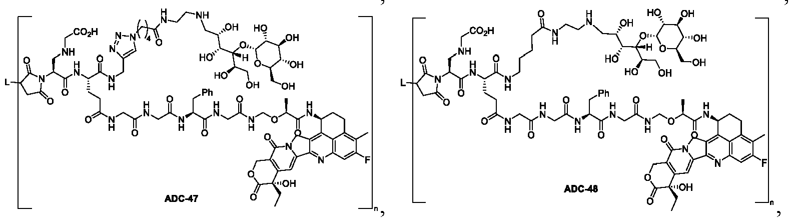 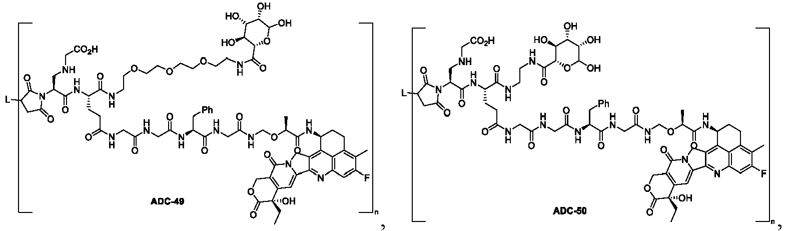 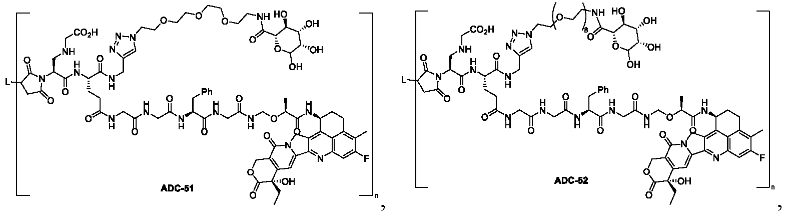 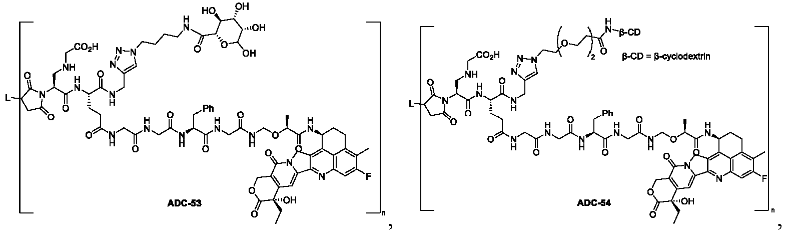 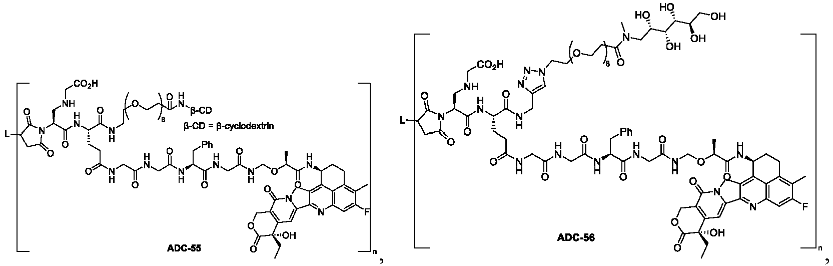 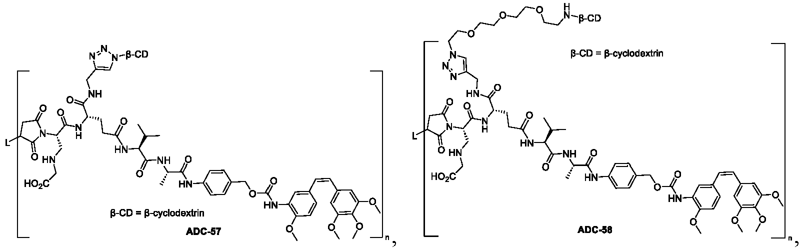 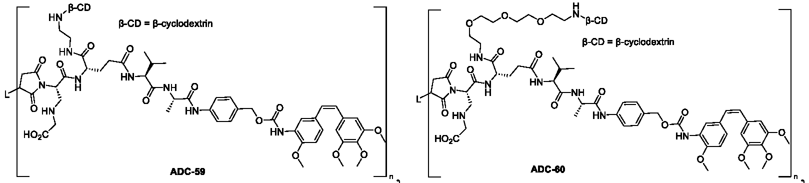 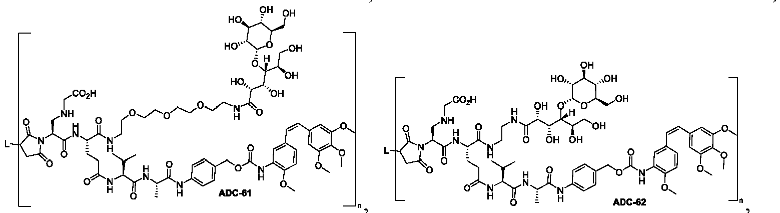 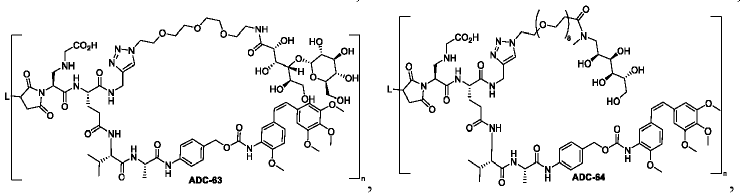 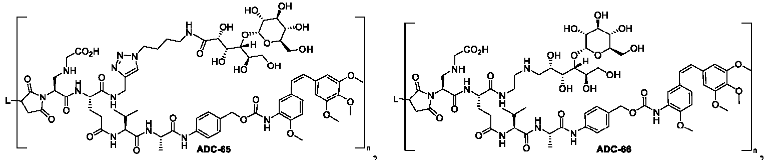 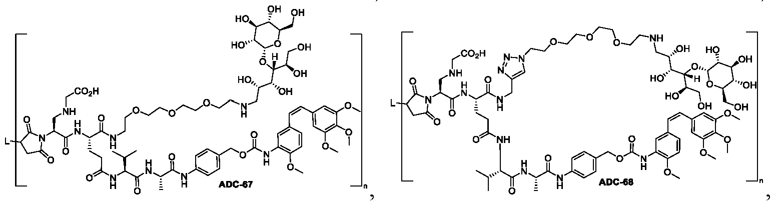 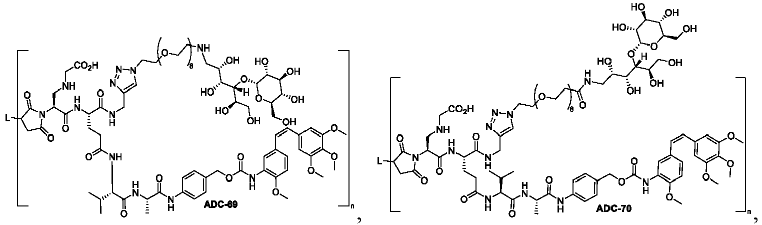 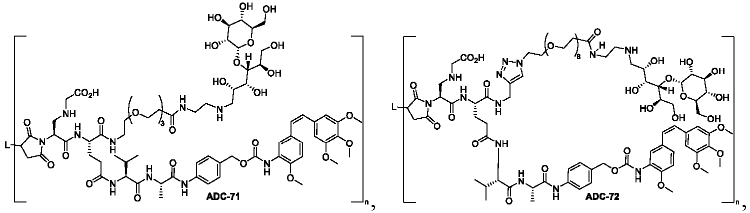 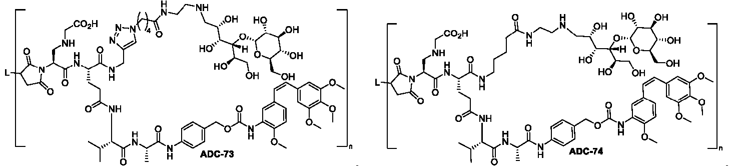 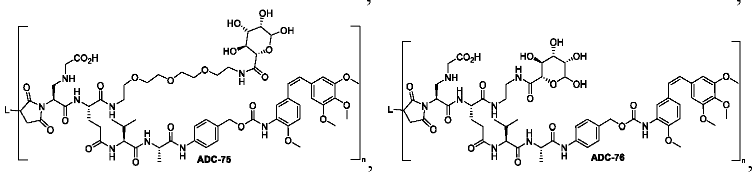 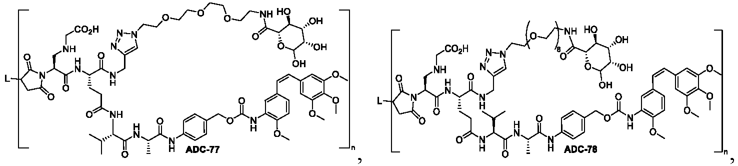 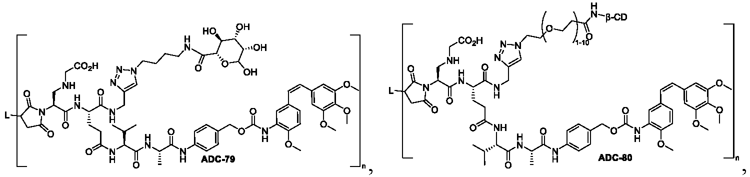 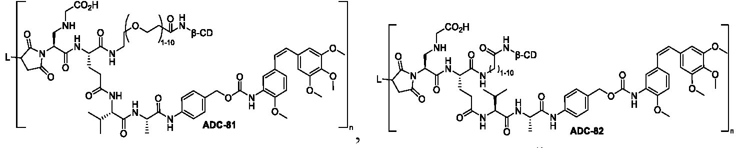 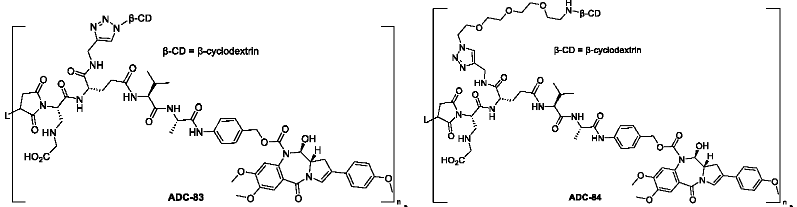 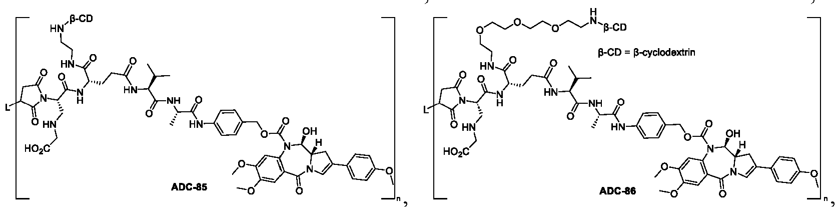 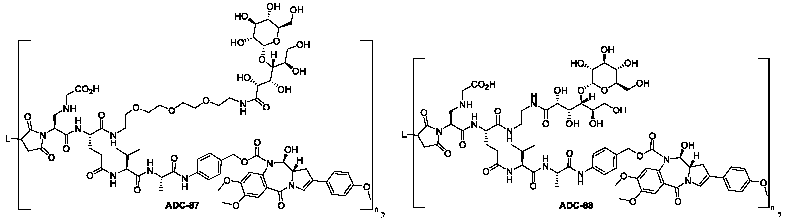 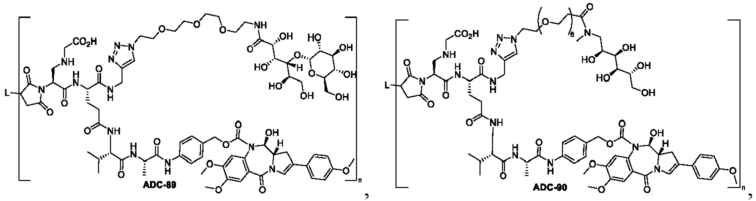 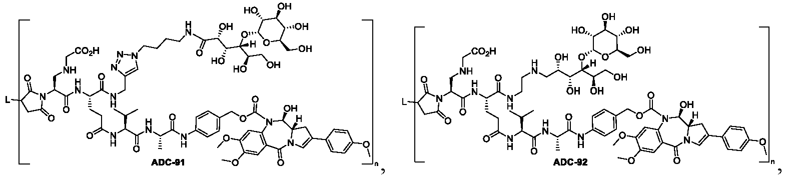 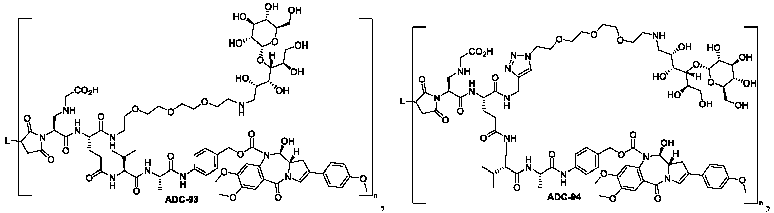 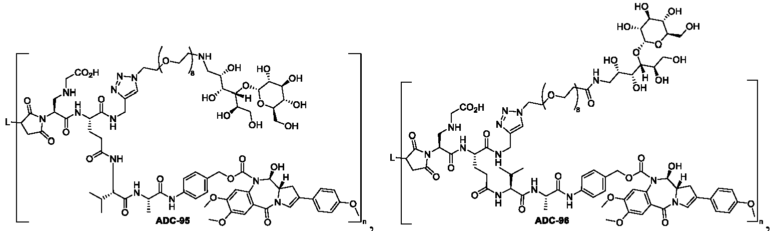 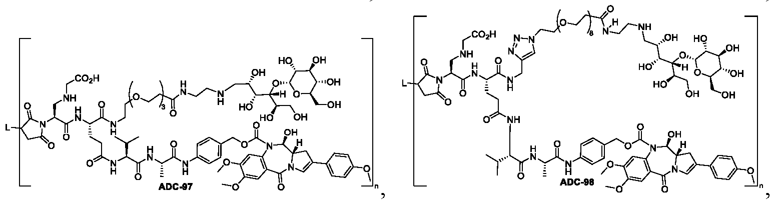 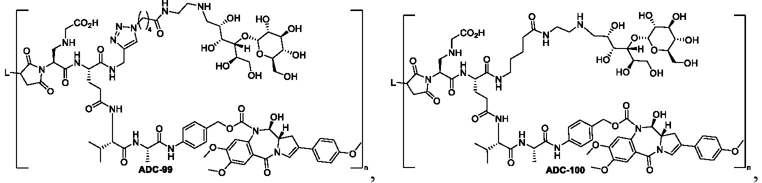 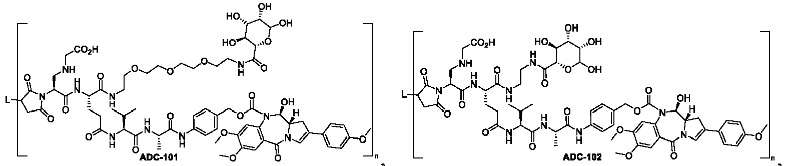 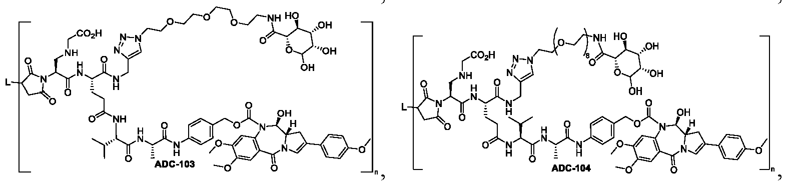 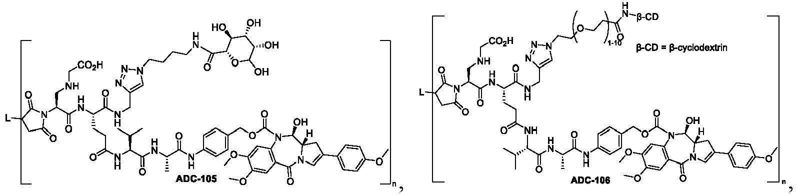 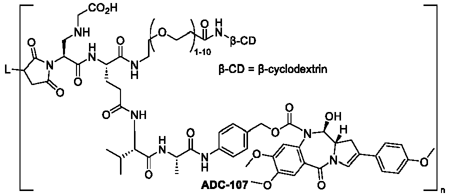 and 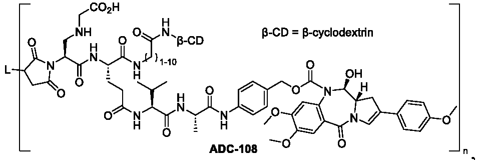 wherein
L is selected from ligands, preferably antibodies;
n is selected from integers of 1 to 10, preferably 2 to 8.

46. The ligand-drug conjugate or a pharmaceutically acceptable salt thereof according to any one of claims 1-20 and 44, or the linker-drug compound or a pharmaceutically acceptable salt or solvate thereof according to any one of claims 21-43, wherein the pharmaceutically acceptable salt is selected from:
a sodium salt, potassium salt, calcium salt or magnesium salt formed by an acidic functional group in the structural formula and an alkali; and
an acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methanesulfonate, ethanesulfonate, benzenesulfonate or p-toluenesulfonate formed by a basic functional group in the structure and an acid.

47. The ligand-drug conjugate or a pharmaceutically acceptable salt according to any one of claims 1-20 and 44, wherein the ligand is selected from monoclonal antibodies and non-restrictively from: an anti-EGFRvIII antibody, an anti-DLL-3 antibody, an anti-PSMA antibody, an anti-CD70 antibody, an anti-MUC16 antibody, an anti-ENPP3 antibody, an anti-TDGF1 antibody, an anti-ETBR antibody, an anti-MSLN antibody, an anti-TIM-1 antibody, an anti-LRRC15 antibody, an anti-LIV-1 antibody, an anti-CanAg/AFP antibody, an anti-cladin18.2 antibody, an anti-Mesothelin antibody, an anti-HER2 (ErbB2) antibody, an anti-EGFR antibody, an anti-C-Met antibody, an anti-SLITRK6 antibody, an anti-KIT/CD117 antibody, an anti-STEAP1 antibody, an anti- SLAMF7/CS1 antibody, an anti-NaPi2B/SLC34A2 antibody, an anti-GPNMB antibody, anti-HER3 (ErbB3) antibody, anti-MUC1/CD227 antibody, an anti-AXL antibody, an anti-CD166 antibody, an anti-B7-H3 (CD276) antibody, an anti-PTK7/CCK4 antibody, an anti-PRLR antibody, an anti-EFNA4 antibody, an anti-5T4 antibody, an anti-NOTCH3 antibody, an anti-Nectin 4 antibody, an anti-Trop-2 antibody, an anti-CD142 antibody, an anti-CA6 antibody, an anti-GPR20 antibody, an anti-CD174 antibody, an anti-CD71 antibody, an anti-EphA2 antibody, an anti-LYPD3 antibody, an anti-FGFR2 antibody, an anti-FGFR3 antibody, an anti-FRα antibody, an anti-CEACAMs antibody an anti-GCC antibody, an anti-Integrin Av antibody, an anti-CAIX antibody, an anti-P-cadherin antibody, an anti-GD3 antibody, an anti-Cadherin 6 antibody, an anti-LAMP1 antibody, an anti-FLT3 antibody, an anti-BCMA antibody, an anti-CD79b antibody, an anti-CD19 antibody, an anti-CD33 antibody, an anti-CD56 antibody Body, an anti-CD74 antibody, an anti-CD22 antibody, an anti-CD30 antibody, an anti-CD37 antibody, an anti-CD138 antibody, an anti-CD352 antibody, an anti-CD25 antibody, and an anti-CD123 antibody;
preferably, the ligand is an anti-Trop-2 antibody or an anti-CD33 antibody.

48. A pharmaceutical composition, containing a therapeutically effective amount of the ligand-drug conjugate or the pharmaceutically acceptable salt of the ligand-drug conjugate or the linker-drug compound or the pharmaceutically acceptable salt or solvate of the linker-drug compound according to any one of claims 1-47, and a pharmaceutically acceptable carrier, diluent or excipient.

49. Use of the ligand-drug conjugate or the pharmaceutically acceptable salt of the ligand-drug conjugate or the linker-drug compound or the pharmaceutically acceptable salt or solvate of the linker-drug compound according to any one of claims 1-48 in preparation of a drug for treating or preventing a tumor or an autoimmune disease.

50. The use according to any one of claims 1-49, wherein the tumor is selected from solid tumors or non-solid tumors, such as breast cancer, ovarian cancer, cervical cancer, uterine cancer, prostate cancer, kidney cancer, urethral cancer, bladder cancer, liver cancer, stomach cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma multiforme, sarcoma, lymphoma and leukemia.
